(19) Europäisches Patentamt
European Patent Office
Office européen des brevets

(11) **EP 0 942 906 B1**

(12) **EUROPEAN PATENT SPECIFICATION**

(45) Date of publication and mention of the grant of the patent:
**03.11.2004 Bulletin 2004/45**

(21) Application number: **97955043.1**

(22) Date of filing: **11.09.1997**

(51) Int Cl.[7]: **C07D 401/04**, A61K 31/55, C07D 401/14 // (C07D401/04, 221:00, 211:00), (C07D401/14, 221:00, 211:00), (C07D401/14, 221:00, 211:00, 207:00)

(86) International application number:
**PCT/US1997/024295**

(87) International publication number:
**WO 1998/030558 (16.07.1998 Gazette 1998/28)**

(54) **COMPOUNDS USEFUL FOR INHIBITION OF FARNESYL PROTEIN TRANSFERASE**

VERBINDUNGEN ALS INHIBITOREN VON FARNESYLPROTEIN-TRANSFERASE

COMPOSES UTILES POUR INHIBER LA FARNESYL-PROTEINE TRANSFERASE

(84) Designated Contracting States:
**AT BE CH DE DK ES FI FR GB GR IE IT LI LU NL PT SE**
Designated Extension States:
**LT LV RO**

(30) Priority: **13.09.1996 US 710225**
**17.06.1997 US 877453**

(43) Date of publication of application:
**22.09.1999 Bulletin 1999/38**

(73) Proprietor: **SCHERING CORPORATION**
**Kenilworth, New Jersey 07033-0530 (US)**

(72) Inventors:
- **NJOROGE, F., George**
**Union, NJ 07083 (US)**
- **TAVERAS, Arthur, G.**
**Rockaway, NJ 07866 (US)**
- **DOLL, Ronald, J.**
**Maplewood, NJ 07040 (US)**
- **LALWANI, Tarik**
**Edison, NJ 08837 (US)**
- **ALVAREZ, Carmen**
**Roselle Park, NJ 07204 (US)**
- **REMISZEWSKI, Stacy, W.**
**Washington Township, NJ 07675 (US)**

(74) Representative: **Ritter, Stephen David et al**
**Mathys & Squire**
**100 Gray's Inn Road**
**London WC1X 8AL (GB)**

(56) References cited:

| | |
|---|---|
| **EP-A- 0 270 818** | **EP-A- 0 396 083** |
| **EP-A- 0 495 484** | **WO-A-95/10514** |
| **WO-A-95/10515** | **WO-A-95/10516** |
| **WO-A-96/30362** | **WO-A-96/30363** |
| **WO-A-96/31478** | **WO-A-97/23478** |

- **BISHOP,W.R. ET AL.: "Novel Tricyclic Inhibitors of Farnesyl Protein Tranferase" J.BIOL.CHEM., vol. 270, no. 51, 22 December 1995, ROCKVILLE PIKE, pages 30611-30618, XP002050604**
- **BUSS,J.E. ET AL.: "Farnesyl transferase inhibitors : the successes and surprises of a new class of potential cancer chemotherapeutics" CHEM.BIOL., vol. 2, December 1995, pages 787-791, XP002056549**
- **NJOROGE,F.G. ET AL.: "Novel Tricyclic Aminoacetyl and Sulfonamide inhibitors of RAS farnesyl protein transferase" BIOORG.&MED.CHEM.LETT., vol. 6, no. 24, December 1996, OXFORD, pages 2977-2982, XP002056550**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 942 906 B1

- **NJOROGE,F.G. ET AL.: "Discovery of a novel Nonpeptide Tricyclic Inhibitors of RAS Farnesyl Transferase" BIOORG.&MED.CHEM.LETT., vol. 5, no. 1, 1997, OXFORD, pages 101-113, XP002056551**

Remarks:

The file contains technical information submitted after the application was filed and not included in this specification

## Description

The present invention relates to inhibitors of farnesyl protein transferase.

BACKGROUND

Bishop *et al*., J. Biol. Chem. (1995) 270, pp 30611-30618 and Buss *et al*., Chem, and Biol. (1995), 2, pp 787-791 discloses the 8-chloro, piperidyl-substituted compound SCH 44342 as an inhibitor of farnesyl protein transferase:

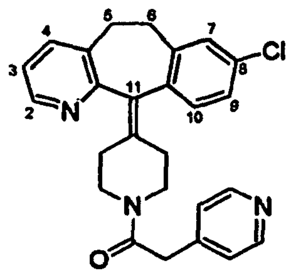

Tricyclic aminoacetyl and sulfonamide inhibitors of farnesyl protein transferase are disclosed in Njoroge *et al*., Bioorg. Med. Chem. Lett., (1996) 6, pp 2977-2982.

Tricyclic compounds useful for the inhibition of farnesyl protein transferase are also disclosed in WO 95/10516, WO 95/10515, WO 95/10514 and Njoroge *et. al*., Bioorg. Med. Chem. Lett., (1997), 5, pp 101-113. WO 95/10516 and WO 95/10514 each disclose, in the specific examples, 3,4,8-tri-halo substituted compounds. In the general formulae disclosed in WO 95/10516, the compounds can contain the group $-(O)CH_2$-(N-substituted piperidyl), said group being attached at the N-atom of the 11-piperidyl/piperazinyl ring. The substituent on the nitrogen atom of the terminal N-substituent can be alkyl, alkoxycarbonyl, haloalkyl or alkylcarbonyl or $-CONHR^{10}$ wherein $R^{10}$ is H or alkyl.

Further tricyclic compounds useful for the inhibition of farnesyl protein transferase are disclosed in WO 96/30363, WO 96/30362, WO 96/31478 and WO 96/23478, each of which were published after the priority date of the present invention.

In view of the current interest in inhibitors of farnesyl protein transferase, a welcome contribution to the art would be further compounds useful for the inhibition of farnesyl protein transferase. Such a contribution is provided by this invention.

SUMMARY OF THE INVENTION

This invention provides compounds useful for the inhibition of farnesyl protein transferase (FPT). The compounds of this invention are represented by the formula:

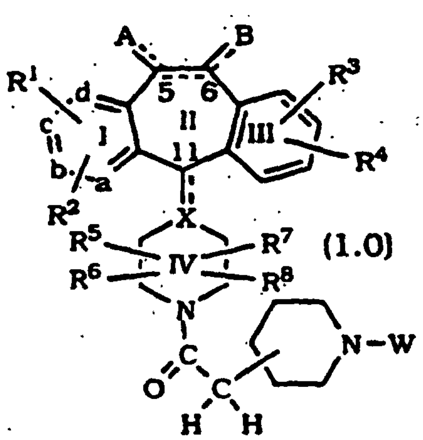

(1.0)

or a pharmaceutically acceptable salt or solvate thereof, wherein: one of a, b, c and d represents N or $NR^9$ wherein $R^9$ is $O^-$, $-CH_3$ or $-(CH_2)_nCO_2H$ wherein n is 1 to 3, and the remaining a, b, c and d groups represent $CR^1$ or $CR^2$; or

each of a, b, c, and d are independently selected from $CR^1$ or $CR^2$;

$R^2$ is H and $R^1$, $R^3$ and $R^4$ are halo;

$R^5$, $R^6$, $R^7$ and $R^8$ each independently represents H, $-CF_3$, $-COR^{10}$, alkyl or aryl, said alkyl or aryl optionally being substituted with $-OR^{10}$, $-SR^{10}$, $-S(O)_tR^{11}$, $-NR^{10}COOR^{11}$, $-N(R^{10})_2$, $-NO_2$, $-COR^{10}$, $-OCOR^{10}$, $-OCO_2R^{11}$, $-CO_2R^{10}$, $OPO_3R^{10}$, or $R^5$ is combined with $R^6$ to represent =O or =S and/or $R^7$ is combined with $R^8$ to represent =O or =S;

$R^{10}$ represents H, alkyl, aryl, or aralkyl (e.g., benzyl);

$R^{11}$ represents alkyl or aryl;

X represents N, CH or C, which C may contain an optional double bond (represented by the dotted line) to carbon atom 11;

the dotted line between carbon atoms 5 and 6 represents an optional double bond such that when a double bond is present. A and B independently represent $-R^{10}$, halo, $-OR^{11}$, $-OCO_2R^{11}$ or $-OC(O)R^{10}$, and when no double bond is present between carbon atoms 5 and 6, A and B each independently represent $H_2$, $-(OR^{11})_2$; H and halo, dihalo, alkyl and H, $(alkyl)_2$, -H and $-OC(O)R^{10}$, H and $-OR^{10}$, =O, aryl and H, $=NOR^{10}$ or -O- $(CH_2)_p$-O- wherein p is 2, 3 or 4; and

W represents a group selected from the group consisting of:

(1)

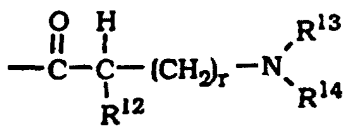;

(2)

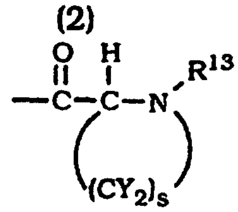;

(3)

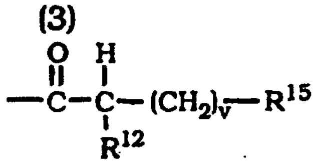

and

(4)

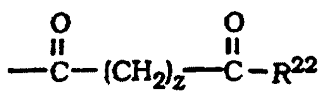

wherein:

$R^{12}$ is selected from the group consisting of: (a) H: (b) alkyl; (c) aralkyl (e.g., benzyl); and (d) heteroarylalkyl (heteroaralkyl) (e.g., $-CH_2$-imidazolyl);

$R^{13}$ and $R^{14}$ are each independently selected from the group consisting of: (a) H; (b) $-C(O)OR^{16}$ wherein $R^{16}$ represents alkyl, aralkyl, and heteroaralkyl; (c) $-SO_2R^{17}$ wherein $R^{17}$ is selected from the group consisting of: $-NH_2$, $-N(alkyl)_2$ wherein each alkyl is the same or different (e.g., $-N(CH_3)_2$), alkyl (e.g., $C_{1-6}$ alkyl, such as methyl), aryl, aralkyl, heteroaryl and heteroaralkyl; (d) $-C(O)R^{18}$ wherein $R^{18}$ is selected from the group consisting of: aryl (e.g., phenyl), alkyl, aralkyl, heteroaryl, and heteroaralkyl: (e) $C_{1-6}$ alkyl; (f) alkaryl; and (g) $C_{3-6}$ cycloalkyl;

r is 0, 1 or 2;

s represents 1, 2, 3, 4, or 5 (preferably 3 or 4), and each Y for each $-CY_2-$ group is independently selected from H or -OH, provided that both Y substituents of each $-CY_2-$ group are not -OH, and provided that for the $-CY_2-$ group alpha to the nitrogen both Y substituents are H, preferably each Y is H such that each $-CY_2-$ group is a $-CH_2-$ group, such that the group

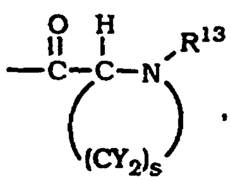, e.g., 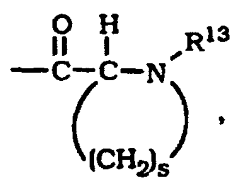,

forms a 3, 4, 5, 6, or 7 (preferably 5 or 6) membered ring (e.g., piperidyl or pyrrolidinyl), ;

v is 0, 1 or 2;

$R^{15}$ is selected from the group consisting of:

(a) heteroaryl (e.g., imidazolyl);

(b) a group selected from:

(1) 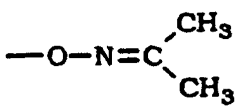 , (2) 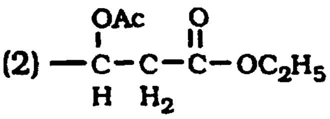 ,

(3) 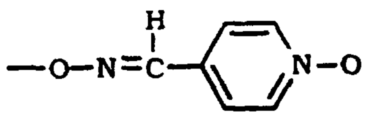 , (4) 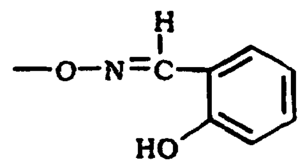 ,

(5) $-CH(OCH_2CH_3)_2$,

(6) -OH, and

(7) -CN: and

(c) heterocycloalkyl selected from the group consisting of:

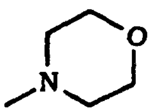 , 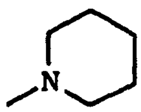 , 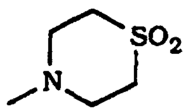 , 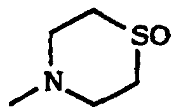 , 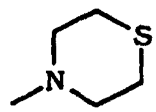 ,

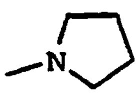 , 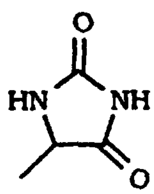 , 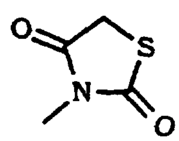 , 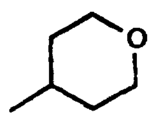 , 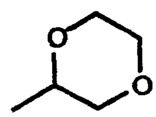 , 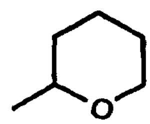

and

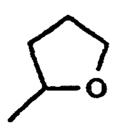 ;

z is 0, 1, 2, 3, 4, or 5 wherein each $-CH_2-$ group is optionally substituted with a -OH group, i.e., each H of each $-CH_2-$ group can optionally be replaced with a -OH group and the optional substitution on each $-CH_2-$ group is independent of the substitution on any other $-CH_2-$ group, generally each $-CH_2-$ is unsubstituted;

$R^{22}$ represents a group selected from:

(1) 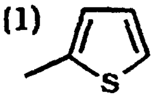, (2) 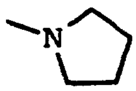, (3) 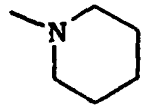, (4) 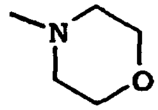,

(5) alkyl (e.g., $-CH_3$),
(6) $-OR^{23}$ wherein $R^{23}$ is selected from the group consisting of: alkyl, aryl and H, and
(7)

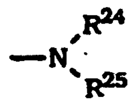

wherein $R^{24}$ and $R^{25}$ are independently selected from the group consisting of: $-NH_2$, alkoxy (e.g., $-OCH_3$), -OH, $-CH_2CO_2H$, $-OCH_2Ph$ (i.e., $-OCH_2C_6H_5$), $-CH(OCH_3)CH(CH_3)_2$

(i.e., 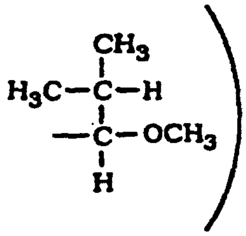),

alkyl, aryl, H, aralkyl, and heteroaralkyl; or $R^{24}$ and $R^{25}$ taken together form a carbon chain having 4 or 5 ($-CH_2-$) groups such that $R^{24}$ and $R^{25}$ taken together with the nitrogen to which they are bound form a 5 or 6 membered heterocycloalkyl ring, wherein "alkenyl", "alkynyl", "alkyl", "arylalkyl", 'aryl", "halo", "heteroaryl", "heteroarylalkyl" are "heterocycloalkyl are defined below

The compounds of this invention: (i) potently inhibit farnesyl protein transferase. but not geranylgeranyl protein transferase I, in vitro; (ii) block the phenotypic change induced by a form of transforming Ras which is a farnesyl acceptor but not by a form of transforming Ras engineered to be a geranylgeranyl acceptor; (iii) block intracellular processing of Ras which is a farnesyl acceptor but not of Ras engineered to be a geranylgeranyl acceptor: and (iv) block abnormal cell growth in culture induced by transforming Ras.

The compounds of this invention inhibit farnesyl protein transferase and the famesylation of the oncogene protein Ras. Thus, this invention further provides a method of inhibiting farnesyl protein transferase, (e.g., ras farnesyl protein transferase) in mammals, especially humans, by the administration of an effective amount of the tricyclic compounds described above. The administration of the compounds of this invention to patients, to inhibit farnesyl protein transferase, is useful in the treatment of the cancers described below.

This invention provides the use of a compound of formula (1.0) for the manufacture of a medicament for inhibiting or treating the abnormal growth of cells, including transformed cells, by administering an effective amount of a compound of this invention. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene: and (3) benign and malignant cells of other proliferative diseases in which aberrant Ras activation occurs.

This invention also provides the use of a compound of formula (1.0) for the manufacture of a medicament for inhibiting or treating tumor growth by administering an effective amount of the tricyclic compounds, described herein, to a mammal (e.g., a human) in need of such treatment. In particular, this invention provides the use of a compound of formula (1.0) for the manufacture of a medicament for inhibiting or treating the growth of tumors expressing an activated Ras oncogene by the administration of an effective amount of the above described compounds. Examples of tumors which may be inhibited or treated include, but are not limited to. lung cancer (e.g., lung adenocarcinoma), pancreatic cancers (e.g., pancreatic carcinoma such as, for example, exocrine pancreatic carcinoma), colon cancers (e.g., colorectal carcinomas, such as, for example, colon adenocarcinoma and colon adenoma), myeloid leukemias (for example, acute myelogenous leukemia (AML)), thyroid follicular cancer, myelodysplastic syndrome (MDS), bladder carcinoma, epidermal carcinoma, breast cancer and prostate cancer.

It is believed that this invention also provides the use of a compound of formula (1.0) for the manufacture of a medicament for inhibiting or treating proliferative diseases, both benign and malignant, wherein Ras proteins are aberrantly activated as a result of oncogenic mutation in other genes--i.e., the Ras gene itself is not activated by mutation to an oncogenic form--with said inhibition or treatment being accomplished by the administration of an effective amount of the tricyclic compounds described herein, to a mammal (e.g., a human) in need of such treatment. For example, the benign proliferative disorder neurofibromatosis, or tumors in which Ras is activated due to mutation or overexpression of tyrosine kinase oncogenes (e.g., neu, src, abl, lck, and fyn). may be inhibited or treated by the tricyclic compounds described herein.

The tricyclic compounds useful in this invention inhibit or treat the abnormal growth of cells. Without wishing to be bound by theory, it is believed that these compounds may function through the inhibition of G-protein function, such as ras p21, by blocking G-protein isoprenylation, thus making them useful in the treatment of proliferative diseases such as tumor growth and cancer. Without wishing to be bound by theory, it is believed that these compounds inhibit ras farnesyl protein transferase, and thus show antiproliferative activity against ras transformed cells.

## DETAILED DESCRIPTION OF THE INVENTION

As used herein, the following terms are used as defined below unless otherwise indicated:

Ac-represents acetyl:
$MH^+$-represents the molecular ion plus hydrogen of the molecule in the mass spectrum;
$M^+$-represents the molecular ion of the molecule in the mass spectrum;
benzotriazol-1-yloxy represents

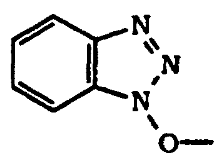

;

1-methyl-tetrazol-5-ylthio represents

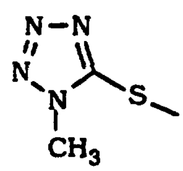

;

alkenyl-represents straight and branched carbon chains having at least one carbon to carbon double bond and containing from 2 to 12 carbon atoms, preferably from 2 to 6 carbon atoms and most preferably from 3 to 6 carbon atoms:
alkynyl-represents straight and branched carbon chains having at least one carbon to carbon triple bond and containing from 2 to 12 carbon atoms, preferably from 2 to 6 carbon atoms;
alkyl-(including the alkyl portions of aralkyl and heteroarylalkyl)-represents straight and branched carbon chains and contains from one to twenty carbon atoms, preferably one to six carbon atoms;
aralkyl-represents an aryl group, as defined below, bound to an alkyl group, as defined above, preferably the alkyl group is $-CH_2-$, (e.g., benzyl);
aryl (including the aryl portion of aralkyl)- represents a carbocyclic group containing from 6 to 15 carbon atoms and having at least one aromatic ring (e.g., aryl is a phenyl ring), with all available substitutable carbon atoms of the carbocyclic group being intended as possible points of attachment, said carbocyclic group being optionally substituted (e.g., 1 to 3) with one or more of halo, alkyl, hydroxy, alkoxy, phenoxy, $CF_3$, amino, alkylamino, dialkylamino, $-COOR^{10}$ or $-NO_2$;
BOC-represents $-C(O)OC(CH_3)_3$;
$-CH_2$-imidazolyl represents an imidazolyl group bound by any substitutable carbon of the imidazole ring to a $-CH_2-$, that is:

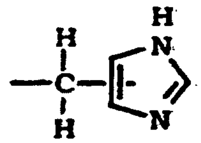,

such as $-CH_2$-(2-, 4- or 5-)imidazolyl, for example

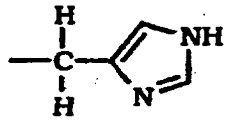

Et-represents ethyl;
halo-represents fluoro, chloro, bromo and iodo:
heteroaryl-represents cyclic groups, optionally substituted with $R^3$, $R^4$; phenyl, and or $-CH_2C(O)OCH_3$, said cyclic groups having at least one heteroatom selected from O, S or N, said heteroatom interrupting a carbocyclic ring structure and having a sufficient number of delocalized pi electrons to provide aromatic character, with the aromatic heterocyclic groups preferably containing from 2 to 14 carbon atoms, e.g., (2-, 4- or 5-)imidazolyl, triazolyl,

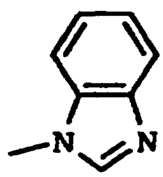, 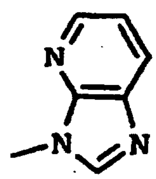,

2-, 3- or 4-pyridyl or pyridyl N-oxide (optionally substituted with $R^3$ and $R^4$), wherein pyridyl N-oxide can be represented as:

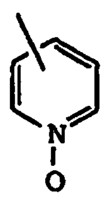 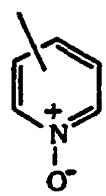 or 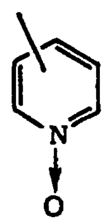 ;

heteroarylalkyl (heteroaralkyl)-represents a heteroaryl group, as defined above, bound to an alkyl group, as defined above, preferably the alkyl group is $-CH_2-$ (e.g., $-CH_2$-(4- or 5-)imidazolyl);
heterocycloalkyl-represents a saturated, branched or unbranched carbocylic ring containing from 3 to 15 carbon atoms, preferably from 4 to 6 carbon atoms, which carbocyclic ring is interrupted by 1 to 3 hetero groups selected from -O-, -S- or - $NR^{10}$-, suitable heterocycloalkyl groups include: (1) 2- or 3-tetrahydrofuranyl, (2) 2- or 3- tetrahydrothienyl, (3) 2-, 3- or 4-piperidinyl, (4) 2- or 3-pyrrolidinyl, (5) 2- or 3-piperizinyl, and (6) 2- or 4-dioxanyl; and
Ph-represents phenyl

The following solvents and reagents are referred to herein by the abbreviations indicated: tetrahydrofuran (THF); isopropanol (iPrOH); ethanol (EtOH); methanol (MeOH); acetic acid (HOAc or AcOH); ethyl acetate (EtOAc); N,N-dimethylformamide (DMF); trifluoroacetic acid (TFA); trifluoro-acetic anhydride (TFAA); 1-hydroxybenzotriazole (HOBT); 1-(3-dimethylaminopropyl)-3-ethyl carbodiimide hydrochloride (DEC); diisobutylaluminum hydride(DIBAL); and 4-methylmorpholine (NMM).
Reference to the position of the substituents $R^1$, $R^2$, $R^3$, and $R^4$ is based on the numbered ring structure:

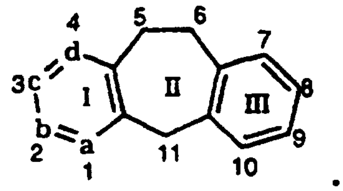

Those skilled in the art will also appreciate that the S and R stereochemistry at the C-11 bond are:

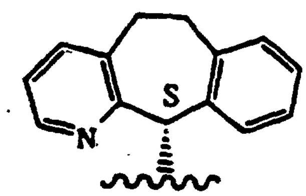

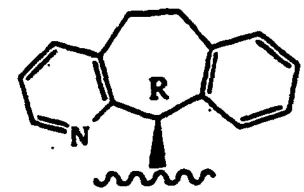

Compounds of Formula 1.0 include compounds wherein the bottom piperidinyl group is a 4- or 3-piperidinyl group, i.e.,

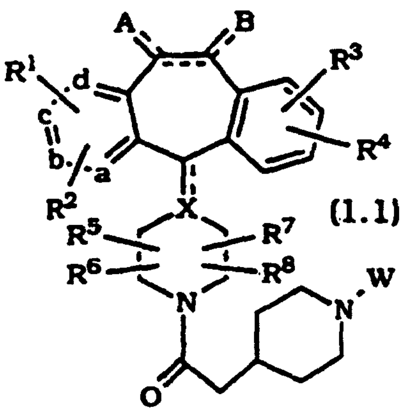

(1.1)

or

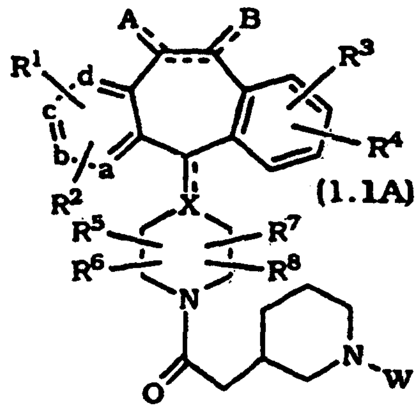

(1.1A)

Compounds of Formula 1.0 also include compounds wherein $R^2$ is H, and $R^1$, $R^3$ and $R^4$ are independently selected from Br or Cl .

Preferably, compounds of Formula 1.0 are represented by compounds of Formula 1.1:

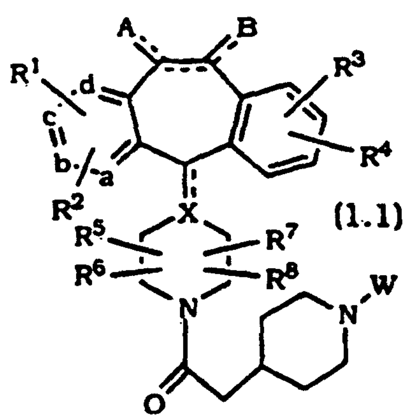

(1.1)

wherein all sinhstituents are as defined for Formula 1.0.

Preferably, $R^2$ is H and $R^1$, $R^3$ and $R^4$ are halo: a is N and b, c and d are carbon; A and B are each $H_2$; the optional bond between C5 and C6 is absent: X is CH; and $R^5$, $R^6$, $R^7$ and $R^8$ are H. More preferably, $R^1$, $R^3$ and $R^4$

are independently selected from Br or Cl. Most preferably, $R^1$ is Br, and $R^3$ and $R^4$ are independently selected from Cl and Br.

More preferably, compounds of Formula 1.0 are represented by compounds of Formula 1.2 and Formula 1.3:

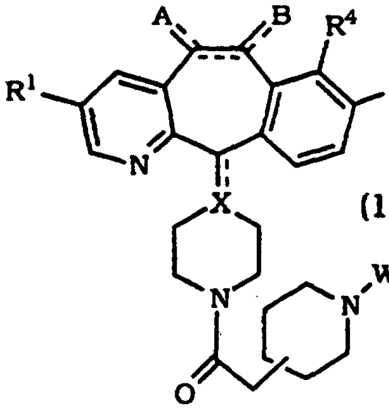

(1.2)

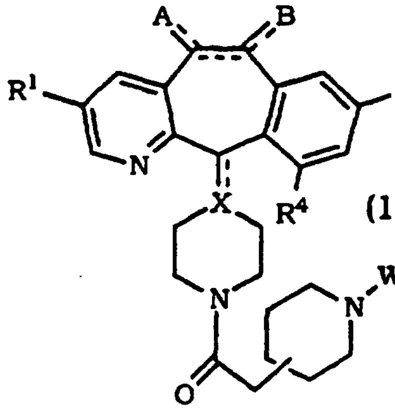

(1.3)

and most preferably, compounds of Formulas 1.4 and 1.5

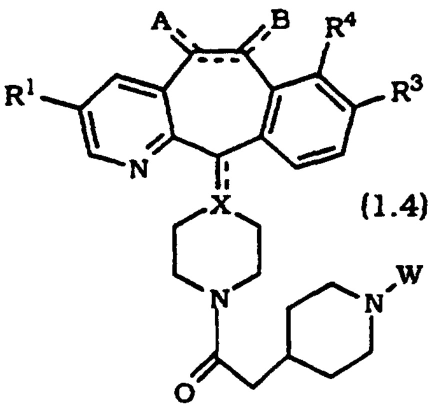

(1.4)

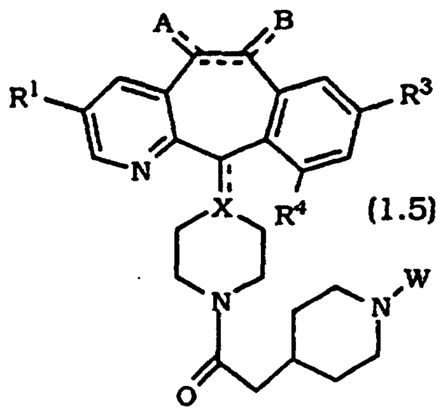

(1.5)

wherein $R^1$, $R^3$ and $R^4$ are each independently selected from halo, preferably, Br or Cl; and A, B, X and W are as defined for Formula 1.0. More preferably, A and B are each $H_2$: the optional bond between C5 and C6 is absent; and X is CH. Most preferably, $R^1$ is Br; $R^3$ and $R^4$ are independently Br or Cl, and still more preferably $R^3$ is Cl and $R^4$ is Br; A and B are each $H_2$; the optional bond between C5 and C6 is absent; X is CH; and $R^5$, $R^6$, $R^7$ and $R^8$ are H.

Examples of $R^{15}$ include:

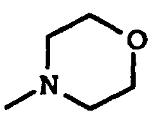 , 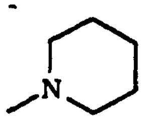 , 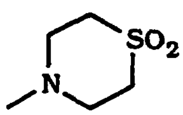 , 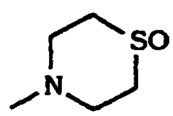 , 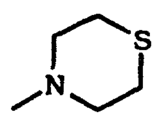 ,

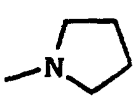 , 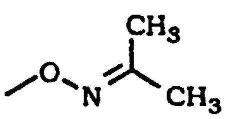 , 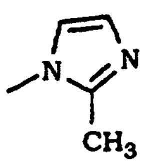 , 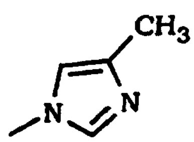 , 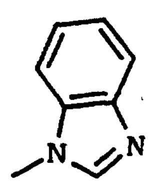 ,

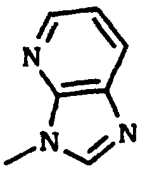, 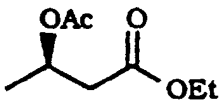, 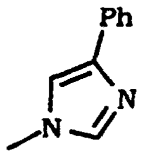, 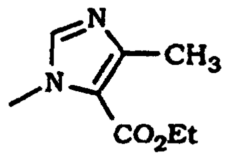,

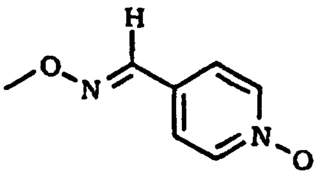, 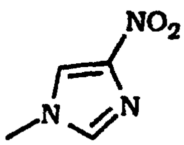, 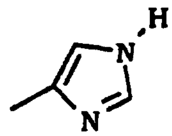, 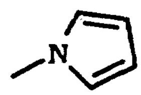,

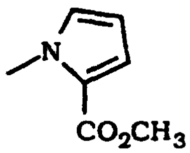, 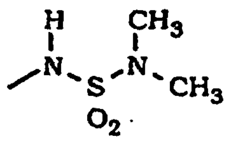, 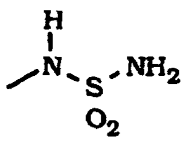, 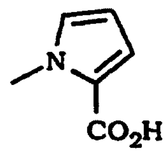,

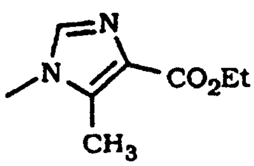, 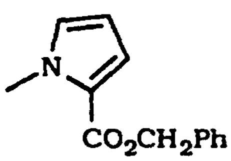, 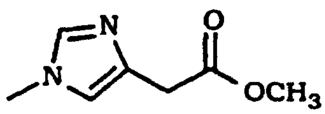, 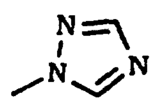,

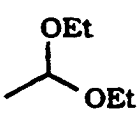, 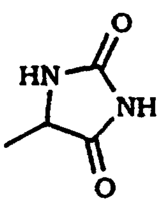, 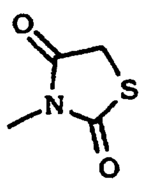, 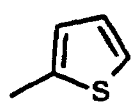, 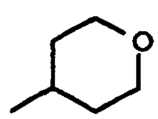,

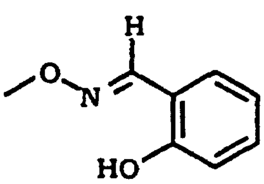, 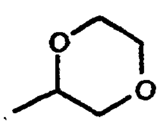, 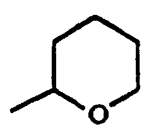, 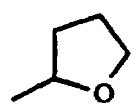,

and -OH, -CN.

When W represents:

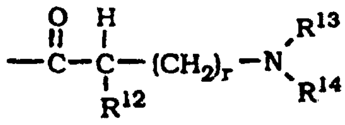

and r is 0: (1) preferably, $R^{12}$ is selected from the group consisting of: (a) H; (b) alkyl; (c) aralkyl; and (d) heteroaralkyl; and most preferably, $R^{12}$ is selected from the group consisting of: (a) H, (b) methyl, (c) $-CH_2$-imidazolyl and (d) benzyl; (2) preferably, $R^{13}$ and $R^{14}$ are independently selected from the group consisting of: (a) H; (b) -C(O)OR$^{16}$ wherein $R^{16}$ is alkyl; (c) $-SO_2R^{17}$ wherein $R^{17}$ is alkyl or aryl; (d) -C(O)R$^{18}$ wherein $R^{18}$ is aryl; and (e) alkyl; and most preferably, $R^{13}$ and $R^{14}$ are independently selected from the group consisting of: (a) H, (b) $-C(O)OC(CH_3)_3$, (c) $-SO_2CH_3$ and (d) -C(O)-phenyl. Preferably, when one of $R^{13}$ or $R^{14}$ is -C(O)OR$^{16}$, $-SO_2R^{17}$, -C(O)R$^{18}$, alkaryl or cycloalkyl, the remaining $R^{13}$ or $R^{14}$ is H. Preferred combinations of substituent groups include: (1) $R^{12}$ being alkyl (more preferred, methyl), $R^{13}$ being -C(O)OR$^{16}$ (more preferred $-C(O)OC(CH_3)_3$) and $R^{14}$ being H; (2) $R^{12}$ being heteroarylalkyl (more preferred $-CH_2$-(4 or 5)-imidazolyl), $R^{13}$ being H or -C(O)OR$^{16}$ (more preferred H or $-C(O)OC(CH_3)_3$) and $R^{14}$ being H; (3) $R^{12}$ being aralkyl (more preferred benzyl), $R^{13}$ being -C(O)OR$^{16}$ (more preferred $-C(O)OC(CH_3)_3$) and $R^{14}$ being H; (4) $R^{12}$ being H, $R^{13}$ being -C(O)OR$^{16}$ (more preferred $-C(O)OC(CH_3)_3$) and $R^{14}$ being H; (5) $R^{12}$ being H, $R^{13}$ being $-SO_2R^{17}$ (more preferred $-SO_2CH_3$) and $R^{14}$ being H; and (6) $R^{12}$ being H, $R^{13}$ being -C(O)R$^{18}$ (more preferred -C(O)-phenyl) and $R^{14}$ being H.

Those skilled in the art will appreciate that the W substituent described in the previous paragraph can be derived from known amino acids having one carboxyl and amino group. Examples of such amino acids include but are not limited to glycine, alanine, phenylalanine, asparagine and histidine. For example, see Morrison and Boyd, Organic Chemistry, Fifth Edition, Allyn and Bacon, Inc., Boston, pages 1346-1347, the disclosure of which is incorporated herein by reference thereto.

When W represents

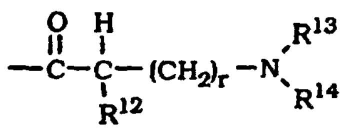

and r is 1 or 2, preferably $R^{12}$ is H, and $R^{13}$ and $R^{14}$ are independently selected from alkyl, most preferably, $R^{13}$ and $R^{14}$ are the same alkyl group (e.g., methyl).

When W represents

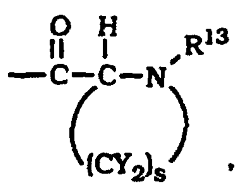 , preferably 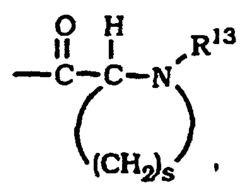 ,

s is preferably 3, such that a pyrrolidone ring is formed, and $R^{13}$ is preferably H or -C(O)OR$^{16}$ wherein $R^{16}$ is alkyl; most preferably, $R^{13}$ is H or $-C(O)OC(CH_3)_3$.

When W represents:

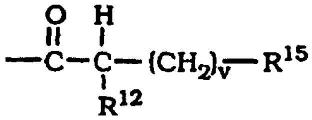

and v is O, preferably $R^{12}$ represents H and $R^{15}$ represents heteroaryl or heterocycloalkyl. Most preferably, when R15 is heteroaryl said heteroaryl is imidazolyl

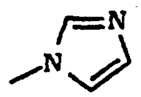

and when $R^{15}$ is heterocycloalkyl said heterocycloalkyl is

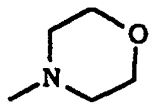 or 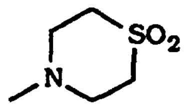 .

When W represents:

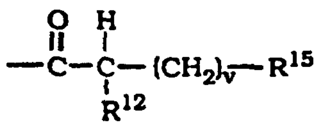

and v is 1 or 2, preferably $R^{12}$ represents H and $R^{15}$ is heterocycloalkyl. Most preferably $R^{12}$ represents H and $R^{15}$ is heterocycloalkyl, e.g.,

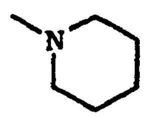 .

When W represents

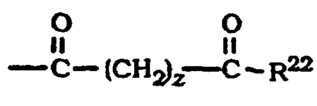

and z is 0, preferably $R^{22}$ represents

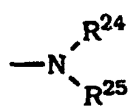

and $R^{24}$ and $R^{25}$ preferably represent H.

When W represents

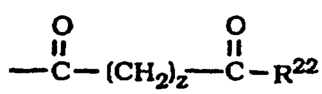

and z is 1, 2, 3, 4 or 5, $R^{22}$ preferably represents $-OR^{23}$ and $R^{23}$ preferably represents alkyl and most preferably methyl.

Compounds of Formulas 1.2A and 1.3A:

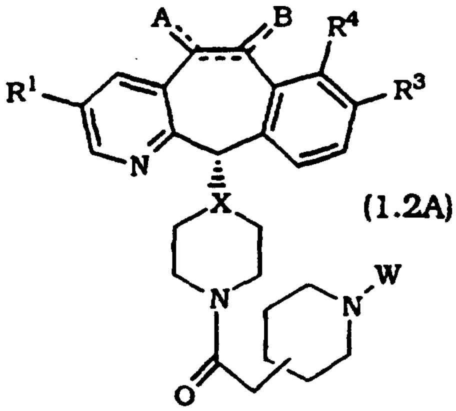

(1.2A)

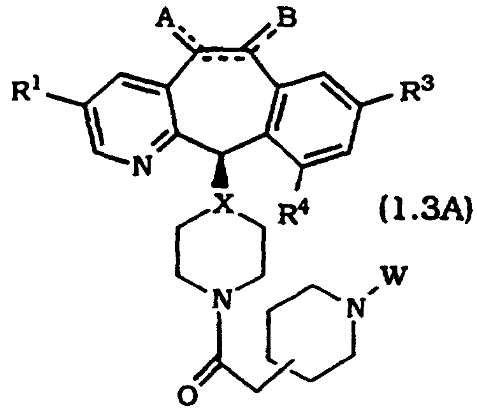

(1.3A)

are preferred when X is CH or N, and $R^1$, $R^3$ and $R^4$ are halo.

The preferred compounds of this invention are represented by the compounds of Formulas:

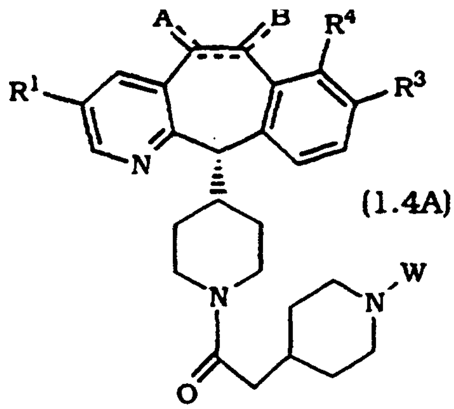

(1.4A)

and

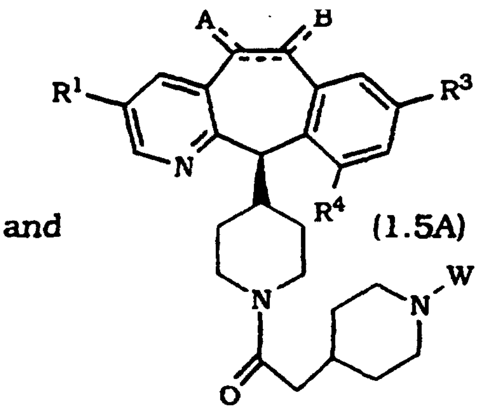

(1.5A)

wherein $R^1$, $R^3$ and $R^4$ are halo and the remaining substituents are as defined above, with the compounds of Formula 1.5A being more preferred.

Those skilled in the art will appreciate that W substituent:

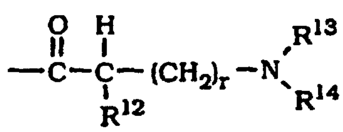

wherein r is 0 includes

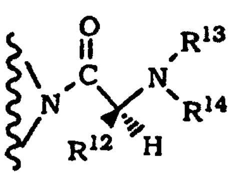

and

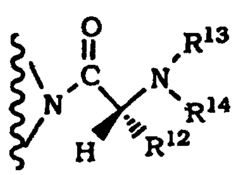

,

and W substituent:

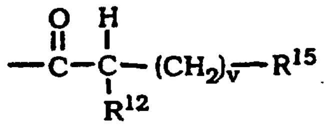

wherein v is 0 includes

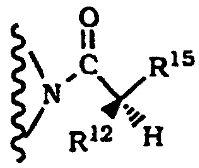

and

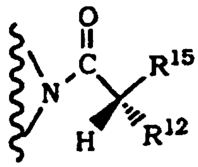

Representative compounds of Formula 1.0 wherein W is

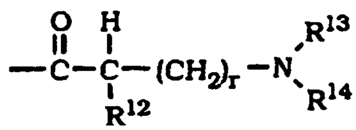

and r is 0 include:

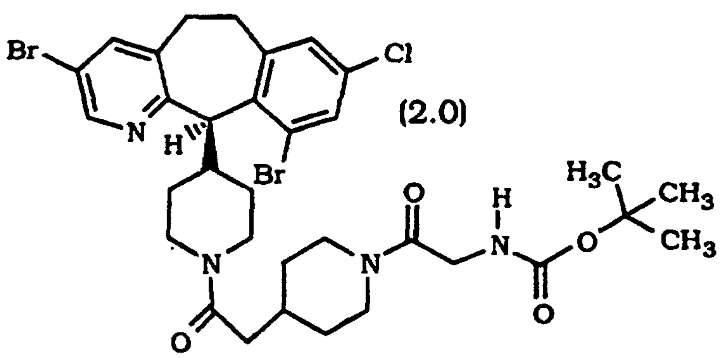

(2.0)

;

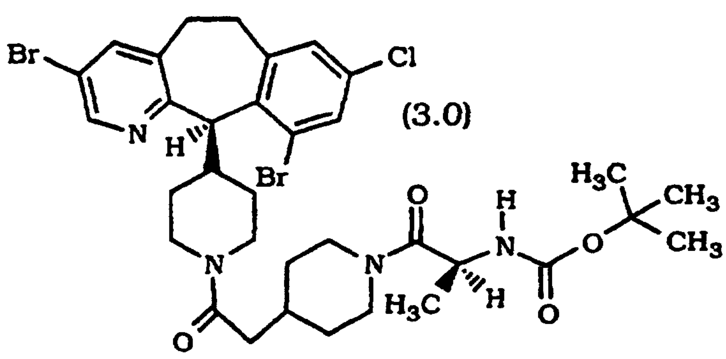

(3.0)

;

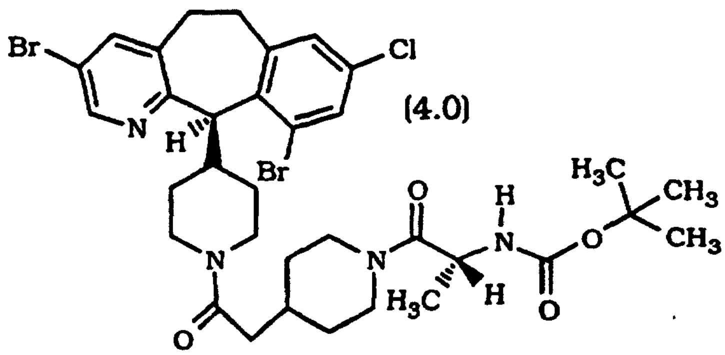

(4.0)

;

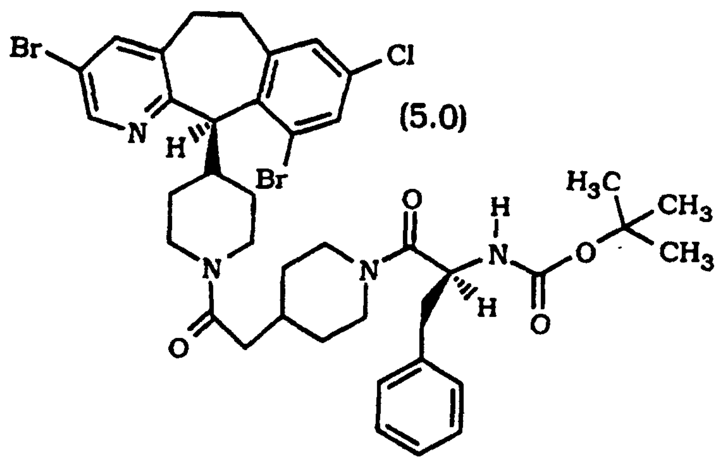

(5.0)

;

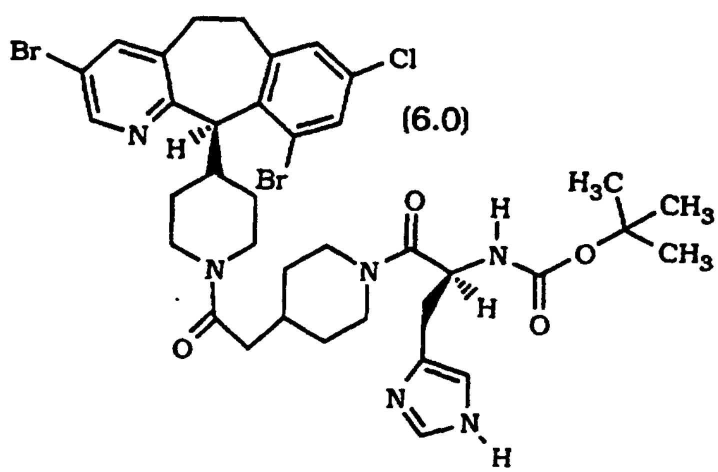

(6.0)

;

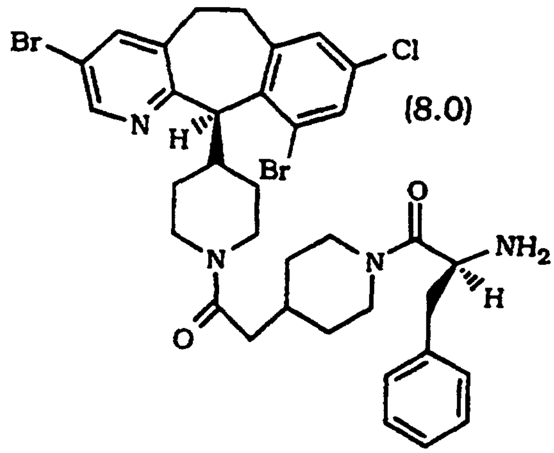

(8.0)

;

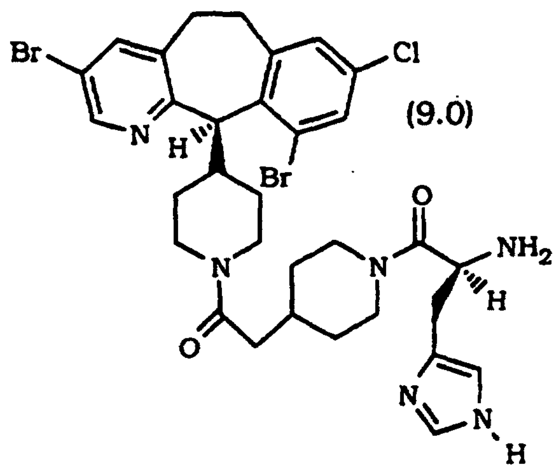

(9.0)

;

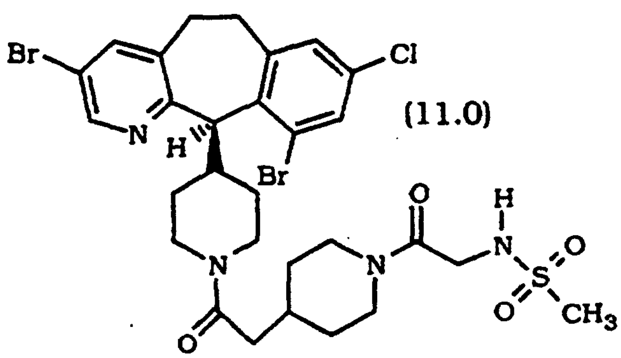

(11.0)

;

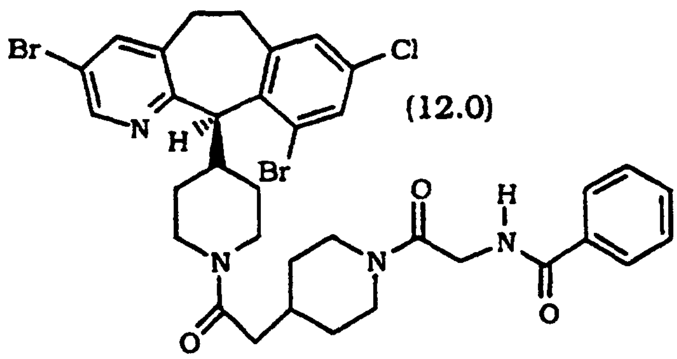

(12.0)

and

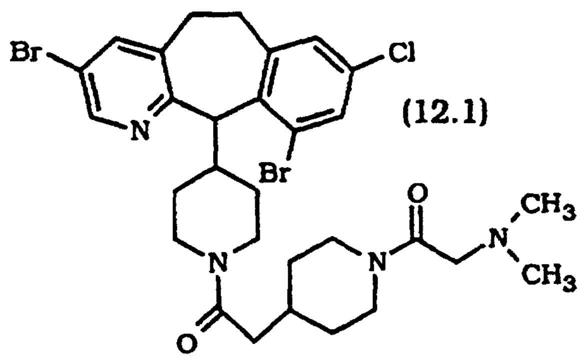

(12.1)

.

Representative compounds of Formula 1.0 wherein W is

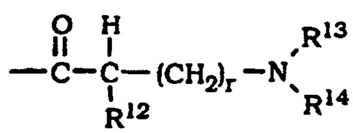

and r is 1 or 2 include:

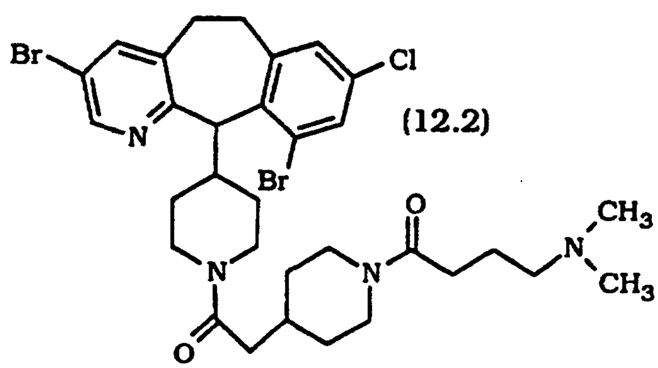

(12.2)

;

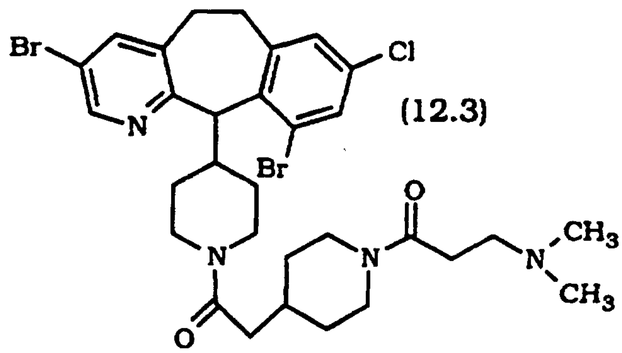

(12.3)

;

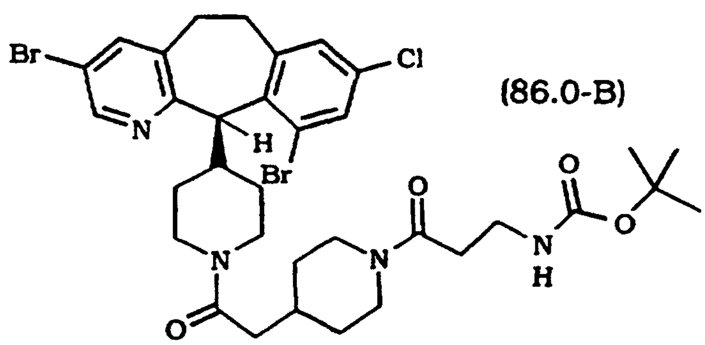

(86.0-B)

;

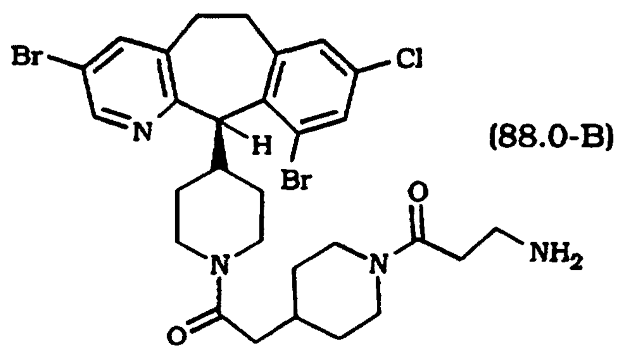

(88.0-B)

;

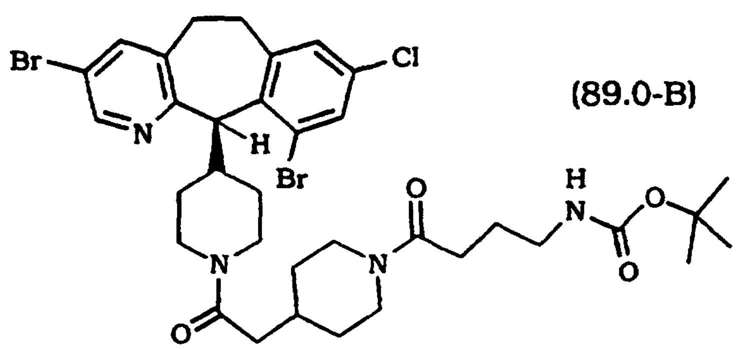

(89.0-B)

and

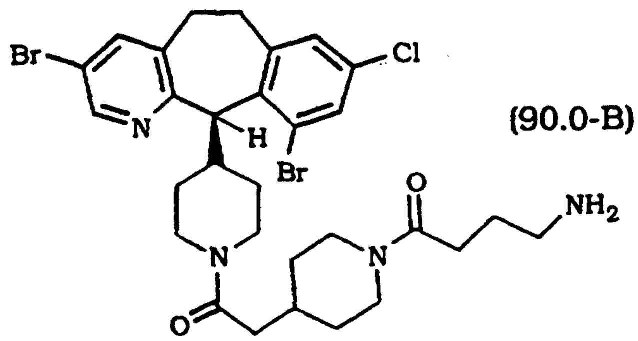

(90.0-B)

.

Representative compounds of Formula 1.0 wherein W is

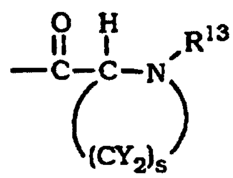

and s is 3 include:

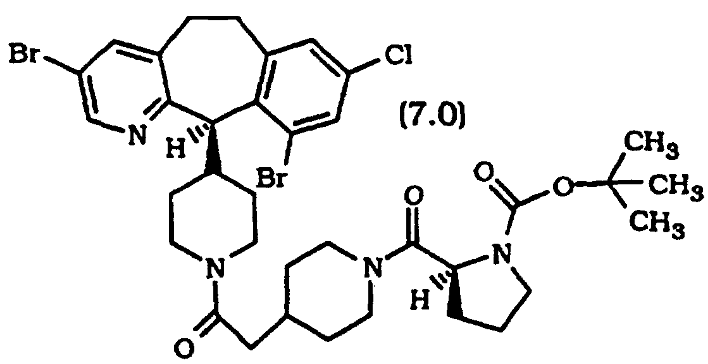

(7.0)

;

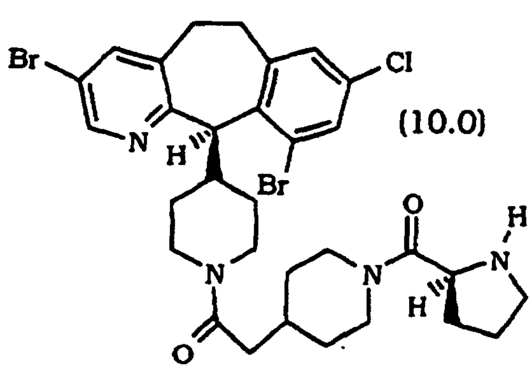

(10.0)

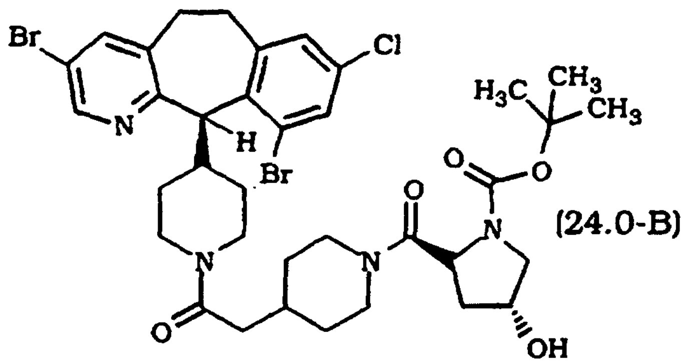

(24.0-B)

and

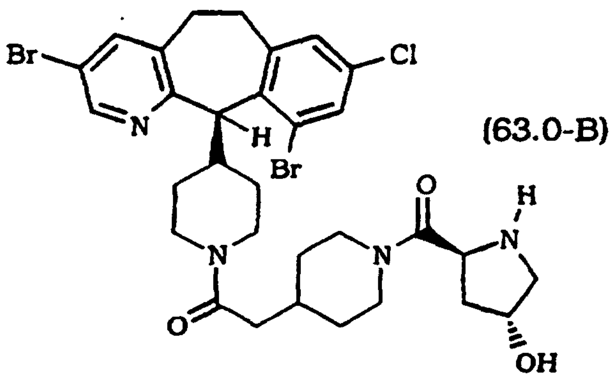

(63.0-B)

.

Representative compounds of Formula 1.0 wherein W is

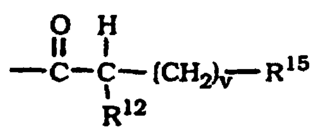

and v is 0 include:

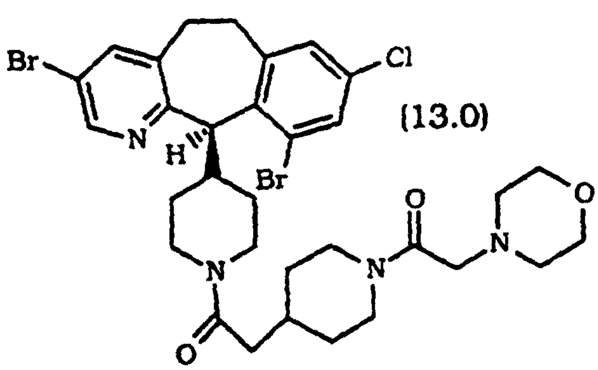

(13.0)

;

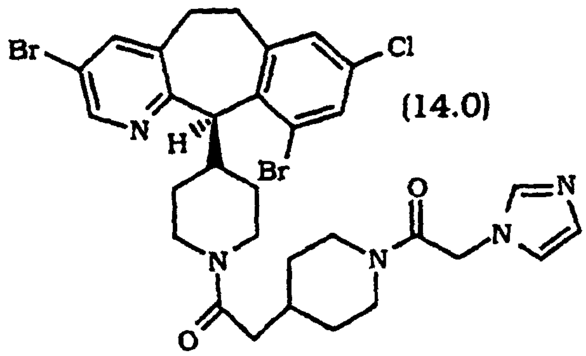

(14.0)

;

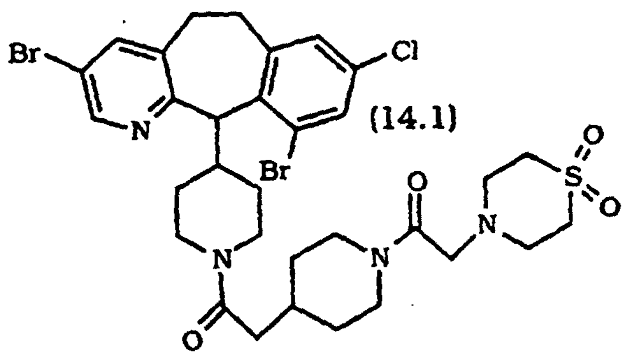

(14.1)

;

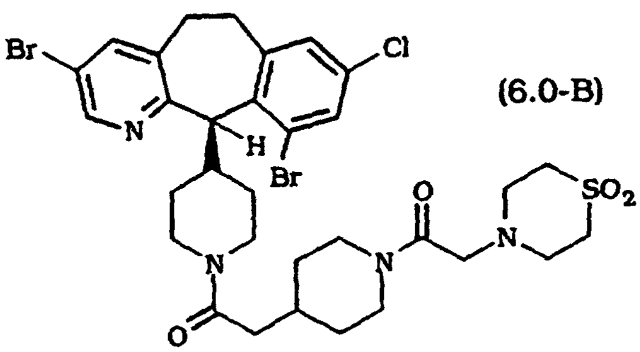

(6.0-B)

;

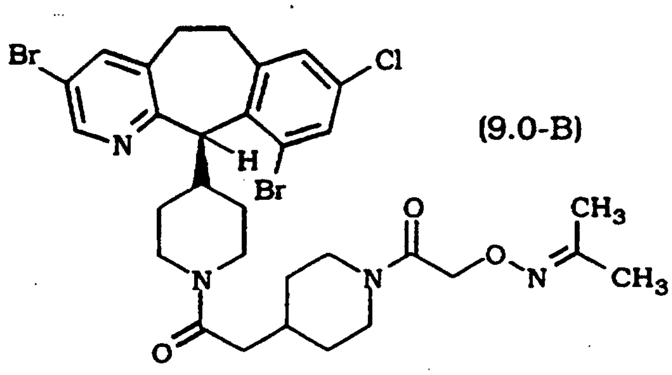

(9.0-B)

;

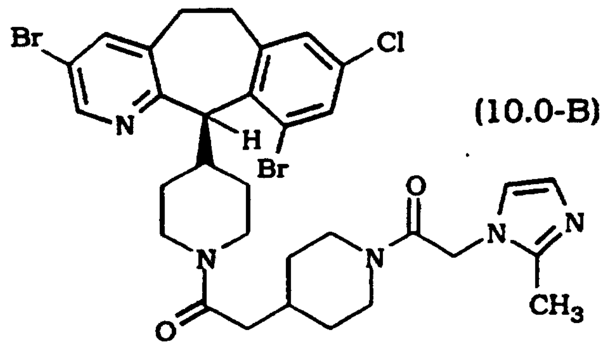

(10.0-B)

;

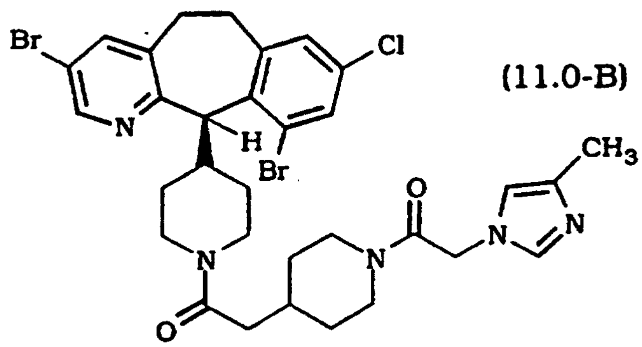

(11.0-B)

;

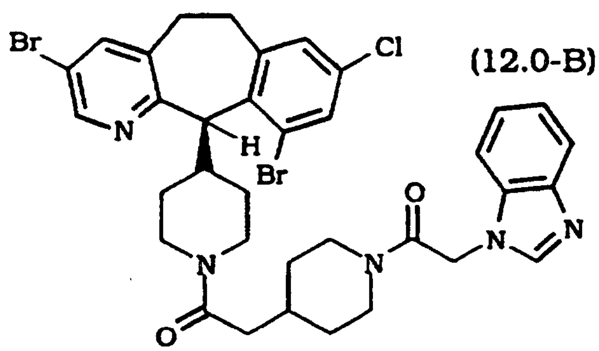

(12.0-B)

;

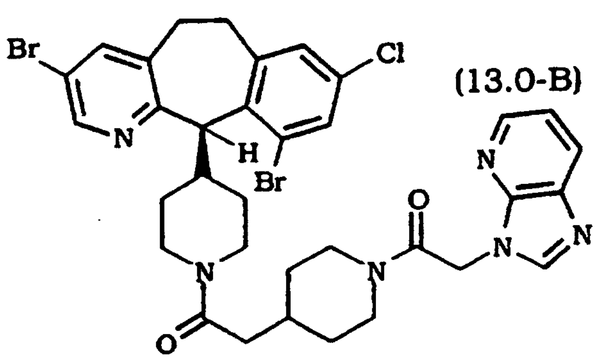

(13.0-B)

;

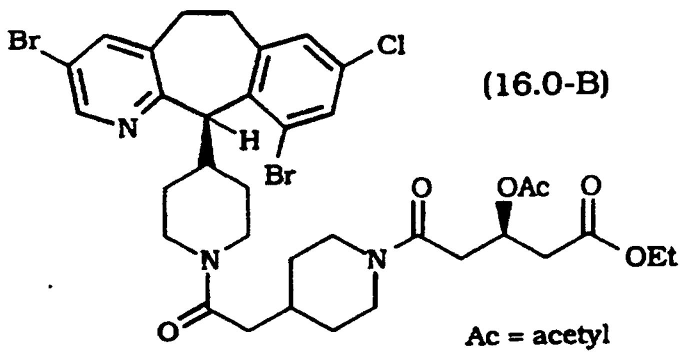

(16.0-B)

Ac = acetyl ;

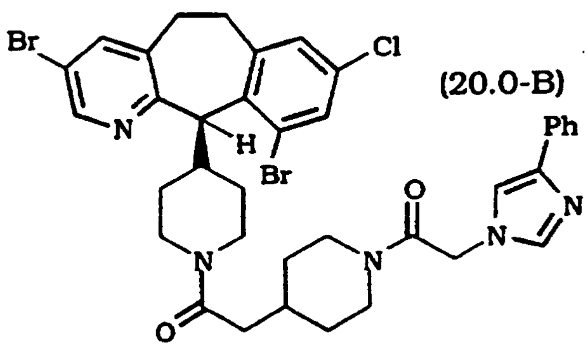

(20.0-B)

;

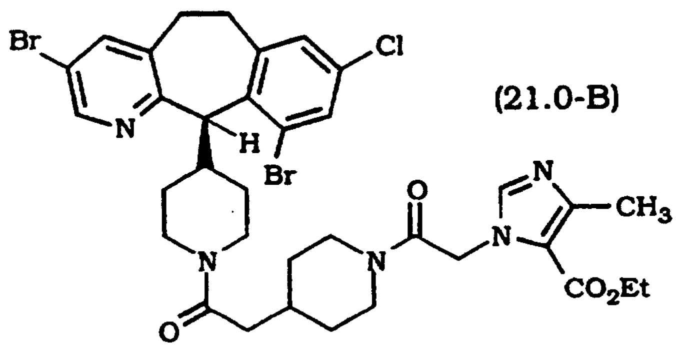

(21.0-B)

;

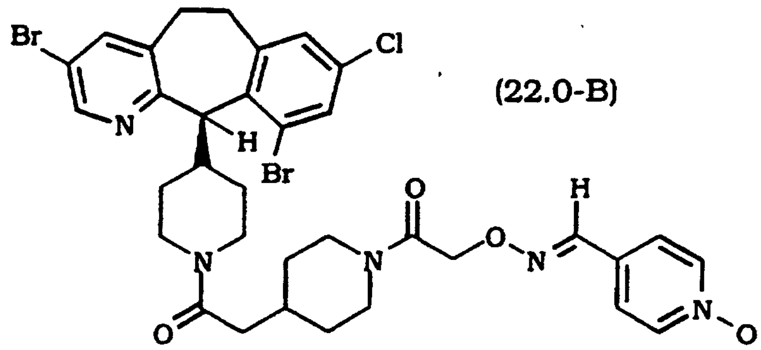

(22.0-B)

;

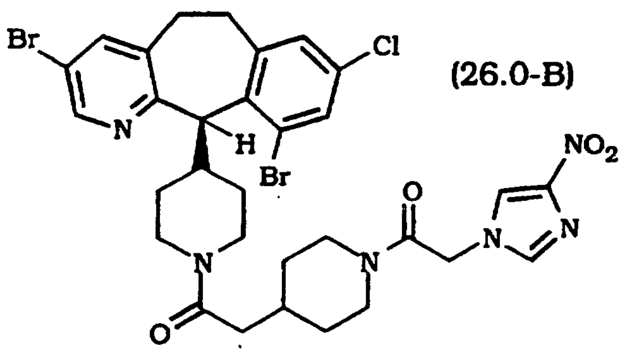

(26.0-B)

;

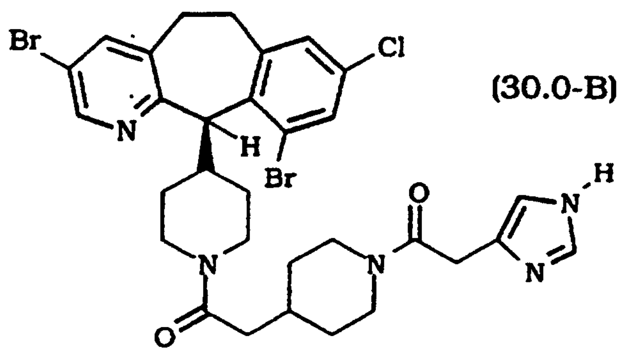

(30.0-B)

;

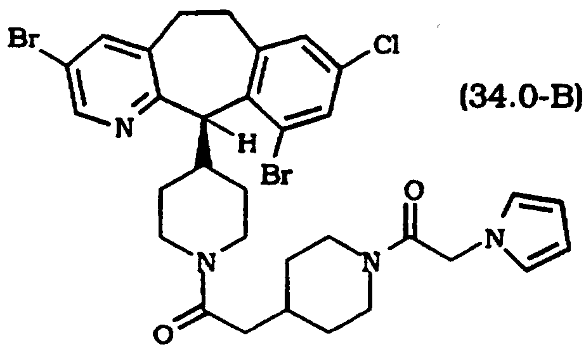

(34.0-B)

;

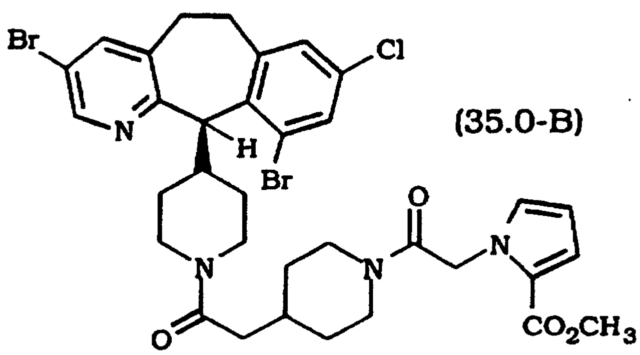

(35.0-B)

;

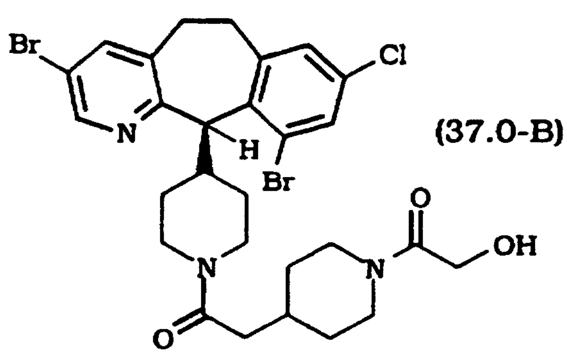

(37.0-B)

;

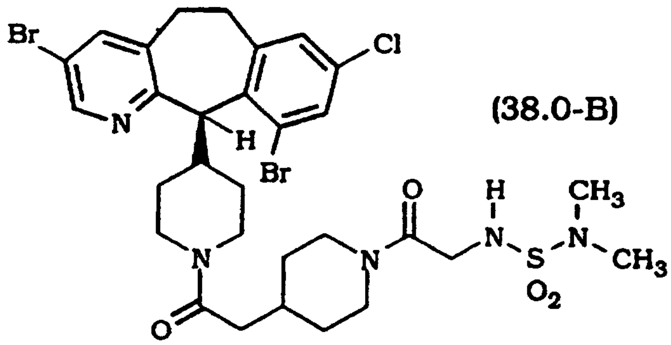

(38.0-B)

;

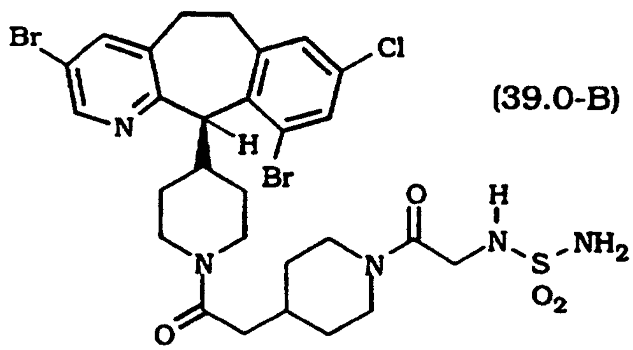

(39.0-B)

;

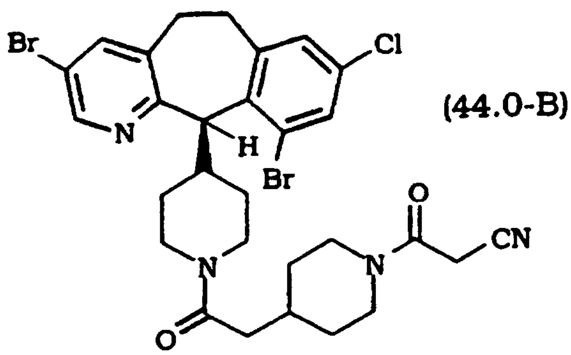

(44.0-B)

;

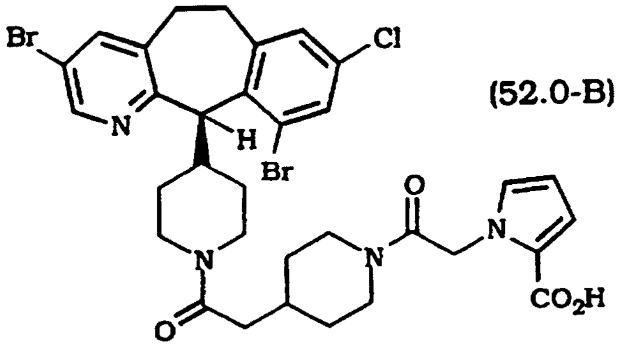

(52.0-B)

;

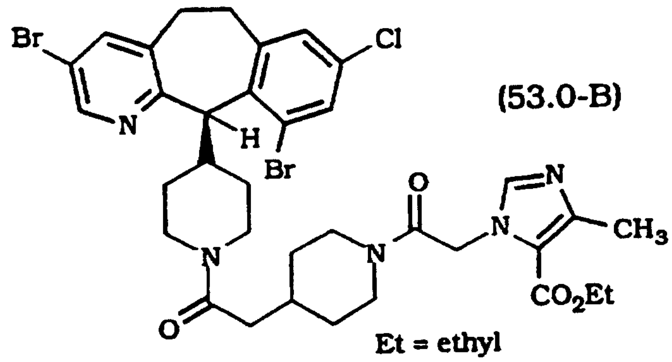

(53.0-B)

Et = ethyl ;

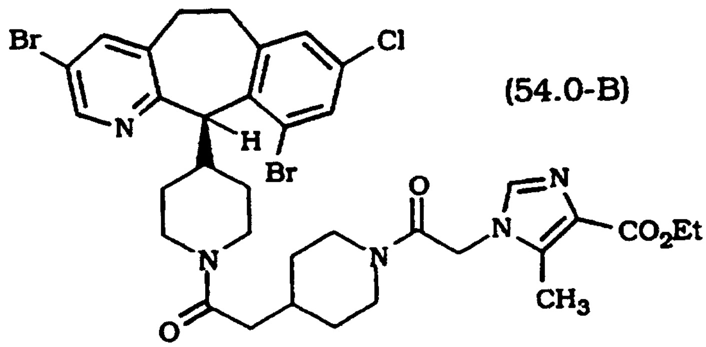

(54.0-B)

;

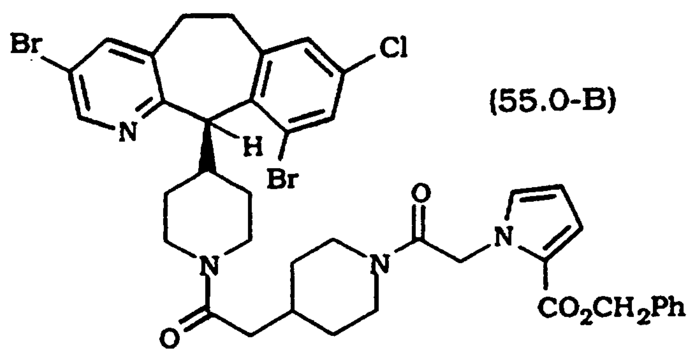

(55.0-B)

Ph = phenyl ;

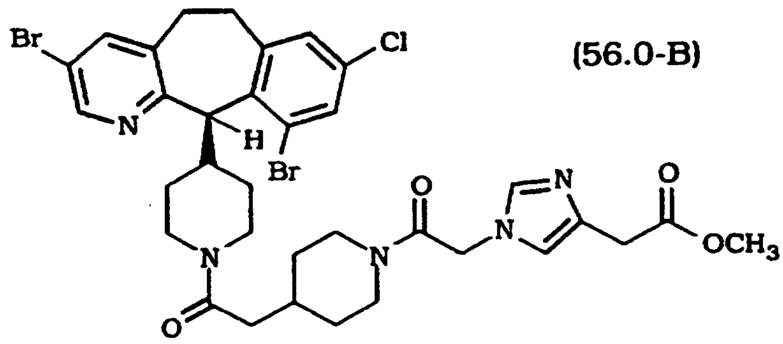

(56.0-B)

;

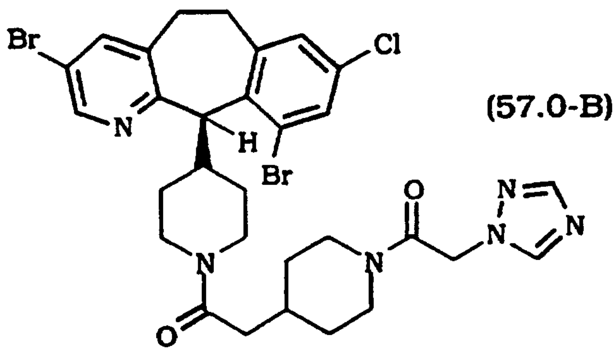

(57.0-B)

;

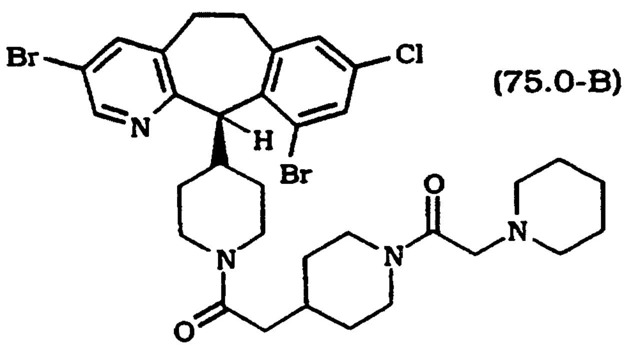

(75.0-B)

;

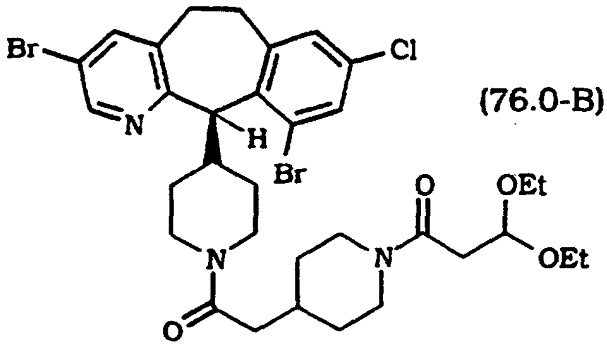

(76.0-B)

;

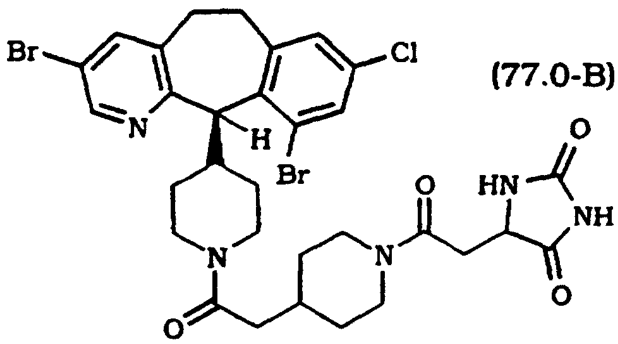

(77.0-B)

;

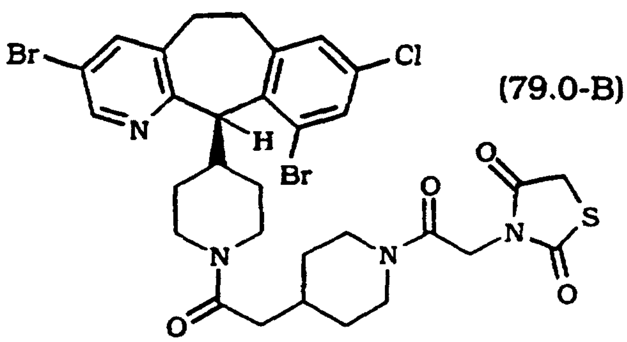

(79.0-B)

;

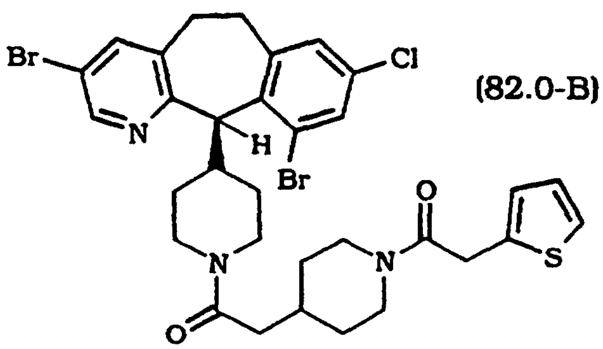

(82.0-B)

;

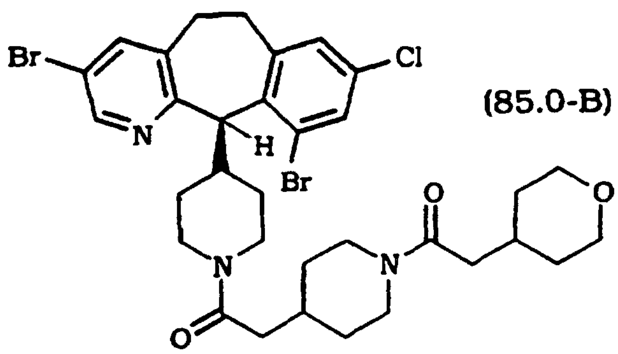

(85.0-B)

;

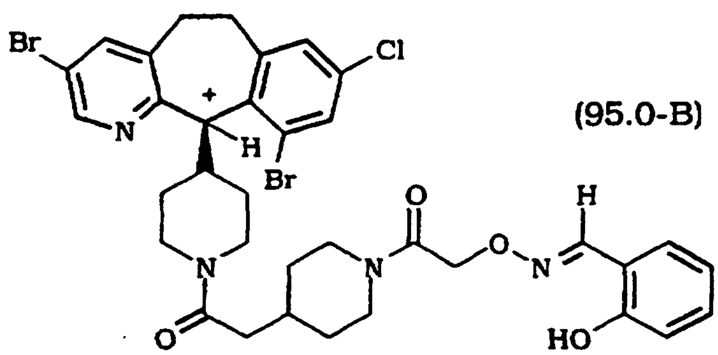

(95.0-B)

;

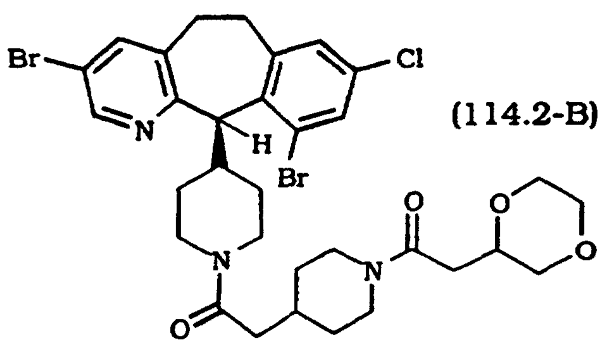

(114.2-B)

;

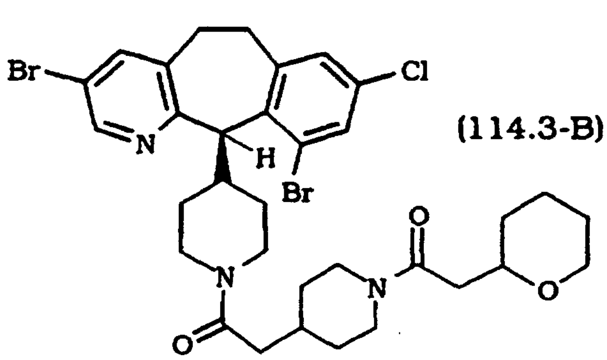

(114.3-B)

and

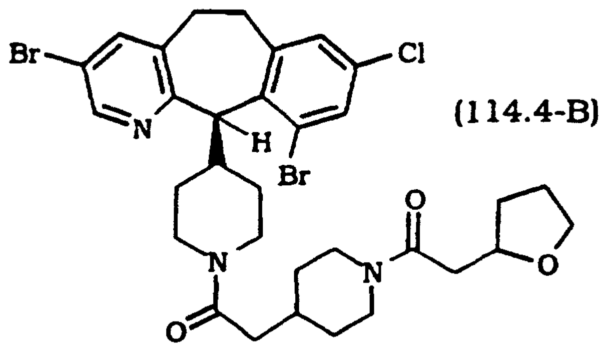

(114.4-B)

.

Representative compounds of Formula 1.0 wherein W is

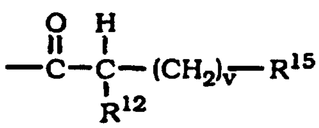

and v is 1 include:

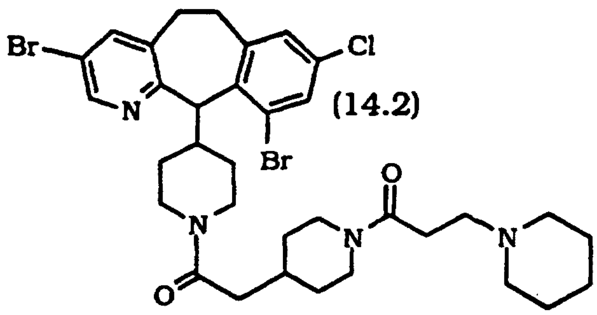

(14.2)

;

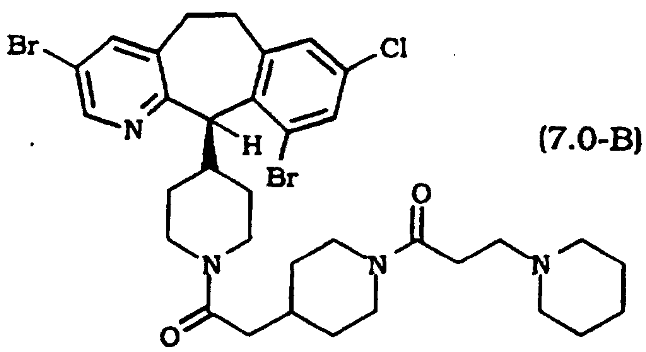

(7.0-B)

and

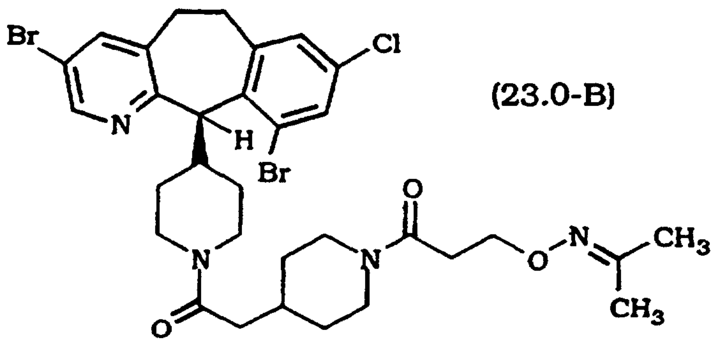

(23.0-B)

.

Compounds of Formula 1.0 wherein W is

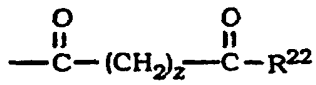

and z is 0 include:

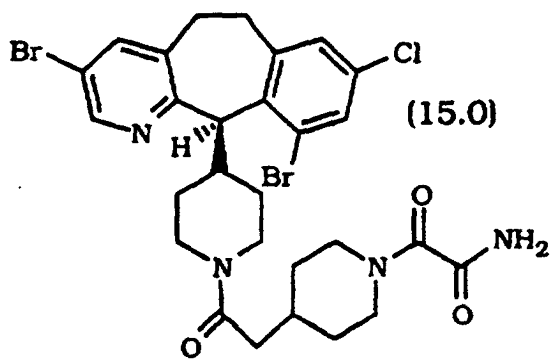

(15.0)

;

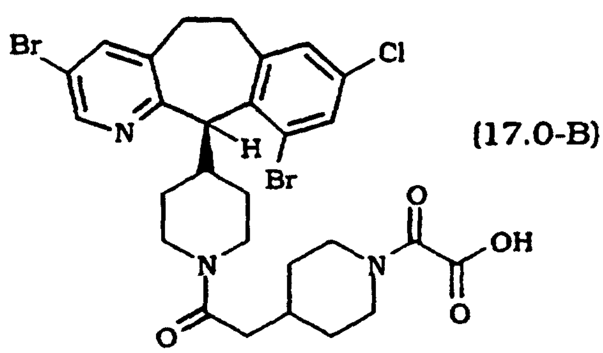

(17.0-B)

;

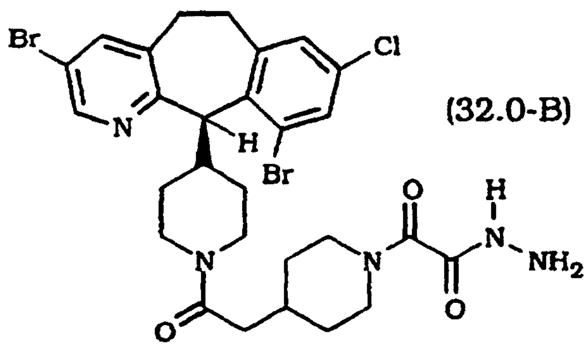

(32.0-B)

;

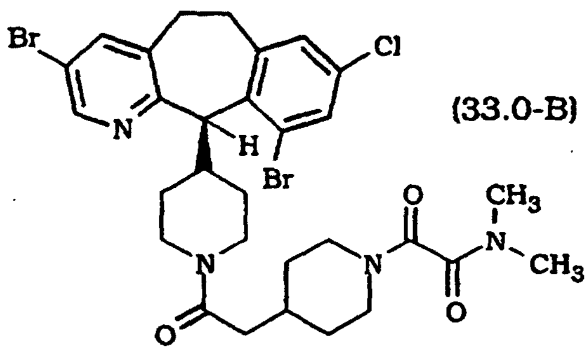

(33.0-B)

;

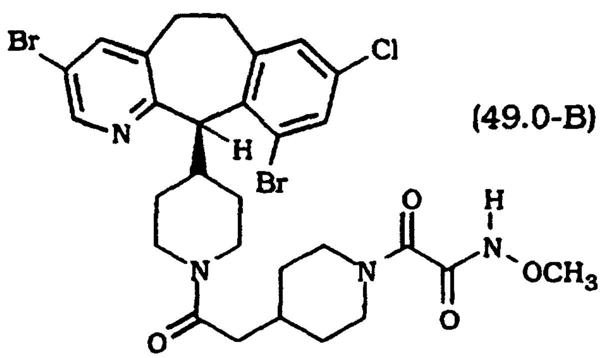

(49.0-B)

;

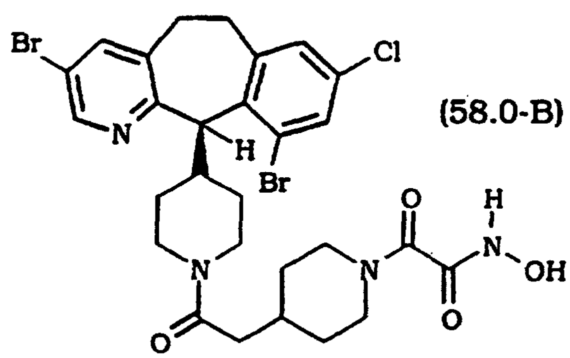

(58.0-B)

;

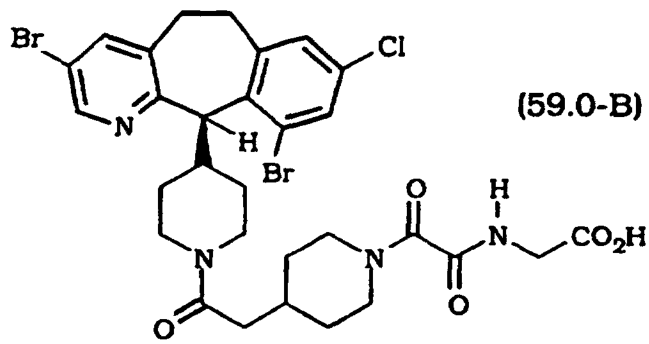

(59.0-B)

;

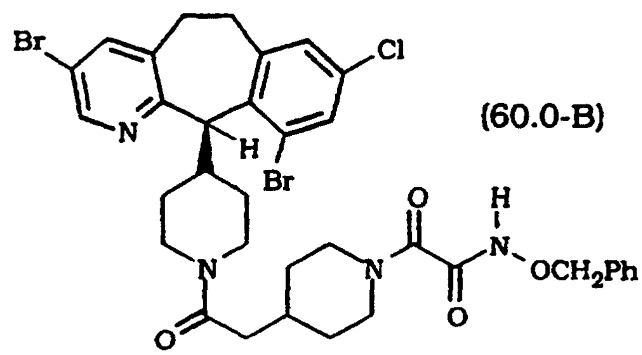

(60.0-B)

;

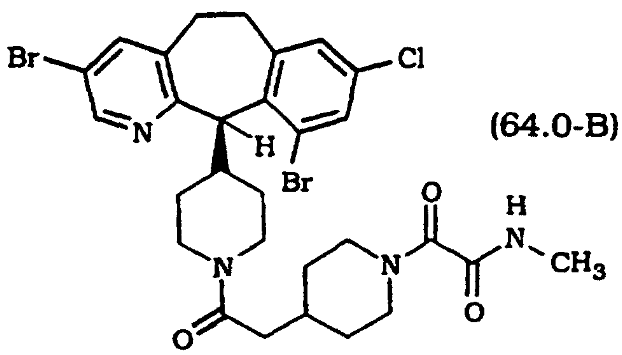

(64.0-B)

;

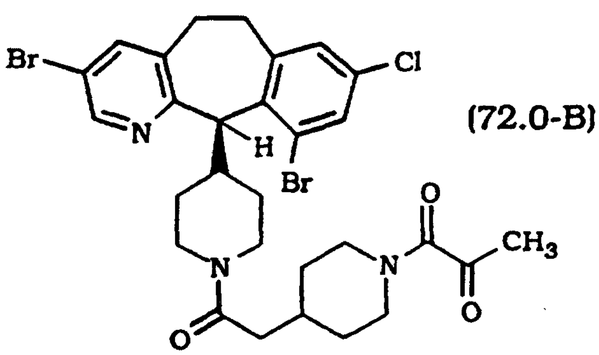

(72.0-B)

;

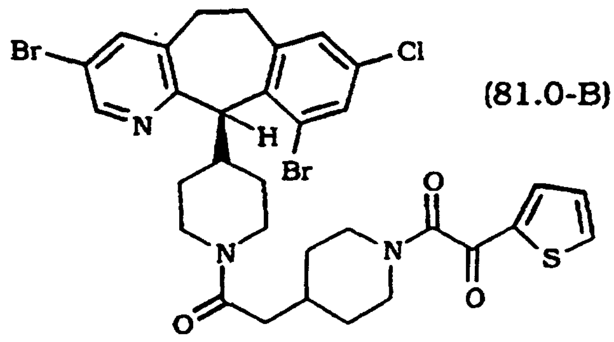

(81.0-B)

and

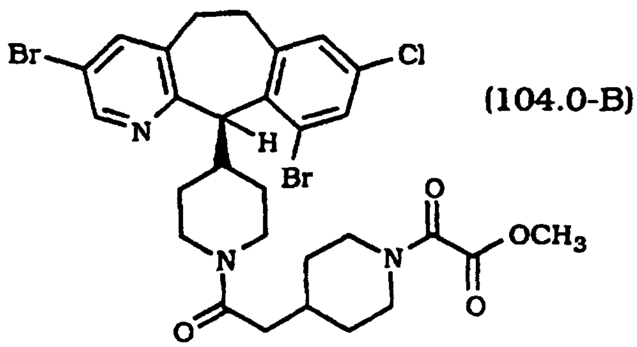

(104.0-B)

Compounds of Formula 1.0 wherein W is

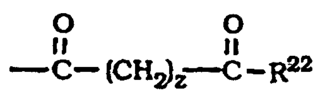

and z is 1, 2, 3, 4 or 5 include:

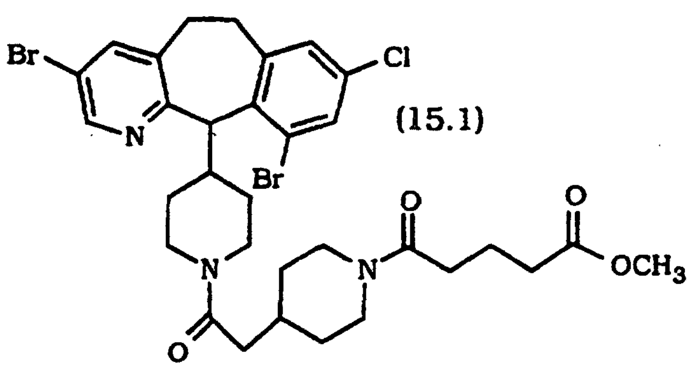

(15.1)

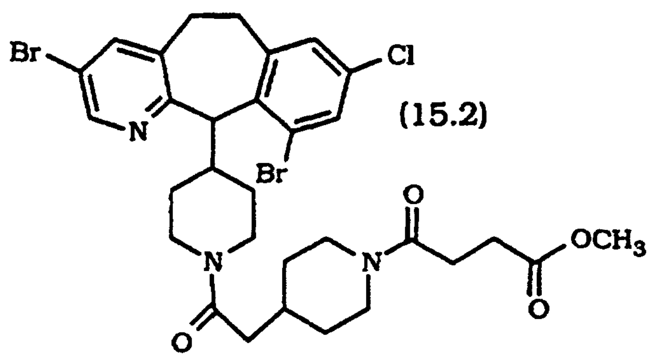

(15.2)

;

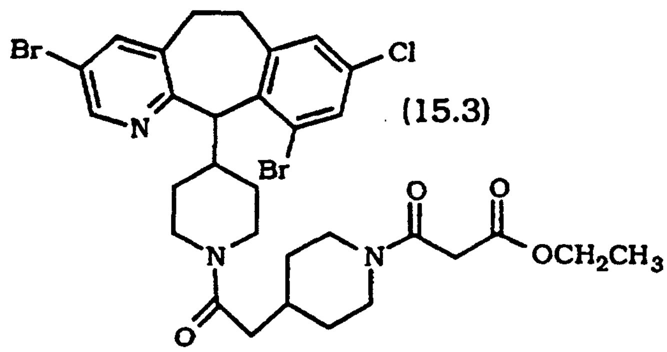

(15.3)

;

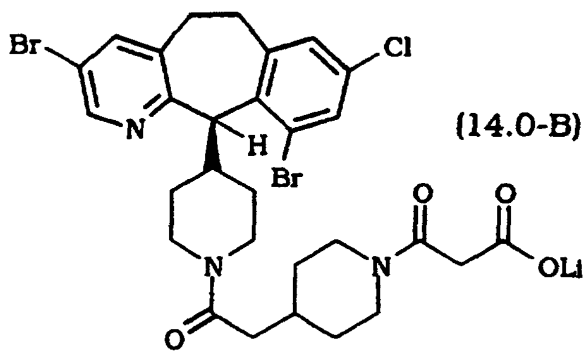

(14.0-B)

;

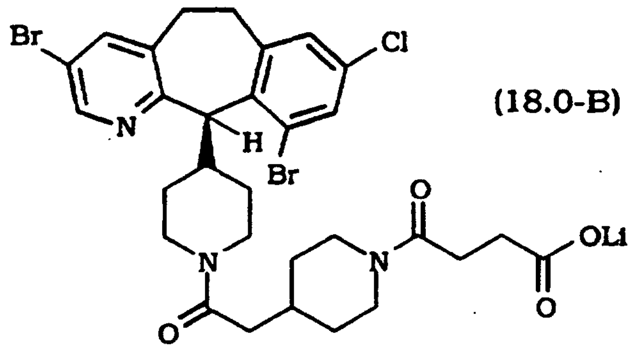

(18.0-B)

;

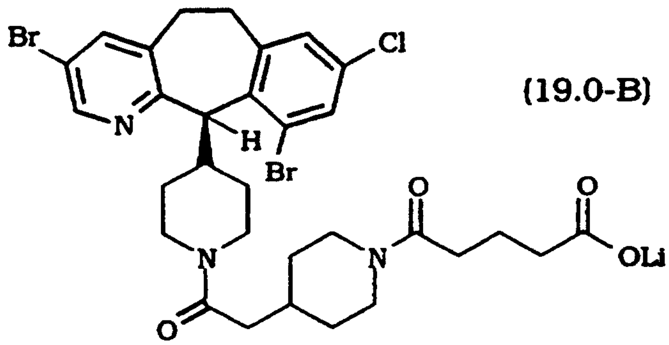

(19.0-B)

;

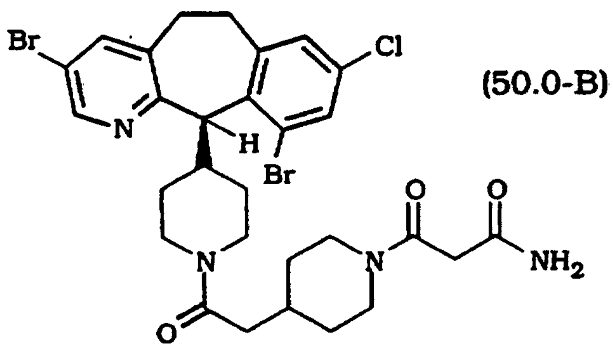

(50.0-B)

;

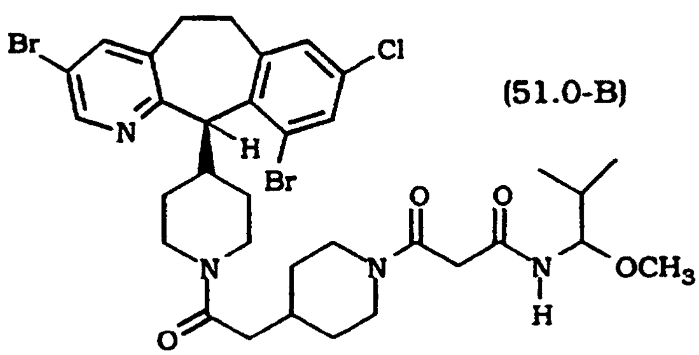

(51.0-B)

;

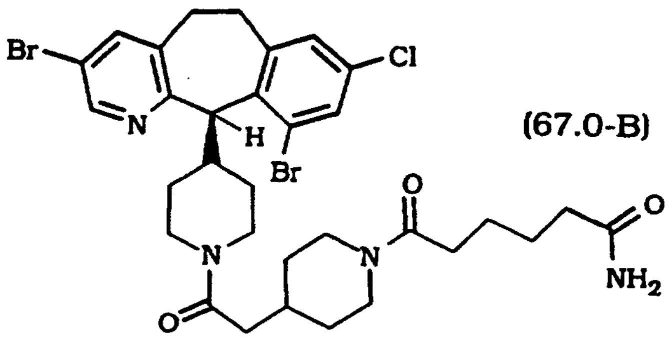

(67.0-B)

;

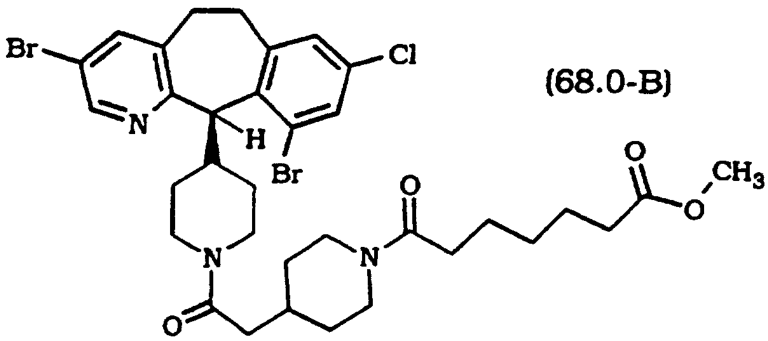

(68.0-B)

;

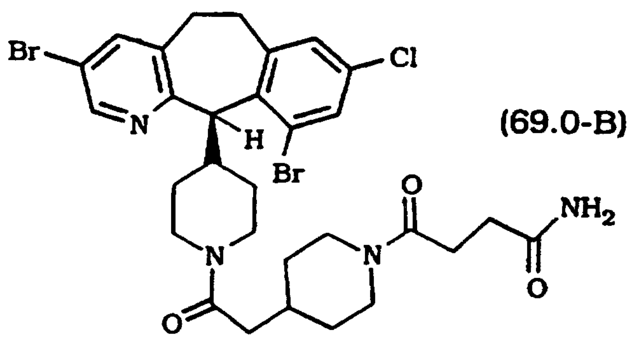

(69.0-B)

;

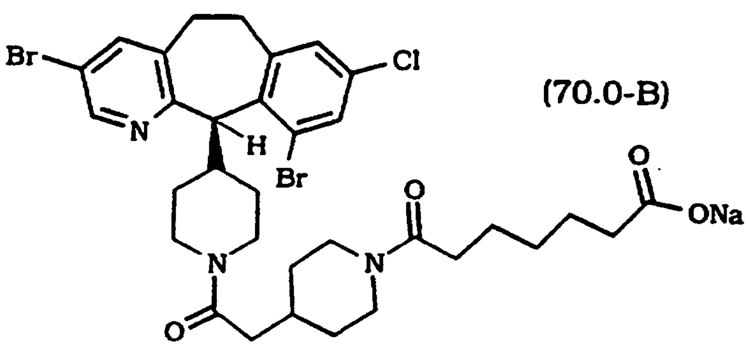

(70.0-B)

;

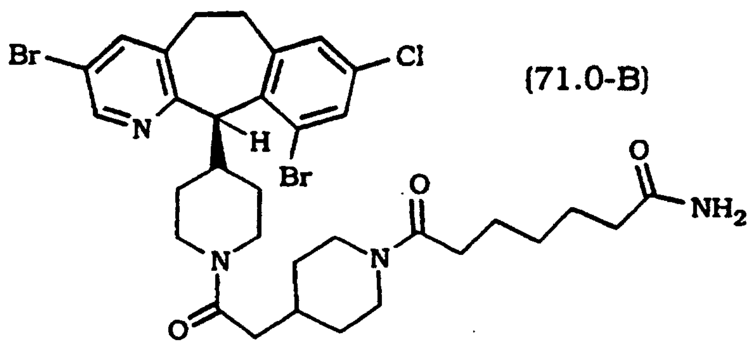

(71.0-B)

;

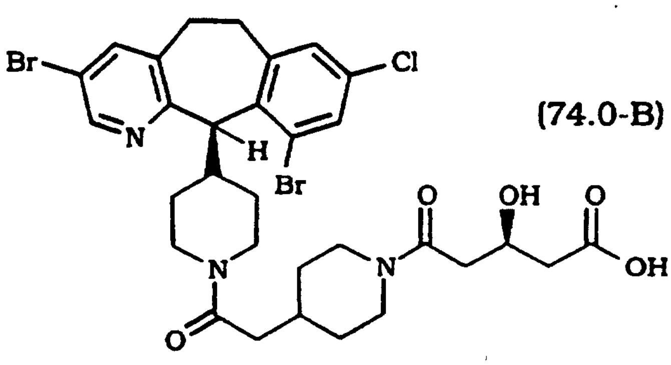

(74.0-B)

;

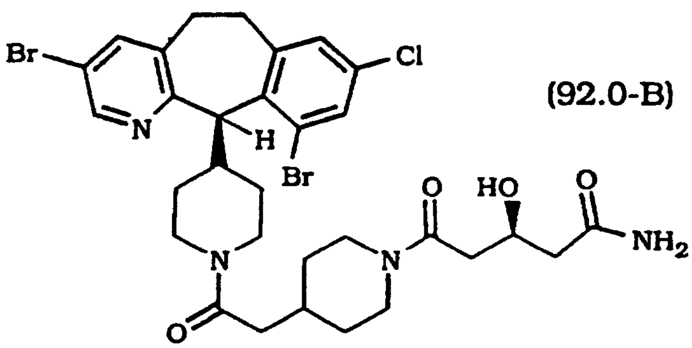

(92.0-B)

;

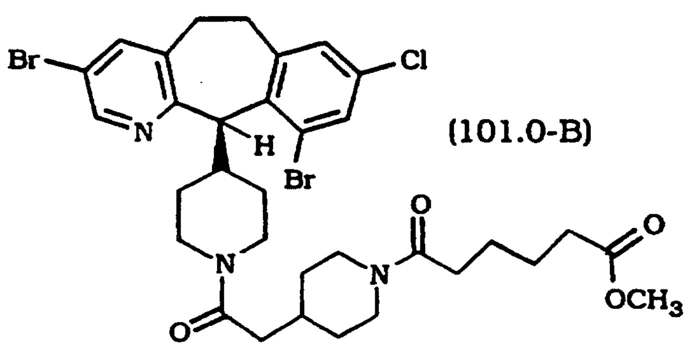

(101.0-B)

;

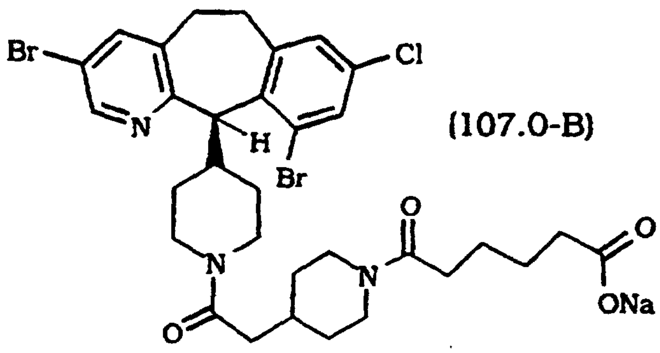

(107.0-B)

;

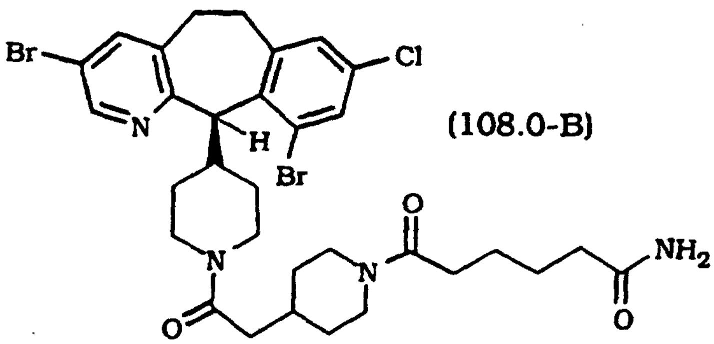

(108.0-B)

;

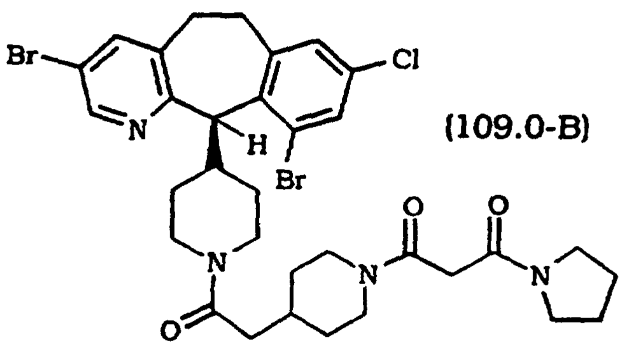

(109.0-B)

;

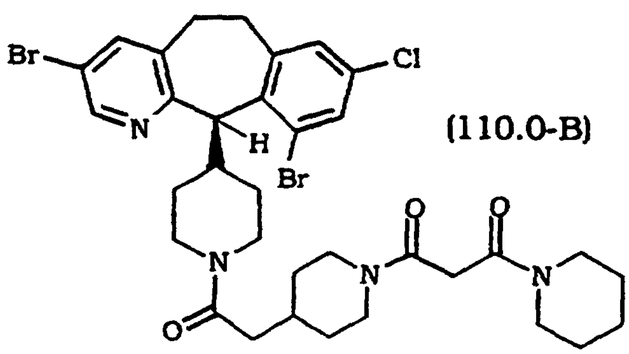

(110.0-B)

;

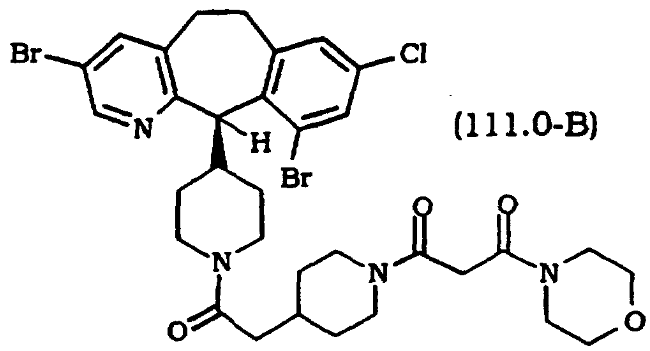

(111.0-B)

;

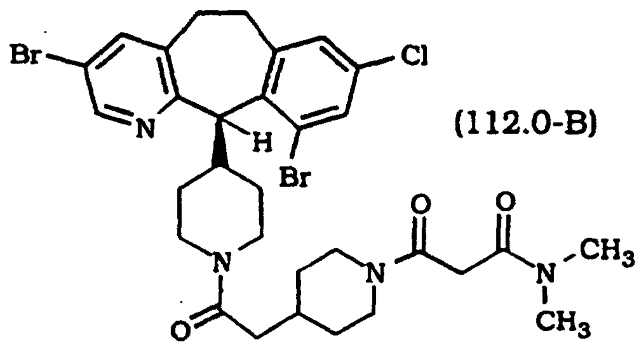

(112.0-B)

and

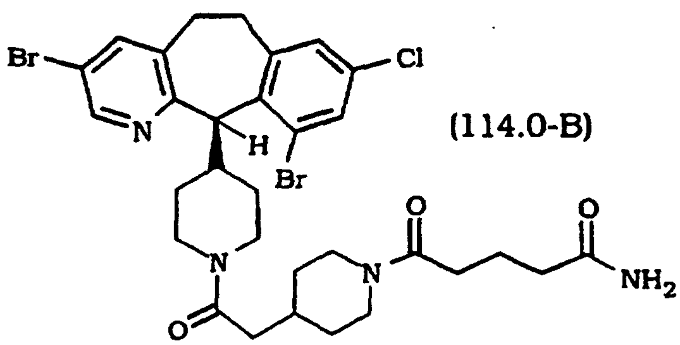

(114.0-B)

.

Compounds of this invention also include:

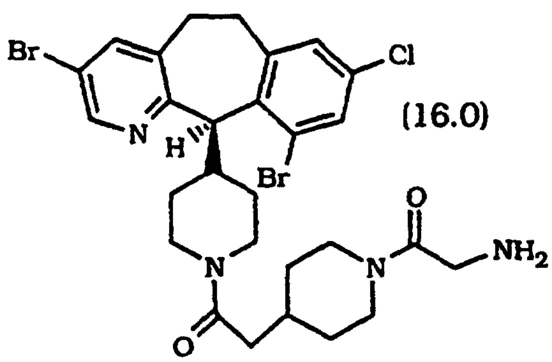

(16.0)

;

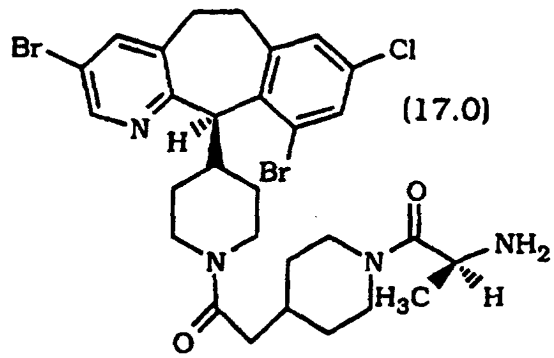

(17.0)

and

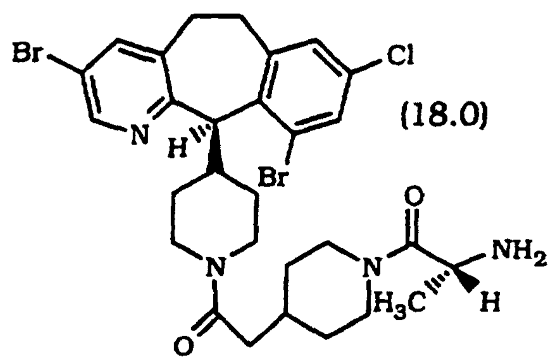

(18.0)

or pharmaceutically acceptable salts or solvates thereof.

The compounds of this invention also include the 1-N-oxides--i.e, for example, compounds of the the formula:

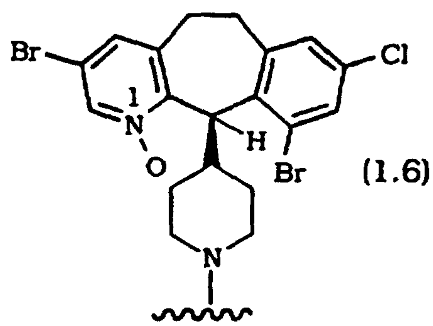

(1.6)

wherein ∿∿∿ represents the remainder of the compound, or pharmaceutically acceptable salts or solvates thereof.

Optical rotation of the compounds ((+)- or (-)-) are measured in methanol or ethanol at 25°C.

This invention includes the above compounds in the amorphous state or in the cyrstalline state.

Lines drawn into the ring systems indicate that the indicated bond may be attached to any of the substitutable ring carbon atoms.

Certain compounds of the present invention may exist in different isomeric forms (e.g., enantiomers or diastereoisomers) including atropisomers (i.e., compounds wherein the 7-membered ring is in a fixed conformation such that the 11-carbon atom is positioned above or below the plane of the fused benzene rings due to the presence of a 10-bromo substituent). The invention contemplates all such isomers both in pure form and in admixture, including racemic mixtures. Enol forms are also included.

Certain tricyclic compounds will be acidic in nature, e.g. those compounds which possess a carboxyl or phenolic hydroxyl group. These compounds may form pharmaceutically acceptable salts. Examples of such salts may

include sodium, potassium, calcium, aluminum, gold, silver and lithium salts. For example, compounds having the $-OR^{23}$ group wherein $R^{23}$ is H can form a sodium or lithium salt--i.e., a compound with a -ONa or -OLi group. Also contemplated are salts formed with pharmaceutically acceptable amines such as ammonia, alkyl amines, hydroxyalkylamines, N-methylglucamine and the like.

Certain basic tricyclic compounds also form pharmaceutically acceptable salts, e.g., acid addition salts. For example, the pyrido-nitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all add and base salts are considered equivalent to the free forms of the corresponding compounds for purposes of the invention.

Compounds of the invention may be prepared according to the procedures described in WO 95/10516 published April 20. 1995. Application Serial No. 08/410.187 filed March 24, 1995, Application Serial No. 08/577,951 filed December 22, 1995 (now abandoned). Application Serial No. 08/615,760 filed March 13. 1996 (now abandoned), WO 97/23478 published July 3, 1997 which discloses the subject matter of Serial No. 08/577.951 and 08/615,760, Application Serial No. 08/710,225 filed September 13, 1996, and Application Serial No. 08/877,453 filed June 17, 1997; and according to the procedures described below.

Compounds of the invention can be prepared by reacting a compound of the formula:

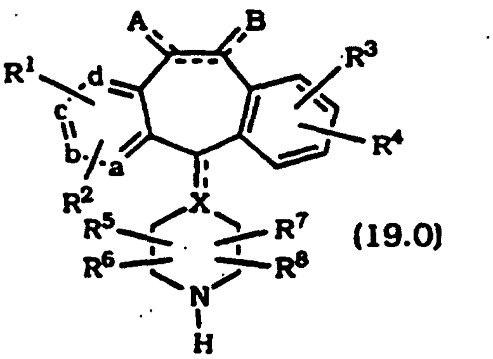

(19.0)

wherein all substituents are as defined for Formula 1.0, with the appropriate protected piperidinyl acetic add (e.g., 1-N-t-butoxycarbonylpiperidinyl acetic acid together with DEC/HOBT/NMM in DMF at about 25°C for about 18 hours to produce a compound of the formula:

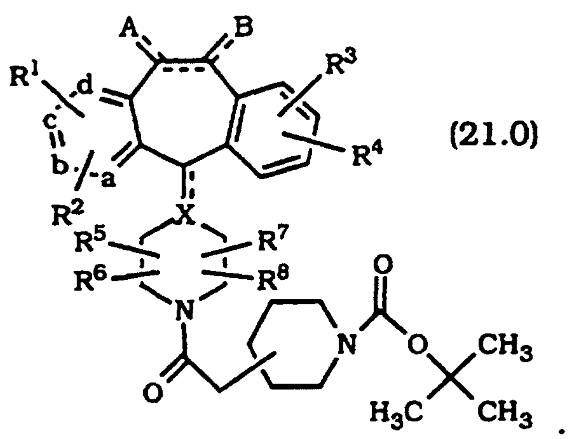

(21.0)

The compound of Formula 21.0 is then reacted either with TFA or 10% sulfuric acid in dioxane and methanol followed by NaOH to produce the compound of Formula 20.0

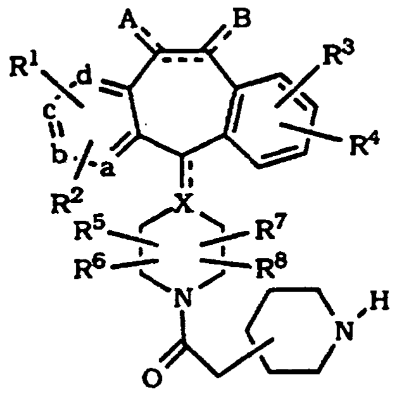

(20.0)

.

For example, the compound of formula

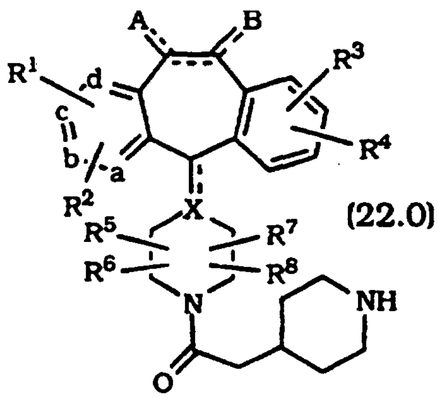

(22.0)

can be prepared by reaction of a compound of Formula 19.0 with 1-N-t-butoxy-carbonylpiperidinyl-4-acetic acid as described above.

For example, compounds of Formula 22.0 include the compounds:

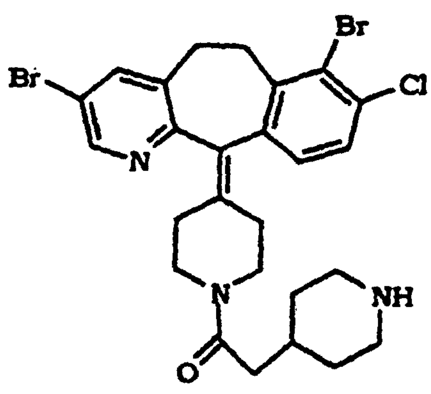

;

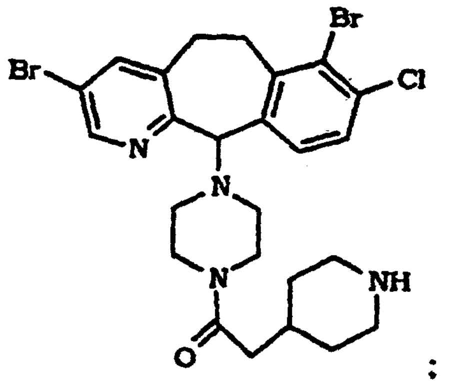

;

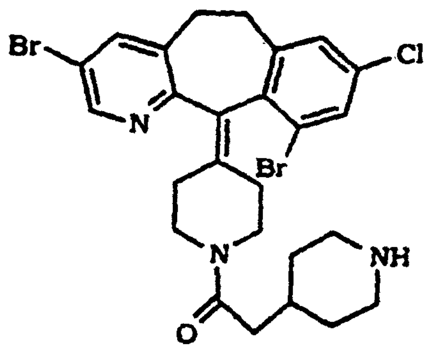

;

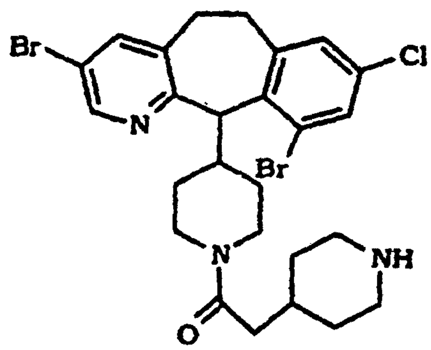

;

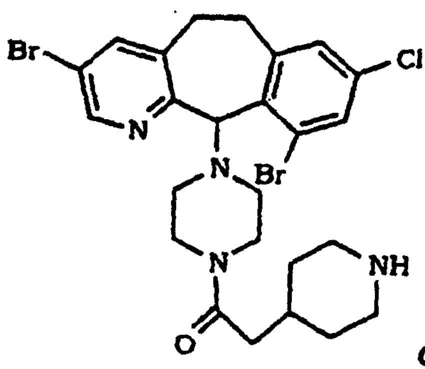

and

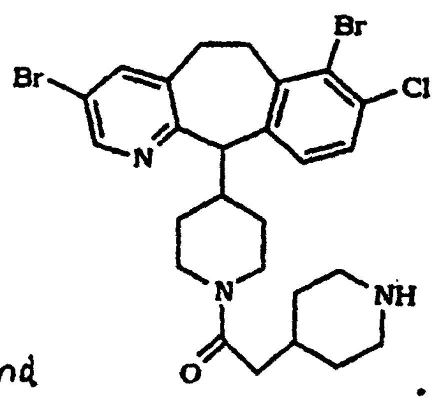

.

The preparation of these compounds are described in Preparative Examples 4, 6, 7, 8, 9, and 10, respectively. below.

The compounds of the invention can be prepared by reacting a compound of the formula:

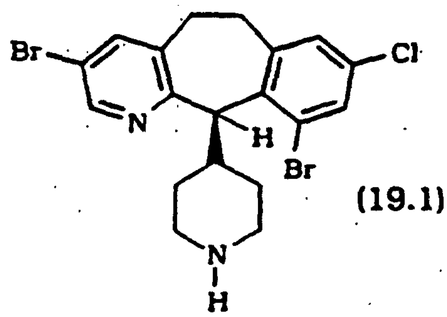

(19.1)

with the appropriate protected piperidinyl acetic acid (e.g., 1-N-t-butoxycarbonylpiperidinyl acetic acid together with DEC/HOBT/NMM in DMF at about 25°C for about 18 hours to produce a compound of the formula:

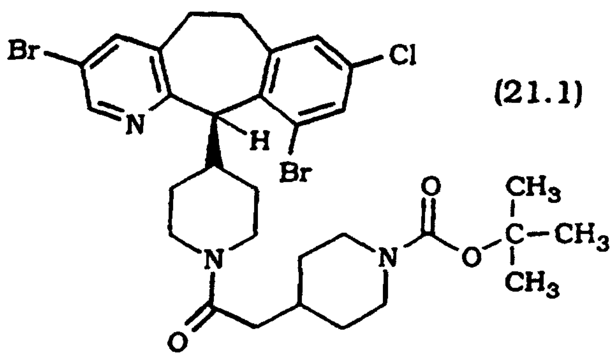

(21.1)

The compound of Formula 21.1 is then reacted either with TFA or 10% sulfuric acid in dioxane and methanol followed by NaOH to produce the compound of Formula 22.1

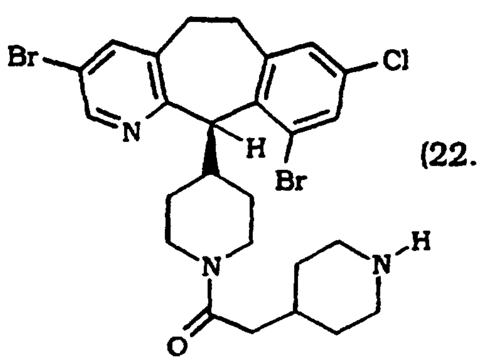

(22.1)

The amide compounds of this invention, represented by Formula 1.7

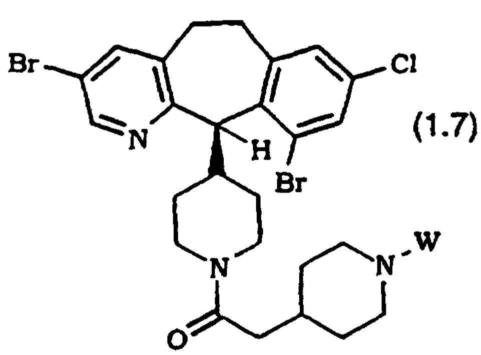

(1.7)

can be prepared by reacting the compound of Formula 22.1 with the appropriate carboxylic acid in the presence of a coupling agent such as DEC and HOBT in dimethylformamide. Alternatively, the compound of Formula 22.1 can be reacted with an acid chloride or anhydride in a solvent such as pyridine.

The W group on Formula 1.7 can contain functionality that can be converted to other functionality by methods, such as hydrolysis, that are well known in the art. For example, the compound of Formula 16.0-B, can be converted to the compound of Formula 74-B, and the compound of Formula 35.0-B to the compound of Formula 52.0-B by treatment with methanolic potassium hydroxide followed by acid. Also, compounds of Formulas 86.0-B and 89.0-B can be converted to compounds of Formulas 88.0-B and 90.0-B, respectively, by treatment with acids such as trifluoroacetic acid or dioxane saturated with HCl gas.

Compounds having an 1-N-O group:

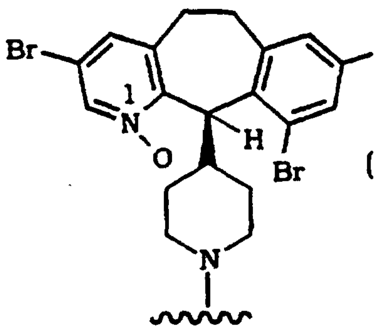

(1.6)

can be prepared from the corresponding pyridyl compounds:

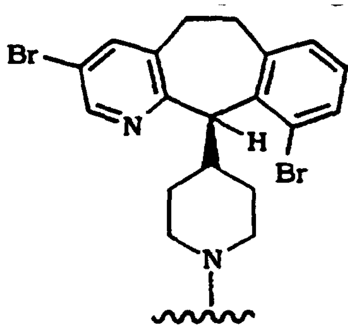

(1.8)

by oxidation with meta-chloroperoxybenzoic acid. This reaction is conducted in a suitable organic solvent, e.g., dichloromethane (usually anhydrous) or methylene chloride, at a suitable temperature, to produce the compounds of the invention having the N-O substituent at position 1 of Ring I of the tricyclic ring system.

Generally, the organic solvent solution of the starting tricyclic reactant is cooled to about 0°C before the m-chloroperoxybenzoic acid is added. The reaction is then allowed to warm to room temperature during the reaction period. The desired product can be recovered by standard separation means. For example, the reaction mixture can be washed with an aqueous solution of a suitable base, e.g., saturated sodium bicarbonate or NaOH (e.g., 1N NaOH), and then dried over anhydrous magnesium sulfate. The solution containing the product can be concentrated in vacuo. The product can be purified by standard means, e.g., by chromatography using silica gel (e.g., flash column chromatography).

Alternatively, N-O compounds can be made from intermediate:

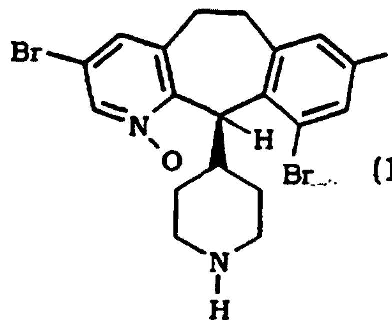

(19.1A)

by the above oxidation procedure with m-chloroperoxybenzoic acid and

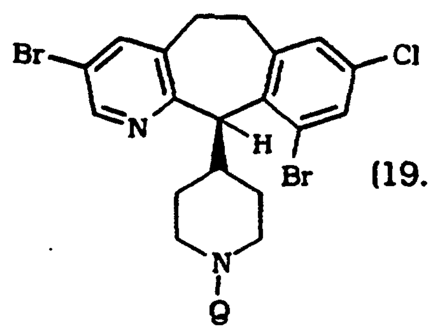

(19.1B)

wherein Q is a protecting group, e.g., BOC. After oxidation the protecting group is removed by techniques well known in the art. The N-O intermediate is then reacted further to produce the compounds of the invention.

Compounds of Formula 19.0 include the compound of Formula 19.1:

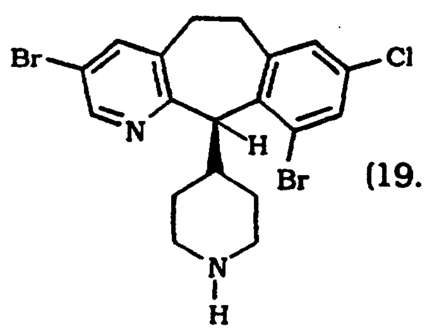

(19.1)

The compound of Formula 19.1 is prepared by methods known in the art, for example by methods disclosed in WO 95/10516, in U.S. 5,151,423 and those described below. The above intermediate compound can also be prepared by a procedure comprising the following steps:

(a) reacting an amide of the formula

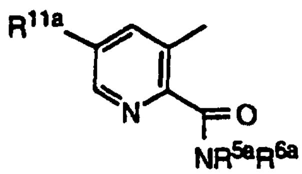

wherein $R^{11a}$ is Br, $R^{5a}$ is hydrogen and $R^{6a}$ is $C_1$-$C_6$ alkyl, aryl or heteroaryl; $R^{5a}$ is $C_1$-$C_6$ alkyl, aryl or heteroaryl and $R^{6a}$ is hydrogen; $R^{5a}$ and $R^{6a}$ are independently selected from the group consisting of $C_1$-$C_6$ alkyl and aryl: or $R^{5a}$ and $R^{6a}$, together with the nitrogen to which they are attached, form a ring comprising 4 to 6 carbon atoms

or comprising 3 to 5 carbon atoms and one hetero moiety selected from the group consisting of -O- and -$NR^{9a}$-, wherein $R^{9a}$ is H, $C_1$-$C_6$ alkyl or phenyl;

with a compound of the formula

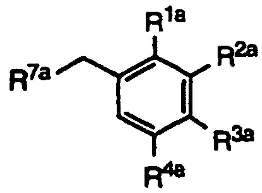

wherein $R^{1a}$, $R^{2a}$, $R^{3a}$ and $R^{4a}$ are independently selected from the group consisting of hydrogen and halo and $R^{7a}$ is Cl or Br, in the presence of a strong base to obtain a compound of the formula

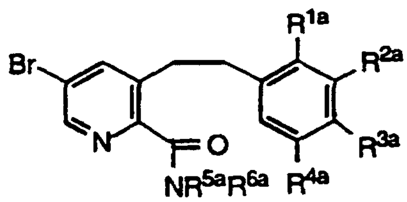

(b) reacting a compound of step (a) with

(i) $POCl_3$ to obtain a cyano compound of the formula

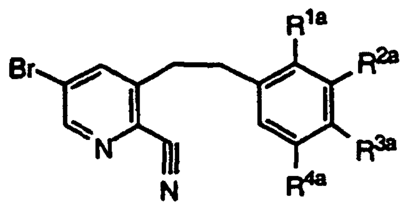

;

or

(ii) DIBALH to obtain an aldehyde of the formula

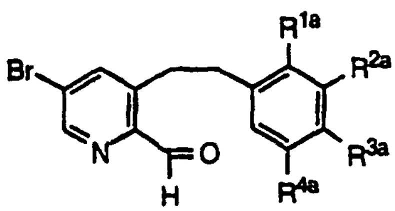

;

(c) reacting the cyano compound or the aldehyde with a piperidine derivative of the formula

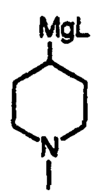

wherein L is a leaving group selected from the group consisting of Cl and Br, to obtain a ketone or an alcohol of

the formula below, respectively:

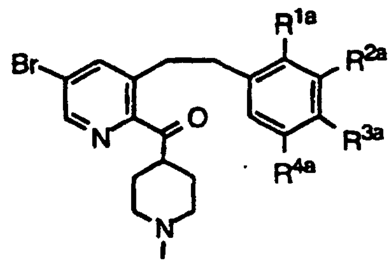

or

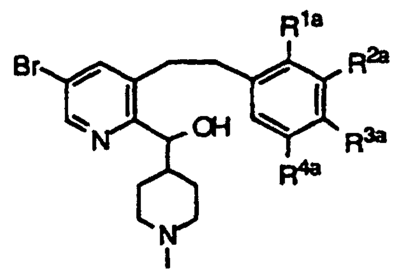

(d)(i) cyclizing the ketone with $CF_3SO_3H$ to obtain a compound of Formula 13.0a wherein the dotted line represents a double bond; or

(d)(ii) cyclizing the alcohol with polyphosphoric acid to obtain an Intermediate compound wherein the dotted line represents a single bond.

Methods for preparing the Intermediate compounds disclosed in WO 95/10516, U.S. 5,151,423 and described below employ a tricyclic ketone intermediate. Such intermediates of the formula

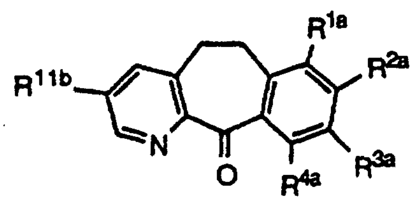

wherein $R^{11b}$, $R^{1a}$, $R^{2a}$, $R^{3a}$ and $R^{4a}$ are independently selected from the group consisting of hydrogen and halo, can be prepared by the following process comprising :

(a) reacting a compound of the formula

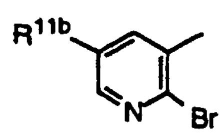

(i) with an amine of the formula $NHR^{5a}R^{6a}$, wherein $R^{5a}$ and $R^{6a}$ are as defined in the process above; in the presence of a palladium catalyst and carbon monoxide to obtain an amide of the formula:

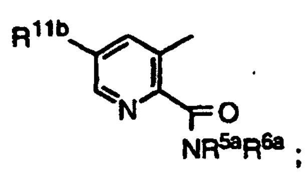

or

(ii) with an alcohol of the formula $R^{10a}OH$, wherein $R^{10a}$ is $C_1$-$C_6$ lower alkyl or $C_3$-$C_6$ cycloalkyl, in the presence of a palladium catalyst and carbon monoxide to obtain the ester of the formula

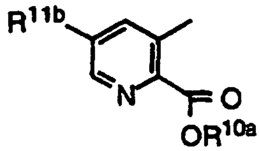

followed by reacting the ester with an amine of formula $NHR^{5a}R^{6a}$ to obtain the amide;

(b) reacting the amide with an iodo-substituted benzyl compound of the formula

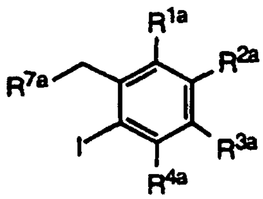

wherein $R^{1a}$, $R^{2a}$, $R^{3a}$, $R^{4a}$ and $R^{7a}$ are as defined above, in the presence of a strong base to obtain a compound of the formula

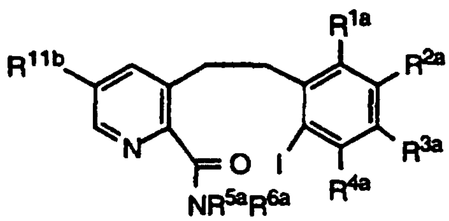

;

and

(c) cyclizing a compound of step (b) with a reagent of the formula $R^{8a}MgL$, wherein $R^{8a}$ is $C_1$-$C_8$ alkyl, aryl or heteroaryl and L is Br or Cl, provided that prior to cyclization, compounds wherein $R^{5a}$ or $R^{6a}$ is hydrogen are reacted with a suitable N-protecting group.

(+)-Isomers of compounds of Formula 19.2

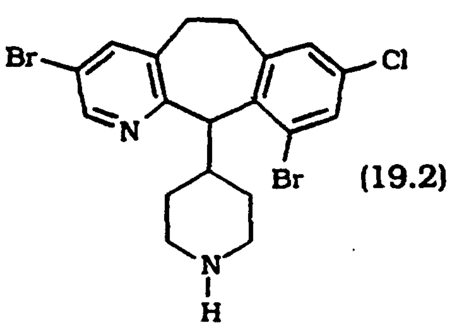

(19.2)

can be prepared with high enantioselectivity by using a process comprising enzyme catalyzed transesterification. Preferably, a racemic compound of Formula 19.3

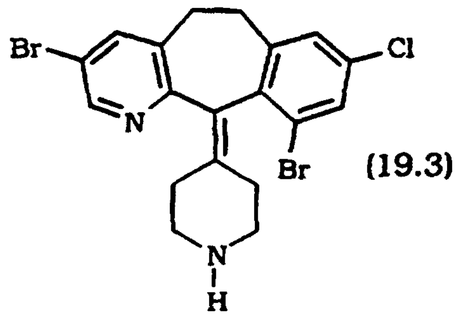

(19.3)

is reacted with an enzyme such as Toyobo LIP-300 and an acylating agent such as trifluoroethly isobutyrate; the resultant (+)-amide is then isolated from the (-)-enantiomeric amine by techniques well known in the art, and then the (+)-amide is hydrolyzed, for example by refluxing with an acid such as $H_2SO_4$, and the resulting compound is then reduced with DIBAL by techniques well known in the art to obtain the corresponding optically enriched (+)-isomer of Formula 19.2. Alternatively, a racemic compound of Formula 19.3, is first reduced to the corresponding racemic compound of Formula 19.2 and then treated with the enzyme (Toyobo LIP-300) and acylating agent as described above to obtain the (+)-amide, which is hydrolyzed to obtain the optically enriched (+)-isomer.

Those skilled in the art will appreciate that compounds of Formula 1.0 having other $R^1$, $R^2$, $R^3$ and $R^4$ substituents may be made by the above enzyme process.

To produce the compounds of Formula 1.0, wherein W is

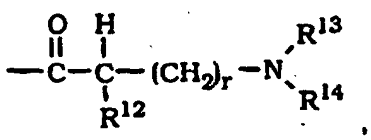

,

r is 0, and $R^{13}$ and $R^{14}$ are selected from H or $-C(O)OR^{16}$, the compounds of Formulas 20.0 or 22.0 are reacted with the appropriate protected amino acid:

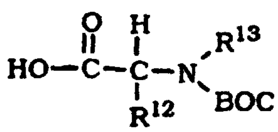

in the presence of DEC and HOBt in dimethylformamide to produce a compound of the formula:

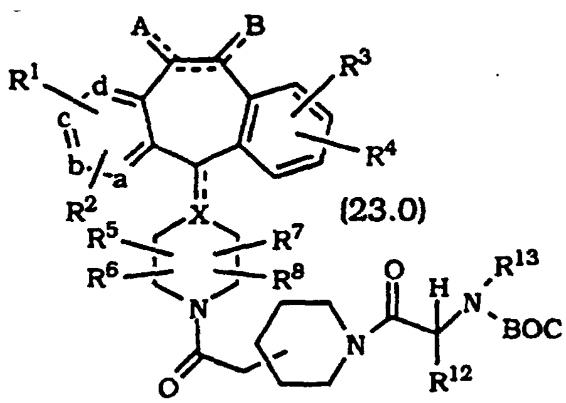

(23.0)

or

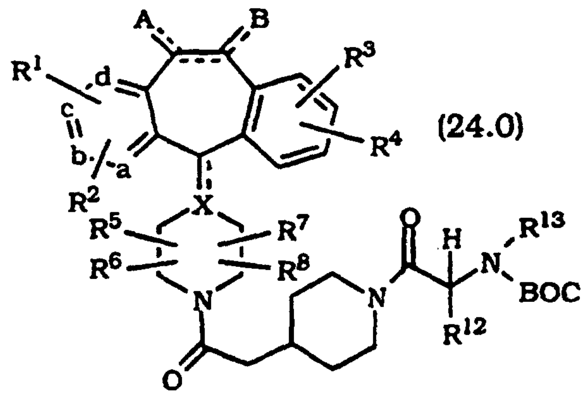

(24.0)

respectively.

Reaction of compounds of Formulas 23.0 or 24.0 with TFA in methylene chloride results in the deprotected compounds:

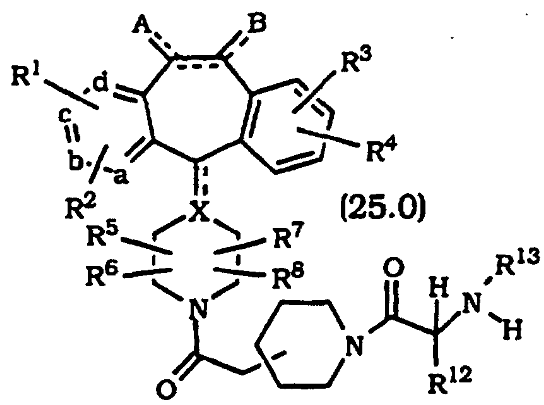

(25.0)

or

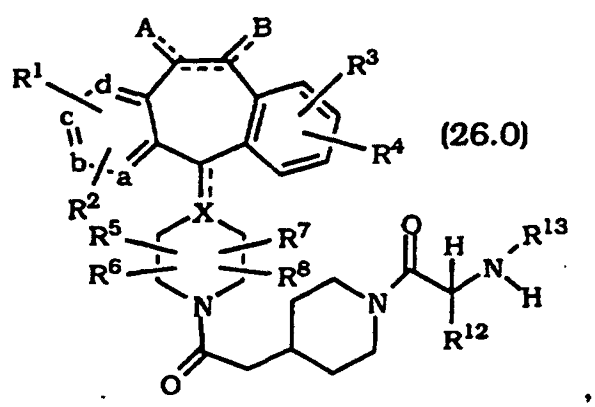

(26.0)

respectively.

Compounds of Formula 1.0 wherein W is

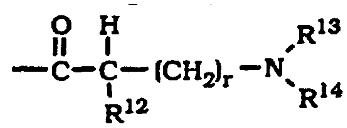

r is 0, $R^{12}$ is H, $R^{13}$ or $R^{14}$ is H, and the remaining $R^{13}$ or $R^{14}$ is -C(O)OR$^{16}$ can be prepared by reacting a compound of Formula 1.0 wherein W is

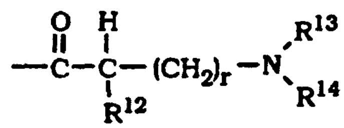

r is 0, $R^{12}$ is H, and $R^{13}$ and $R^{14}$ are both H, with the appropriate chloroformate

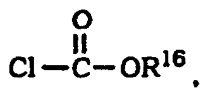

TEA and $CH_2Cl_2$.

Compounds of Formulas 25.0 or 26.0 wherein $R^{13}$ is selected from -$SO_2R^{17}$ or -C(O)$R^{18}$ can be prepared by reacting a compound of Formula 25.0 or 26.0 with a suitable sulfonyl chloride ($R^{17}SO_2Cl$) or a suitable acyl chloride ($R^{18}$C(O)Cl) with TEA in a suitable organic solvent (e.g., $CH_2Cl_2$).

Compounds of Formula 1.0 wherein W is

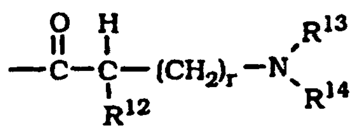

r is 1 or 2 and $R^{12}$ is H can be prepared by reacting a compound of Formula 20.0 or 22.0 with the appropriately substitued carboxylic acid and, for example DEC, HOBT and N-methylmorpholine, or by reacting a compound of Formula 20.0 or 22.0 with the appropriately substituted acid chloride.

For example, a compound of Formula 20.0 or 22.0 can be reacted with

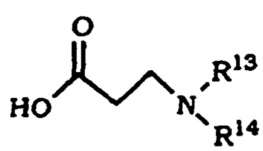

from propionic acid, wherein $R^{13}$ and $R^{14}$ are, for example, alkyl (e.g., methyl). Where the amino carboxylic acid is not commercially available, it can be prepared by reaction of ethyl acrylate with the appropriate amino compound (as described by Ahn, K.H. et al., Tetrahedron Letters , 35. 1875-1878 (1994)) with subsequent hydrolysis of the ester to the desired aminocarboxylic acid.

Also, for example, a compound of Formula 20.0 or 22.0 can be reacted with

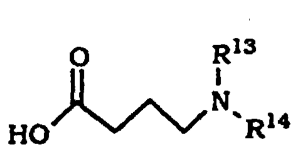

from butyric acid, wherein $R^{13}$ and $R^{14}$ are, for example, alkyl (e.g., methyl). Where the amino carboxylic acid is not

commercially available, the appropriate acid chloride can be prepared in a manner similar to that described by Goel, O.P. et al., Synthesis, p. 538 (1973). The acid chloride is then reacted with a compound of Formula 20.0 or 22.0 to give the compound

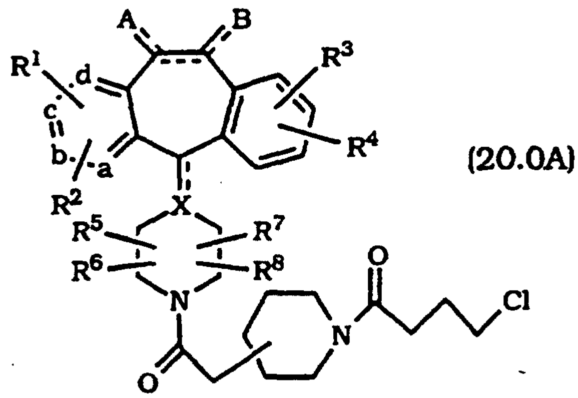

(20.0A)

or

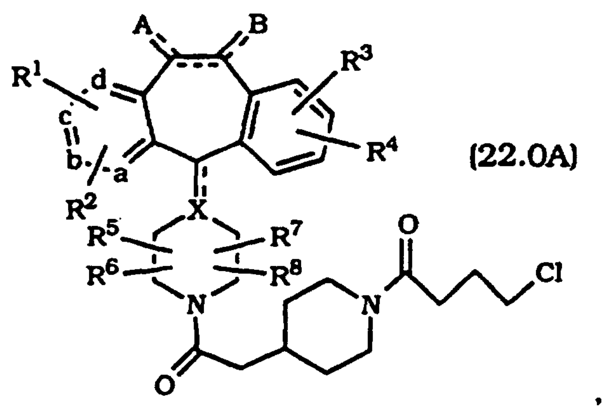

(22.0A)

respectively. The chloro atom can then be displaced with the appropriate amine to give the desired compound.

Where either $R^{13}$ or $R^{14}$ is H, then the starting material would be a protected amino carboxylic acid

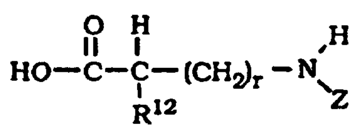

wherein Z is an appropriater protecting group (e.g., BOC, CBZ (carbonylbenzyloxy) or TFA). Coupling this protected amino carboxylic acid with a compound of Formula 20.0 or 22.0 would then give the amino protected intermediate

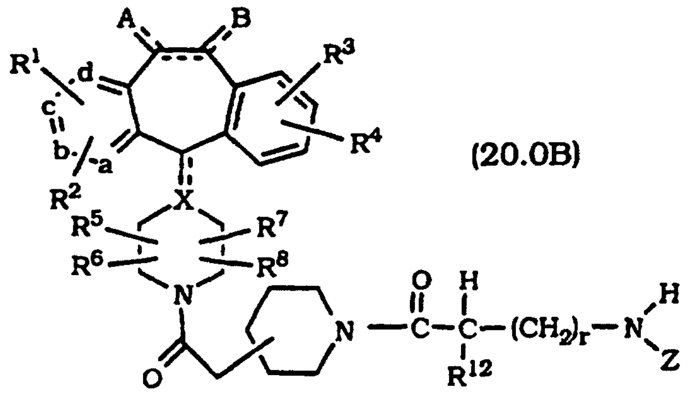

(20.0B)

or

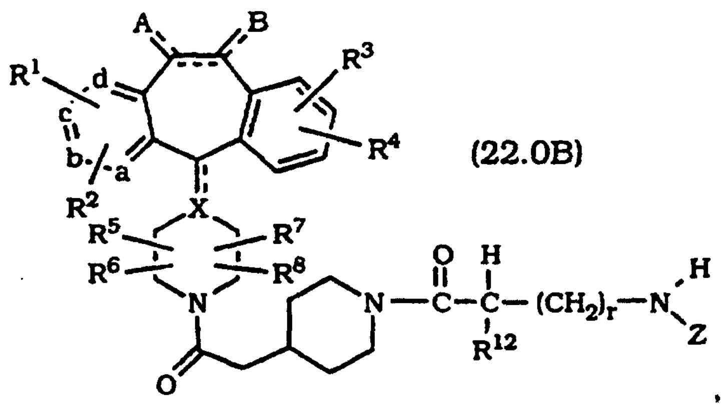

(22.0B)

,

respectively. The amino protected intermediate (20.0B or 22.0B) would then be alkylated, and then the protecting group removed, using standard procedures known in the art.

Compounds of Formula 1.0 wherein W is

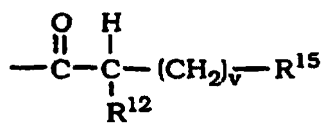

v is 0, and $R^{12}$ is H can be prepared by reacting a compound of Formula 20.0 or 22.0 with chloroacetylchloride, TEA and $CH_2Cl_2$ to produce a compound of the formula:

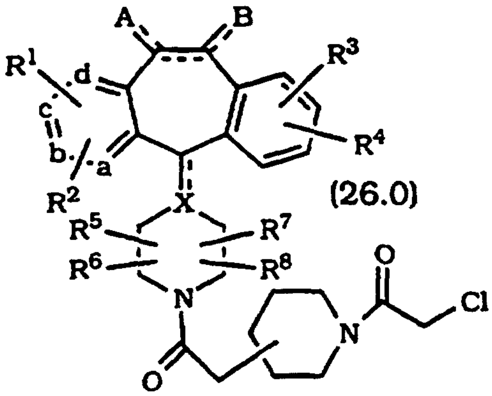

(26.0)

or

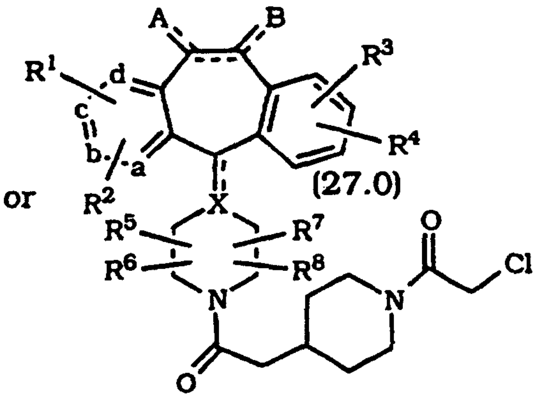

(27.0)

.

The chloro atom in the $-C(O)CH_2Cl$ group in the compound of Formula 26.0 or 27.0 is then displaced with an appropriate nucleophile, $R^{15}$, using a suitable base, e.g., sodium carbonate, and optionally, a suitable suitable solvent (e.g., DMF).

Compounds of Formula 1.0 wherein W is

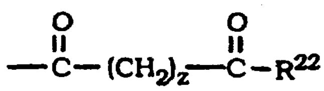

z is 0, and $R^{22}$ is

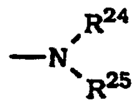

can be prepared from compounds of Formula 20.0 or 22.0 by reaction with oxallyl chloride and an excess of the amine

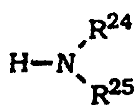

Compounds of Formula 1.0 wherein W is

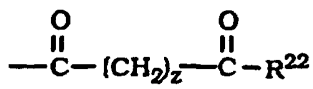

z is 1, 2, 3, 4 or 5 and $R^{22}$ is $-OR^{23}$, and $R^{23}$ is, for example, alkyl, can be prepared by reaction of a compound of Formula 20.0 or 22.0 with the appropriate substituted dicarboxylic acid which is protected as a mono ester with an appropriate alkyl or aryl group. The corresponding acids (i.e., $R^{23}$ is H) can be obtained by base hydrolysis (e.g., NaOH) of the ester. The compounds, wherein $R^{22}$ is $-NR^{24}R^{25}$, can be prepared by reacting the appropriately substituted amine with the carboxylic acid generated above using DEC, HOBT and NMM. For example, for compounds wherein z is 3 a glutarate

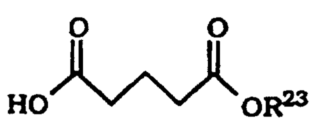

(wherein $R^{23}$ is alkyl, e.g., methyl) can be used, and for compounds wherein z is 2 a succinate

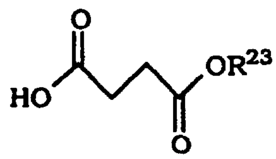

(wherein $R^{23}$ is alkyl, e.g., methyl) can be used, and for compounds wherein z is 1 a malonate

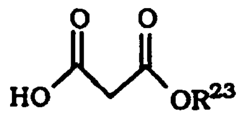

(wherein $R^{23}$ is alkyl, e.g., ethyl) can be used.

Reaction Scheme 1 illustrates the preparation of compounds of this invention.

SCHEME 1

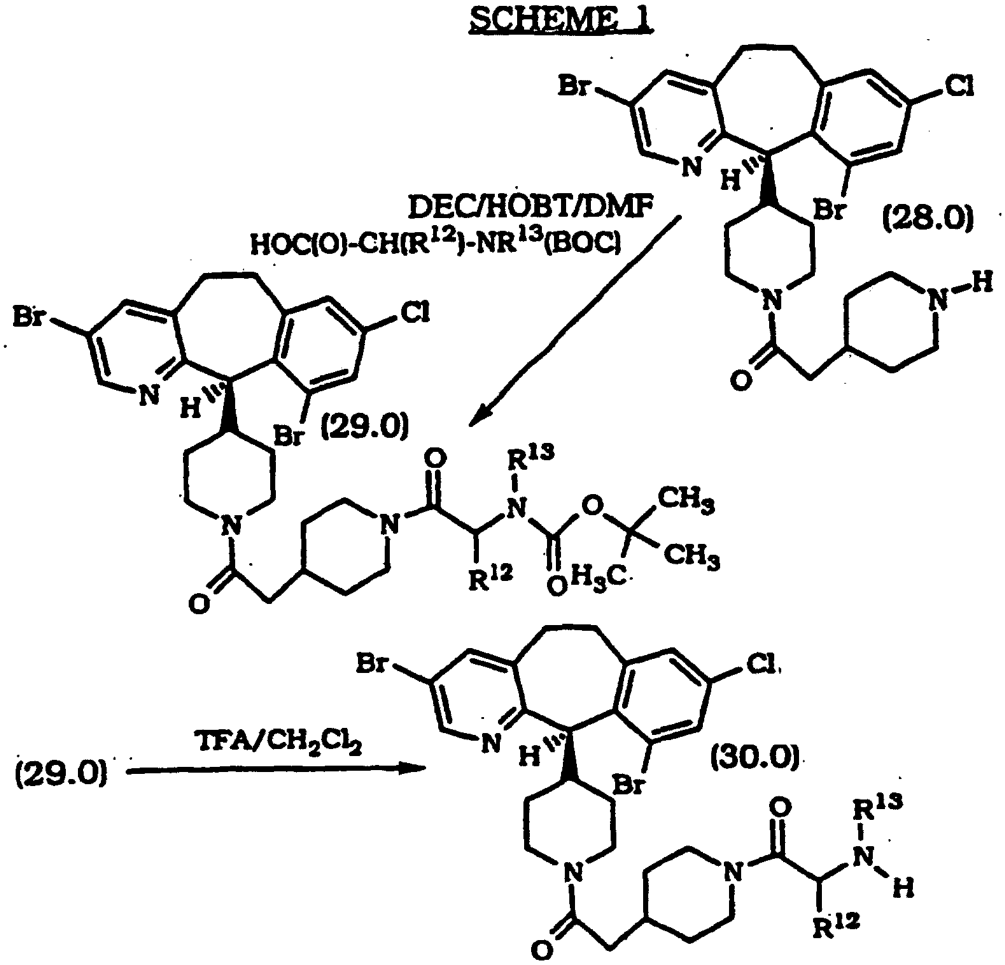


Compounds useful in this invention are exemplified by the following examples. Examples marked with an asterisk (*) are not within the scope of the invention, and are provide by way of illustrating analogous procedures by which compounds of the invention may be made.

PREPARATIVE EXAMPLE 1

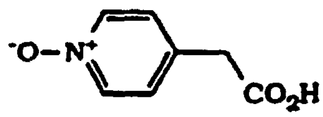

Step A:

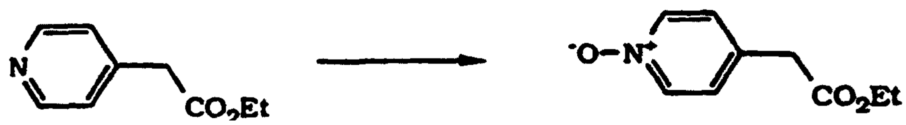

Combine 10 g (60.5 mmol) of ethyl 4-pyridylacetate and 120 mL of dry $CH_2Cl_2$ at -20°C, add 10.45 g (60.5 mmol) of MCPBA and stir at -20°C for 1 hour and then at 25°C for 67 hours. Add an additional 3.48 g (20.2 mmoles) of MCPBA and stir at 25°C for 24 hours. Dilute with $CH_2Cl_2$ and wash with saturated $NaHCO_3$ (aqueous) and then water. Dry over $MgSO_4$, concentrate *in vacuo* to a residue, and chromatograph (silica gel, 2%-5.5% (10% $NH_4OH$ in MeOH)/$CH_2Cl_2$)to give 8.12 g of the product compound. Mass Spec.: $MH^+$ = 182.15

Step B:

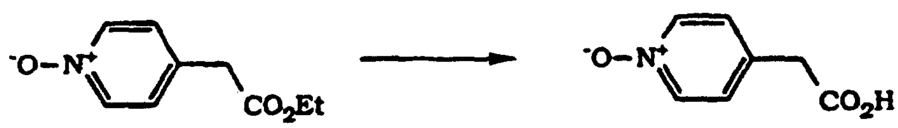

Combine 3.5 g (19.3 mmol) of the product of Step A, 17.5 mL of EtOH and 96.6 mL of 10% NaOH (aqueous) and heat the mixture at 67°C for 2 hours. Add 2 N HCl (aqueous) to adjust to pH = 2.37 and concentrate *in vacuo* to a residue. Add 200 mL of dry EtOH, filter through celite® and wash the filter cake with dry EtOH (2X50 ml). Concentrate the combined filtrates *in vacuo* to give 2.43 g of the title compound.

PREPARATIVE EXAMPLE 2

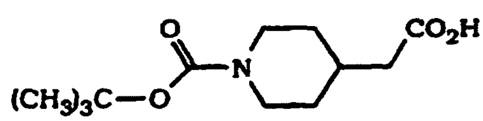

The title compound is prepared via the process disclosed in PCT International Publication No. WO95/10516.

PREPARATIVE EXAMPLE 3*

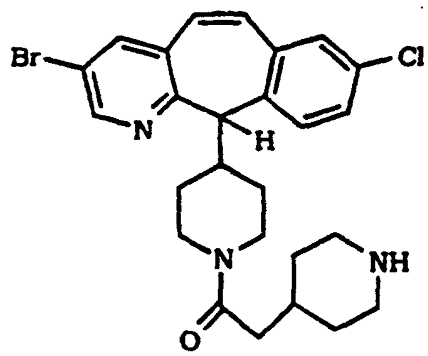

Step A:

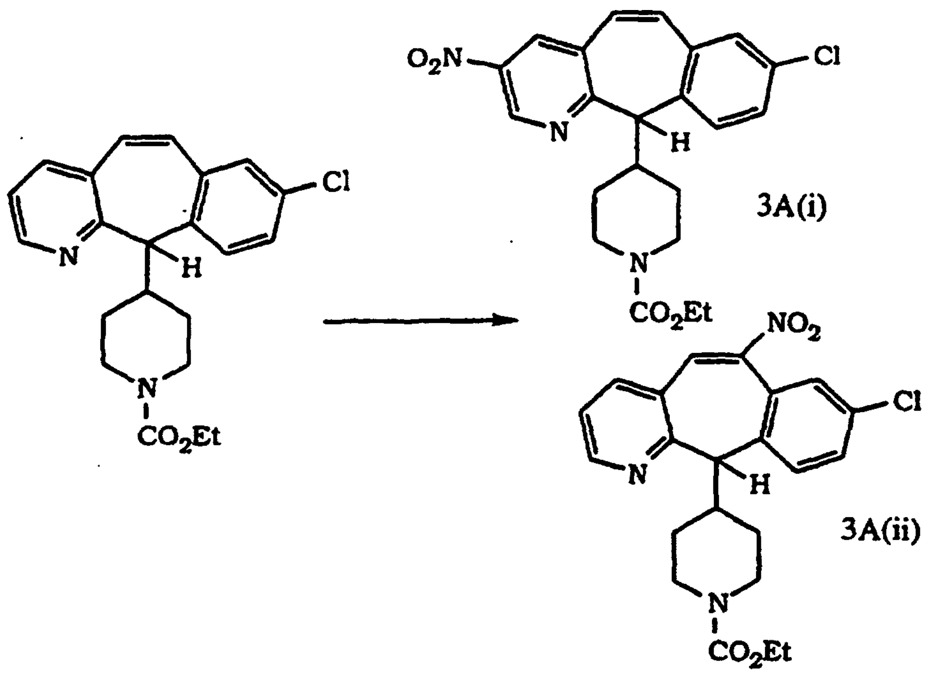

3A(i)

3A(ii)

Combine 14.95 g (39 mmol) of 8-chloro-11-(1-ethoxycarbonyl-4-piperidinyl)-11H-benzo[5,6]cyclohepta[1,2-b] pyridine and 150 mL of $CH_2Cl_2$, then add 13.07 g (42.9 mmol) of $(nBu)_4NNO_3$ and cool the mixture to 0°C. Slowly add (dropwise) a solution of 6.09 mL (42.9 mmol) of TFAA in 20 mL of $CH_2Cl_2$ over 1.5 hours. Keep the mixture at 0°C overnight, then wash successively with saturated $NaHCO_3$ (aqueous), water and brine. Dry the organic solution over $Na_2SO_4$, concentrate *in vacuo* to a residue and chromatograph the residue (silica gel, EtOAc/hexane gradient) to give 4.32 g and 1.90 g of the two product compounds 3A(i) and 3A(ii), respectively.

Mass Spec. for compound 3A(i): $MH^+$ = 428.2;

Mass Spec. for compound 3A(ii): $MH^+$ = 428.3.

Step B:

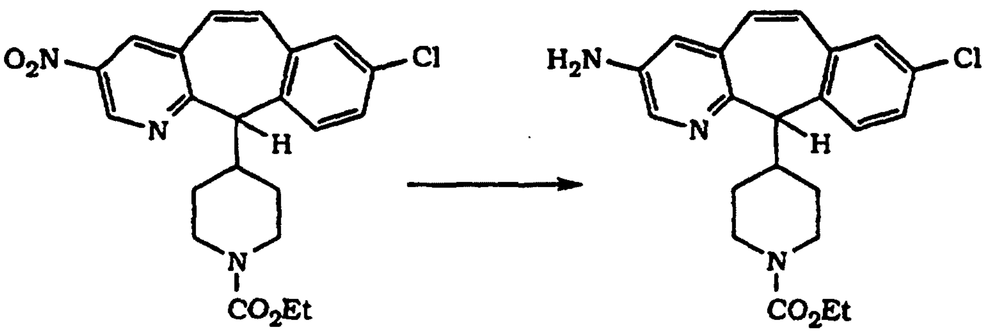

Combine 22.0 g (51.4 mmol) of the product 3A(i) from Step A, 150 mL of 85% EtOH (aqueous), 25.85 g (0.463 mole) of Fe powder and 2.42 g (21.8 mmol) of $CaCl_2$, and heat at reflux overnight. Add 12.4 g (0.222 mole) of Fe powder and 1.2 g (10.8 mmol) of $CaCl_2$ and heat at reflux for 2 hours. Add another 12.4 g (0.222 mole) of Fe powder and 1.2 g (10.8 mmol) of $CaCl_2$ and heat at reflux for 2 hours more. Filter the hot mixture through celite®, wash the celite® with 50 mL of hot EtOH and concentrate the filtrate *in vacuo* to a residue. Add 100 mL of anhydrous EtOH, concentrate to a residue and chromatograph the residue (silica gel, $MeOH/CH_2Cl_2$ gradient) to give 16.47 g of the product compound.

Step C:

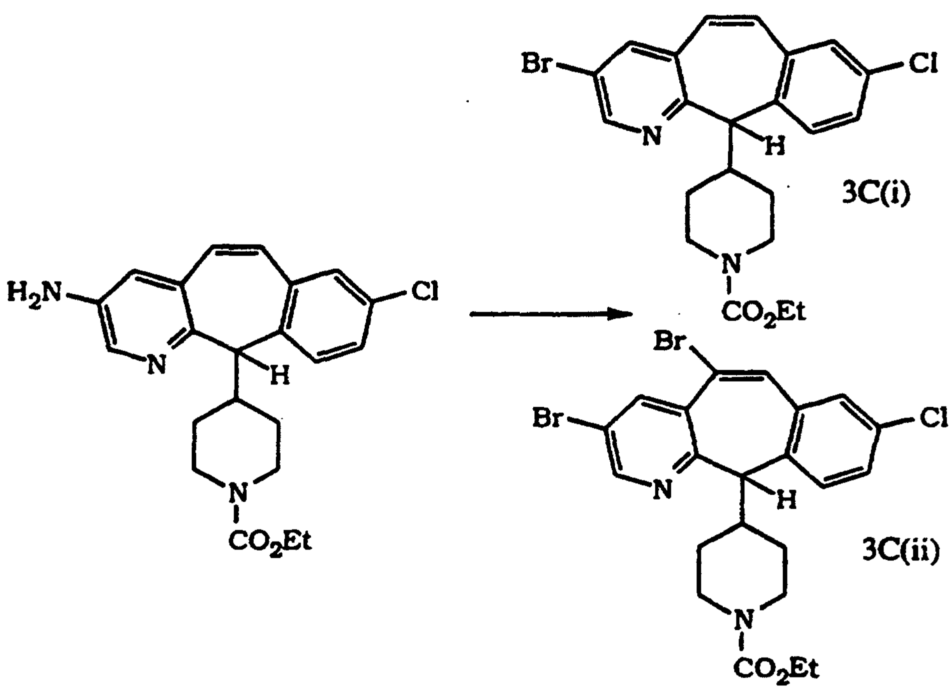

3C(i)

3C(ii)

Combine 16.47 g (41.4 mmol) of the product from Step B, and 150 mL of 48% HBr (aqueous) and cool to -3°C. Slowly add (dropwise) 18 mL of bromine, then slowly add (dropwise) a solution of 8.55 g (0.124 mole) of $NaNO_2$ in 85 mL of water. Stir for 45 minutes at -3° to 0°C, then adjust to pH = 10 by adding 50% NaOH (aqueous). Extract with EtOAc, wash the extracts with brine and dry the extracts over $Na_2SO_4$. Concentrate to a residue and chromatograph (silica gel, EtOAc/hexane gradient) to give 10.6 g and 3.28 g of the two product compounds 3C(i) and 3C(ii),

respectively.

Mass Spec. for compound 3C(i): $MH^+$ = 461.2;

Mass Spec. for compound 3C(ii): $MH^+$ = 539.

Step D:

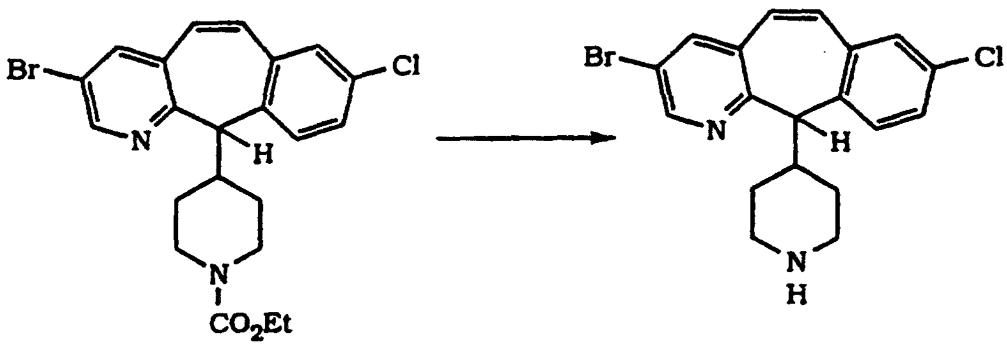

Hydrolyze the product 3C(i) of Step C by dissolving in concentrated HCl and heating to about 100°C for @ 16 hours. Cool the mixture, the neutralize with 1 M NaOH (aqueous). Extract with $CH_2Cl_2$, dry the extracts over $MgSO_4$, filter and concentrate *in vacuo* to the title compound.

Mass Spec.: $MH^+$ = 466.9.

Step E:

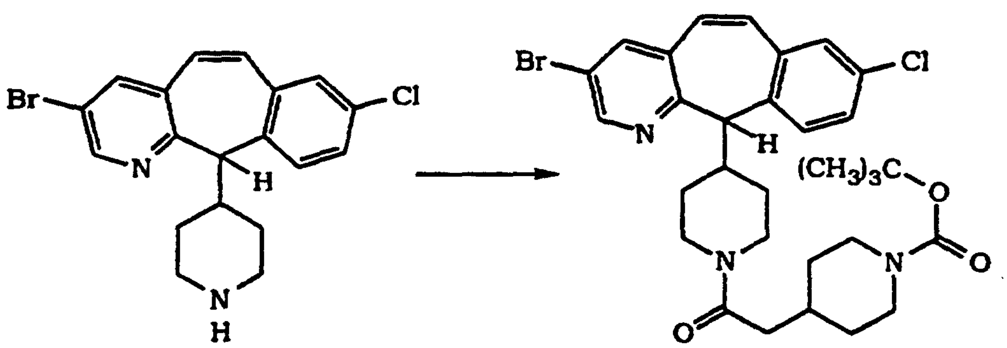

Dissolve 1.160 g (2.98 mmol) of the title compound from Step D in 20 mL of DMF, stir at room temperature, and add 0.3914 g (3.87 mmol) of 4-methyl-morpholine, 0.7418 g (3.87 mmol) of DEC, 0.5229 g (3.87 mmol) of HOBT, and 0.8795 g (3.87 mmol) of 1-N-t-butoxycarbonyl-piperidinyl-4-acetic acid. Stir the mixture at room temperature for 2 days, then concentrate *in vacuo* to s residue and partition the residue between $CH_2Cl_2$ and water. Wash the organic phase successively with saturated $NaHCO_3$ (aqueous), 10% $NaH_2PO_4$ (aqueous) and brine. Dry the organic phase over $MgSO_4$, filter and concentrate *in vacuo* to a residue. Chromatograph the residue (silica gel, 2% MeOH/ $CH_2Cl_2$ + $NH_3$) to give 1.72 g of the product. m.p. = 94.0-94.5°C, Mass Spec.: $MH^+$ = 616.3,

| | | | | |
|---|---|---|---|---|
| elemental analysis | calculated - | C. 60.54; | H, 6.06; | N, 6.83 |
| | found - | C, 59.93; | H, 6.62; | N, 7.45. |

Step F:

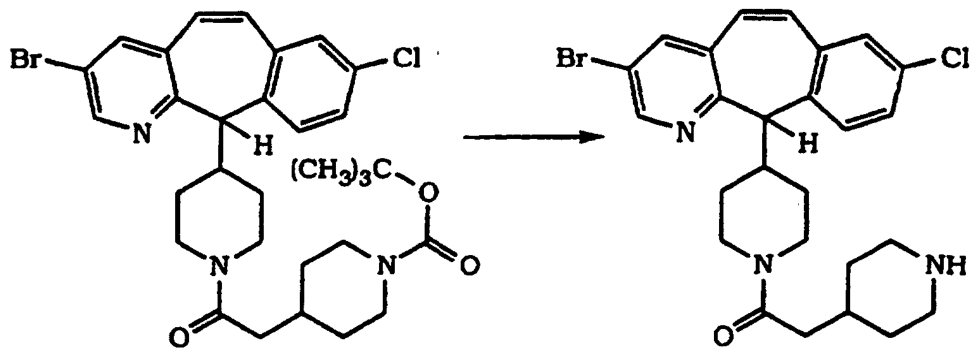

Combine 1.67 g (2.7 mmol) of the product of Step E and 20 mL of $CH_2Cl_2$ and stir at 0°C. Add 20 mL of TFA, stir the mixture for 2 hours, then basify the mixture with 1 N NaOH (aqueous). Extract with $CH_2Cl_2$, dry the organic phase over $MgSO_4$, filter and concentrate *in vacuo* to give 1.16 g of the product. m.p. = 140.2-140.8°C. Mass Spec.: $MH^+$ = 516.2.

PREPARATIVE EXAMPLE 4

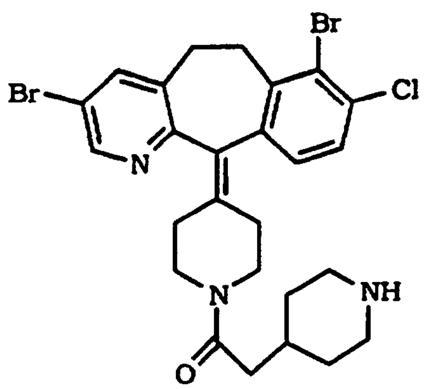

Step A:

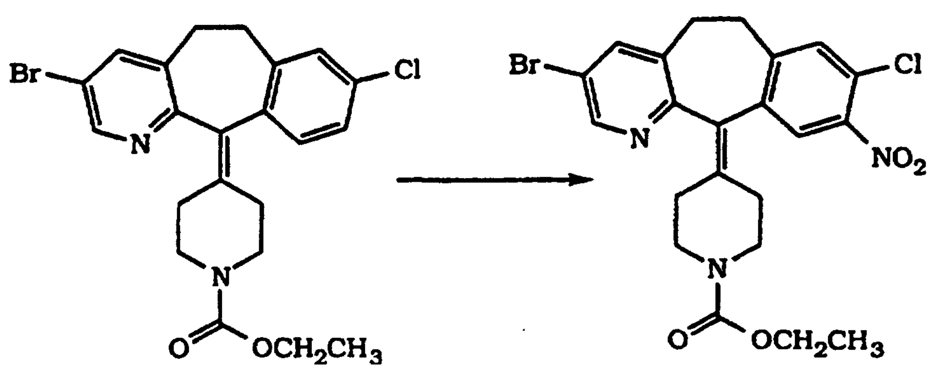

Combine 25.86 g (55.9 mmol) of 4-(8-chloro-3-bromo-5,6-dihydro-11H-benzo[5,6]cyclohepta[1,2-b]pyridin-11-ylidene)-1-piperidine-1-carboxylic acid ethyl ester and 250 mL of concentrated $H_2SO_4$ at -5°C, then add 4.8 g (56.4 mmol) of $NaNO_3$ and stir for 2 hours. Pour the mixture into 600 g of ice and basify with concentrated $NH_4OH$ (aqueous). Filter the mixture, wash with 300 mL of water, then extract with 500 mL of $CH_2Cl_2$. Wash the extract with 200 mL of water, dry over $MgSO_4$, then filter and concentrate *in vacuo* to a residue. Chromatograph the residue (silica gel, 10% EtOAc/ $CH_2Cl_2$) to give 24.4 g (86% yield) of the product. m.p. = 165-167°C, Mass Spec.: $MH^+$ = 506 (CI),

| | | | | |
|---|---|---|---|---|
| elemental analysis | calculated - | C, 52.13; | H, 4.17; | N, 8.29 |
| | found - | C. 52.18; | H, 4.51; | N, 8.16. |

Step B:

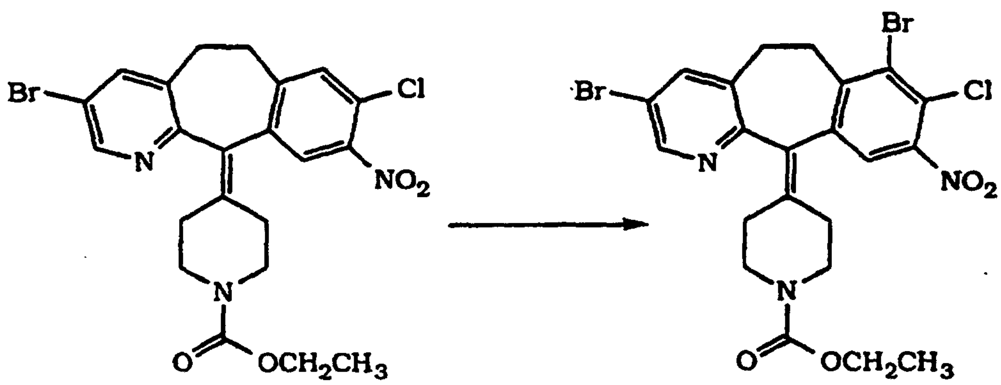

Combine 20 g (40.5 mmol) of the product of Step A and 200 mL of concentrated $H_2SO_4$ at 20°C, then cool the mixture to 0°C. Add 7.12 g (24.89 mmol) of 1.3-dibromo-5.5-dimethylhydantoin to the mixture and stir for 3 hours at 20°C. Cool to 0°C, add an additional 1.0 g (3.5 mmol) of the dibromohydantoin and stir at 20°C for 2 hours. Pour the mixture into 400 g of ice, basify with concentrated $NH_4OH$ (aqueous) at 0°C, and collect the resulting solid by filtration. Wash the solid with 300 mL of water, slurry in 200 mL of acetone and filter to provide 19.79 g (85.6% yield) of the product. m.p. = 236-237°C, Mass Spec.: $MH^+$ = 584 (CI),

| | | | | |
|---|---|---|---|---|
| elemental analysis | calculated - | C, 45.11; | H, 3.44; | N, 7.17 |
| | found - | C. 44.95; | H, 3.57; | N, 7.16. |

Step C:

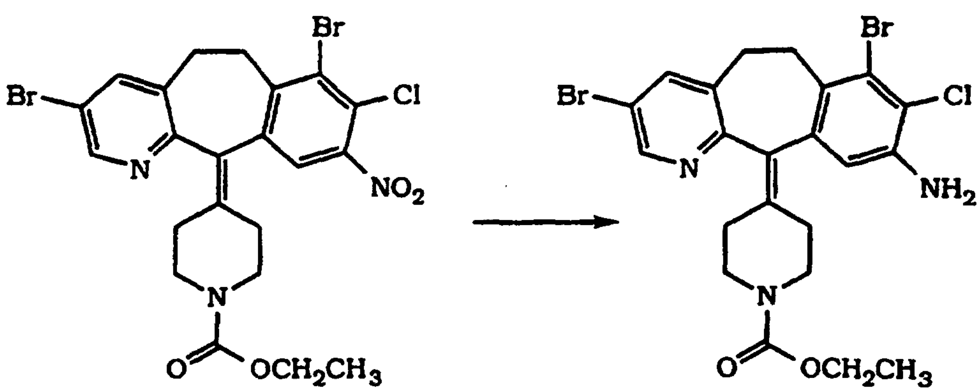

Combine 25 g (447 mmol) of Fe filings, 10 g (90 mmol) of $CaCl_2$ and a suspension of 20 g (34.19 mmol) of the product of Step B in 700 mL of 90:10 EtOH/water at 50°C. Heat the mixture at reflux overnight, filter through Celite®

and wash the filter cake with 2 X 200 mL of hot EtOH. Combine the filtrate and washes, and concentrate *in vacuo* to a residue. Extract the residue with 600 mL of $CH_2Cl_2$, wash with 300 mL of water and dry over $MgSO_4$. Filter and concentrate *in vacuo* to a residue, then chromatograph (silica gel, 30% EtOAc/$CH_2Cl_2$) to give 11.4 g (60% yield) of the product. m.p. = 211-212°C,
Mass Spec.: $MH^+$ = 554 (CI),

| | | | | |
|---|---|---|---|---|
| elemental analysis | calculated - | C, 47.55; | H, 3.99; | N, 7.56 |
| | found - | C, 47.45; | H, 4.31; | N, 7.49. |

Step D:

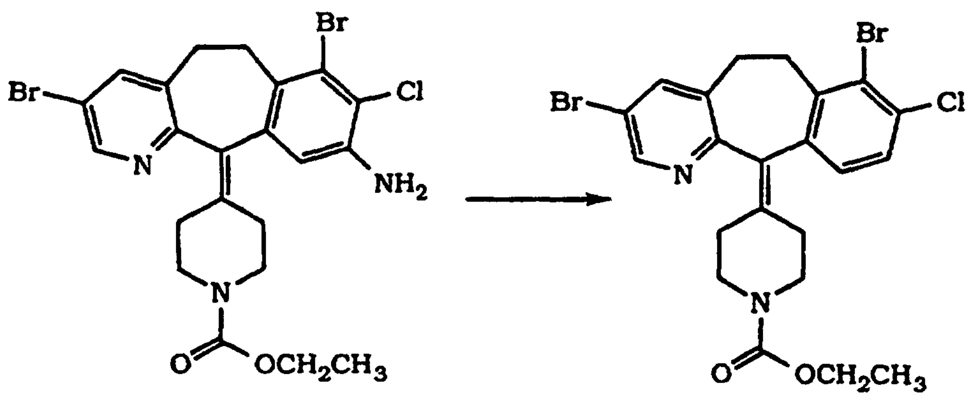

Slowly add (in portions) 20 g (35.9 mmol) of the product of Step C to a solution of 8 g (116 mmol) of $NaNO_2$ in 120 mL of concentrated HCl (aqueous) at -10°C. Stir the resulting mixture at 0°C for 2 hours, then slowly add (dropwise) 150 mL (1.44 mole) of 50% $H_3PO_2$ at 0°C over a 1 hour period. Stir at 0°C for 3 hours, then pour into 600 g of ice and basify with concentrated $NH_4OH$ (aqueous). Extract with 2 X 300 mL of $CH_2Cl_2$, dry the extracts over $MgSO_4$, then filter and concentrate *in vacuo* to a residue. Chromatograph the residue (silica gel, 25% EtOAc/hexanes) to give 13.67 g (70% yield) of the product. m.p. = 163-165°C, Mass Spec.: $MH^+$ = 539 (CI),

| | | | | |
|---|---|---|---|---|
| elemental analysis | calculated - | C. 48.97; | H, 4.05; | N, 5.22 |
| | found - | C. 48.86; | H, 3.91; | N, 5.18. |

Step E:

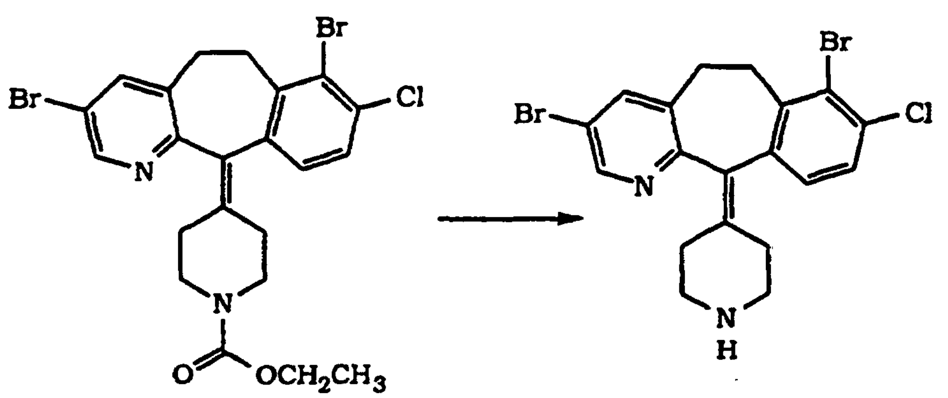

Combine 6.8 g (12.59 mmol) of the product of Step D and 100 mL of concentrated HCl (aqueous) and stir at 85°C overnight. Cool the mixture, pour it into 300 g of ice and basify with concentrated $NH_4OH$ (aqueous). Extract with

2 x 300 mL of $CH_2Cl_2$, then dry the extracts over $MgSO_4$. Filter, concentrate *in vacuo* to a residue, then chromatograph (silica gel, 10% MeOH/EtOAc + 2% $NH_4OH$ (aqueous)) to give 5.4 g (92% yield) of the title compound. m.p. = 172-174°C, Mass Spec.: $MH^+$ = 467 (FAB),

| | | | | |
|---|---|---|---|---|
| elemental analysis | calculated - | C, 48.69; | H, 3.65; | N, 5.97 |
| | found - | C, 48.83; | H, 3.80; | N, 5.97. |

Step F:

Following essentially the same procedure as Step C of Preparative Example 5 below, the title compound from Step E above is reacted with 1-N-t-butoxycarbonylpiperidinyl-4-acetic acid to produce the compound

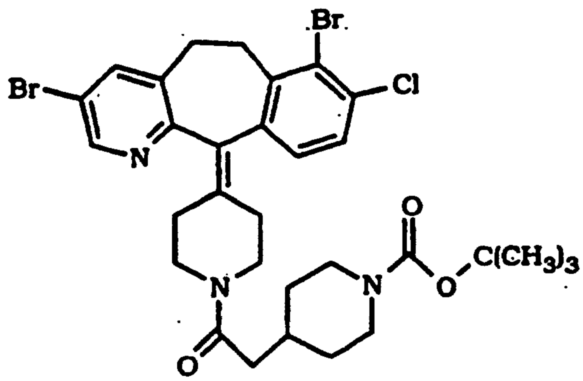

Step G:

Following, essentially the same procedure as Step D of Preparative Example 5 below, the title compound from Step F above is deprotected to yield the title compound of Preparative Example 4.

PREPARATIVE EXAMPLE 5*

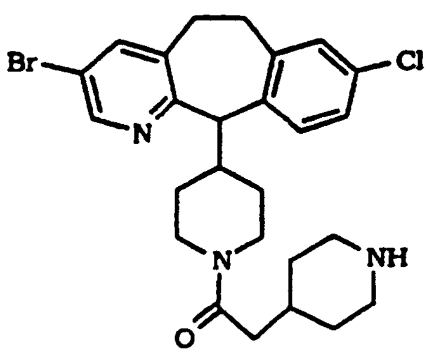

Step A:

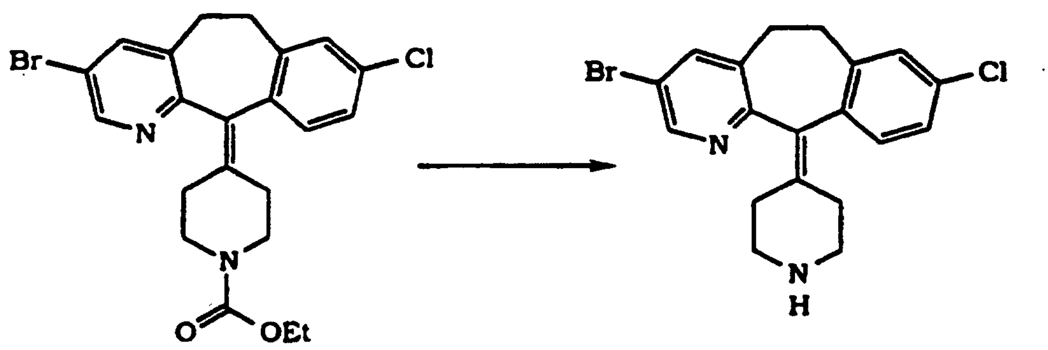

Hydrolyze 2.42 g of 4-(8-chloro-3-bromo-5,6-dihydro-11H-benzo[5,6]cyclohepta[1,2-b]pyridin-11-ylidene)-1-piperidine-1-carboxylic acid ethyl ester via substantially the same procedure as described in Preparative Example 3, Step D, to give 1.39 g (69% yield) of the product.

Step B:

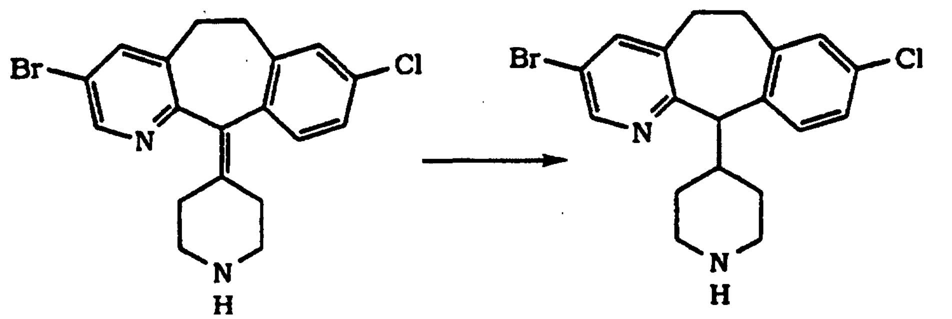

Combine 1 g (2.48 mmol) of the product of Step A and 25 mL of dry toluene, add 2.5 mL of 1 M DIBAL in toluene and heat the mixture at reflux. After 0.5 hours, add another 2.5 mL of 1 M DIBAL in toluene and heat at reflux for 1 hour. (The reaction is monitored by TLC using 50% $MeOH/CH_2Cl_2$ $+NH_4OH$ (aqueous).) Cool the mixture to room temperature, add 50 mL of 1 N HCl (aqueous) and stir for 5 min. Add 100 mL of 1 N NaOH (aqueous), then extract with EtOAc (3 X 150 mL). Dry the extracts over $MgSO_4$, filter and concentrate *in vacuo* to give 1.1 g of the title compound.

Step C:

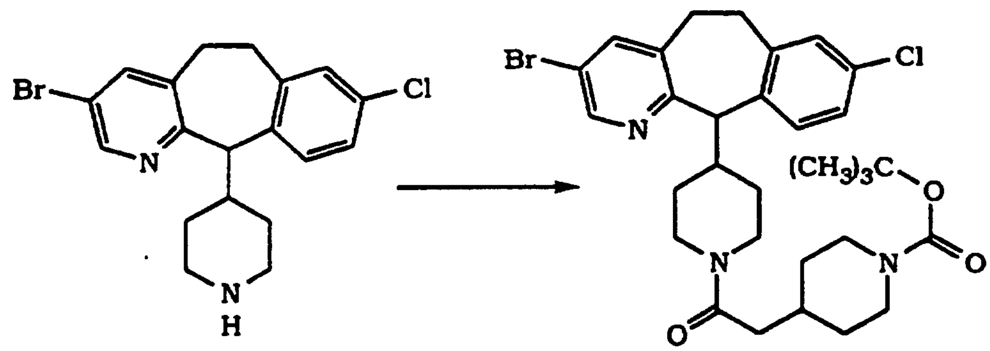

Combine 0.501 g (1.28 mmol) of the title compound of Step B and 20 mL of dry DMF, then add 0.405 g (1.664 mmol) of 1-N-t-butoxycarbonylpiperidinyl-4-acetic acid, 0.319 g (1.664 mmol) of DEC, 0.225 g (1.664 mmol) of HOBT, and 0.168 g (1.664 mmol) of 4-methylmorpholine and stir the mixture at room temperature overnight. Concentrate the mixture *in vacuo* to a residue, then partition the residue between 150 mL of $CH_2Cl_2$ and 150 mL of saturated $NaHCO_3$ (aqueous). Extract the aqueous phase with another 150 mL of $CH_2Cl_2$. Dry the organic phase over $MgSO_4$, and concentrate *in vacuo* to a residue.
Chromatograph the residue (silica gel, 500 mL hexane, 1 L of 1% $MeOH/CH_2Cl_2$ + 0.1% $NH_4OH$ (aqueous), then 1 L of 2% $MeOH/CH_2Cl_2$ + 0.1% $NH_4OH$ (aqueous)) to give 0.575 g of the product. m.p. = 115°-125°C; Mass Spec.: $MH^+$ = 616.

Step D:

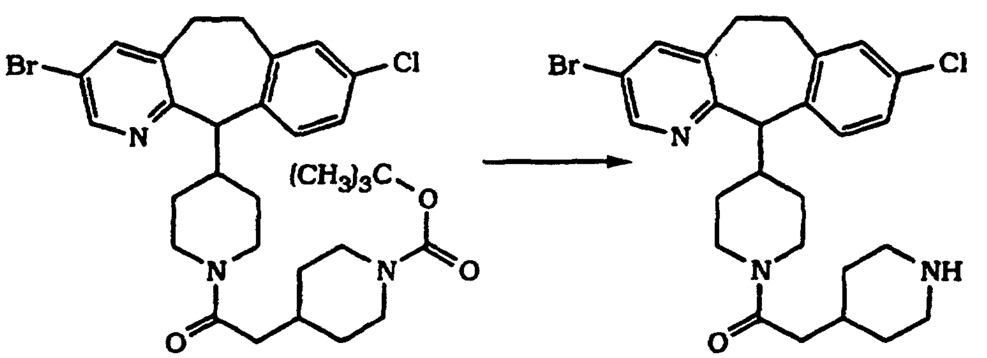

Combine 0.555 g (0.9 mmol) of the product of Step C and 15 mL of $CH_2Cl_2$ and cool the mixture to 0°C. Add 15 mL of TFA and stir at 0°C for 2 hours. Concentrate *in vacuo* at 40-45°C to a residue, then partition the residue between 150 mL of $CH_2Cl_2$ and 100 mL of saturated $NaHCO_3$ (aqueous). Extract the aqueous layer with 100 mL of $CH_2Cl_2$, combine the extracts and dry over $MgSO_4$. Concentrate *in vacuo* to give 0.47 g of the product. m.p. = 140°-150°C; Mass Spec.: $MH^+$ = 516.

PREPARATIVE EXAMPLE 6

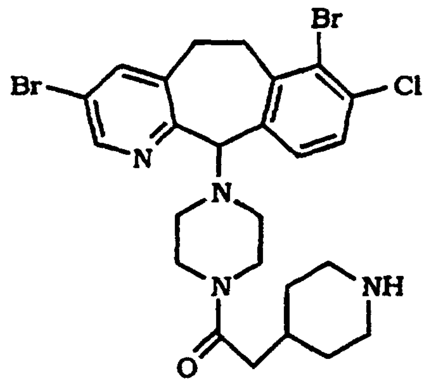

[racemic as well as (+)- and (-)-isomers]

Step A:

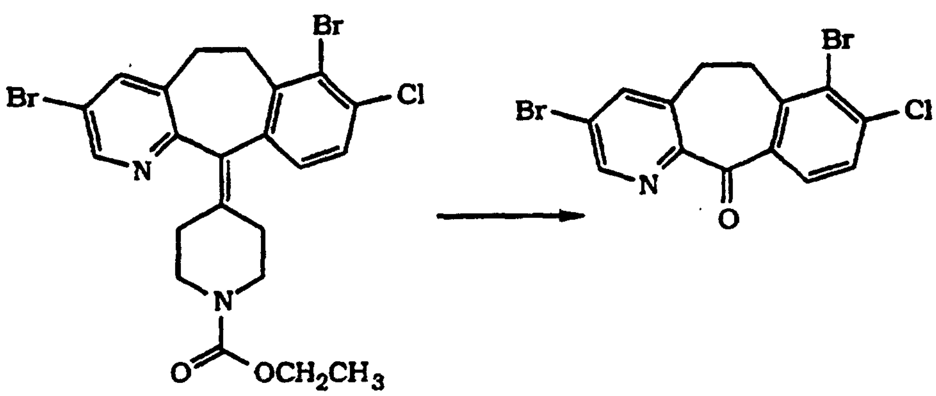

Combine 16.6 g (0.03 mole) of the product of Preparative Example 4, Step D, with a 3:1 solution of $CH_3CN$ and water (212.65 mL $CH_3CN$ and 70.8 mL of water) and stir the resulting slurry overnight at room temperature. Add 32.833 g (0.153 mole) of $NaIO_4$ and then 0.31 g (2.30 mmol) of $RuO_2$ and stir at room temperature give 1.39 g (69% yield) of the product. (The addition of RuO is accompanied by an exothermic reaction and the temperature climbs from 20° to 30°C.) Stir the mixture for 1.3 hrs. (temperature returned to 25°C after about 30 min.), then filter to remove the solids and wash the solids with $CH_2Cl_2$. Concentrate the filtrate *in vacuo* to a residue and dissolve the residue in $CH_2Cl_2$. Filter to remove insoluble solids and wash the solids with $CH_2Cl_2$. Wash the filtrate with water, concentrate to a volume of about 200 mL and wash with bleach, then with water. Extract with 6 N HCl (aqueous). Cool the aqueous extract to 0°C and slowly add 50% NaOH (aqueous) to adjust to pH = 4 while keeping the temperature <30°C. Extract twice with $CH_2Cl_2$, dry over $MgSO_4$ and concentrate *in vacuo* to a residue. Slurry the residue in 20 mL of EtOH and cool to 0°C. Collect the resulting solids by filtration and dry the solids *in vacuo* to give 7.95 g of the product. $^1$H NMR ($CDCl_3$, 200 MHz): 8.7 (s, 1H); 7.85 (m, 6H); 7.5 (d, 2H); 3.45 (m, 2H); 3.15 (m, 2H).

Step B:

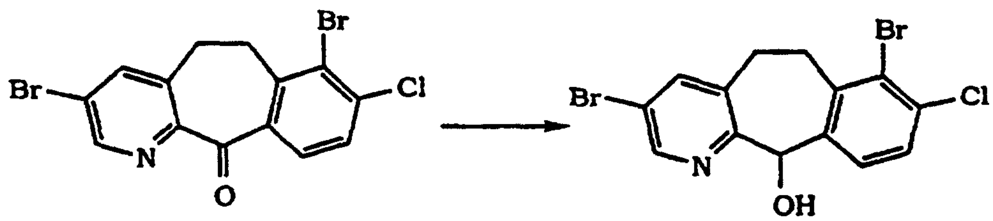

Combine 21.58 g (53.75 mmol) of the product of Step A and 500 mL of an anhydrous 1:1 mixture of EtOH and toluene, add 1.43 g (37.8 mmol) of $NaBH_4$ and heat the mixture at reflux for 10 min. Cool the mixture to 0°C, add 100 mL of water, then adjust to pH= 4-5 with 1 M HCl (aqueous) while keeping the temperature <10°C. Add 250 mL of EtOAc and separate the layers. Wash the organic layer with brine (3 X 50 mL) then dry over $Na_2SO_4$. Concentrate *in vacuo* to a residue (24.01 g) and chromatograph the residue (silica gel, 30 % hexane/$CH_2Cl_2$) to give the product. Impure fractions were purified by rechromatography. A total of 18.57 g of the product was obtained. $^1H$ NMR (DMSO-$d_6$, 400 MHz): 8.5 (s, 1H); 7.9 (s, 1H); 7.5 (d of d, 2H); 6.2 (s, 1H): 6.1 (s, 1H); 3.5 (m, 1H); 3.4 (m, 1H); 3.2 (m, 2H).

Step C:

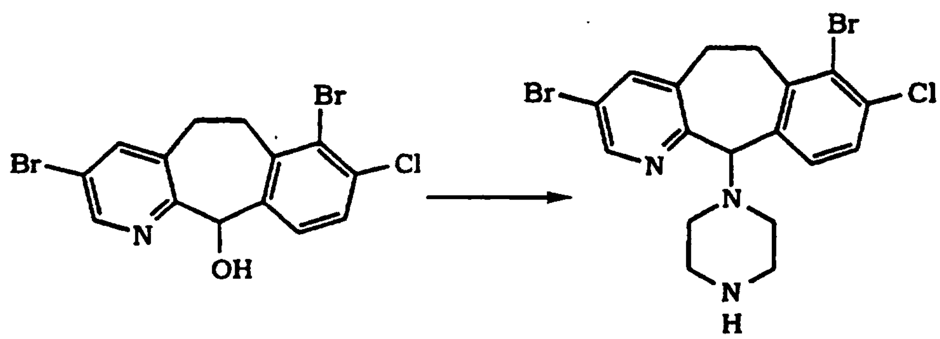

Combine 18.57 g (46.02 mmol) of the product of Step B and 500 mL of $CHCl_3$, then add 6.70 mL (91.2 mmol) of $SOCl_2$, and stir the mixture at room temperature for 4 hrs. Add a solution of 35.6 g (0.413 mole) of piperazine in 800 mL of THF over a period of 5 min. and stir the mixture for 1 hr. at room temperature. Heat the mixture at reflux overnight, then cool to room temperature and dilute the mixture with 1 L of $CH_2Cl_2$. Wash with water (5 X 200 mL), and extract the aqueous wash with $CHCl_3$ (3 X 100 mL). Combine all of the organic solutions, wash with brine (3 X 200 mL) and dry over $MgSO_4$. Concentrate *in vacuo* to a residue and chromatograph (silica gel, gradient of 5%, 7.5%, 10% MeOH/$CH_2Cl_2$ + $NH_4OH$) to give 18.49 g of the title compound as a racemic mixture.

Step D - Separation of Enantiomers;

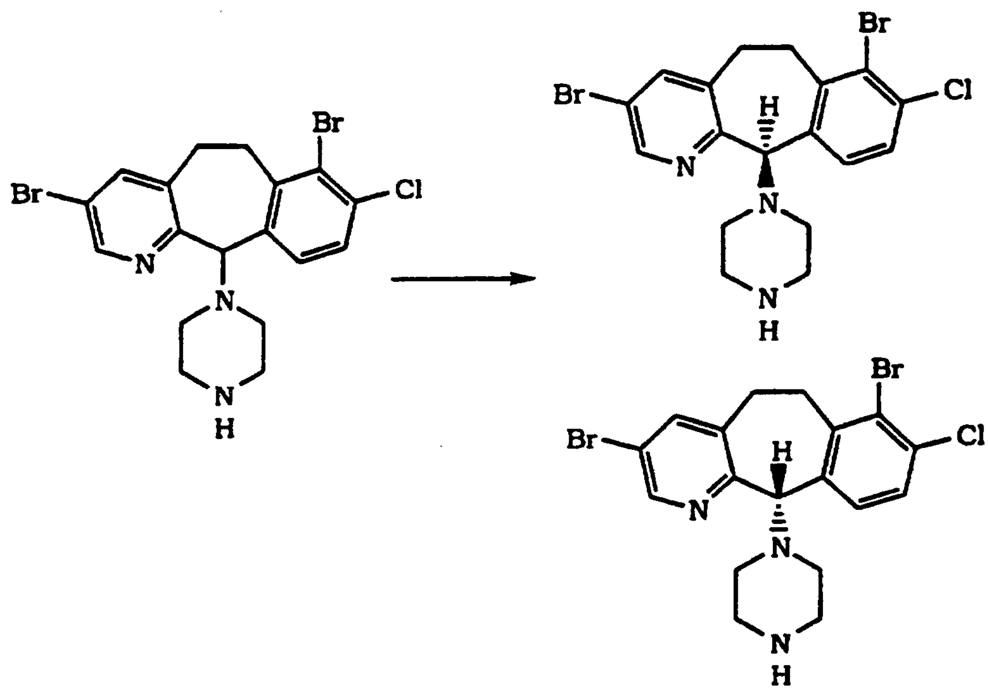

The racemic title compound of Step C is separated by preparative chiral chromatography (Chiralpack AD, 5 cm X 50 cm column, flow rate 100 mL/min., 20% iPrOH/hexane + 0.2% diethylamine), to give 9.14 g of the (+)-isomer and 9.30 g of the (-)-isomer.

Physical chemical data for (+)-isomer: m.p. = 74.5°-77.5°C; Mass Spec. $MH^+$ = 471.9; $[\alpha]_D^{25}$ = +97.4° (8.48 mg/ 2mL MeOH).

Physical chemical data for (-)-isomer: m.p. = 82.9°-84.5°C; Mass Spec. $MH^+$ = 471.8; $[\alpha]_D^{25}$ = -97.4° (8.32 mg/ 2mL MeOH).

Step E:

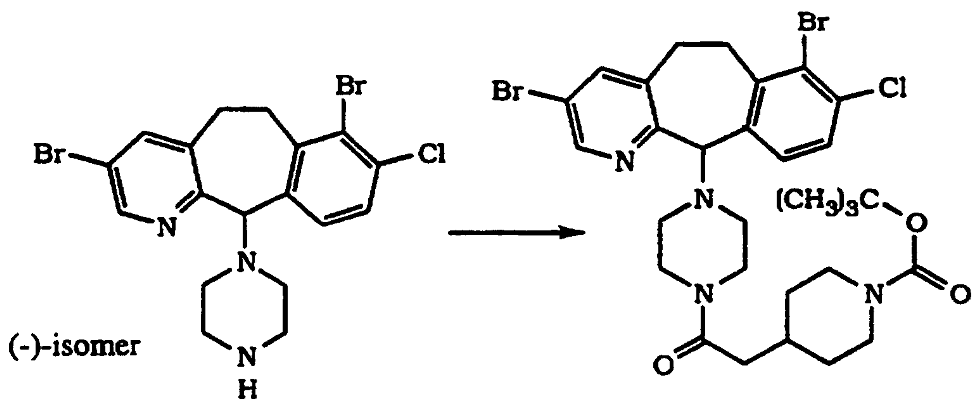


Combine 3.21 g (6.80 mmol) of the (-)-isomer product of Step D and 150 mL of anhydrous DMF. Add 2.15 g (8.8 mmol) of 1-N-t-butoxycarbonylpiperidinyl-4-acetic acid, 1.69 g (8.8 mmol) of DEC, 1.19 g (8.8 mmol) of HOBT and 0.97 mL (8.8 mmol) of N-methylmorpholine and stir the mixture at room temperature overnight. Concentrate *in vacuo*

to remove the DMF and add 50 mL of saturated $NaHCO_3$ (aqueous). Extract with $CH_2Cl_2$ (2 X 250 mL), wash the extracts with 50 mL of brine and dry over $MgSO_4$. Concentrate *in vacuo* to a residue and chromatograph (silica gel, 2% MeOH/$CH_2Cl_2$ + 10% $NH_4OH$) to give 4.75 g of the product. m.p. = 75.7°-78.5°C; Mass Spec.: $MH^+$ = 697; $[\alpha]_D^{25}$ = -5.5° (6.6 mg/2 mL MeOH).

Step F:

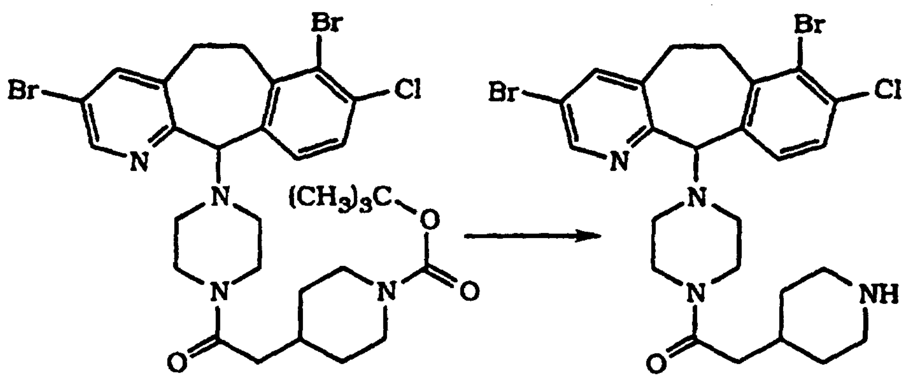

Combine 4.70 g (6.74 mmol) of the product of Step E and 30 mL of MeOH, then add 50 mL of 10% $H_2SO_4$/dioxane in 10 mL aliquots over a 1 hr. period. Pour the mixture into 50 mL of water and add 15 mL of 50% NaOH (aqueous) to adjust to pH= 10-11. Filter to remove the resulting solids and extract the filtrate with $CH_2Cl_2$ (2 X 250 mL). Concentrate the aqueous layer *in vacuo* to remove the MeOH and extract again with 250 mL of $CH_2Cl_2$. Dry the combined extracts over $MgSO_4$ and concentrate *in vacuo* to give the product. m.p. = 128.1°-131.5°C; Mass Spec.: $MH^+$ = 597; $[\alpha]_D^{25}$ = -6.02° (9.3 mg/2 mL MeOH).

PREPARATIVE EXAMPLE 7

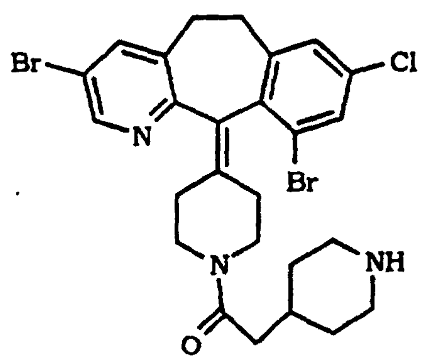

Step A:

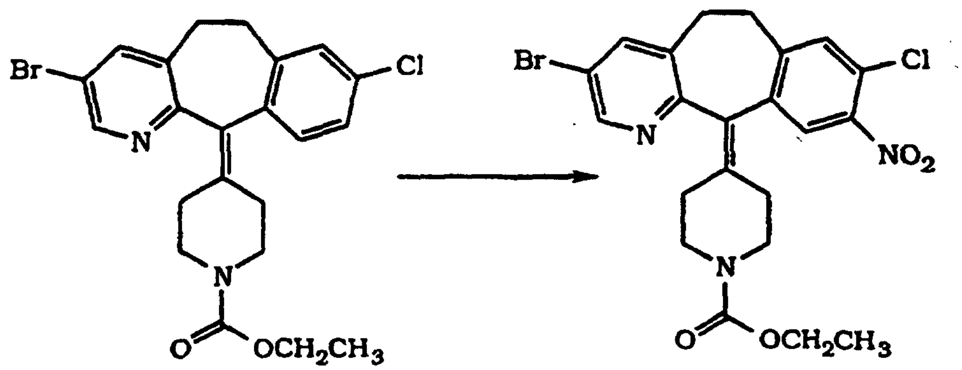

Combine 15 g (38.5 mmol) of 4-(8-chloro-3-bromo-5,6-dihydro-11H-benzo[5,6]cyclohepta[1,2-b]pyridin-11-ylidene)-1-piperidine-1-carboxylic acid ethyl ester and 150 mL of concentrated $H_2SO_4$ at -5°C, then add 3.89 g (38.5 mmol) of $KNO_3$ and stir for 4 hours. Pour the mixture into 3 L of ice and basify with 50% NaOH (aqueous). Extract with $CH_2Cl_2$, dry over $MgSO_4$, then filter and concentrate *in vacuo* to a residue. Recrystallize the residue from acetone to give 6.69 g of the product. $^1H$ NMR ($CDCl_3$, 200 MHz): 8.5 (s, 1H); 7.75 (s, 1H); 7.6 (s, 1H); 7.35 (s, 1H); 4.15 (q, 2H); 3.8 (m, 2H); 3.5-3.1 (m, 4H); 3.0-2.8 (m, 2H); 2.6-2.2 (m. 4H); 1.25 (t, 3H).

Step B:

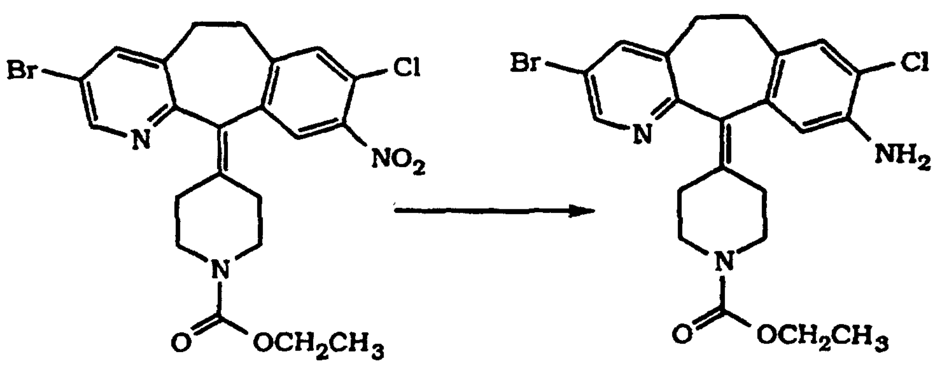

Combine 6.69 g (13.1 mmol) of the product of Step A and 100 mL of 85% EtOH/water, then add 0.66 g (5.9 mmol) of $CaCl_2$ and 6.56 g (117.9 mmol) of Fe and heat the mixture at reflux overnight. Filter the hot reaction mixture through celite® and rinse the filter cake with hot EtOH. Concentrate the filtrate *in vacuo* to give 7.72 g of the product. Mass Spec.: $MH^+$ = 478.0.

Step C:

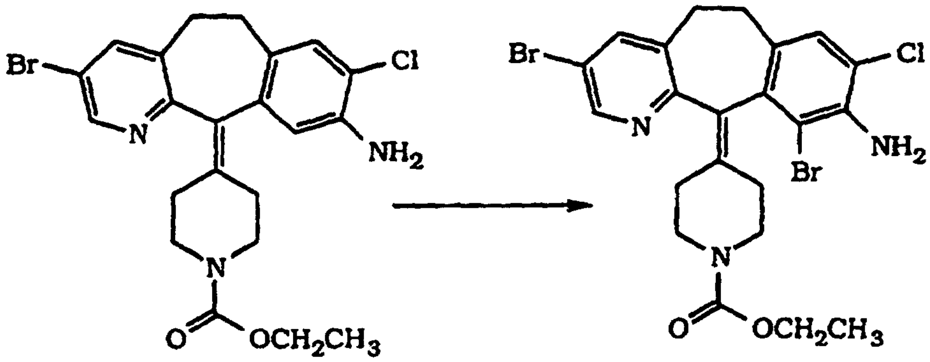

Combine 7.70 g of the product of Step B and 35 mL of HOAc, then add 45 mL of a solution of $Br_2$ in HOAc and stir the mixture at room temperature overnight. Add 300 mL of 1 N NaOH (aqueous) , then 75 mL of 50% NaOH (aqueous) and extract with EtOAc. Dry the extract over $MgSO_4$ and concentrate *in vacuo* to a residue. Chromatograph the residue (silica gel, 20%-30% EtOAc/hexane) to give 3.47 g of the product (along with another 1.28 g of partially purified product). Mass Spec.: $MH^+$ = 555.9.

$^1$H NMR ($CDCl_3$, 300 MHz): 8.5 (s, 1H); 7.5 (s, 1H); 7.15 (s, 1H); 4.5 (s, 2H); 4.15 (m, 3H); 3.8 (br s, 2H); 3.4-3.1 (m, 4H); 9-2.75 (m, 1H); 2.7-2.5 (m, 2H); 2.4-2.2 (m, 2H); 1.25 (m, 3H).

Step D:

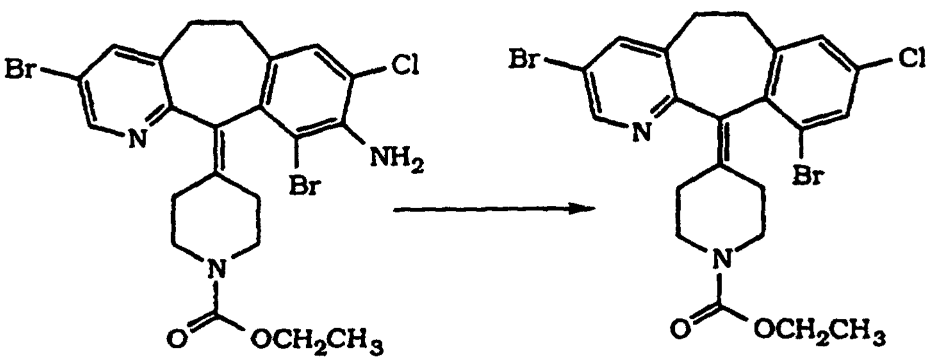

Combine 0.557 g (5.4 mmol) of t-butylnitrite and 3 mL of DMF, and heat the mixture at to 60°-70°C. Slowly add (dropwise) a mixture of 2.00 g (3.6 mmol) of the product of Step C and 4 mL of DMF, then cool the mixture to room temperature. Add another 0.64 mL of t-butylnitrite at 40°C and reheat the mixture to 60°-70°C for 0.5 hrs. Cool to room temperature and pour the mixture into 150 mL of water. Extract with $CH_2Cl_2$, dry the extract over $MgSO_4$ and concentrate *in vacuo* to a residue. Chromatograph the residue (silica gel, 10%-20% EtOAc/hexane) to give 0.74 g of the product. Mass Spec.: $MH^+$ = 541.0.

$^1$H NMR (CDCl3, 200 MHz): 8.52 (s, 1H); 7.5 (d, 2H); 7.2 (s, 1H); 4.15 (q. 2H); 3.9-3.7 (m, 2H); 3.5-3.1 (m, 4H); 3.0-2.5 (m, 2H); 2.4-2.2 (m, 2H); 2.1-1.9 (m, 2H); 1.26 (t, 3H).

Step E:

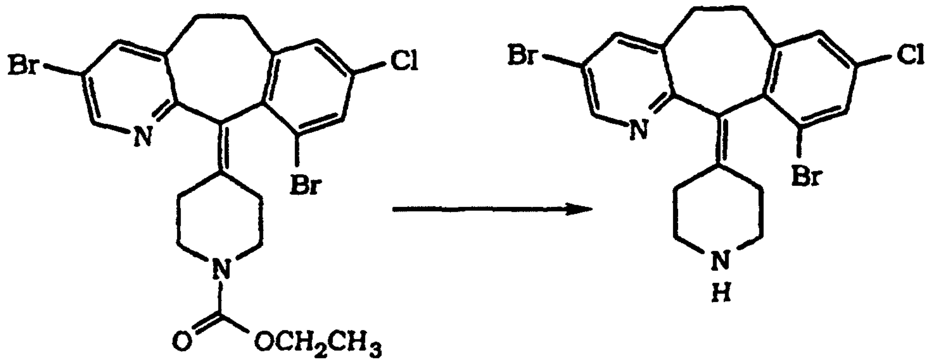

Combine 0.70 g (1.4 mmol) of the product of Step D and 8 mL of concentrated HCl (aqueous) and heat the mixture at reflux overnight. Add 30 mL of 1 N NaOH (aqueous), then 5 mL of 50% NaOH (aqueous) and extract with $CH_2Cl_2$. Dry the extract over $MgSO_4$ and concentrate *in vacuo* to give 0.59 g of the title compound. Mass Spec.: $M^+$ = 468.7. m.p. = 123.9°-124.2°C.

Step F:

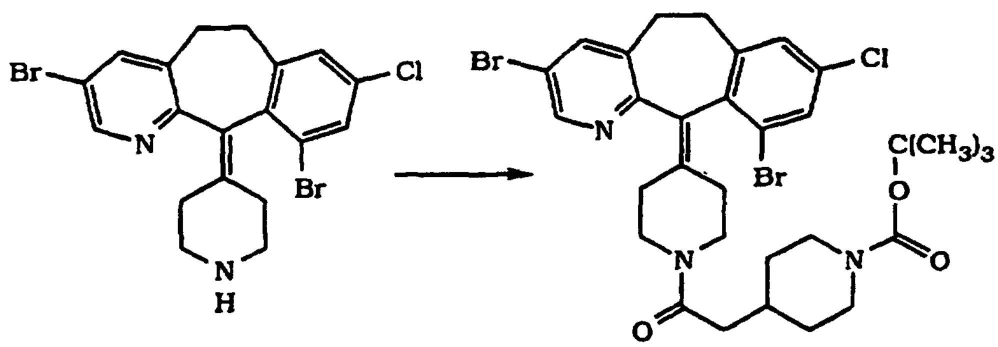

React 6.0 g (12.8 mmol) of the title compound from Step E and with 3.78 g (16.6 mmol) of 1-N-t-butoxycarbonylpiperidinyl-4-acetic acid using substantially the same procedures as described for Preparative Example 5, Step C, to give 8.52 g of the product. Mass Spec.: $MH^+$ = 694.0 (FAB). $^1H$ NMR ($CDCl_3$, 200 MHz): 8.5 (d, 1H); 7.5 (d, 2H); 7.2 (d, 1H); 4.15-3.9 (m, 3H); 3.8-3.6 (m, 1H); 3.5-3.15 (m, 3H); 2.9 (d, 2H); 2.8-2.5 (m, 4H); 2.4-1.8 (m, 6H); 1.8-1.6 (br d, 2H); 1.4 (s, 9H); 1.25-1.0 (m, 2H).

Step G:

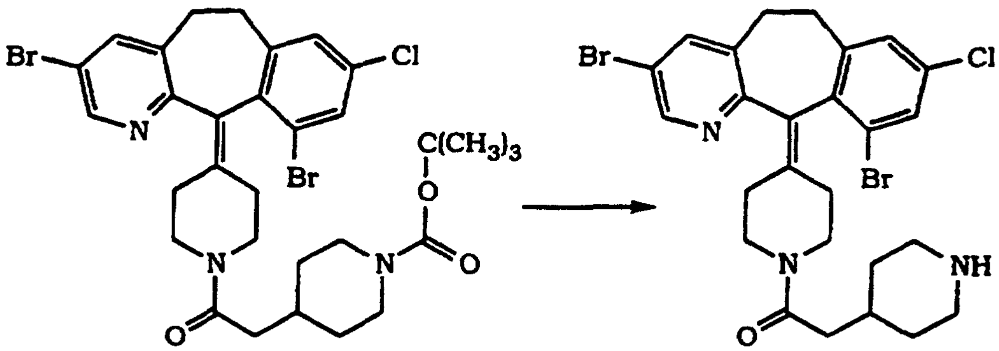

Combine 8.50 g of the product of Step F and 60 mL of $CH_2Cl_2$, then cool to 0°C and add 55 mL of TFA. Stir the mixture for 3 h at 0°C, then add 500 mL of 1 N NaOH (aqueous) followed by 30 mL of 50% NaOH (aqueous). Extract with $CH_2Cl_2$, dry over $MgSO_4$ and concentrate *in vacuo* to give 7.86 g of the product.
Mass Spec.: $M^+$ = 593.9 (FAB). $^1H$ NMR ($CDCl_3$, 200 MHz): 8.51 (d, 1H); 7.52 (d of d, 2H); 7.20 (d, 1H); 4.1-3.95 (m, 2H); 3.8-3.65 (m, 2H); 3.5-3.05 (m, 5H); 3.0-2.5 (m, 6H); 2.45-1.6 (m, 6H);1.4-1.1 (m, 2H).

PREPARATIVE EXAMPLE 8

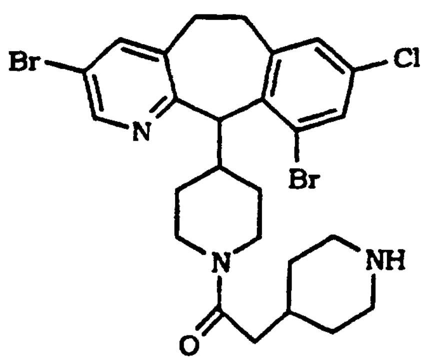

[racemic as well as (+)- and (-)-isomers]

Step A:

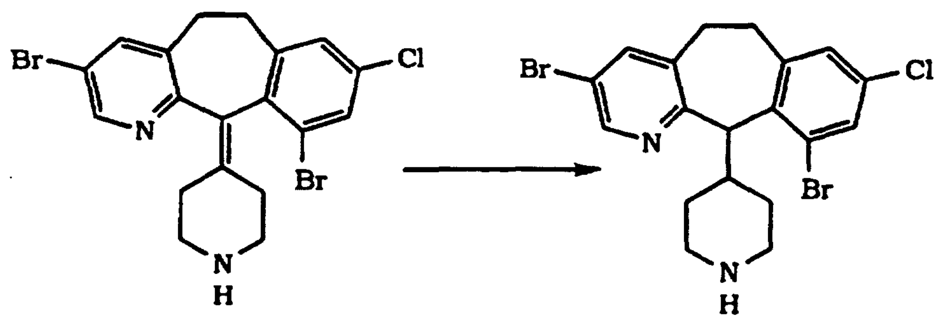

Prepare a solution of 8.1 g of the title compound from Preparative Example 7, Step E, in toluene and add 17.3 mL of a 1M solution of DIBAL in toluene. Heat the mixture at reflux and slowly add (dropwise) another 21 mL of 1 M DIBAL/toluene solution over a period of 40 min. Cool the reaction mixture to about 0°C and add 700 mL of 1 M HCl (aqueous). Separate and discard the organic phase. Wash the aqueous phase with $CH_2Cl_2$, discard the extract, then basify the aqueous phase by adding 50% NaOH (aqueous). Extract with $CH_2Cl_2$, dry the extract over $MgSO_4$ and concentrate *in vacuo* to give 7.30 g of the title compound, which is a racemic mixture of enantiomers.

Step B - Separation of Enantiomers:

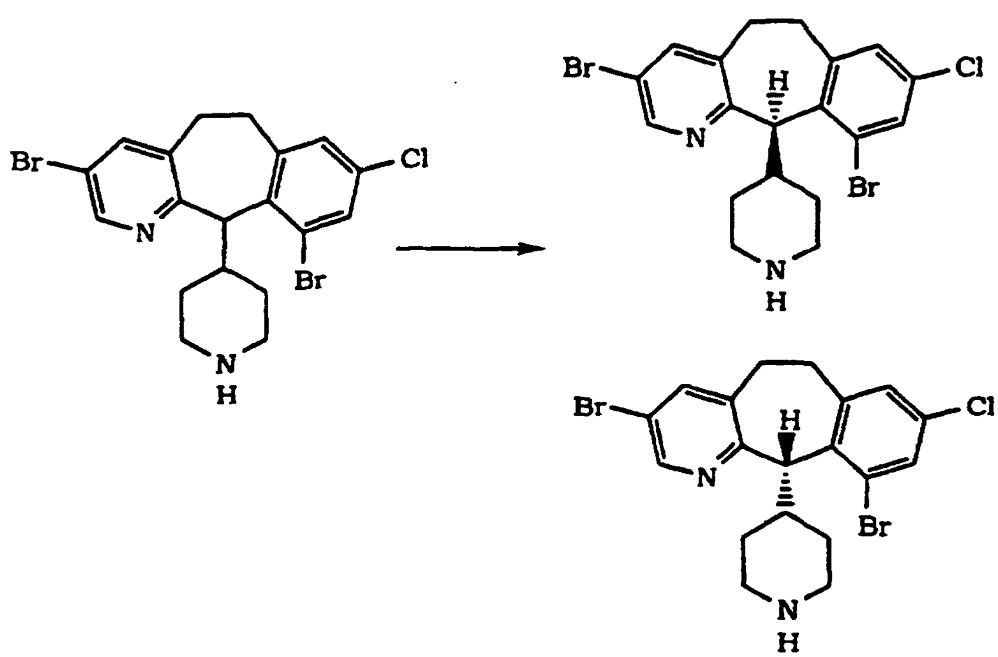

The racemic title compound of Step A is separated by preparative chiral chromatography (Chiralpack AD, 5 cm X 50 cm column, using 20% iPrOH/hexane + 0.2% diethylamine), to give the (+)-isomer and the (-)-isomer of the title compound.

Physical chemical data for (+)-isomer: m.p. = 148.8°C: Mass Spec. $MH^+$ = 469; $[\alpha]_D^{25}$ = +65.6° (12.93 mg/ 2mL MeOH).

Physical chemical data for (-)-isomer. m.p. = 112°C: Mass Spec. $MH^+$ = 469; $[\alpha]_D^{25}$ = -65.2° (3.65 mg/ 2mL MeOH).

Step C:

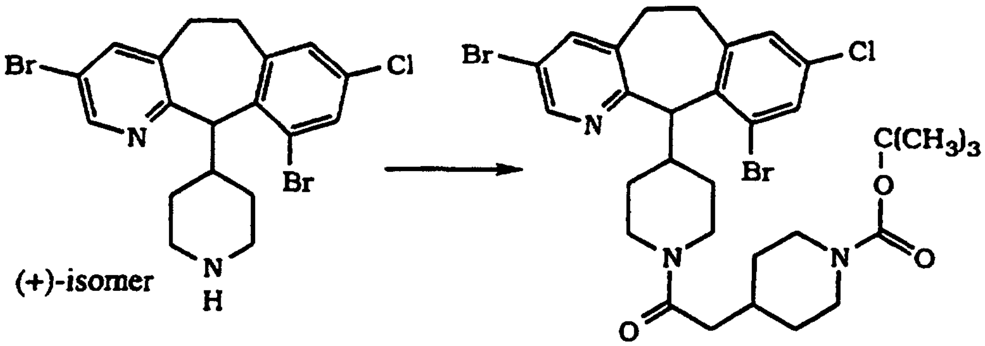

React 1.33 g of the (+)-isomer of the title compound of Preparative Example 8, Step B, with 1.37 g of 1-N-t-butoxycarbonylpiperidinyl-4-acetic acid using substantially the same procedures as described for Preparative Example 5. Step C, to give 2.78 g of the product. Mass Spec.: $MH^+$ = 694.0 (FAB); $[\alpha]_D^{25}$ = +34.1° (5.45 mg/2 mL. MeOH).

Step D:

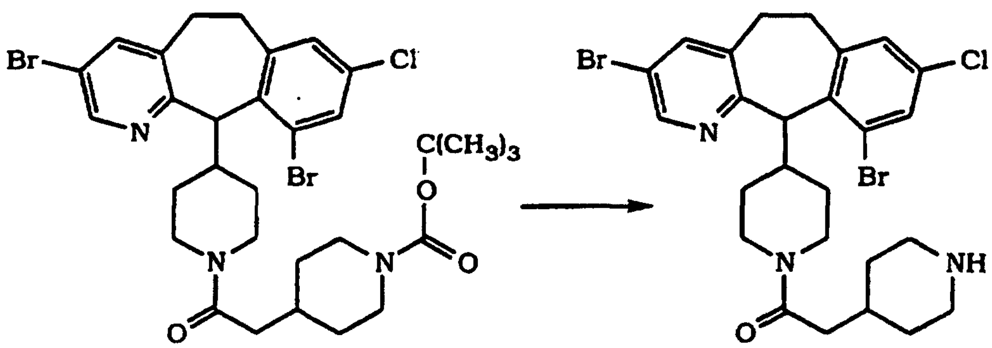

Treat 2.78 g of the product of Step C via substantially the same procedure as described for Preparative Example 5, Step D, to give 1.72 g of the product. m.p. = 104.1°C; Mass Spec.: $MH^+$ = 594; $[\alpha]_D^{25}$ = +53.4° (11.42 mg/2 mL, MeOH).

PREPARATIVE EXAMPLE 9

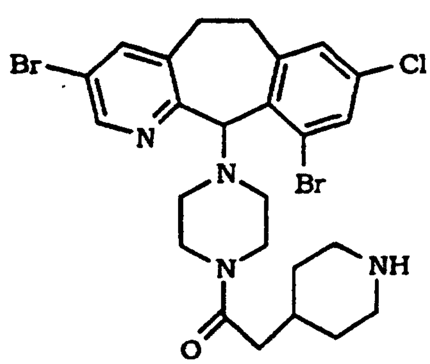

[racemic as well as (+)- and (-)-isomers)

Step A:

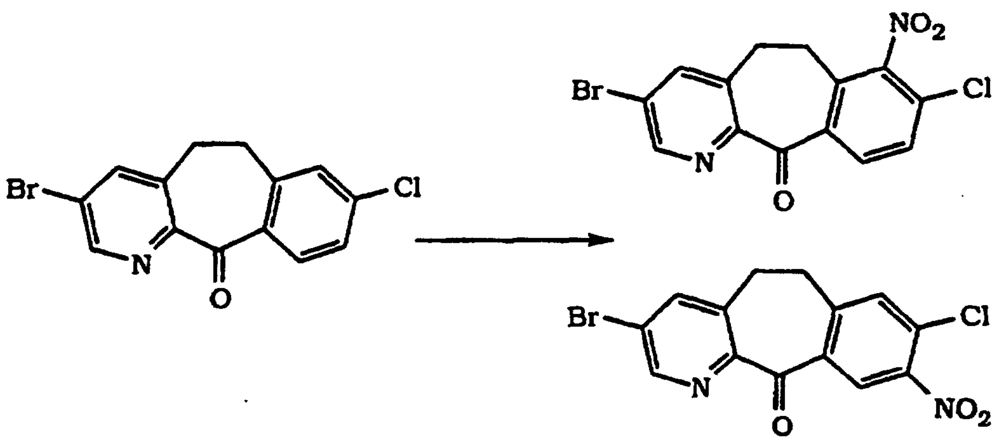

Combine 40.0 g (0.124 mole) of the starting ketone and 200 mL of $H_2SO_4$ and cool to 0°C. Slowly add 13.78 g (0.136 mole) of $KNO_3$ over a period of 1.5 hrs., then warm to room temperature and stir overnight. Work up the reaction using substantially the same procedure as described for Preparative Example 4, Step A. Chromatograph (silica gel, 20%, 30%, 40%. 50% EtOAc/hexane, then 100% EtOAc) to give 28 g of the 9-nitro product, along with a smaller quantity of the 7-nitro product and 19 g of a mixture of the 7-nitro and 9-nitro compounds.

Step B:

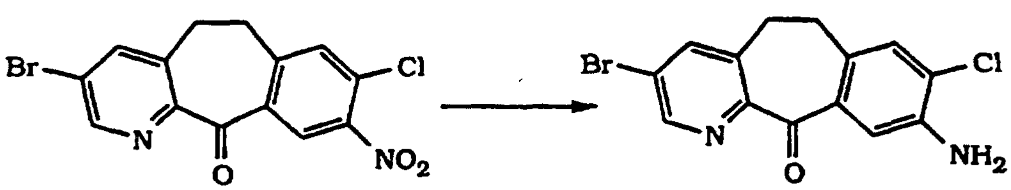

React 28 g (76.2 mmol) of the 9-nitro product of Step A, 400 mL of 85% EtOH/water, 3.8 g (34.3 mmol) of $CaCl_2$ and 38.28 g (0.685 mole) of Fe using substantially the same procedure as described for Preparative Example 4, Step C, to give 24 g of the product

Step C:

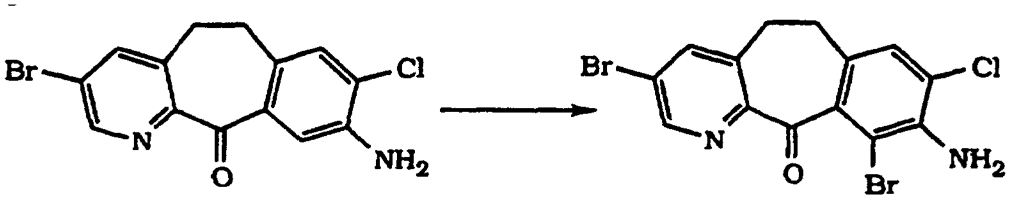

Combine 13 g (38.5 mmol) of the product of Step B, 140 mL of HOAc and slowly add a solution of 2.95 mL (57.8 mmol) of $Br_2$ in 10 mL of HOAc over a period of 20 min. Stir the reaction mixture at room temperature, then

concentrate *in vacuo* to a residue. Add $CH_2Cl_2$ and water, then adjust to pH = 8-9 with 50% NaOH (aqueous). Wash the organic phase with water, then brine and dry over $Na_2SO_4$. Concentrate *in vacuo* to give 11.3 g of the product.

Step D:

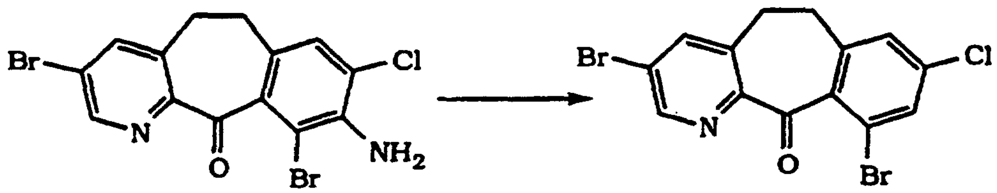

Cool 100 mL of concentrated HCl (aqueous) to 0°C, then add 5.61 g (81.4 mmol) of $NaNO_2$ and stir for 10 min. Slowly add (in portions) 11.3 g (27.1 mmol) of the product of Step C and stir the mixture at 0°-3°C for 2.25 hrs. Slowly add (dropwise) 180 mL of 50% $H_3PO_2$ (aqueous) and allow the mixture to stand at 0°C overnight. Slowly add (dropwise) 150 mL of 50% NaOH over 30 min., to adjust to pH = 9, then extract with $CH_2Cl_2$. Wash the extract with water, then brine and dry over $Na_2SO_4$. Concentrate *in vacuo* to a residue and chromatograph (silica gel, 2% EtOAc/$CH_2Cl_2$) to give 8.6 g of the product.

Step E:

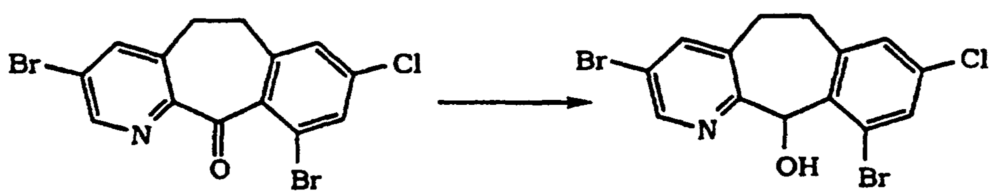

Combine 8.6 g (21.4 mmol) of the product of Step D and 300 mL of MeOH and cool to 0°-2°C. Add 1.21 g (32.1 mmol) of $NaBH_4$ and stir the mixture at ~0°C for 1 hr. Add another 0.121 g (3.21 mmol) of $NaBH_4$, stir for 2 hr. at 0°C, then let stand overnight at 0°C. Concentrate *in vacuo* to a residue then partition the residue between $CH_2Cl_2$ and water. Separate the organic phase and concentrate *in vacuo* (50°C) to give 8.2 g of the product.

Step F:

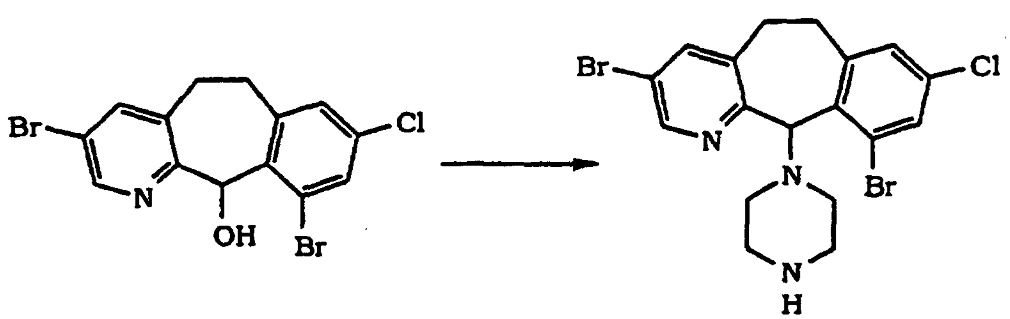

Combine 8.2 g (20.3 mmol) of the product of Step E and 160 mL of $CH_2Cl_2$, cool to 0°C, then slowly add (dropwise) 14.8 mL (203 mmol) of $SOCl_2$ over a 30 min. period. Warm the mixture to room temperature and stir for 4.5 hrs., then concentrate *in vacuo* to a residue, add $CH_2Cl_2$ and wash with 1 N NaOH (aqueous) then brine and dry over $Na_2SO_4$. Concentrate *in vacuo* to a residue, then add dry THF and 8.7 g (101 mmol) of piperazine and stir at room

temperature overnight. Concentrate *in vacuo* to a residue, add $CH_2Cl_2$, and wash with 0.25 N NaOH (aqueous), water, then brine. Dry over $Na_2SO_4$ and concentrate *in vacuo* to give 9.46 g of the crude product. Chromatograph (silica gel, 5% MeOH/$CH_2Cl_2$ + $NH_3$) to give 3.59 g of the title compound, as a racemate. $^1H$ NMR ($CDCl_3$, 200 MHz): 8.43 (d, 1H); 7.55 (d, 1H); 7.45 (d, 1H); 7.11 (d, 1H); 5.31 (s, 1H); 4.86-4.65 (m, 1H); 3.57-3.40 (m, 1H); 2.98-2.55 (m, 6H); 2.45-2.20 (m, 5H).

Step G - Separation of Enantiomers:

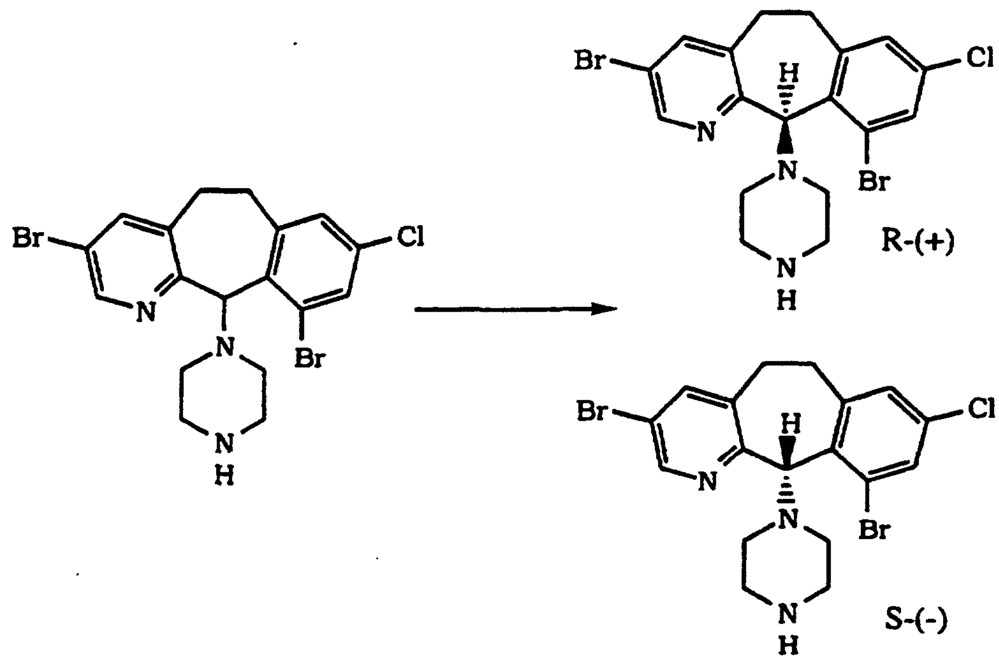

R-(+)

S-(-)

The racemic title compound from Step F (5.7 g) is chromatographed as described for Preparative Example 6, Step D, using 30% iPrOH/hexane + 0.2% diethylamine, to give 2.88 g of the R-(+)-isomer and 2.77 g of the S-(-)-isomer of the title compound.

Physical chemical data for the R-(+)-isomer: Mass Spec. $MH^+$ = 470.0; $[\alpha]_D^{25}$ = +12.1° (10.9 mg/ 2mL MeOH).

Physical chemical data for the S-(-)-isomer: Mass Spec. $MH^+$ = 470.0: $[\alpha]_D^{25}$ = -13.2° (11.51 mg/ 2mL MeOH).

Step H:

Following essentially the same procedure as Preparative Example 5, Steps C and D, the racemic title compound of Preparative Example 9 is obtained from the racemic compound of Step F. Similarly, using the (-)- or (+)-isomer from Step G, the (-)- or (+)-isomer of the title compound of Preparative Example 9 is obtained, respectively.

PREPARATIVE EXAMPLE 10

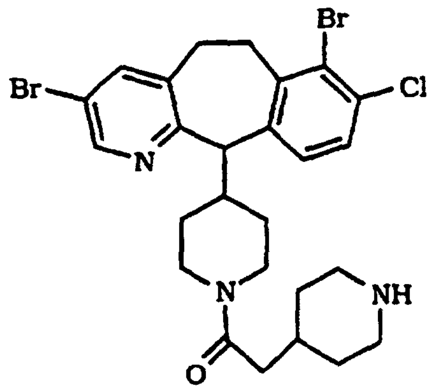

[racemic as well as (+)- and (-)-isomers]

Step A:

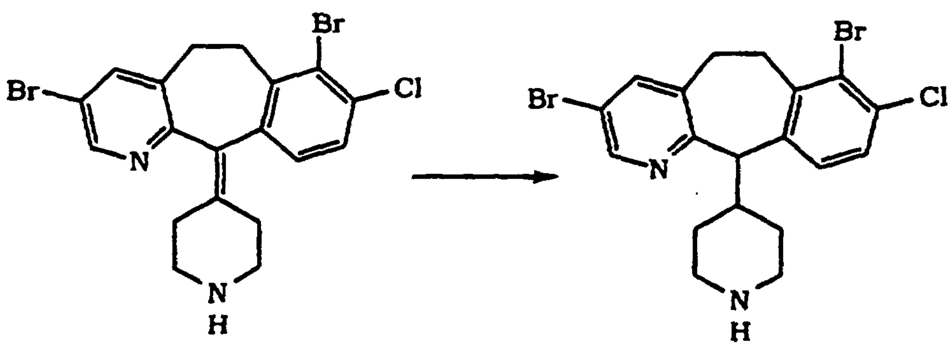

Combine 13 g (33.3 mmol) of the title compound from Preparative Example 4, Step E, and 300 mL of toluene at 20°C, then add 32.5 mL (32.5 mmol) of a 1 M solution of DIBAL in toluene. Heat the mixture at reflux for 1 hr., cool to 20°C, add another 32.5 mL of 1 M DIBAL solution and heat at reflux for 1 hr. Cool the mixture to 20°C and pour it into a mixture of 400 g of ice, 500 mL of EtOAc and 300 mL of 10% NaOH (aqueous). Extract the aqueous layer with $CH_2Cl_2$ (3 x 200 mL), dry the organic layers over $MgSO_4$, then concentrate *in vacuo* to a residue. Chromatograph (silica gel, 12% MeOH/$CH_2Cl_2$ + 4% $NH_4OH$) to give 10.4 g of the title compound as a racemate. Mass Spec.: $MH^+$ = 469 (FAB). partial $^1H$ NMR ($CDCl_3$, 400 MHz): 8.38 (s, 1H); 7.57 (s, 1H); 7.27 (d, 1H); 7.06 (d, 1H); 3.95 (d, 1H).

Step B - Separation of Enantiomers:

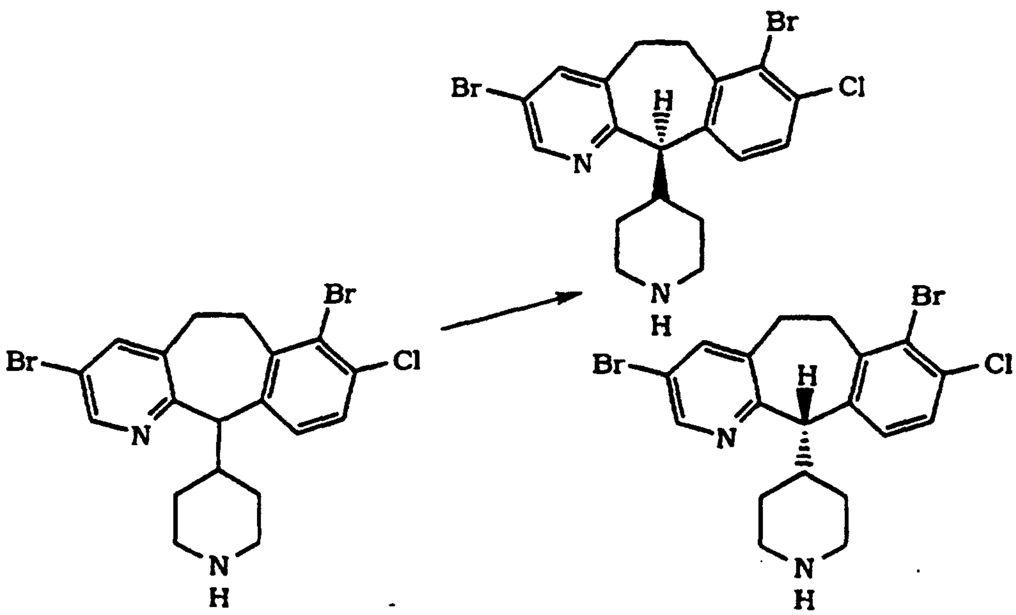

The racemic title compound of Step A is separated by preparative chiral chromatography (Chiralpack AD, 5 cm X 50 cm column, using 5% iPrOH/hexane + 0.2% diethylamine), to give the (+)-isomer and the (-)-isomer of the title compound.

Physical chemical data for (+)-isomer: Mass Spec. $MH^+$ = 469 (FAB); $[\alpha]_D^{25}$ = +43.5° (c=0.402. EtOH); partial $^1H$ NMR ($CDCl_3$, 400 MHz): 8.38 (s, 1H); 7.57 (s, 1H); 7.27 (d, 1H); 7.05 (d, 1H); 3.95 (d, 1H).

Physical chemical data for (-)-isomer: Mass Spec. $MH^+$ = 469 (FAB); $[\alpha]_D^{25}$ = -41.8° (c=0.328 EtOH); partial $^1H$ NMR ($CDCl_3$, 400 MHz): 8.38 (s, 1H); 7.57 (s, 1H); 7.27 (d, 1H); 7.05 (d, 1H); 3.95 (d, 1H).

Step C:

Following the procedure of Preparative Example 9, Step H, the racemic compound, the (+)-isomer or the (-)-isomer of the title compound of Preparative Example 10 can be obtained.

PREPARATIVE EXAMPLE 11*

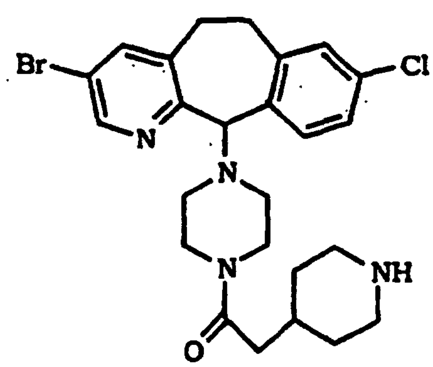

[racemic as well as R-(+)- and S-(-)-isomers]

The compound

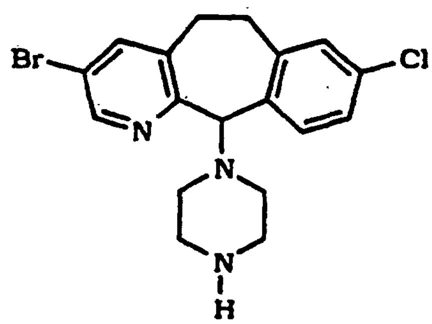

is prepared according to the procedures of Preparative Example 40 of WO 95/10516 (published April 20, 1995), by following the procedures described in Example 193 of WO 95/10516.

The (+)- and (-)-isomers can be separated by following essentially the same procedure as Step D of Preparative Example 6.

Physical chemical data for the R-(+)-isomer: $^{13}C$ NMR ($CDCl_3$): 155.8 (C); 146.4 (CH); 140.5 (CH); 140.2 (C); 136.2 (C); 135.3 (C); 133.4 (C); 132.0 (CH); 129.9 (CH); 125.6 (CH); 119.3 (C); 79.1 (CH); 52.3 ($CH_2$); 52.3 (CH); 45.6 ($CH_2$); 45.6 ($CH_2$); 30.0 ($CH_2$); 29.8 (CH2). $[\alpha]_D^{25}$ = +25.8° (8.46 mg/2 mL MeOH).

Physical chemical data for the S-(-)-isomer: $^{13}C$ NMR ($CDCl_3$): 155.9 (C); 146.4 (CH); 140.5 (CH); 140.2 (C); 136.2 (C); 135.3 (C); 133.3 (C); 132.0 (CH); 129.9 (CH); 125.5 (CH); 119.2 (C); 79.1 (CH); 52.5 ($CH_2$); 52.5 (CH); 45.7 ($CH_2$); 45.7 ($CH_2$); 30.0 ($CH_2$); 29.8 ($CH_2$), $[\alpha]_D^{25}$ = -27.9° (8.90 mg/2 mL MeOH).

Following essentially the same procedure as Preparative Example 5. Steps C and D, the racemic compound, (+)-isomer or (-)-isomer of the title compound of Preparative Example 11 can be obtained from the corresponding racemic compound, (+)-isomer or (-)-isomer of the compound

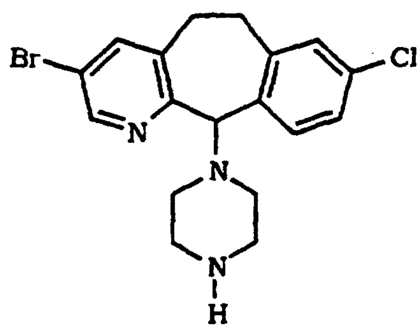

.

PREPARATIVE EXAMPLE 12

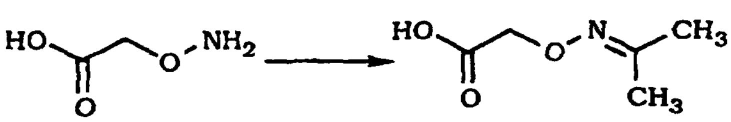

Follow a procedure outlined in Collect. Czech. Chem. Comm. (1990) 55, 2086. Dissolve 0.2 g (0.915 mmol) of (aminooxy)acetic acid hemihydrochloride and 0.2 g (3 mmol) of acetone in 2 mL of pyridine and allow to stand for 18 hr. Concentrate under vacuum and partition the residue between ethyl acetate and 1 N Hcl. Dry the organic layer over magnesium sulfate and concentrate under vacuum to give a white solid mp = 77.3-78°C.

PREPARATIVE EXAMPLE 13

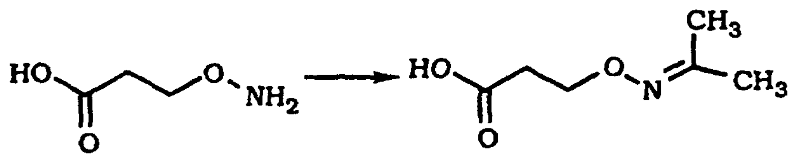

Follow the procedure of Preparative Example 12 but use 2-aminooxypropionic acid hemihydrochloride instead of (aminooxy)acetic acid to obtain the product as a colorless oil.

PREPARATIVE EXAMPLE 14

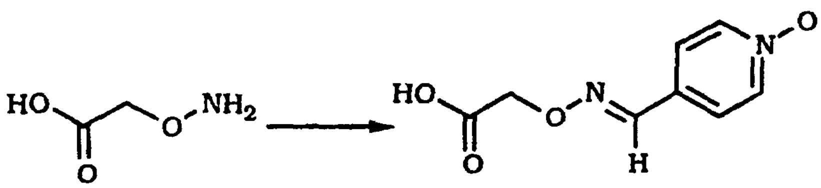

Follow the procedure of Preparative Example 12 but use 4-pyridinecarboxaldehyde N-oxide instead of acetone to obtain the product that was recrystallized from water to give a white solid mp = 227-228°C.

PREPARATIVE EXAMPLE 15

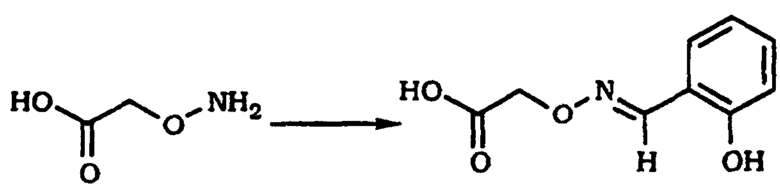

Follow the procedure of Preparative Example 12 but use 2-hydroxybenzaldehyde instead of acetone to obtain the product as a white solid mp = 152-153.5°C.

EXAMPLE 1

(28.0)

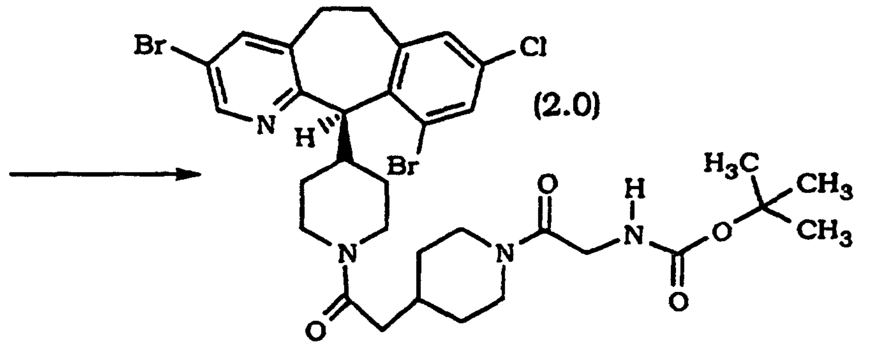

(2.0)

The compound of Formula 28.0

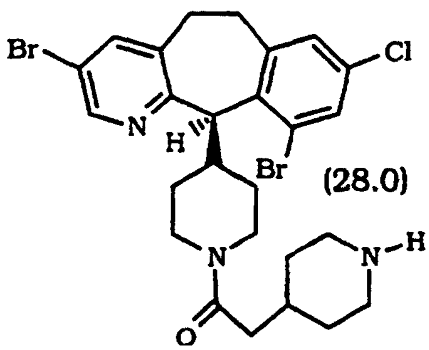

(28.0)

(Preparative Example 8) (0.149 g, 0.25 mmol) was combined with 1-hydroxybenzotriazole hydrate (0.067 g, 0.5 mmol), 1-(3-dimethylaminopropyl)-3-ethylcarbodiimide hydrochloride (0.096 g, 0.5 mmol), N-BOC-glycine (0.087 g, 0.5 mmol) and anhydrous dimethylformamide (5 mL) and the resulting mixture was stirred at room temperature under nitrogen overnight. Concentration in vacuo provided an oil which was diluted with dichloromethane, washed with 1*M* hydrochloric acid and 1 *M* aqueous sodium hydroxide, then dried over anhydrous magnesium sulfate. Filtration and concentration *in vacuo* afforded the compound of Formula 2.0 (+-isomer)(0.16 g, 85%, mp 116 - 123 °C).

EXAMPLE 2

(2.0)

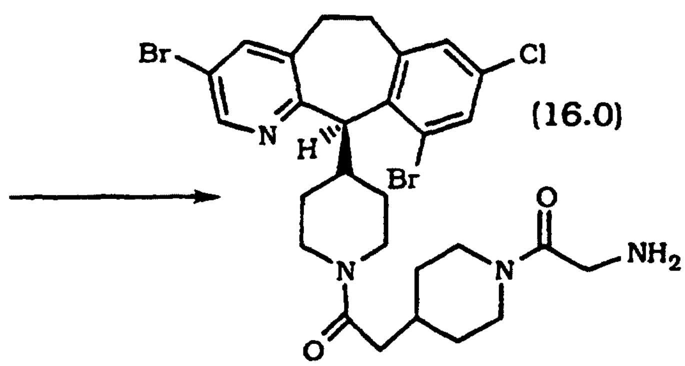

(16.0)

To the compound of Formula 2.0 (Example 1) (0.145 g) dissolved in anhydrous dichloromethane (10 mL) was added trifluoroacetic acid (2 mL) and the resulting solution was stirred at room temperature for 1 hour. 50% Aqueous

sodium hydroxide was added slowly followed by dichloromethane and brine. The mixture was shaken well, the organic phase was separated and dried over anhydrous magnesium sulfate. Filtration and concentration *in vacuo* afforded the compound of Formula 16.0 (+-isomer) (0.086 g, 68%, mp 131 - 138°C).

EXAMPLE 3

(28.0)

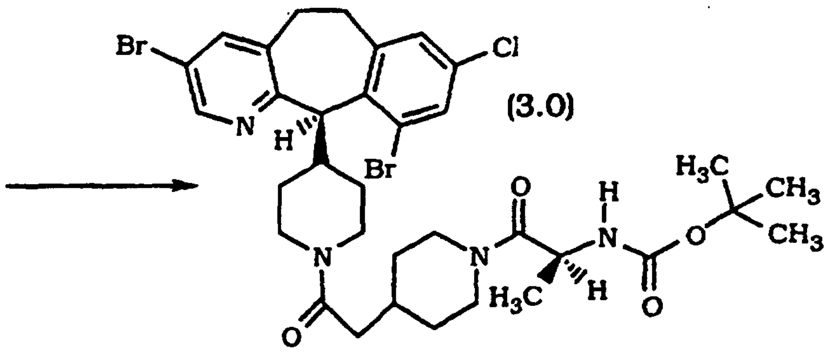

(3.0)

The compound of Formula 28.0 (Preparative Example 8) (0.10 g, 0.17 mmol) was combined with 1-hydroxybenzotriazole hydrate (0.045 g, 0.34 mmol), 1-(3-dimethylaminopropyl)-3-ethylcarbodiimide hydrochloride (0.064 g, 0.34 mmol), N-tert-butoxycarbonyl-L-alanine (0.064 g, 0.34 mmol) and anhydrous dimethylformamide (10 mL) and the resulting mixture was stirred at room temperature under nitrogen overnight. Concentration *in vacuo* provided an oil which was diluted with dichloromethane, washed with 1*M* hydrochloric acid and 1 *M* aqueous sodium hydroxide, then dried over anhydrous magnesium sulfate. Filtration and concentration *in vacuo* afforded the compound of Formula 3.0 (+-isomer) (0.095 g, 74%, mp 135 - 142 °C).

EXAMPLE 4

(3.0)

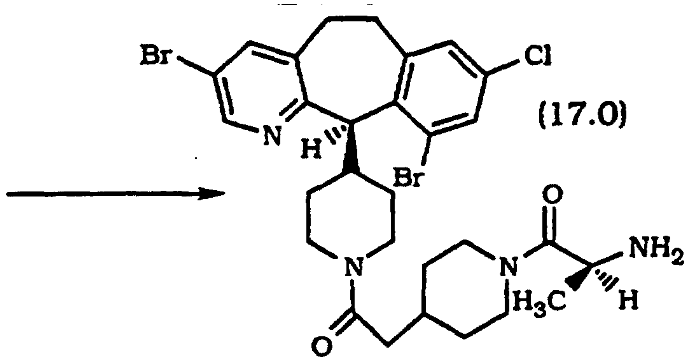

(17.0)

To the compound of Formula 3.0 (Example 3) (0.09 g) dissolved in anhydrous dichloromethane (10 mL) was added trifluoroacetic acid (1 mL) and the resulting solution was stirred at room temperature for 1 hour. 50% Aqueous sodium hydroxide was added slowly followed by dichloromethane and brine. The mixture was shaken well, the organic phase was separated and dried over anhydrous magnesium sulfate. Filtration and concentration *in vacuo* afforded the compound of Formula 17.0 (+-isomer) (0.053 g, 68%, mp 122.7 - 128°C).

EXAMPLE 5

(28.0)

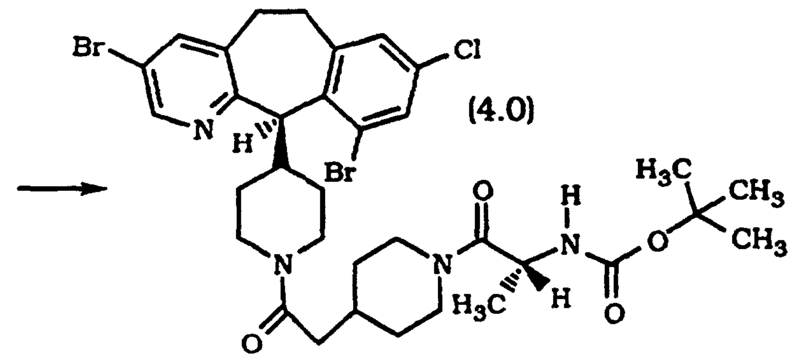

(4.0)

The compound of Formula 28.0 (Preparative Example 8) (0.10 g, 0.17 mmol) was combined with 1-hydroxybenzotriazole hydrate (0.045 g, 0.34 mmol), 1-(3-dimethylaminopropyl)-3-ethylcarbo-diimide hydrochloride (0.064 g, 0.34 mmol), N-tert-butoxy-carbonyl-D-alanine (0.064 g, 0.34 mmol) and anhydrous dimethylformamide (10 mL) and the resulting mixture was stirred at room temperature under nitrogen overnight. Concentration *in vacuo* provided an oil which was diluted with dichloromethane, washed with 1*M* hydrochloric acid and 1 *M* aqueous sodium hydroxide, then dried over anhydrous magnesium sulfate. Filtration and concentration in vacuo afforded the compound of Formula 4.0 (+-isomer) (0.104 g, 81%, mp 135.1 - 142.3 °C).

EXAMPLE 6

(4.0)

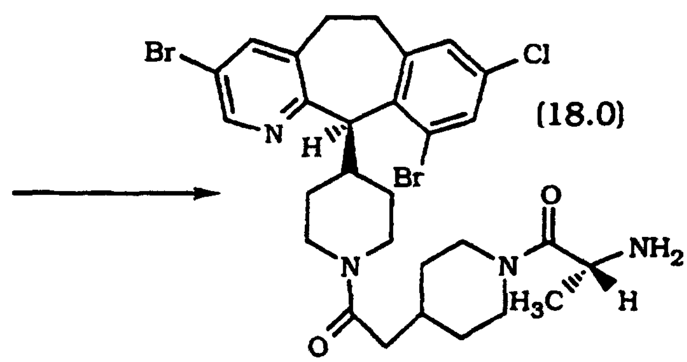

(18.0)

To the compound of Formula 4.0 (Example 5) (0.10 g) dissolved in anhydrous dichloromethane (10 mL) was added trifluoroacetic acid (1 mL) and the resulting solution was stirred at room temperature for 1 hour. 50% Aqueous sodium hydroxide was added slowly followed by dichloromethane and brine. The mixture was shaken well, the organic phase was separated and dried over anhydrous magnesium sulfate. Filtration and concentration *in vacuo* afforded the compound of formula 18.0 (+-isomer) (0.056 g, 64%, mp 103°C (DEC)).

EXAMPLE 7

(28.0)

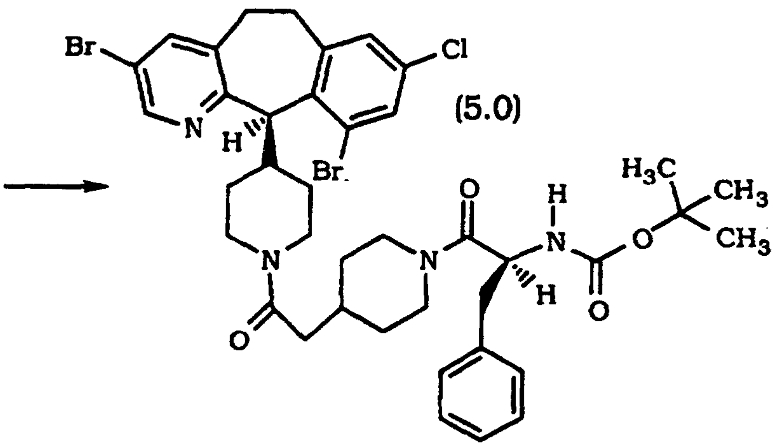

(5.0)

The compound of Formula 5.0 (+-isomer) was prepared, according to procedures similar to those of Examples 1, 3, and 5, by reacting the compound of Formula 28.0 (Preparative Example 8) with the amino acid N-tert-butoxycarbonyl-L-phenylalanine. Yield: 76%, mp: 128.6-134°C.

EXAMPLE 8

(28.0)

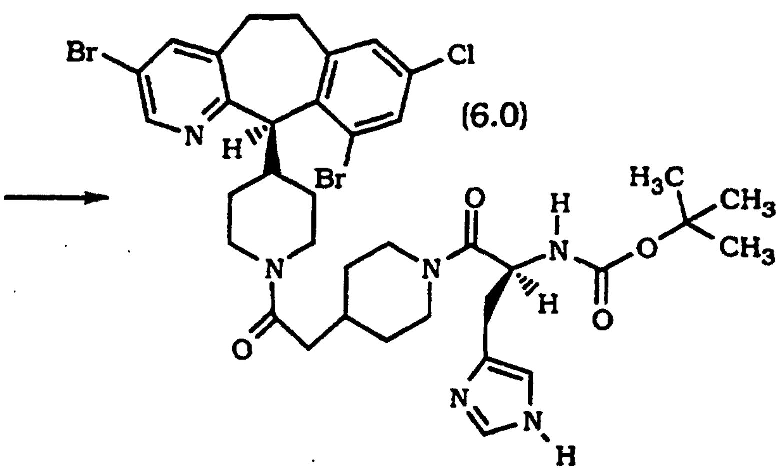

(6.0)

The compound of Formula 6.0 (+-isomer) was prepared, according to procedures similar to those of Examples 1, 3, and 5, by reacting the compound of Formula 28.0 (Preparative Example 8) with the amino acid N-(alpha)-tert-butoxycarbonyl-L-histidine. Yield: 32%, mp: 96.0-99.7°C.

EXAMPLE 9

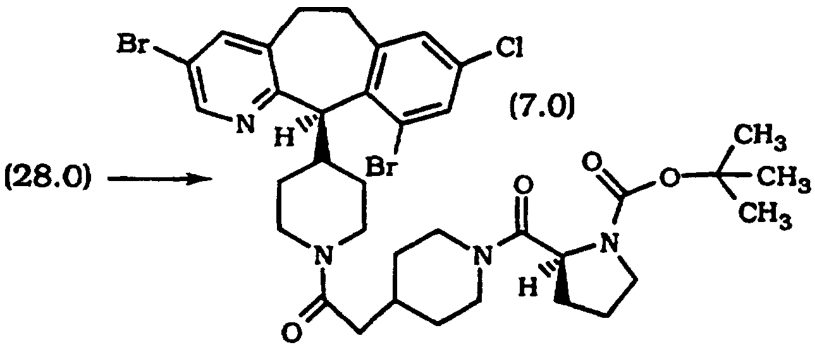

(28.0)

(7.0)

The compound of Formula 7.0 (+-isomer) was prepared, according to procedures similar to those of Examples 1, 3, and 5, by reacting the compound of Formula 28.0 (Preparative Example 8) with the amino acid N-(alpha)-tert-butoxycarbonyl-L-proline. Yield: 52%, mp: 110°C.

EXAMPLE 10

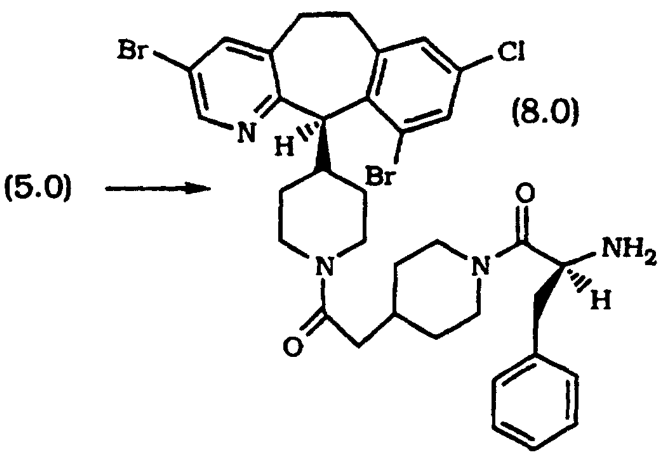

(5.0)

(8.0)

The compound of Formula 8.0 (+-isomer) was prepared, according to procedures similar to those of Examples 2, 4, and 6, from the compound of Formula 5.0 (Example 7). Yield: 70%, mp: 116-119°C.

EXAMPLE 11

(6.0)

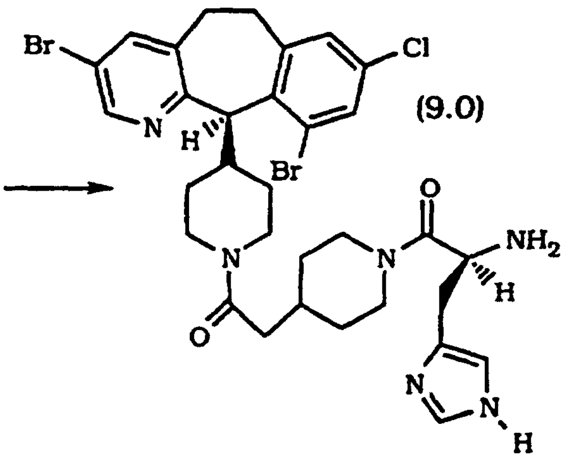

(9.0)

The compound of Formula 9.0 (+-isomer) was prepared, according to procedures similar to those of Examples 2, 4, and 6, from the compound of Formula 6.0 (Example 8). Yield: 51%, mp: 101°C.

EXAMPLE 12

(7.0)

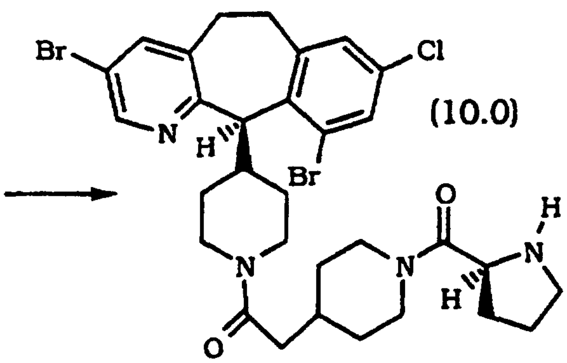

(10.0)

The compound of Formula 10.0 (+-isomer) was prepared, according to procedures similar to those of Examples 2, 4, and 6, from the compound of Formula 7.0 (Example 9). Yield: 46%, mp: 131.6°C.

EXAMPLE 13

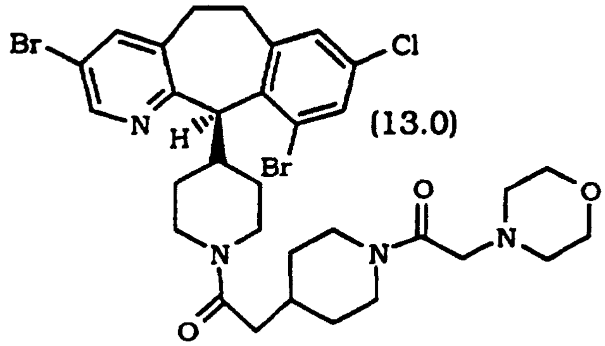

(13.0)

STEP A:

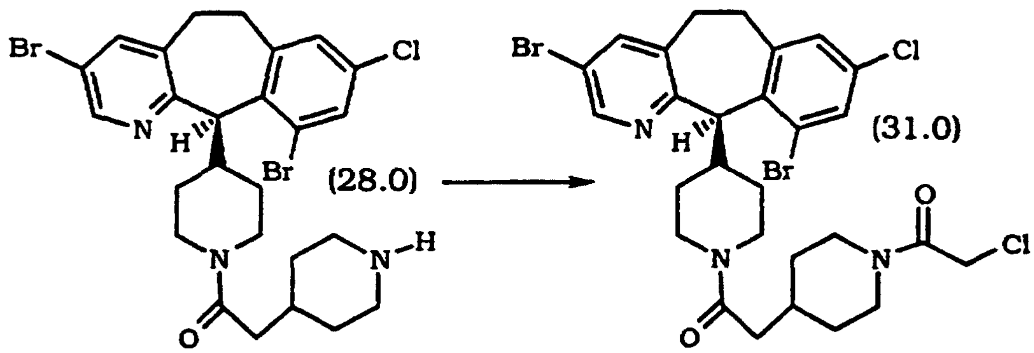

(28.0)

(31.0)

To the compound of Formula 28.0 (Preparative Example 8) (0.51 g, 0.85 mmol) and triethylamine (0.18 mL, 1.3 mmol) dissolved in anhydrous dichloromethane (50 mL) was added $ClCH_2C(O)Cl$ (chloroacetyl chloride) (0.28 mL, 1.2 eq) dissolved in dichloromethane (10 mL) at 0°C. After stirring for 1.5 hours, 1 *M* hydrochloric acid was added and the mixture was shaken. The organic phase was separated and washed with 1 *N* aqueous sodium hydroxide, then brine, and dried over anhydrous magnesium sulfate. Filtration and concentration *in vacuo* afforded the compound of Formula 31.0 (0.58 g, 100%, mp 124.0-134.5°C).

STEP B

(31.0)

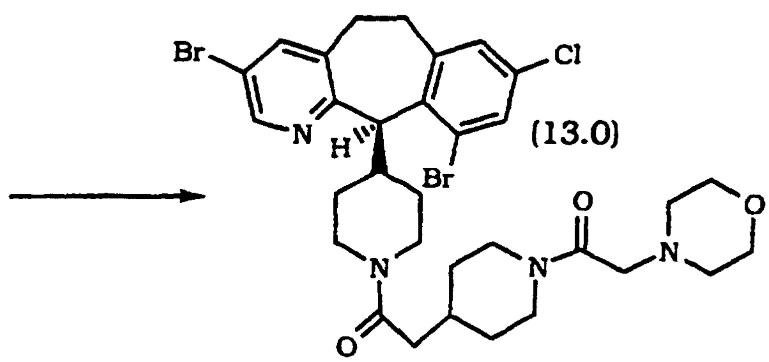

(13.0)

The compound of Formula 31.0 (0.12 g, 0.18 mmol), morpholine (5 mL) and anhydrous sodium carbonate (0.038 g, 2 eq) were stirred at 130°C overnight. After concentration *in vacuo*, the residue was diluted with dichloromethane, washed with water and dried over anhydrous magnesium sulfate. Filtration and concentration in vacuo afforded a yellow residue (0.17 g) which was purified by preparative plate chromatography (silica gel) using 5% methanol-dichloromethane and concentrated ammonium hydroxide to provide the compound of Formula 13.0 (0.096 g, 75%, mp 116.6°C).

EXAMPLE 14

(31.0)

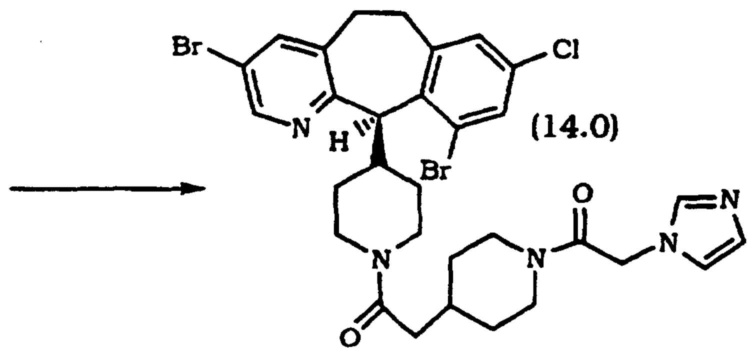

(14.0)

The compound of Formula 31.0 (Example 13) (0.12 g, 0.18 mmol), anhydrous dimethylformamide (10 mL), imidazole (0.037 g, 0.54 mmol) and anhydrous sodium carbonate (0.057 g, 0.54 mmol) were stirred at 130°C overnight. The mixture was cooled to room temperature, diluted with water, filtered and the solids washed with water. The solids were diluted with dichloromethane, washed with water and then with 1 N aqueous sodium hydroxide. The organic phase was separated, dried over anhydrous magnesium sulfate, filtered and concentrated *in vacuo* to afford a solid (0.084 g) which was purified by preparative plate chromatography (silica gel) using 5% methanol-dichloromethane and concentrated ammonium hydroxide to provide the compound of Formula 14.0 (0.06 g, 48%, mp 148.9°C).

EXAMPLE 15

(28.0)

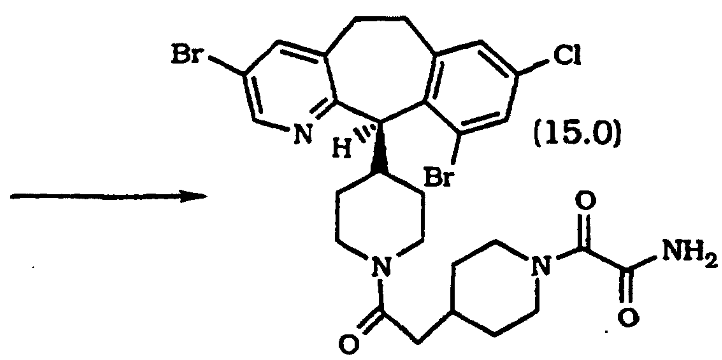

(15.0)

The compound of Formula 28.0 (Preparative Example 8) (0.21 g, 0.34 mmol) dissloved in anhydrous dichloromethane (10 mL) was added to a dichloromethane solution (10 mL) of oxallyl chloride (1.0 mL) and pyridine ( 0.08 mL, 3 eq) at 0°C. After stirring the resulting solution for 5 min, concentrated ammonium hydroxide was added and the mixture was allowed to stir overnight. The mixture was diluted with dichloromethane and water, shaken and then the phases were separated. The organic phase was washed with brine, then with 1 M hydrochloric acid, 1 N aqueous sodium hydroxide and brine. The organic phase was separated, dried over anhydrous magnesium sulfate, filtered and concentrated *in vacuo* to afford a solid (0.17 g) which was purified by preparative plate chromatography (silica gel) using 5% methanol-dichloromethane and concentrated ammonium hydroxide to provide the compound of Formula 15.0 (0.086 g, 37%, mp 152.8°C).

EXAMPLE 16

(16.0)

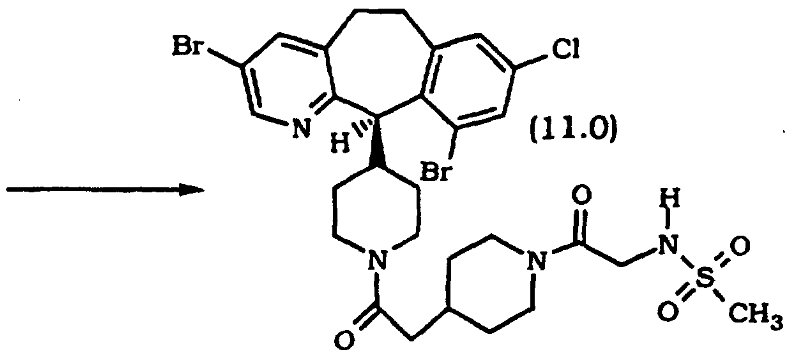

(11.0)

To the compound of Formula 16.0 (Example 2) (0.10 g) dissolved in anhydrous dichloromethane (10 mL) was added triethylamine (0.032 mL, 1.5 eq)) and methanesulfonyl chloride (0.014 mL, 1.2 eq) and the resulting solution was stirred at room temperature overnight. The solution was diluted with dichloromethane and washed with 1M hydrochloric acid and then with 1 *M* aqueous sodium hydroxide. The organic phase was separated and dried over anhydrous magnesium sulfate. Filtration and concentration *in vacuo* afforded the compound of Formula 11.0 (0.099 g, 89%, mp 116°C).

EXAMPLE 17

(16.0)

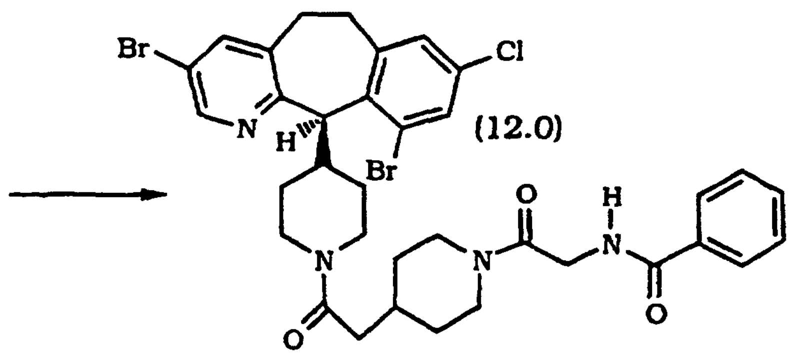

(12.0)

To the compound of Formula 16.0 (Example 2) (0.07 g) dissolved in anhydrous dichloromethane (10 mL) was added triethylamine (0.022 mL, 1.5 eq)) and benzoyl chloride (0.014 mL, 1.2 eq) and the resulting solution was stirred at room temperature overnight. The solution was diluted with dichloromethane and washed with 1*M* hydrochloric acid and then with 1 *M* aqueous sodium hydroxide. The organic phase was separated and dried over anhydrous magnesium sulfate. Filtration and concentration *in vacuo* afforded the compound of formula 12.0 (0.066 g, 85%, mp 117.2°C).

EXAMPLE 18

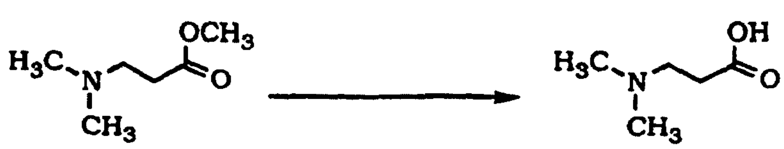

Dissolve 2 g (15 mmol) of methyl 3-(dimethyl amino) propionate in 20 mL of EtOH and then add 20 mL of 1M

LiOH. Stir the reaction mixture at room temperature for 16 h. Strip off the solvents. Dissolve the resulting material in water and adjust pH to ~ 6. Concentrate the reaction mixture to give the product. Mass Spec.: $MH^+$ = 118.

EXAMPLE 19

(+)-4-[3,10-DIBROMO-8-CHLORO-6,11-DIHYDRO-5H-BENZO[5,6]CYCLOHEPTA[1,2-b]PYRIDIN-11-YL]-1-[4-(DIMETHYLAMINO)-1-OXOBUTYL]-4-PIPERIDINYL]ACETYL]-PIPERIDINE

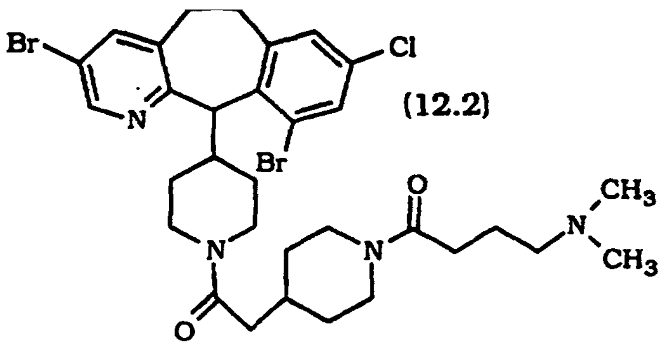

(12.2)

Dissolve 0.1 g (0.23 mmol) of the product of Preparative Example 8

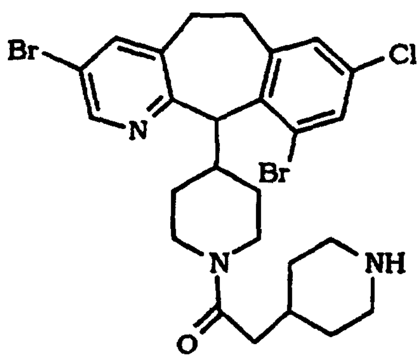

in 8 mL DMF, add 0.04 g (0.22 mmol) 4-(dimethylamino) butyric acid hydrochloride, 0.04 g (0.22 mmol) of DEC, 0.03 g (0.22 mmol) of HOBT and 0.1 mL of N-methyl morpholine at about 0 to about 4°C. Stir the reaction mixture overnight letting it warm to room temperature. Remove all the volatiles and then partition between $H_2O$-$CH_2Cl_2$. Extract the aqueous phase with $CH_2Cl_2$. Combine the $CH_2Cl_2$ fractions and dry over $MgSO_4$ and concentrate. Purify by flash chromatography, first eluting with 5% MeOH-($NH_3$)-$CH_2Cl_2$ and then 10% MeOH-($NH_3$)-$CH_2Cl_2$ to obtain the compound of Formula 12.2. Mass Spec. MH+ = 709, mp = 69-71 °C.

EXAMPLE 20

(+)-4-(3,10-DIBROMO-8-CHLORO-6,11-DIHYDRO-5H-BENZO[5,6]CYCLOHEPTA[1,2-b]PYRIDIN-11-YL)-1-[4-(DIMETHYLAMINO)-1-OXOPROPYL]-4-PIPERIDINYL]ACETYL]-PIPERIDINE

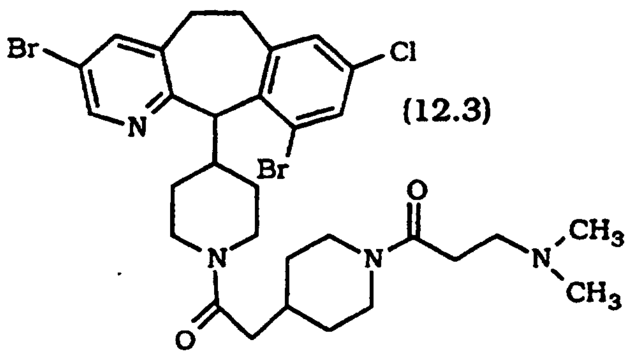

(12.3)

By following essentially the same procedure as described in Example 19 above, but using 3-(dimethylamino) propionic acid (Example 18) instead of 4-(dimethylamino) butyric acid hydrochloride, the compound of Formula 12.3 was prepared. FAB-MS - MH+ = 695, mp = 82-84°C.

EXAMPLE 21

(+)-4-(3,10-DIBROMO-8-CHLORO-6,11-DIHYDRO-5H-BENZO[5,6]CYCLOHEPTA[1,2-b]PYRIDIN-11-YL)-1-[4-(DIMETHYLAMINO)-1-OXOETHYL]-4-PIPERIDINYL]ACETYL]-PIPERIDINE

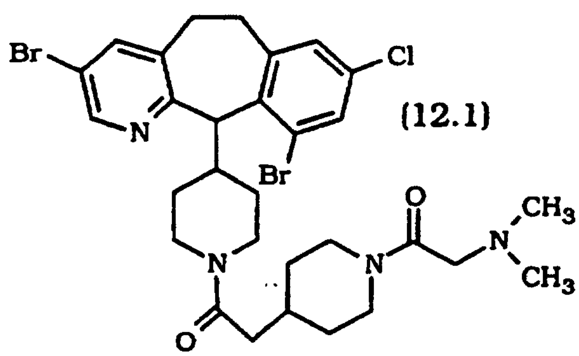

(12.1)

By following essentially the same procedure as described in Example 19 above, but using N,N-dimethyl glycine instead of 4-(dfmethylamino) butyric acid hydrochloride, the compound of Fromula 12.1 was prepared. FAB-MS - MH+ = 681, mp = 123-124°C.

EXAMPLE 22

(+)-4-(3,10-DIBROMO-8-CHLORO-6,11-DIHYDRO-5H-BENZO[5,6]CYCLOHEPTA[1,2-b]PYRIDIN-11-YL)-1-[4-(PIPERIDINYL)-1-OXOETHYL]-4-PIPERIDINYL] ACETYL]-PIPERIDINE

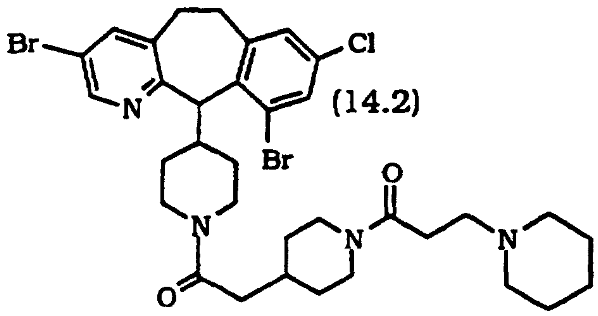

(14.2)

By following essentially the same procedure as described in Example 19 above, but using 1-piperidine propionic acid instead of 4-(dimethylamino) butyric acid hydrochloride, the compound of Formula 14.2 was prepared. FAB-MS: $MH^+$ 735, mp=127-128°C.

EXAMPLE 23

(+)-4-[3,10-DIBROMO-8-CHLORO-6,11-DIHYDRO-5H-BENZO[5,6]CYCLOHEPTA[1,2-b]PYRIDIN-11-YL)-1-[4-[TETRAHYDRO-2H-1,4-THIAZIN-4-YL)-1-OXOETHYL 1-1-DIOXIDE]-4-PIPERIDINYL]ACETYL]PIPERIDINE

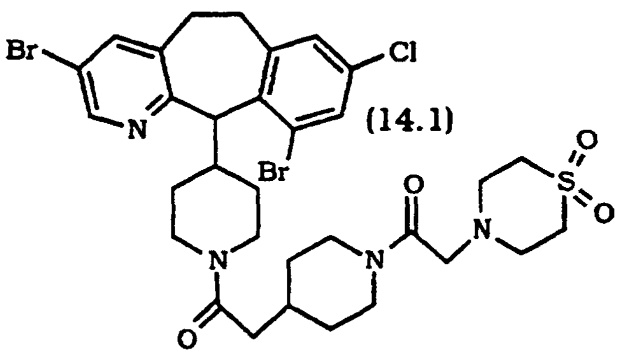

(14.1)

By following essentially the same procedure as described in Example 19 above, but using thiomorpholine S-dioxide acetic acid instead of 4-(dimethylamino) butyric acid hydrochloride, the compound of Formula 14.1 was prepared. mp = 140-141°C.

EXAMPLE 24

(+)- METHYL-4-[2-[4-[(3,10-DIBROMO-8-CHLORO-6,11-DIHYDRO-5H-BENZO[5,6]CYCLOHEPTA[1,2-b]PYRIDIN-11-YL)-1-PIPERIDINYL]-2-OXOETHYL]-DELTA-OXO-1- PIPERIDINE-PENTANOATE

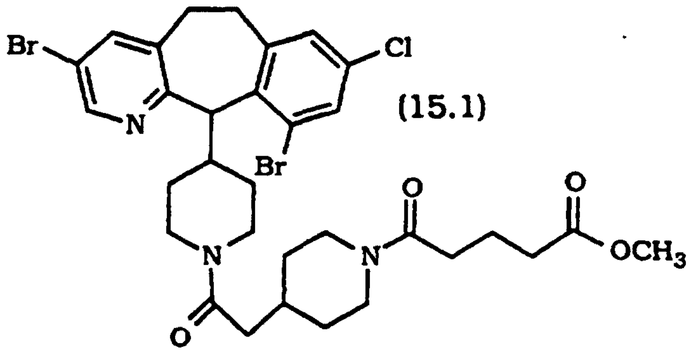

(15.1)

By following essentially the same procedure as described in Example 19 above, but using monomethyl glutarate instead of 4-(dimethylamino) butyric acid hydrochloride, the compound of Formula 15.1 was prepared. FAB-MS - MH+ = 724, mp = 101-102°C.

EXAMPLE 25

(+)- METHYL-4-[2-[4-[(3,10-DIBROMO-8-CHLORO-6,11-DIHYDRO-5H-BENZO[5,6]CYCLOHEPTA[1,2-b]PYRIDIN-11-YL)-1-PIPERIDINYL]-2-OXOETHYL]-GAMMA-OXO-1- PIPERIDINE-BUTANOATE

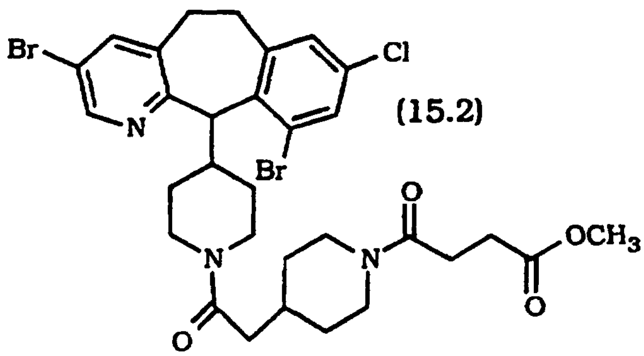

(15.2)

By following essentially the same procedure as described in Example 19 above, but using monomethyl succinate instead of 4-(dimethylamino) butyric acid hydrochloride, the compound of Formula 15.2 was prepared. FAB-MS - MH+ = 710, mp = 114-115 °C.

EXAMPLE 26

(+)- ETHYL-4-[2-[4-[(3,10-DIBROMO-8-CHLORO-6,11-DIHYDRO-5H-BENZO[5,6]CYCLOHEPTA[1,2-b]PYRIDIN-11-YL)-1-PIPERIDINYL]-2-OXOETHYL]-BETA-OXO-1- PIPERIDINE-BUTANOATE

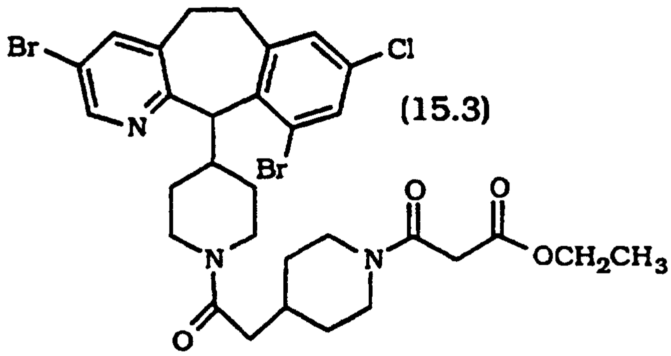

(15.3)

By following essentially the same procedure as Example 19 above, but using monoethyl malonate instead of 4-(dimethylamino) butyric acid hydrochloride, the compound of Formula 15.3 was obtained. FAB-MS - $MH^+$ = 710, m. p. = 77-78°C

EXAMPLE 27

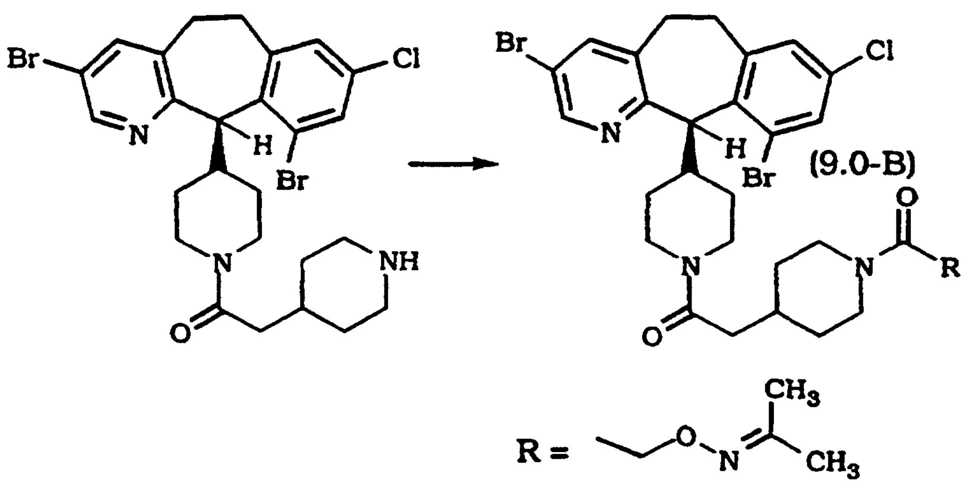

(9.0-B)

Dissolve the (+) product of Preparative Example 8, Step D (0.01 g, 0.017 mmol) in 0.5 mL of DMF, stir at room temperature and add 0.003 g (0.017 mmol) of DEC, 0.002 g (0.017 mmol) of HOBT and 0.003 g (0.017 mmole) of the product of Preparative Example 12. Stir the mixture at room temperature for 18 hr, then concentrate in vacuo to a residue and partition between ethyl acetate and water. Wash the organic phase with aqueous sodium bicarbonate solution then brine. Dry the organic phase over magnesium sulfate, filter and concentrate in vacuo to a residue. Chromatograph the residue on silica gel, eluting with dichloromethane (saturated with ammonia) - methanol (95% - 5%) to yield the product (0.01 g) as a white solid. M.p. = 84°-90°C, Mass Spec.: MH+ = 709.

EXAMPLES 28-60

Follow the procedure of Example 27 but use the acid shown in Table 1 below instead of the product of Preparative Example 12 to obtain the compounds of Formula 1.7

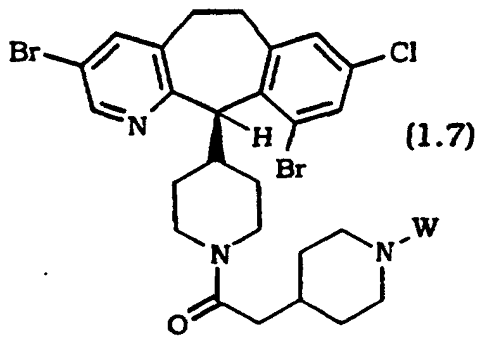

(1.7)

wherein W is defined in Table 1. The Formula number of the compound formed is given in parenthesis below the W substituent.

TABLE 1

| EX. | Acid | W | mp (°C) |
|---|---|---|---|
| 28 | 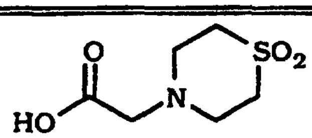 | 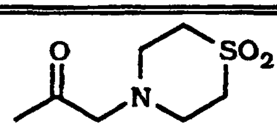 (6.0-B) | 140-140.8 |
| 29 | 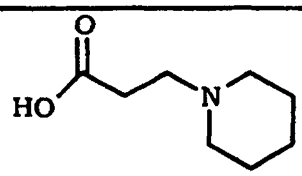 | 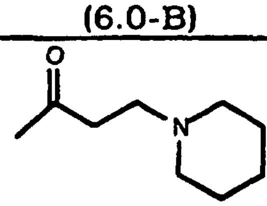 (7.0-B) | 127.2-127.7 |

TABLE 1 - continued

| EX. | Acid | W | mp (°C) |
|---|---|---|---|
| 30 | 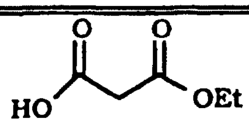 | 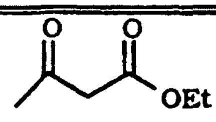 (8.0-B) | 77.2-78.1 |
| 31 | 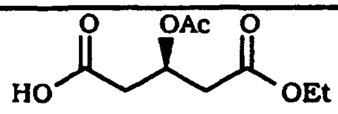 | 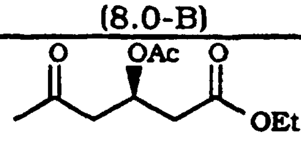 (16.0-B) | 103.5-106.4 |
| 32 | 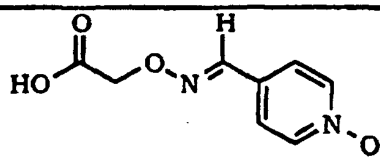 Preparative Ex. 4 | 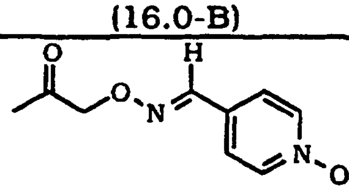 (22.0-B) | 129 (d) |
| 33 | 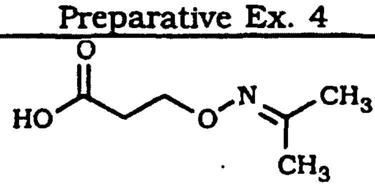 Preparative Ex. 3 | 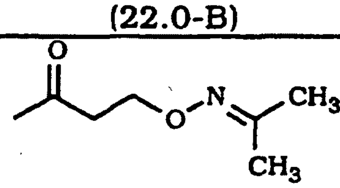 (23.0-B) | 75 |
| 34 | 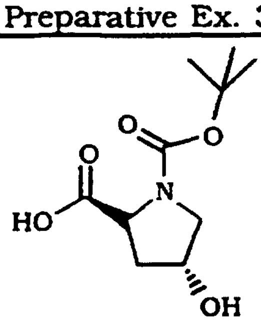 | 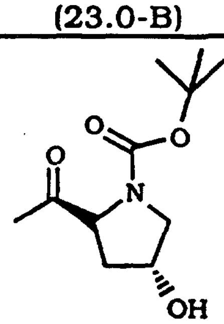 (24.0-B) | 145.8-147.7 |
| 35 | 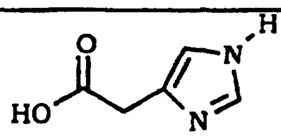 | 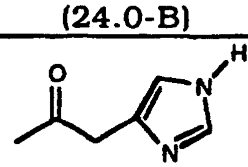 (30.0-B) | 125.8-127.3 |
| 36 | 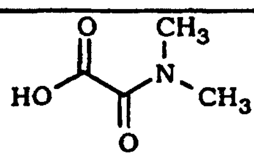 | 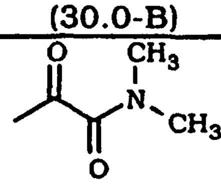 (33.0-B) | 95-143 |
| 37 | 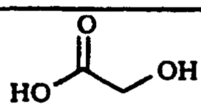 | 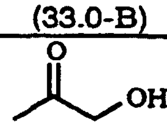 (37.0-B) | ----- |

TABLE 1 - continued

| EX. | Acid | W | mp (°C) |
|---|---|---|---|
| 38 | 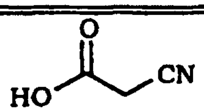 | 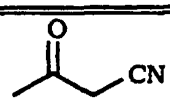 (44.0-B) | 124-125 |
| 39 | 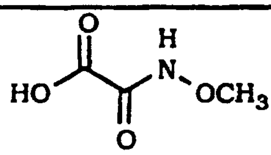 | 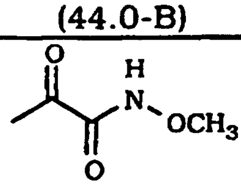 (49.0-B) | 204.5 |
| 40 | 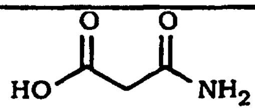 | 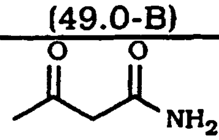 (50.0-B) | 137.4-138 |
| 41 | 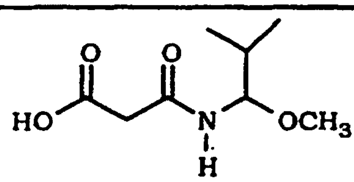 | 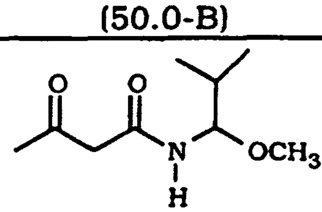 (51.0-B) | 115.8-116.4 |
| 42 | 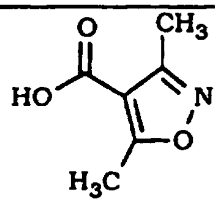 | 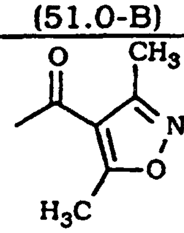 (65.0-B) | ------ |
| 43 | 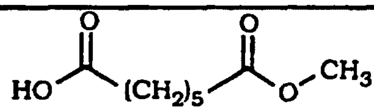 | 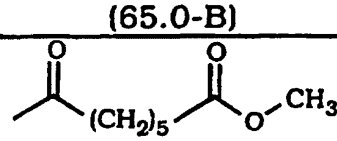 (68.0-B) | ------ |
| 44 | 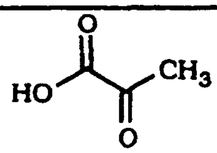 | 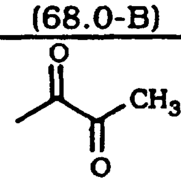 (72.0-B) | 113-120 |
| 45 | 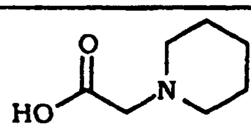 | 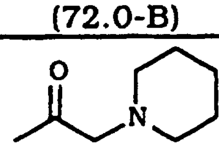 (75.0-B) | 95-100 |

TABLE 1 - continued

| EX. | Acid | W | mp (°C) |
|---|---|---|---|
| 46 | 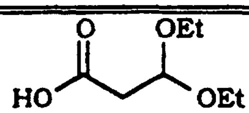 | 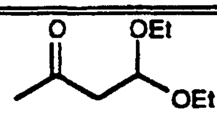 (76.0-B) | 100-108 |
| 47 | 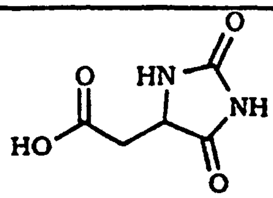 | 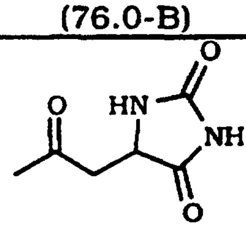 (77.0-B) | 192-203 |
| 48 | 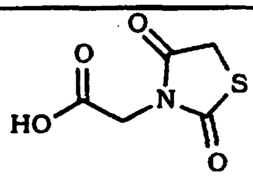 | 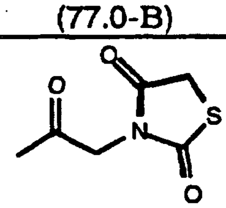 (79.0-B) | 172-190 |
| 49 | 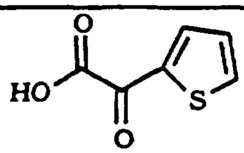 | 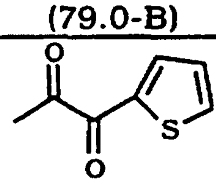 (81.0-B) | 154-163 |
| 50 | 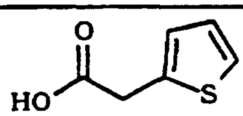 | 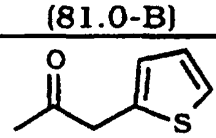 (82.0-B) | 129-139 |
| 51 | 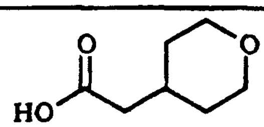 J. Med. Chem. (1993), 36, 2300 | 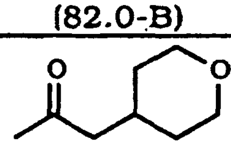 (85.0-B) | 114.5-119.3 |
| 52 | 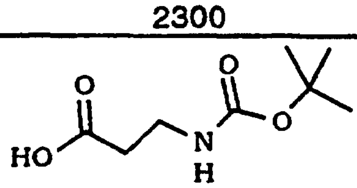 | 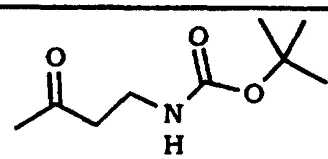 (86.0-B) | 87-88 |
| 53 | 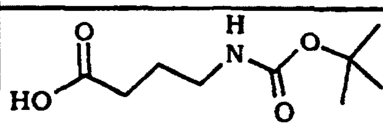 | 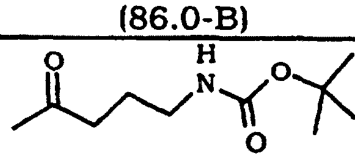 (89.0-B) | 102.7-103.3 |

TABLE 1 - continued

| EX. | Acid | W | mp (°C) |
|---|---|---|---|
| 54 | 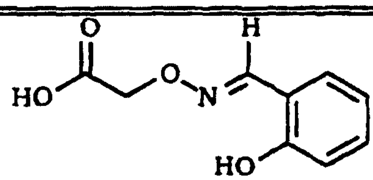 Preparative Ex. 5 | 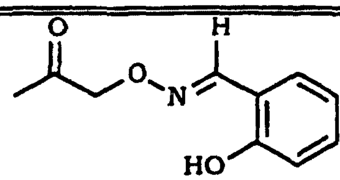 (95.0-B) | 145 (d) |
| 55 | 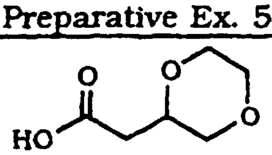 Tetrahedron (1989), 45, 69 | 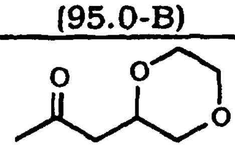 (114.2-B | 129-143 |
| 56 | 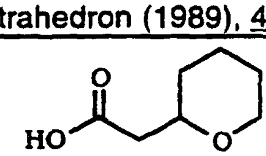 Tetrahedron (1989), 45, 69 | 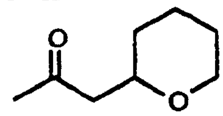 (114.3-B) | 124-132 |
| 57 | 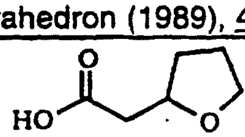 Tetrahedron (1989), 45, 69 | 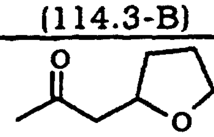 (114.4-B) | 127-136 |
| 58 | 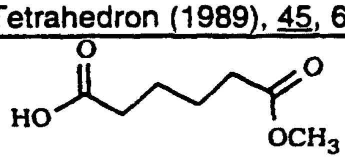 | 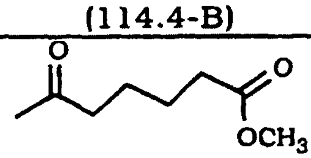 (101.0-B) | 74-75 |
| 59 | 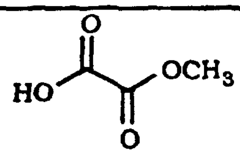 | 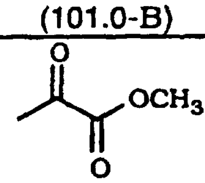 (104.0-B) | ------ |
| 60 | 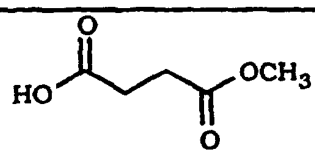 | 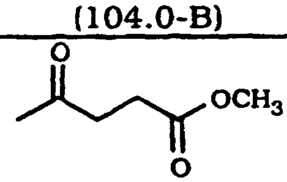 (105.0-B) | 114-115 |
| 61 | 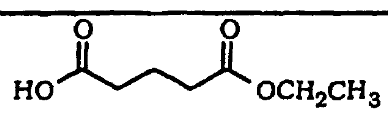 | 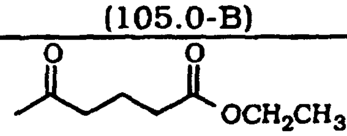 (106.0-B) | 101.1-101.5 |

EXAMPLE 62

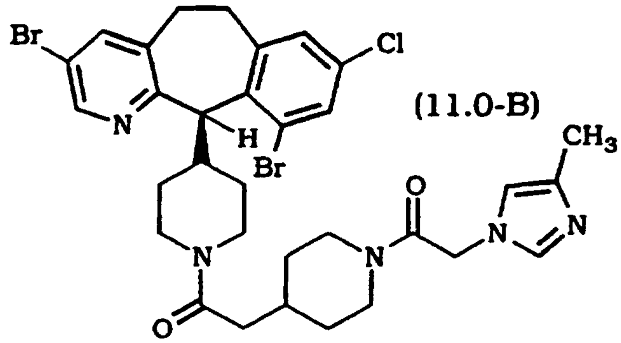

(11.0-B)

Step A

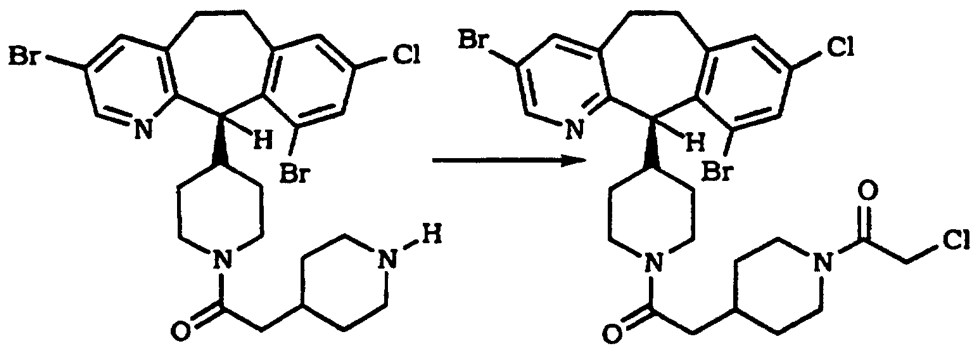

Dissolve the (+) product of Preparative Example 8, Step D (0.744 g, 1.25 mmol) in 20 mL of dichloromethane containing 0.348 mL (2.5 mmol) of triethylamine, stir at room temperature and add 0.1 mL (1.26 mmol) of chloroacetylchloride. Stir for 10 hr then add 20 mL of 1N HCl. Wash the organic layer with aqueous sodium bicarbonate, dry over magnesium sulfate, and concentrate under vacuum to give 0.71 g of the product.

Step B

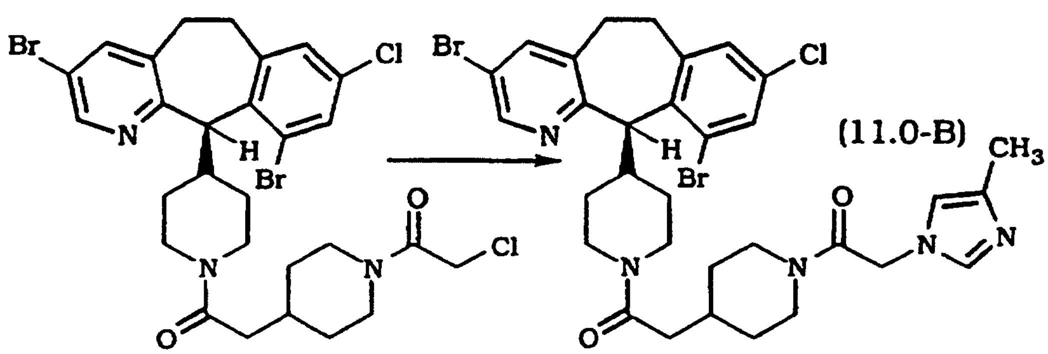

(11.0-B)

Dissolve 0.120 g (0.78 mmol) of the product from Step A, 0.0365 g (0.535 mmol) of 4-methylimidazole and 0.057 g (0.535 mmol) of sodium carbonate in 10 mL of DMF and stir at 120-130°C for 18 hr. Cool to 25°C and 30 mL

of water and filter the precipitated solid. Dissolve the solid in 50 mL of dichloromethane and wash with 1N NaOH. Dry the organic layer over magnesium sulfate and concentrate under vacuum. Chromatograph the residue on a silica gel TLC plate using methanol-dichloromethane saturated with ammonia (5-95) to give 0.06 g of the product as a white solid mp = 148.9°C.

EXAMPLES 63-75

Follow the procedure of Example 62, but use the amine shown in Table 2 below instead of 4-methylimidazole, to obtain the compounds of Formula 1.7

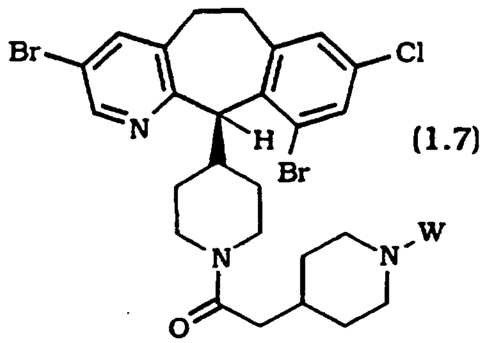

(1.7)

wherein W is defined in Table 2. The Formula number of the compound formed is given in parenthesis below the W substituent.

TABLE 2

| EX. | Amine | W | mp (°C) |
|---|---|---|---|
| 63 | 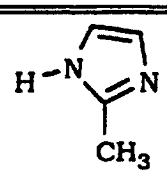 | 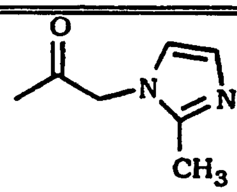 (10.0-B) | 137.8 |
| 64 | 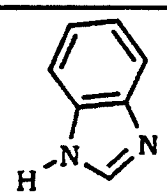 | 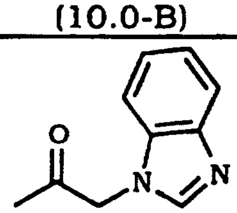 (12.0-B) | 151.3 |

TABLE 2 - continued

| EX. | Amine | W | mp (°C) |
|---|---|---|---|
| 65 | 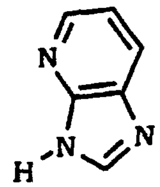 | 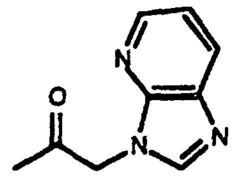 (13.0-B) | 154.2 |
| 66 | 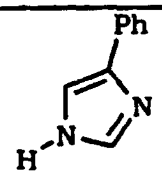 | 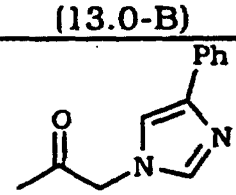 (20.0-B) | 150.3 (d) |
| 67 | 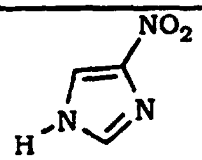 | 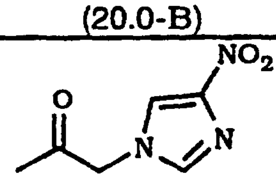 (26.0-B) | 168.1 |
| 68 | 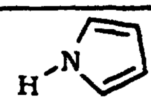 | 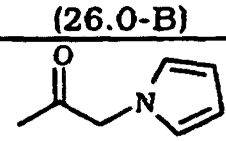 (34.0-B) | 123.4 |
| 69 | 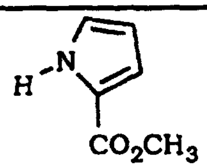 | 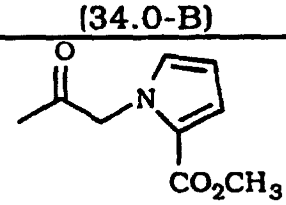 (35.0-B) | 184-190 |
| 70 | 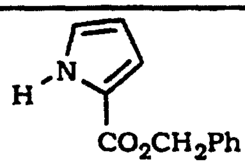 | 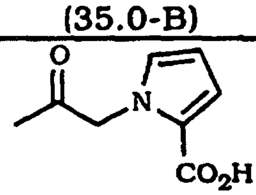 (52.0-B) | 178.6 |
| 71 | 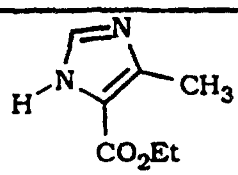 | 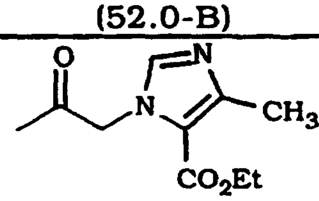 (53.0-B) | 156.1 |

TABLE 2 - continued

| EX. | Amine | W | mp (°C) |
|---|---|---|---|
| 72 | 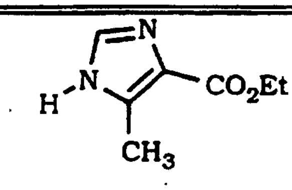 | 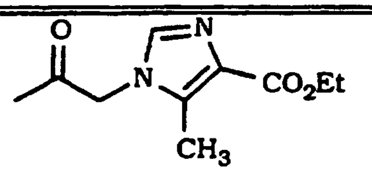 (54.0-B) | 158.7 |
| 73 | 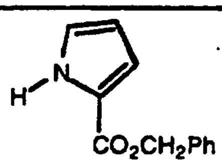 | 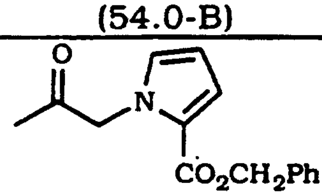 (55.0-B) | 130.4 |
| 74 | 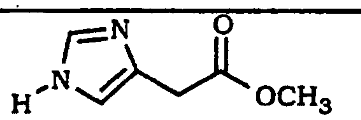 | 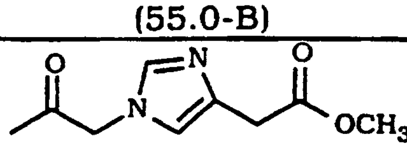 (56.0-B) | 122.8 |
| 75 | 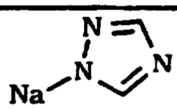 | 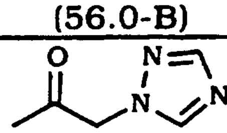 (57.0-B) | 125.3 |

EXAMPLE 76

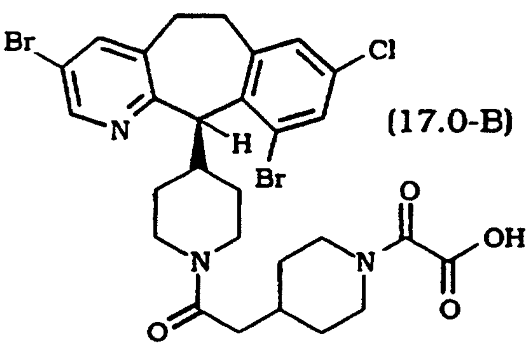

(17.0-B)

Dissolve 1 equivalent of the product of Example 59 (Compound 104.0-B, Table 1) in methanol containing 1.2 equivalents of 1N KOH in methanol and stir for 48 hr at 25°C. Acidify to pH 2 with 1N HCl and extract with dichloromethane. Dry the organic layer over magnesium sulfate and concentrate under vacuum. Purify the residue by preparative silica gel TLC using methanol-dichloromethane-acetic acid (5-94-1) to give the product as a white solid mp = 240.1°C.

EXAMPLE 77

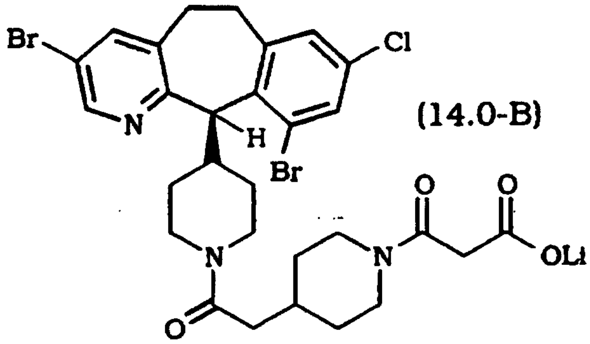

(14.0-B)

Dissolve 1 equivalent of the prduct of Example 30 (Compound 8.0-B, Table 1) in 95% aqueous ethanol containing 1.1 equivalents of LiOH and stir for 16 hr at 25°C. Concentrate under vacuum to give the product as a white solid.

EXAMPLE 78

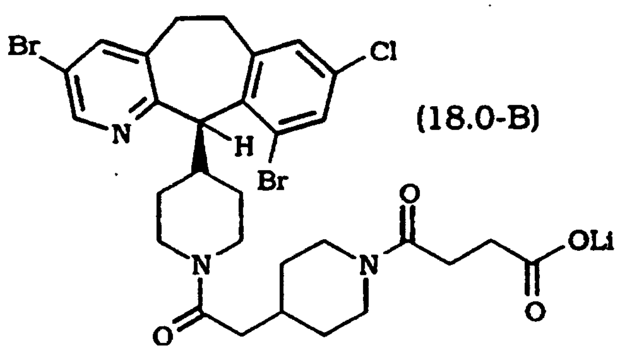

(18.0-B)

Follow the procedure of Example 77 but use the product of Example 60 (Compound 105.0-B, Table 1) instead of the product of Example 59 (Compound 104.0-B, Table 1) to obtain the product as a white solid.

EXAMPLE 79

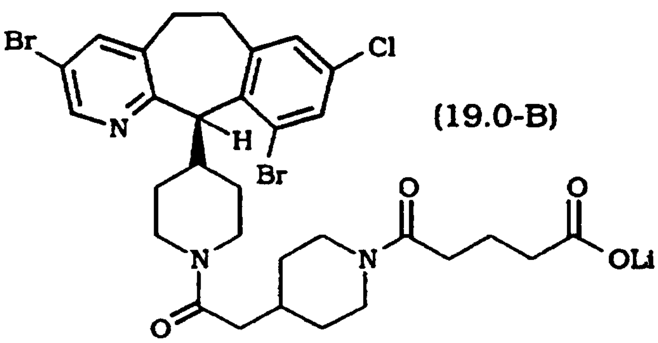

(19.0-B)

Follow the procedure of Example 77 but use the product of Example 61 (Compound 106.0-B, Table 1) instead

of the product of Example 59 (Compound 104.0-B, Table 1) to obtain the product as a white solid.

EXAMPLE 80

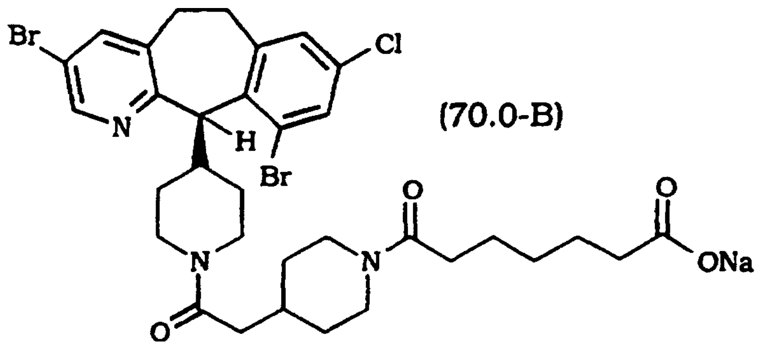

(70.0-B)

Dissolve 1 equivalent of the product of Example 43 (Compound 68.0-B. Table 1) in 95% aqueous methanol containing 1.1 equivalents of NaOH and stir for 16 hr at 25°C. Concentrate under vacuum to give the product as a white solid. mp = 215.5-216.2°C

EXAMPLE 81

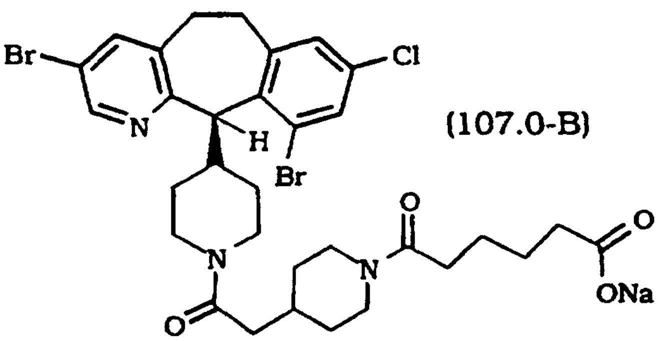

(107.0-B)

Dissolve 1 equivalent of the prduct of Example 58 (Compound 101.0-B, Table 1) in 95% aqueous methanol containing 1.1 equivalents of NaOH and stir for 16 hr at 25°C. Concentrate under vacuum to give the product as a white solid. mp = 240°C (d).

EXAMPLE 82

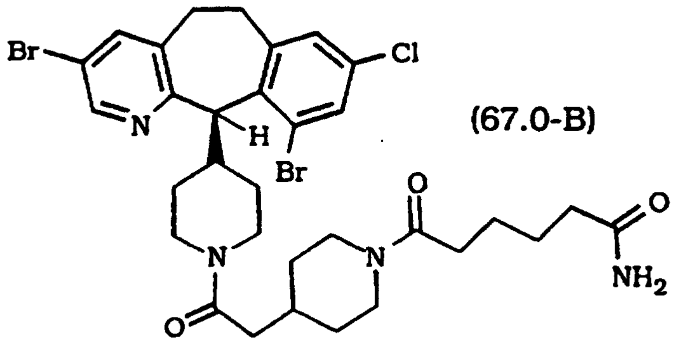

(67.0-B)

Dissolve 1.0 equivalent of the product of Example 81 (Compound 107.0-B) in DMF containing 5.0 equivalents of ammonium chloride and 1.0 equivalent each of DEC, HOBT and N-methylmorpholine. Stir the mixture at room temperature for 18 hr, then concentrate in vacuo to a residue and partition between ethyl acetate and water. Wash the organic phase with aqueous sodium bicarbonate solution then brine. Dry the organic phase over magnesium sulfate, filter and concentrate in vacuo to a residue. Chromatograph the residue on silica gel to obtain the product as a white solid mp = 125.5-126.5°C.

EXAMPLE 83

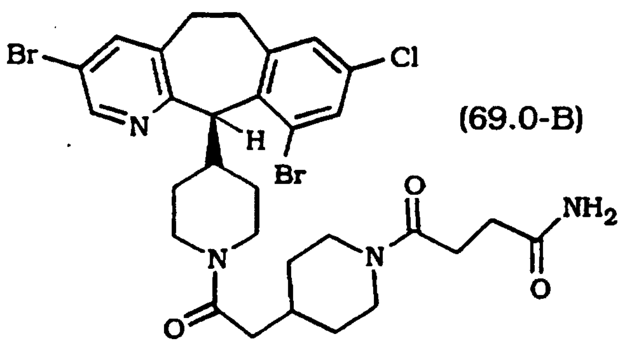

(69.0-B)

Dissolve 1.0 equivalent of the product of Example 78 (Compound 18.0-B) in DMF containing 5.0 equivalents of ammonium chloride and 1.0 equivalent each of DEC, HOBT and N-methylmorpholine. Stir the mixture at room temperature for 18 hr, then concentrate in vacuo to a residue and partition between ethyl acetate and water. Wash the organic phase with aqueous sodium bicarbonate solution then brine. Dry the organic phase over magnesium sulfate, filter and concentrate in vacuo to a residue. Chromatograph the residue on silica gel to obtain the product as a white solid mp =142.8-143.3°C.

EXAMPLE 84

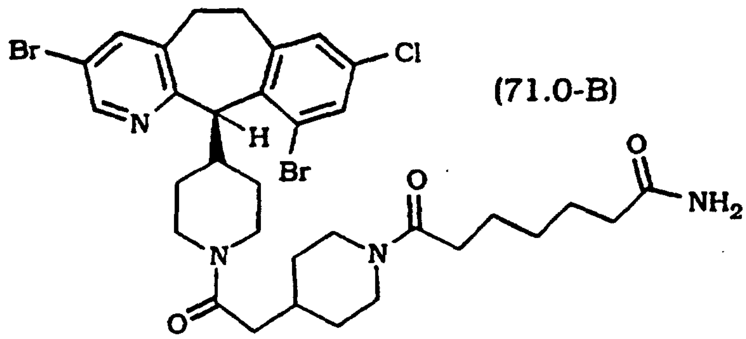

(71.0-B)

Dissolve 1.0 equivalent of the product of Example 80 . (Compound 70.0-B) in DMF containing 5.0 equivalents of ammonium chloride and 1.0 equivalent each of DEC, HOBT and N-methylmorpholine. Stir the mixture at room temperature for 18 hr, then concentrate in vacuo to a residue and partition between ethyl acetate and water. Wash the organic phase with aqueous sodium bicarbonate solution then brine. Dry the organic phase over magnesium sulfate, filter and concentrate in vacuo to a residue. Chromatograph the residue on silica gel to obtain the product as a white solid mp =119.2-120°C.

EXAMPLE 85

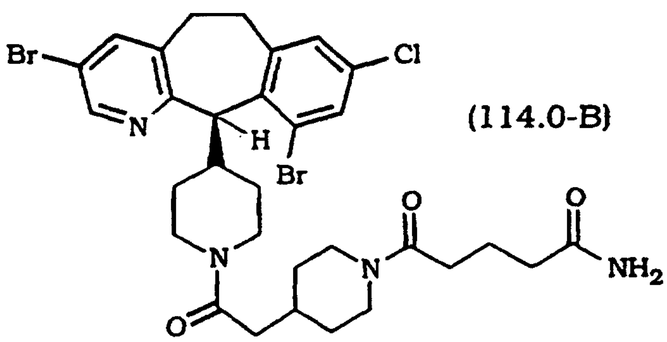

(114.0-B)

Dissolve 1.0 equivalent of the product of Example 81 (Compound 107.0-B) in DMF containing 5.0 equivalents of ammonium chloride and 1.0 equivalent each of DEC, HOBT and N-methylmorpholine. Stir the mixture at room temperature for 18 hr, then concentrate in vacuo to a residue and partition between ethyl acetate and water. Wash the organic phase with aqueous sodium bicarbonate solution then brine. Dry the organic phase over magnesium sulfate, filter and concentrate in vacuo to a residue. Chromatograph the residue on silica gel to obtain the product as a white solid.

EXAMPLE 86

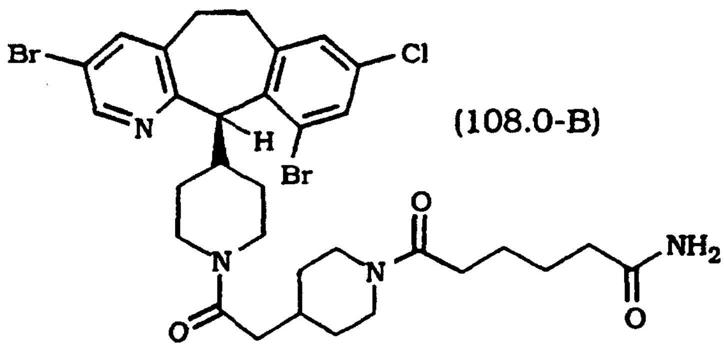

(108.0-B)

Dissolve 1.0 equivalent of the product of Example 79 (Compound 19.0-B) in DMF containing 5.0 equivalents of ammonium chloride and 1.0 equivalent each of DEC, HOST and N-methylmorpholine. Stir the mixture at room temperature for 18 hr, then concentrate in vacuo to a residue and partition between ethyl acetate and water. Wash the organic phase with aqueous sodium bicarbonate solution then brine. Dry the organic phase over magnesium sulfate, filter and concentrate in vacuo to a residue. Chromatograph the residue on silica gel to obtain the product as a white solid.

EXAMPLE 87

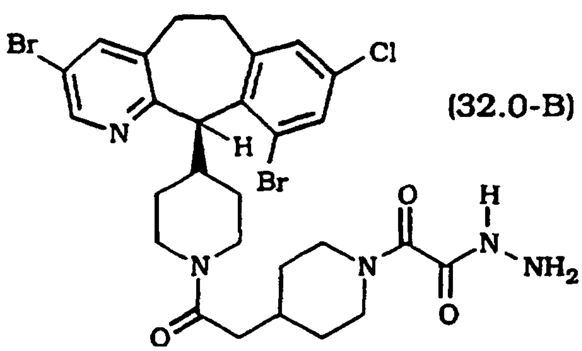

(32.0-B)

Dissolve 1.0 equivalent of the product of Example 59 (Compound 104.0-B, Table 1) in dichloromethane containing 4.0 equivalents of anhydrous hydrazine and stir for 48 hr. Concentrate under vacuum and chromatograph the residue on preparative silica gel TLC using methanol-dichloromethane 5-95) to yield the product as a yellow solid, mp = 90°C.

EXAMPLE 88

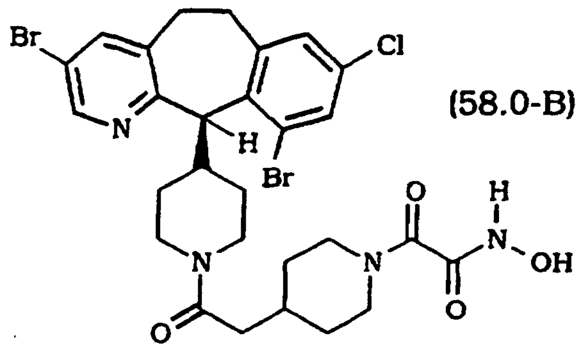

(58.0-B)

Dissolve 1.0 equivalent of the product of Example 59 (Compound 104.0-B, Table 1) in methanol containing 1.4 equivalents of LiOH and stir for 18 hr. Add DMF containing 1.0 equivalent each of DEC, HOBT and N-methylmorpholine and O-tert-butyldimethylsilylhydroxylamine. Stir the mixture at room temperature for 48 hr, then concentrate in vacuo to a residue and partition between ethyl acetate and water. Dry the organic phase over magnesium sulfate, filter and chromatograph on silica gel using methanol-dichloromethane (5-95) to obtain the product as a white solid, mp = 103.0°C.

EXAMPLE 89

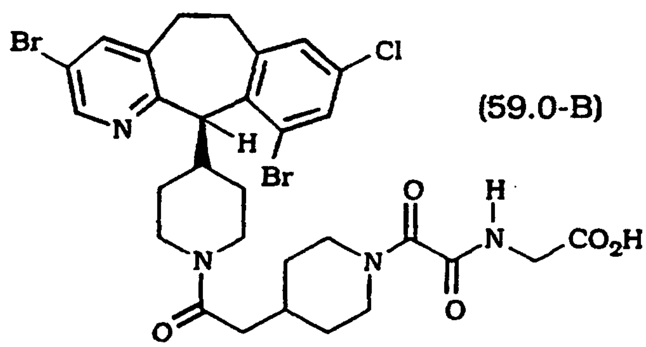

(59.0-B)

Dissolve 1.0 equivalent the product of Example 59 (Compound 104.0-B, Table 1) in methanol containing 3.0 equivalents of KOH and 3.0 equivalent of glycine hydrochloride tert-butyl ester and stir for 7 days. Concentrate under vacuum and chromatograph the residue on silica gel using methanol-dichloromethane (5-95 to obtain the product as a yellow solid, mp = 108°C.

EXAMPLE 90

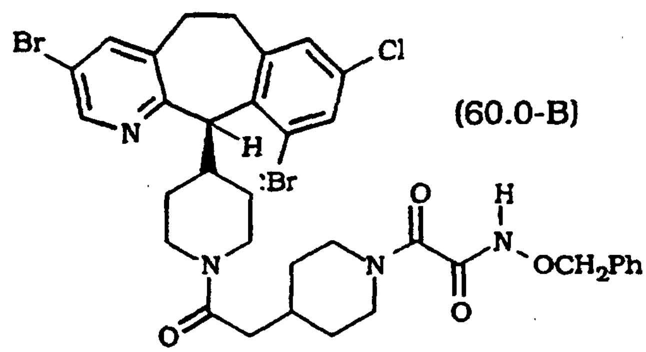

(60.0-B)

Ph = phenyl

Dissolve 1.0 equivalent the product of Example 59 (Compound 104.0-B. Table 1) in methanol containing 3.0 equivalents of KOH and 3.0 equivalent of O-benzylhydroxyl-amine hydrochloride and stir for 48 hr. Concentrate under vacuum and chromatograph the residue on silica gel using methanol-dichloromethane-acetic acid (10-89.5-0.5) to obtain the product as a yellow solid mp = 75°C.

EXAMPLE 91

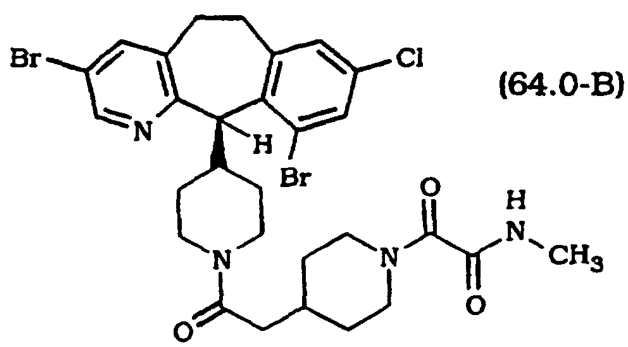

(64.0-B)

Dissolve 1.0 equivalent the product of Example 59 (Compound 104.0-B, Table 1) in methanol 4.0 equivalent of methylamine and stir for 18 hr. Concentrate under vacuum and chromatograph the residue on silica gel using methanol-dichloromethane saturated with ammonia (5-95) to obtain the product as a yellow solid mp = 86-132°C.

EXAMPLE 92

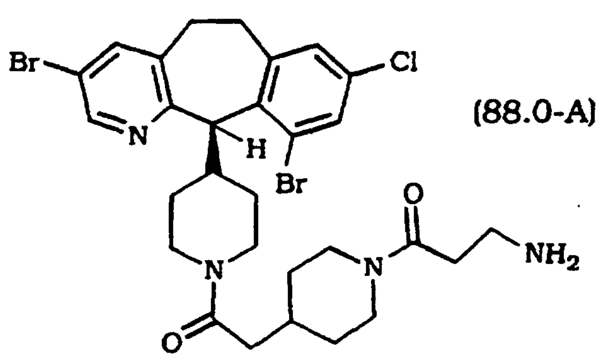

(88.0-A)

Dissolve 1.0 equivalent of the product from Example 52 (Compound 86.0-B, Table 1) in dichloromethane containing 2 equivalents of trifluoroacetic acid and stir for 2 hr. Concentrate under vacuum and partition the residue between dichloromethane and aqueous sodium bicarbonate. Dry the organic layer over magnesium sulfate and concentrate under vacuum to yield the product as a white solid, mp = 120.6-120.8°C.

EXAMPLE 93

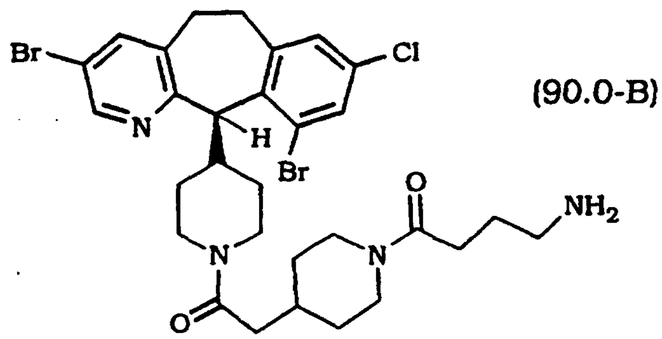

(90.0-B)

Dissolve 1.0 equivalent of the product from Example 53 (Compound 89.0-B, Table 1) in dichloromethane containing 2 equivalents of trifluoroacetic acid and stir for 2 hr. Concentrate under vacuum and partition the residue between dichloromethane and aqueous sodium bicarbonate. Dry the organic layer over magnesium sulfate and concentrate under vacuum to yield the product as a white solid, mp = 114-115°C.

EXAMPLE 94

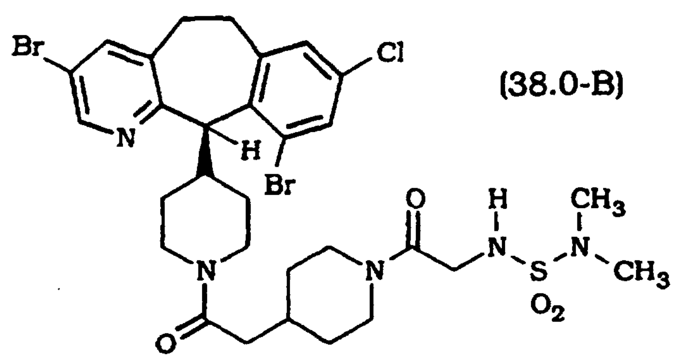

(38.0-B)

Dissolve 1.0 equivalent of the product from Example 1 in dichloromethane containing 2 equivalents of trifluoroacetic acid and stir for 2 hr. Concentrate under vacuum and partition the residue between dichloro-methane and aqueous sodium bicarbonate. Dry the organic layer over magnesium sulfate and concentrate under vacuum. Dissolve the residue in dichloromethane containing 1.5 equivalents of triethyl amine and 1.2 equivalents of dimethylsulfamoyl chloride. Stir for 18 hr then wash with 1N HCl followed by 1N NaOH. Dry the organic layer over magnesium sulfate and concentrate under vacuum to obtain the produce, mp = 124.4-130°C.

EXAMPLE 95

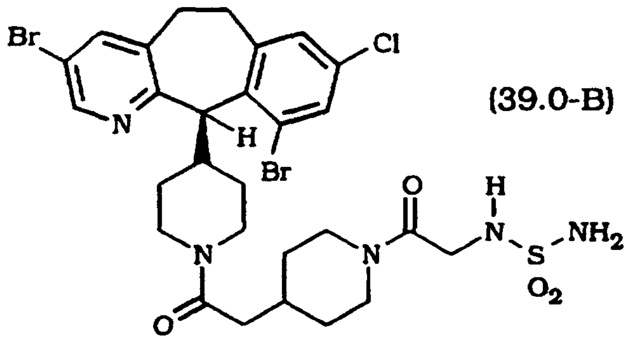

(39.0-B)

Dissolve 1.0 equivalent of the product from Example 1 in dichloromethane containing 2 equivalents of trlfluoroacetic acid and stir for 2 hr. Concentrate under vacuum and partition the residue between dichloro-methane and aqueous sodium bicarbonate. Dry the organic layer over magnesium sulfate and concentrate under vacuum. Dissolve the residue in 10.0 equivalents of aqueous sulfamide and reflux for 48 hr. Concentrate under vacuum and chromatograph the residue on silica gel using methanol-dichloromethane saturated with ammonia (5-95)to obtain the product, mp = 151.9°C.

EXAMPLE 96

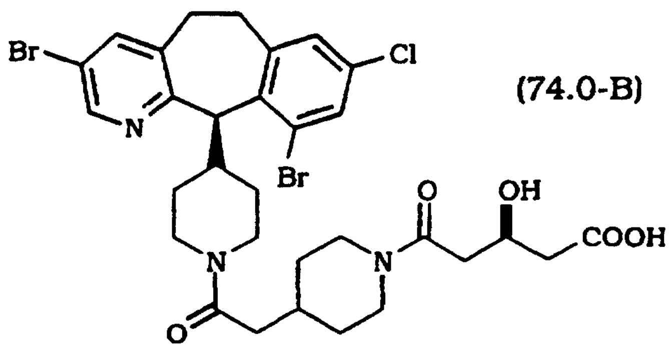

(74.0-B)

The product of Example 31 (Compound16.0-B, Table 1) (372.1 mg, 0.468 mmol) was dissolved in 3 mL of 6 M HCl and the solution stirred at room temperature overnight. The reaction mixture was added to 25 mL water, and the resulting precipitate was filtered and washed with 0.1 M HCl. The filtrate was saturated with NaCl and extracted continuously for 48 h to provide additional crude product. Purify the combined crude material by flash chromatoghrapy (C-18 reverse phase silica, gradient of 50% MeOH/0.17 M HOAc to 90% MeOH/0.17 M HOAc). The resulting material was dissolved in MeOH and added to water and the resulting suspension evaporated to dryness to give the title compound as a white solid (mp 133.5° - 141.2° C, heating 2° - 3° C/min).

EXAMPLE 97

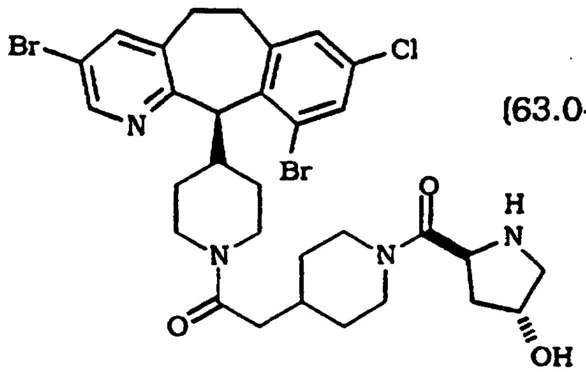

(63.0-B)

The product of Example 34 (Compound 24.0-B, Table 1) (450.0 mg, 0.56 mmol) was dissolved in 20 mL $CH_2Cl_2$, cooled to 0° C, and 8 mL of trifluoroacetic acid was added slowly. After 1h the cold mixture was diluted with 50% NaOH (aq) and water. The mixture was extracted with $CH_2Cl_2$, which was then dried ($MgSO_4$) and evaporated to give the title compound as a yellow solid (230 mg, mp 161.0° -163.0° C).

EXAMPLE 98

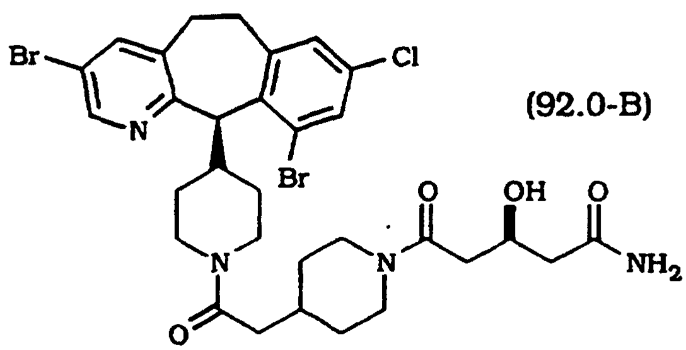

(92.0-B)

The product of Example 96 (Compound 74.0-B) (93.6 mg, 0.129 mmol) and 1-hydroxybenzotriazole (27.3 mg, 0.202 mmol) were dissolved in 1 mL of DMF. $NH_4Cl$ (14.8 mg, 0.276 mmol), N-methylmorpholine (70 μL) and DEC•HCl (30.8 mg, 0.161 mmol) were added. After 4 h the mixture was evaporated and the residue purified by flash chromatoghrapy (C-18 reverse phase silica, gradient of 50% MeOH/0.17 M HOAc to 90% MeOH/0.17 M HOAc). The resulting material was lyophilized from $HOAc/H_2O$ to give the title compound as a tan solid (67.7 mg, mp 115.2° - 122.0° C, heating 2° - 3° C/min).

EXAMPLE 99

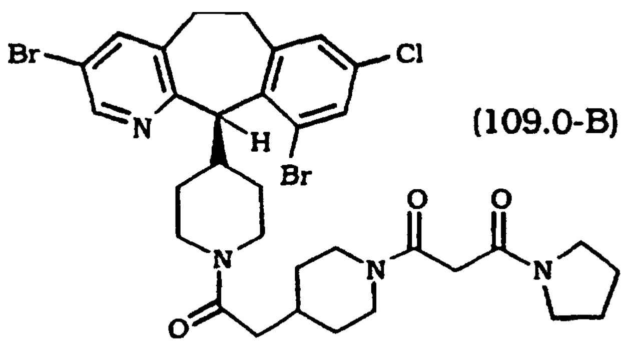

(109.0-B)

Dissolve 1.0 equivalent of the product of Example 77 (Compound 14.0-B) in DMF containing 1.0 equivalent each of DEC, HOBT, N-methylmorpholine and pyrrolidine. Stir the mixture at room temperature for 18 hr, then concentrate in vacuo to a residue and partition between ethyl acetate and water. Wash the organic phase with aqueous sodium bicarbonate solution then brine. Dry the organic phase over magnesium sulfate, filter and concentrate in vacuo to a residue. Chromatograph the residue on silica gel to obtain the product as a white solid.

EXAMPLE 100

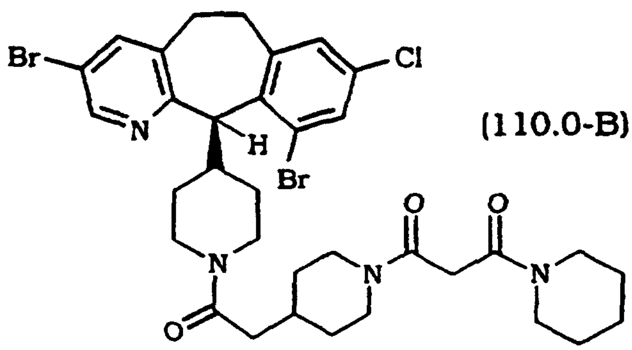

(110.0-B)

Dissolve 1.0 equivalent of the product of Example 77 (Compound 14.0-B) in DMF containing 1.0 equivalent each of DEC, HOBT, N-methylinorpholine and piperidine. Stir the mixture at room temperature for 18 hr, then concentrate in vacuo to a residue and partition between ethyl acetate and water. Wash the organic phase with aqueous sodium bicarbonate solution then brine. Dry the organic phase over magnesium sulfate, filter and concentrate in vacuo to a residue. Chromatograph the residue on silica gel to obtain the product as a white solid mp =135-136°C.

EXAMPLE 101

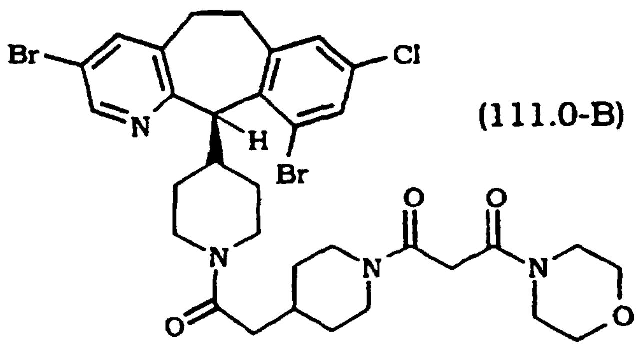

(111.0-B)

Dissolve 1.0 equivalent of the product of Example 77 (Compound 14.0-B) in DMF containing 1.0 equivalent each of DEC, HOBT, N-methylmorpholine and morpholine. Stir the mixture at room temperature for 18 hr, then concentrate in vacuo to a residue and partition between ethyl acetate and water. Wash the organic phase with aqueous sodium bicarbonate solution then brine. Dry the organic phase over magnesium sulfate, filter and concentrate in vacuo to a residue. Chromatograph the residue on silica gel to obtain the product as a white solid mp =35-136°C.

EXAMPLE 102

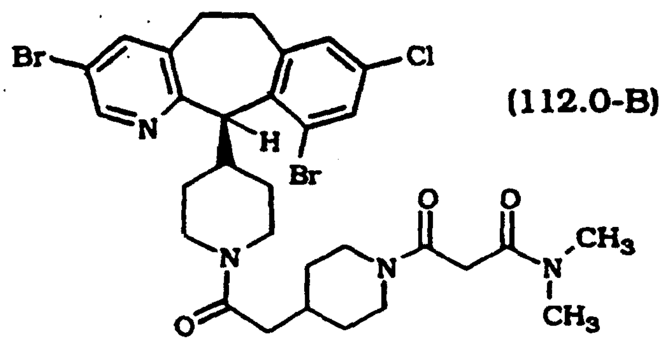

(112.0-B)

Dissolve 1.0 equivalent of the product of Example 77 (Compound 14.0-B) in DMF containing 1.0 equivalent each of DEC, HOBT, N-methylmorpholine and dimethylamine. Stir the mixture at room temperature for 18 hr, then concentrate in vacuo to a residue and partition between ethyl acetate and water, Wash the organic phase with aqueous sodium bicarbonate solution then brine. Dry the organic phase over magnesium sulfate, filter and concentrate in vacuo to a residue. Chromatograph the residue on silica gel to obtain the product as a white solid mp =133-134°C.

ASSAYS

FPT $IC_{50}$ (inhibition of farnesyl protein transferase, in vitro enzyme assay) and COS Cell $IC_{50}$ (Cell-Based Assay) were determined following the assay procedures described in WO 95/10516, published April 20, 1995. GGPT $IC_{50}$ (inhibition of geranylgeranyl protein transferase, in vitro enzyme assay), Cell Mat Assay, and anti-tumor activity (in vivo anti-tumor studies) could be determined by the assay procedures described in WO 95/10516.

Additional assays can be carried out by following essentially the same procedure as described above, but with substitution of alternative indicator tumor cell lines in place of the T24-BAG cells. The assays can be conducted using either DLD-1-BAG human colon carcinoma cells expressing an activated K-ras gene or SW620-BAG human colon carcinoma cells expressing an activated K-ras gene. Using other tumor cell lines known in the art, the activity of the compounds of this invention against other types of cancer cells could be demonstrated.

Soft Agar Assay:

Anchorage-independent growth is a characteristic of tumorigenic cell lines. Human tumor cells are suspended in growth medium containing 0.3% agarose and an indicated concentration of a farnesyl transferase inhibitor. The solution is overlayed onto growth medium solidified with 0.6% agarose containing the same concentration of farnesyl transferase inhibitor as the top layer. After the top layer is solidified, plates are incubated for 10-16 days at 37°C under 5% $CO_2$ to allow colony outgrowth. After incubation, the colonies are stained by overlaying the agar with a solution of MTT (3-[4,5-dimethylthiazol-2-yl]-2.5-diphenyltetrazolium bromide, Thiazolyl blue) (1 mg/mL in PBS). Colonies can be counted and the $IC_{50}$'s can be determined.

Compounds 2.0, 3.0, 4.0, 5.0, 6.0, 7.0, 8.0, 9.0, 10.0, 11.0. 12.0, 12.1, 12.2, 12.3, 13.0, 14.1, 14.2, 15.1, 15.2, 15.3, 16.0, 17.0-B. 18.0-B, 23.0-B, 79.0-B, 104.0-B and 108.0-B had an FPT $IC_{50}$ (H-ras) within the range of <2 to 31.7 nM (nanomolar).

Compounds 6.0, 7.0, 8.0, 10.0, 11.0, 12.0, 12.1, 12.3, 13.0, 14.1, 14.2, 15.1, 15.2 and 15.3 had a Cos Cell $IC_{50}$ within the range of 10 to 700 nM.

Compounds 6.0-B, 7.0-B, 8.0-B, 9.0-B, 10.0-B, 11.0-B, 12.0-B, 13.0-B, 14.0-B, 16.0-B, 17.0-B. 18.0-B, 19.0-B, 20.0-B. 21.0-B, 22.0-B, 23.0-B, 24.0-B, 26.0-B, 30.0-B, 32.0-B, 33.0-B, 34.0-B, 35.0-B, 37.0-B. 38.0-B, 39.0-B, 44.0-B, 49.0-B, 50.0-B, 51.0-B, 52.0-B, 53.0-B, 54.0-B, 55.0-B, 56.0-B, 57.0-B, 58.0-B, 59.0-B, 60.0-B, 63.0-B, 64.0B, 67.0-B, 68.0-B, 69.0-B, 70.0-B, 71.0-B, 72.0-B, 74.0-B, 75.0-B, 76.0-B, 77.0-B, 79.0-B, 81.0-B, 82.0-B, 85.0-B, 86.0-B, 88.0-B, 89.0-B, 90.0-B, 92.0-B, 95.0-B. 101.0-B, 107.0-B, 114.0-B, 114.2-B, 114.3-B and 114.4-B had an FPT $IC_{50}$ within the range of 0.7-18nM.

Compounds 2.0, 3.0, 4.0, 6.0, 7.0, 8.0, 9.0, 10.0, 12.1, 12.3, 16.0, 6.0-B, 7.0-B, 9.0-B, 10.0-B, 11.0-B, 12.0-B, 13.0-B, 17.0-B and 18.0-B had an FPT $IC_{50}$ (k-ras) within the range of 14.5-71.2nM.

Compounds 6.0, 16.0, 17.0, 18.0, 6.0-B, 7.0-B. 9.0-B. 10.0-B, 11.0-B, 12.0-B, 13.0-B, 14.0-B, 16.0-B, 17.0-B, 18.0-B, 19.0-B, 22.0-B. 23.0-B, 24.0-B, 26.0-B, 30.0-B, 32.0-B, 33.0-B, 34.0-B. 35.0-B, 37.0-B, 38.0-B, 39.0-B, 44.0-B, 49.0-B, 50.0-B, 51.0-B, 52.0-B, 53.0-B, 54.0-B, 55.0-B, 56.0-B, 57.0-B, 58.0-B, 59.0-B, 60.0-B, 63.0-B, 64.0B, 68.0-B, 69.0-B, 70.0-B, 71.0-B, 72.0-B, 74.0-B, 75.0-B, 77.0-B, 79.0-B, 81.0-B, 82.0-B. 85.0-B, 88.0-B, 89.0-B, 92.0-B, 95.0-B, 101.0-B, 104.0-B, 107.0-B, 108.0-B, 114.0-B, 114.2-B, 114.3-B and 114.4-B had a Cos Cell within the range of 9 to >1000nM.

Compounds 10.0, 12.1, 12.3, 15.2, 16.0, 18.0, 6.0-B, 9.0-B, 10.0-B, 11.0-B, 12.0-B, 13.0-B, 14.0-B, 16.0-B, 17.0-B, 18.0-B, 19.0-B, 21.0-B, 22.0-B, 23.0-B, 24.0-B, 26.0-B, 30.0-B, 32.0-B, 33.0-B, 34.0-B, 35.0-B, 37.0-B, 38.0-B, 39.0-B, 44.0-B, 49.0-B, 50.0-B, 52.0-B, 53.0-B, 54.0-B, 55.0-B, 56.0-B. 57.0-B, 59.0-B, 60.0-B, 63.0-B, 64.0B, 68.0-B, 69.0-B, 70.0-B, 71.0-B, 72.0-B, 74.0-B, 75.0-B, 77.0-B, 79.0-B, 81.0-B, 82.0-B, 85.0-B, 88.0-B, 89.0-B, 92.0-B, 95.0-B, 101.0-B, 104.0-B, 107.0-B, 114.0-B, 114.2-B, 11.4.3-B and 114.4-B had a Soft Agar within the range of 19.5 to >500nM.

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 70 percent active ingredient. Suitable solid carriers are known in the art, e.g. magnesium carbonate, magnesium stearate, talc, sugar, lactose. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection.

Liquid form preparations may also include solutions for intranasal administration.

Aerosol preparations suitable for inhalation may include solutions and solids in powder form, which may be in combination with a pharmaceutically acceptable carrier, such as an inert compressed gas.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in unit dosage form. In such form, the preparation is subdivided into unit doses containing appropriate quantities of the active component, e.g., an effective amount to achieve the desired purpose.

The quantity of active compound in a unit dose of preparation may be varied or adjusted from about 0.1 mg

to 1000 mg, more preferably from about 1 mg. to 300 mg, according to the particular application.

The actual dosage employed may be varied depending upon the requirements of the patient and the severity of the condition being treated. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The amount and frequency of administration of the compounds of the invention and the pharmaceutically acceptable salts thereof will be regulated according to the judgment of the attending clinician considering such factors as age, condition and size of the patient as well as severity of the symptoms being treated. A typical recommended dosage regimen is oral administration of from 10 mg to 2000 mg/day preferably 10 to 1000 mg/day, in two to four divided doses to block tumor growth. The compounds are non-toxic when administered within this dosage range.

The following are examples of pharmaceutical dosage forms which contain a compound of the invention. The scope of the invention in its pharmaceutical composition aspect is not to be limited by the examples provided.

Pharmaceutical Dosage Form Examples

EXAMPLE A

Tablets

| No. | Ingredients | mg/tablet | mg/tablet |
|---|---|---|---|
| 1. | Active compound | 100 | 500 |
| 2. | Lactose USP | 122 | 113 |
| 3. | Corn Starch, Food Grade, as a 10% paste in Purified Water | 30 | 40 |
| 4. | Corn Starch. Food Grade | 45 | 40 |
| 5. | Magnesium Stearate | 3 | 7 |
| | Total | 300 | 700 |

Method of Manufacture

Mix Item Nos. 1 and 2 in a suitable mixer for 10-15 minutes. Granulate the mixture with Item No. 3. Mill the damp granules through a coarse screen (e.g., 1/4". 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weigh on a suitable tablet machine.

EXAMPLE B

Capsules

| No. | Ingredient | mg/capsule | mg/capsule |
|---|---|---|---|
| 1. | Active compound | 100 | 500 |
| 2. | Lactose USP | 106 | 123 |
| 3. | Corn Starch, Food Grade | 40 | 70 |
| 4. | Magnesium Stearate NF | 7 | 7 |
| | Total | 253 | 700 |

Method of Manufacture

Mix Item Nos. 1, 2 and 3 in a suitable blender for 10-15 minutes. Add Item No. 4 and mix for 1-3 minutes. Fill

the mixture into suitable two-piece hard gelatin capsules on a suitable encapsulating machine.

## Claims

**1.** A compound of the formula:

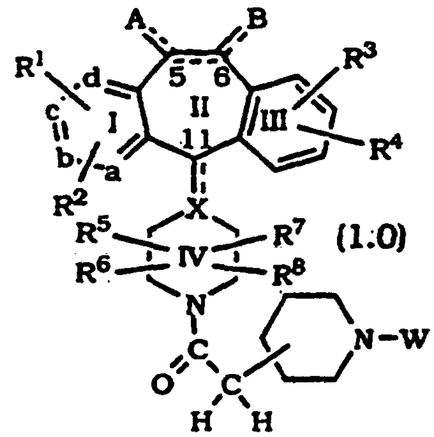

(1.0)

or a pharmaceutically acceptable salt or solyate thereof, wherein: one of a, b, c and d represents N or $NR^9$ wherein $R^9$ is $O^-$, $-CH_3$ or $-(CH_2)_nCO_2H$ wherein n is 1 to 3, and the remaining a, b, c and d groups represent $CR^1$ or $CR^2$; or each of a, b, c, and d are independently selected from $CR^1$ or $CR^2$;

$R^2$ is H and $R^1$, $R^3$ and $R^4$ are halo;

$R^5$, $R^6$, $R^7$ and $R^8$ each independently represents H, $-CF_3$, $-COR^{10}$, alkyl or aryl, said alkyl or aryl optionally being substituted with $-OR^{10}$, $-SR^{10}$, $-S(O)_tR^{11}$, $-NR^{10}COOR^{11}$, $-N(R^{10})_2$, $-NO_2$, $-COR^{10}$, $-OCOR^{10}$, $-OCO_2R^{11}$, $-CO_2R^{10}$, $OPO_3R^{10}$, or $R^5$ is combined with $R^6$ to represent =O or =S and/or $R^7$ is combined with $R^8$ to represent =O or =S;

$R^{10}$ represents H, alkyl, aryl, or aralkyl;

$R^{11}$ represents alkyl or aryl;

X represents N, CH or C, which C may contain an optional double bond (represented by the dotted line) to carbon atom 11;

the dotted line between carbon atoms 5 and 6 represents an optional double bond, such that when a double bond is present, A and B independently represent $-R^{10}$, halo, $-OR^{11}$, $-OCO_2R^{11}$ or $-OC(O)R^{10}$, and when no double bond is present between carbon atoms 5 and 6, A and B each independently represent $H_2$, $-(OR^{11})_2$; H and halo, dihalo, alkyl and H, $(alkyl)_2$, -H and $-OC(O)R^{10}$, H and $-OR^{10}$, =O, aryl and H, $=NOR^{10}$ or $-O-(CH_2)_p-O-$ wherein p is 2, 3 or 4; and

W represents a group selected from the group consisting of:

(1)

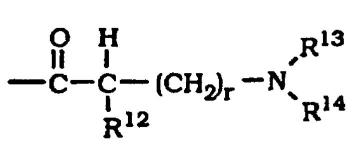

;

(2)

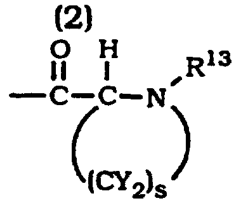

;

(3)

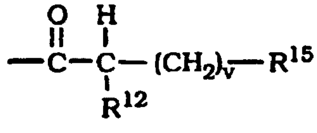

and

(4)

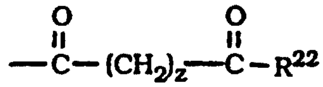

wherein:

$R^{12}$ is selected from the group consisting of: (a) H; (b) alkyl; (c) aralkyl; and (d) heteroarylalkyl;
$R^{13}$ and $R^{14}$ are each independently selected from the group consisting of: (a) H; (b) -C(O)O$R^{16}$ wherein $R^{16}$ represents alkyl, aralkyl, and heteroaralkyl; (c) -$SO_2R^{17}$ wherein $R^{17}$ is selected from the group consisting of: $NH_2$, -N(alkyl)$_2$ wherein each alkyl is the same or different, alkyl, aryl, aralkyl, heteroaryl and heteroaralkyl; (d) -C(O)$R^{18}$ wherein $R^{18}$ is selected from the group consisting of: aryl, alkyl, aralkyl, heteroaryl, and heteroaralkyl; (e) $C_{1-6}$ alkyl; (f) alkaryl; and (g) $C_{3-6}$ cycloalkyl;
r is 0, 1 or 2;
s represents 1, 2, 3, 4, or 5, and each Y for each -$CY_2$- group is independently selected from H or -OH, provided that both Y substituents of each -$CY_2$- group are not -OH, and provided that for the -$CY_2$- group alpha to the nitrogen both Y substituents are H, such that the group

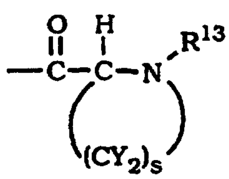

forms a 3, 4, 5, 6, or 7 membered ring;
v is 0, 1 or 2;
$R^{15}$ is selected from the group consisting of:

(a) heteroaryl;
(b) a group selected from:

(1)

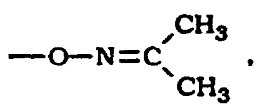

(2)

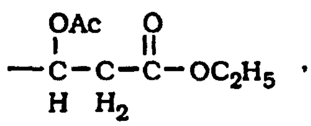

(3)

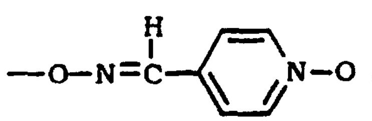

(4)

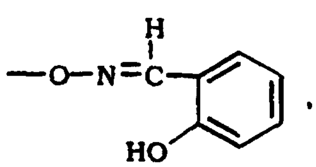

(5) $-CH(OCH_2CH_3)_2$,
(6) -OH, and
(7) -CN; and

(c) heterocycloalkyl selected from the group consisting of:

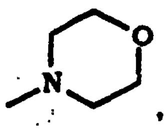, 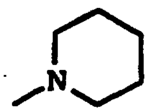, 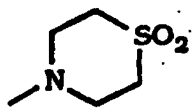, 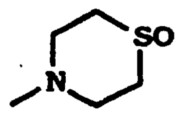, 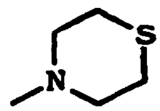,

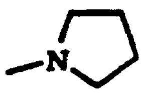, 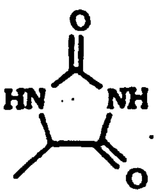, 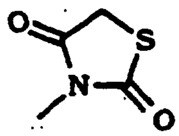, 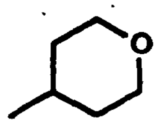, 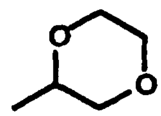, 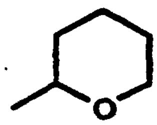,

and

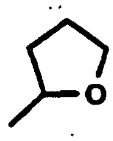;

z is 0, 1, 2. 3, 4, or 5 wherein each $-CH_2-$ group is optionally substituted with a -OH group;
$R^{22}$ represents a group selected from:

(1) 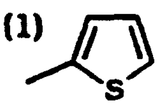, (2) 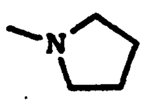, (3) 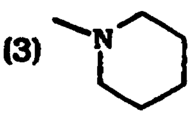, (4) 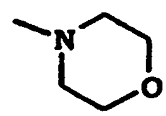,

(5) alkyl (e.g., $-CH_3$),
(6) $-OR^{23}$ wherein $R^{23}$ is selected from the group consisting of: alkyl, aryl and H, and
(7)

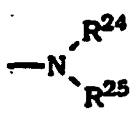

wherein $R^{24}$ and $R^{25}$ are independently selected from the group consisting of: $-NH_2$, alkoxy, -OH, $-CH_2CO_2H$, $-OCH_2Ph$, $-CH(OCH_3)CH(CH_3)_2$, alkyl, aryl, H, aralkyl, and heteroaralkyl; or R24 and R25 taken together form a carbon chain having 4 or 5 ($-CH_2-$) groups such that $R^{24}$ and $R^{25}$ taken together with the nitrogen to which they are bound form a 5 or 6 membered heterocycloalkyl ring;

wherein, unless stated otherwise:

alkenyl-represents straight and branched carbon chains having at least one carbon to carbon double bond and containing from 2 to 12 carbon atoms;

alkynyl-represents straight and branched carbon chains having at least one carbon to carbon triple bond and containing from 2 to 12 carbon atoms;

alkyl-(including the alkyl portions of aralkyl and heteroarylalkyl)-represents straight and branched carbon chains and contains from one to twenty carbon atoms;

aralkyl-represents an aryl group, as defined below, bound to an alkyl group, as defined above;

aryl (including the aryl portion of aralkyl represents a carbocyclic group containing from 6 to 15 carbon atoms and having at least one aromatic ring (e.g., aryl is a phenyl ring), with all available substitutable carbon atoms of the carbocyclic group being intended as possible points of attachment, said carbocyclic group being optionally substituted (e.g., 1 to 3) with one or more of halo, alkyl, hydroxy, alkoxy, phenoxy, $CF_3$, amino, alkylamino, dialkylamino, -$COOR^{10}$ or -$NO_2$;

halo-represents fluoro, chloro, bromo and iodo;

heteroaryl-represents cyclic groups, optionally substituted with $R^3$, $R^4$, phenyl, and or -$CH_2C(O)OCH_3$, said cyclic groups having at least one heteroatom selected from O. S or N, said heteroatom interrupting a carbocyclic ring structure and having a sufficient number of delocalized pi electrons to provide aromatic character, with the aromatic heterocyclic groups containing from 2 to 14 carbon atoms;

heteroarylalkyl (heteroaralkyl)-represents a heteroaryl group, as defined above, bound to an alkyl group, as defined above;

heterocycloalkyl-represents a saturated, branched or unbranched carbocyclic ring containing from 3 to 15 carbon atoms, preferably from 4 to 6 carbon atoms, which carbocyclic ring is interrupted by 1 to 3 hetero groups selected from -O-, -S- or - $NR^{10}$-.

**2.** The compound of Claim 1 wherein a is N and b c and d are carbon; A and B are each $H_2$; the optional bond between C5 and C6 is absent; X is CH; and $R^5$, $R^6$, $R^7$ and $R^8$ are H.

**3.** The compound of any of Claims 1-2 wherein W is selected from the group consisting of:

**(A)**

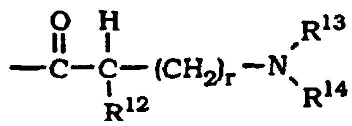

wherein:

(1) r is 0;
(2) $R^{12}$ is selected from the group consisting of: (a) H; (b) alkyl; (c) aralkyl; and (d) heteroaralkyl; and
(3) $R^{13}$ and $R^{14}$ are independently selected from the group consisting of: (a) H; (b) -$C(O)OR^{16}$ wherein $R^{16}$ is alkyl; (c) -$SO_2R^{17}$ wherein $R^{17}$ is alkyl or aryl; (d) -$C(O)R^{18}$

wherein $R^{18}$ is aryl: and (e) alkyl;

**(B)**

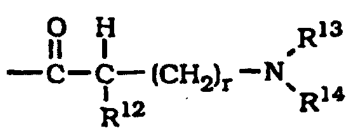

wherein:

(1) r is 1 or 2;
(2) $R^{12}$ is H; and
(3) $R^{13}$ is alkyl and $R^{14}$ is H, alkyl or -$C(O)OR^{16}$

wherein $R^{16}$ is alkyl;

(C)

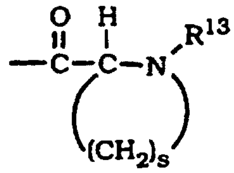

wherein:

(1) s is 1, 2, 3, 4 or 5; and
(2) $R^{13}$ is selected from the group consisting of: (a) H and -C(O)OR$^{16}$ wherein $R^{16}$ is alkyl;

(D)

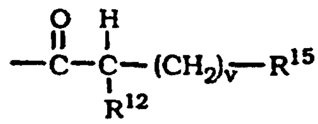

wherein:

(1) v is 0;
(2) $R^{12}$ is H; and
(3) $R^{15}$ is selected from the group consisting of:

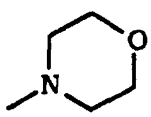 , s 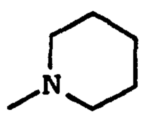 , 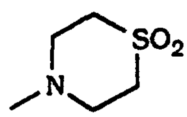 , 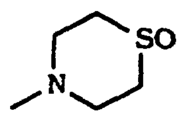 ,

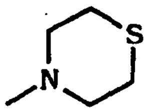 , 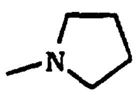 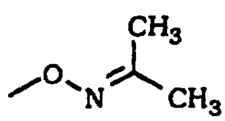 , 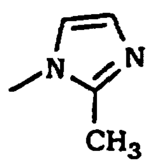 ,

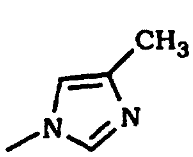 , 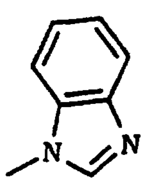 , 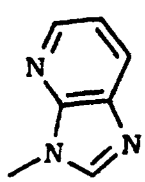 , 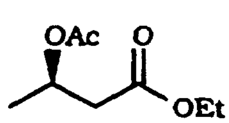 ,

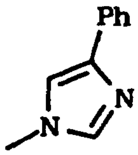, 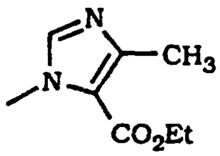, 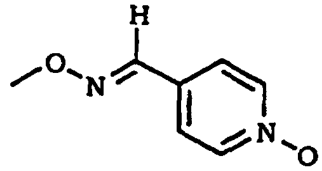,

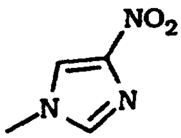, 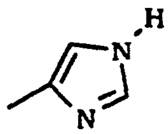, 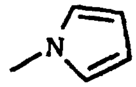, 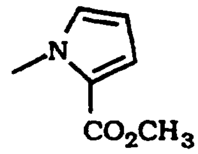,

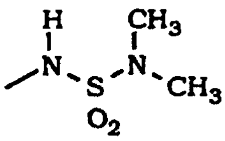, 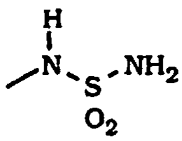, 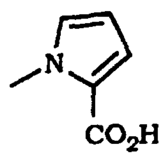, 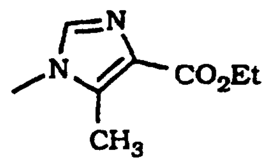,

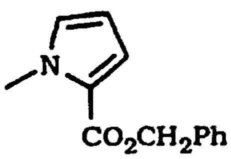, 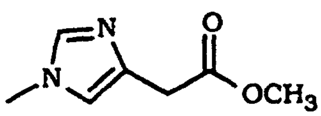, 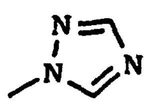, 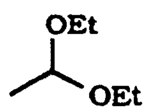,

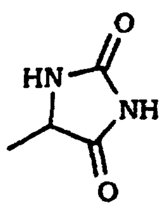, 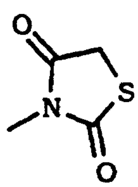, 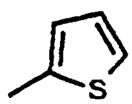, 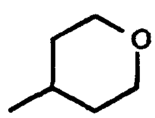,

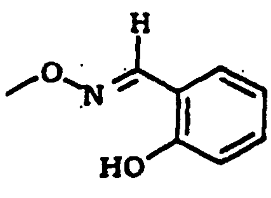, 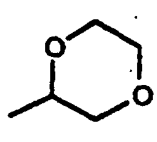, 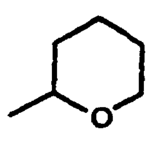, 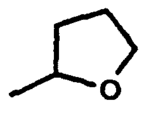,

and -OH, -CN;

(E)

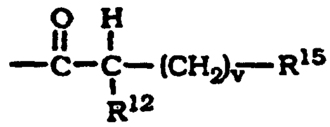

wherein:

(1) v is 1 or 2;
(2) $R^{12}$ is H; and
(3) $R^{15}$ is heterocycloalkyl;

(F)

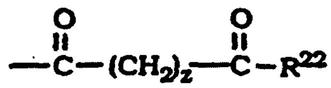

wherein:

(1) z is O;
(2) $R^{22}$ is $-NR^{24}R^{25}$; and
(3) $R^{24}$ and $R^{25}$ are independently selected from: H, $-NH_2$, alkyl, alkoxy, -OH, $-CH_2CO_2H$, or $-OCH_2C_6H_5$; and

(G)

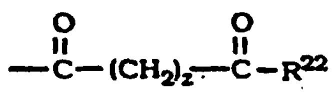

wherein:

(1) z is 1, 2, 3, 4 or 5;
(2) $R^{22}$ is selected from:$-OR^{23}$, -ONa, -OLi, alkyl, $-NR^{24}R^{25}$ or

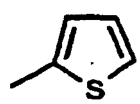 ;

(3) $R^{23}$ is alkyl; and
(4) $R^{24}$ and $R^{25}$ are independently selected from H, $-CH(OCH_3)CH(CH_3)_2$,

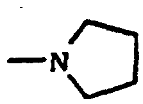 , 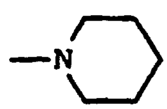 , or 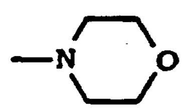 .

**4.** The compound of any of Claims 1-3 wherein $R^1$, $R^3$ and $R^4$ is selected from the group consisting of: Cl or Br.

**5.** The compound of any of Claims 1-4 wherein X is CH.

**6.** The compound of any of Claims 1-5 selected from:

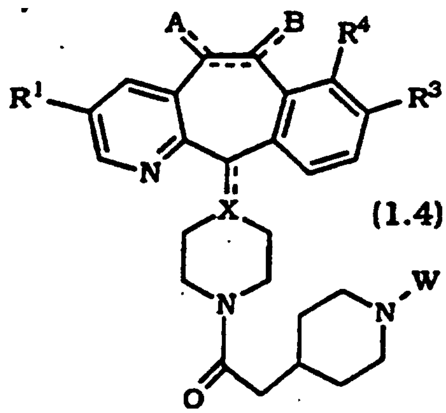

(1.4) or

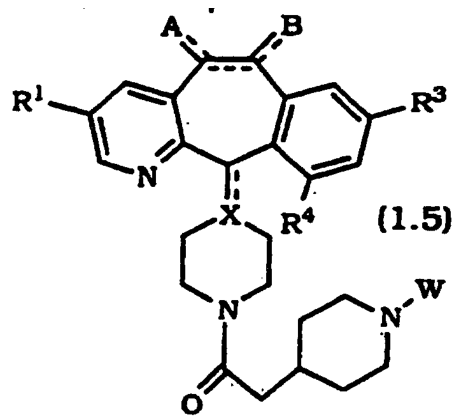

(1.5)

wherein A, B, X and W are as defined in Claim 1.

**7.** The compound of any of Claims 1-6 wherein $R^1$ is Br; $R^3$ is Cl; and $R^4$ is Br.

**8.** The compound of any of Claims 1-7 wherein said compound is a compound of the formula:

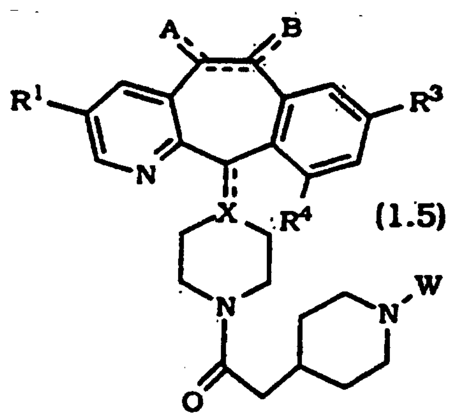

(1.5)

**9.** The compound of Claim 1 selected from:

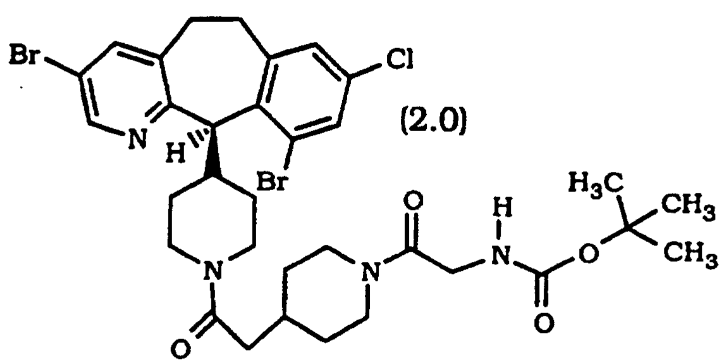

(2.0)

;

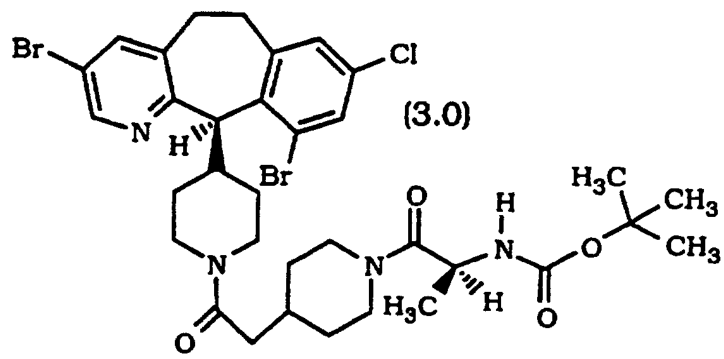

(3.0)

;

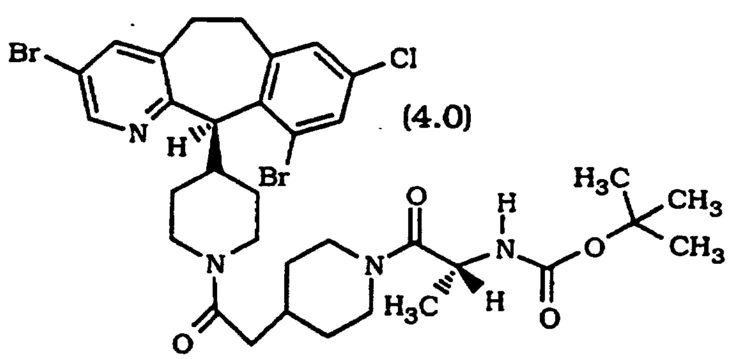

(4.0)

;

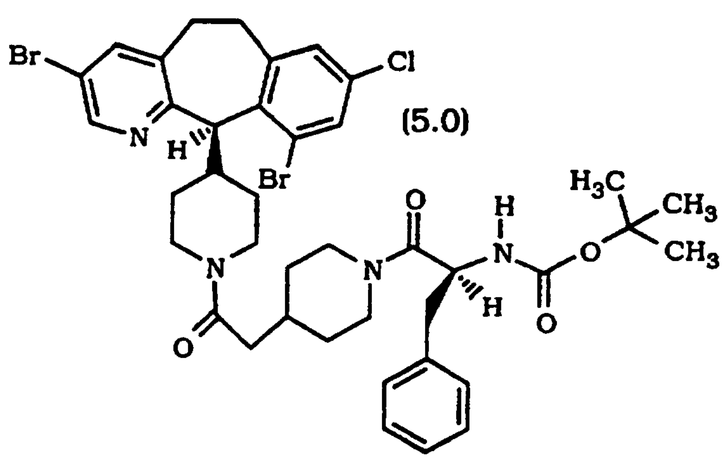

(5.0)

;

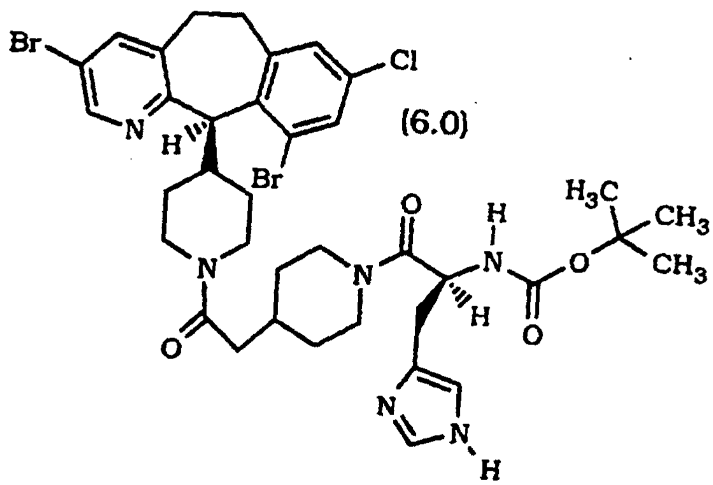

(6.0)

;

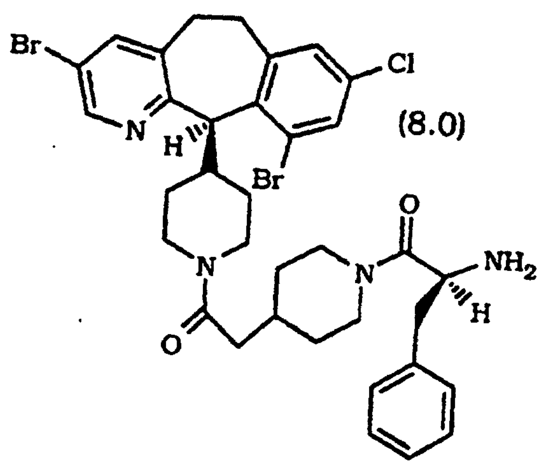

(8.0)

;

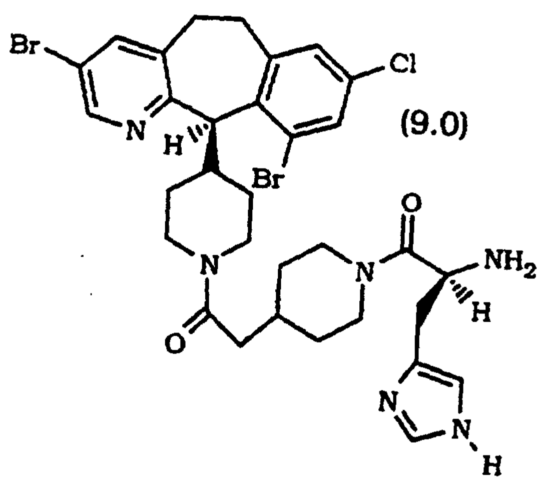

(9.0)

;

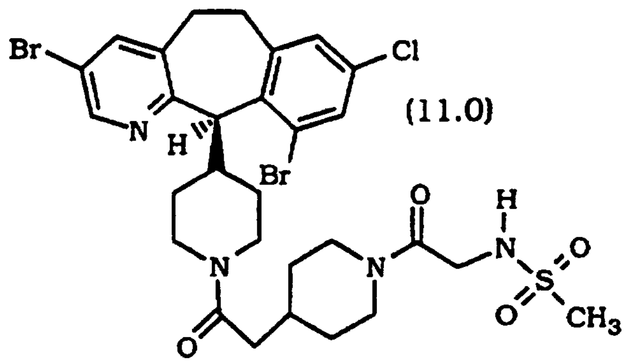

(11.0)

;

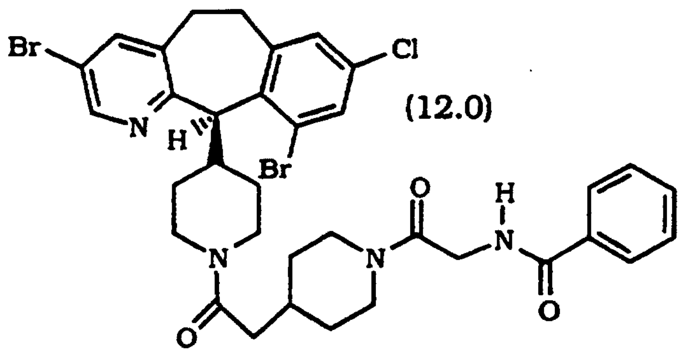

(12.0)

;

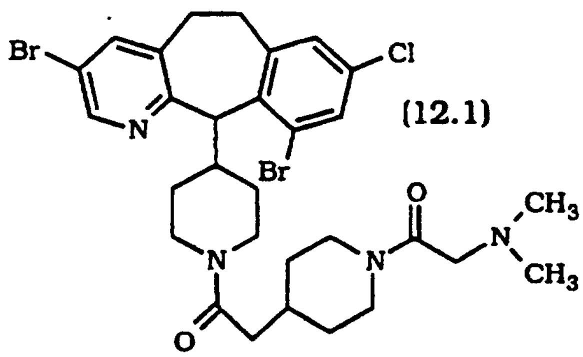

(12.1)

;

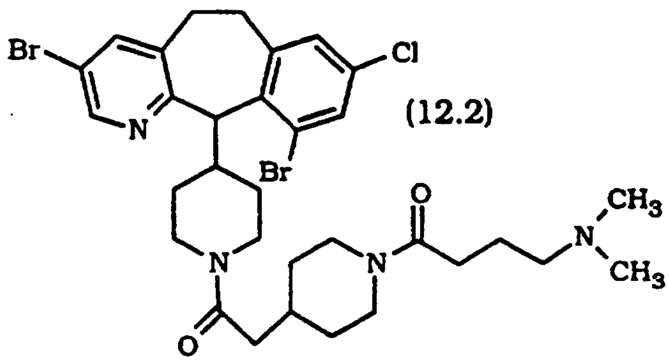

(12.2)

;

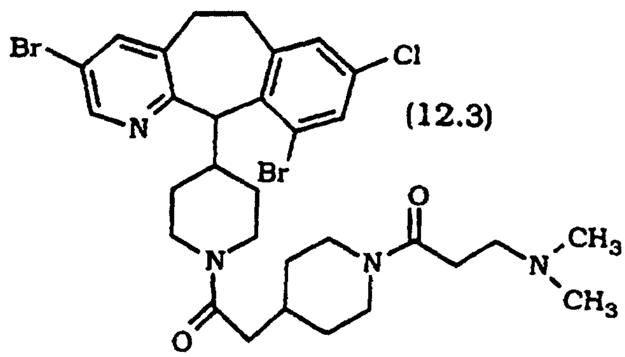

(12.3)

;

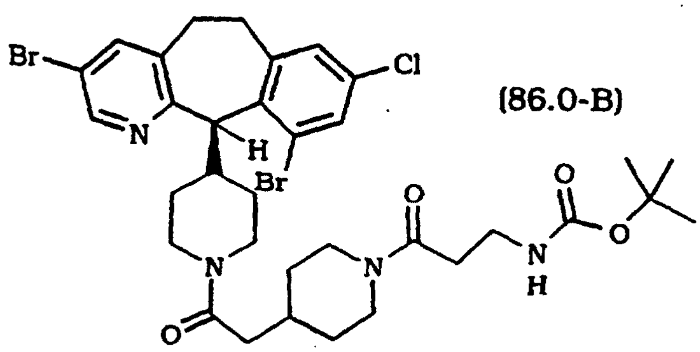

(86.0-B)

;

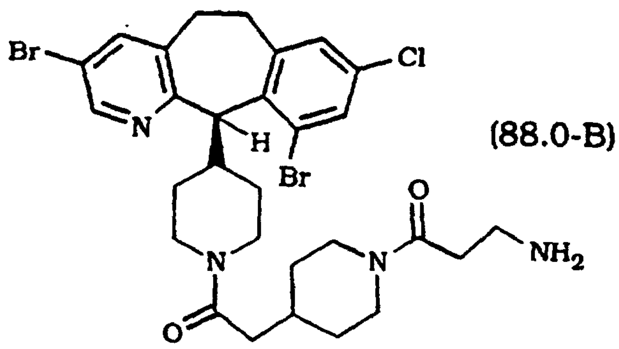

(88.0-B)

;

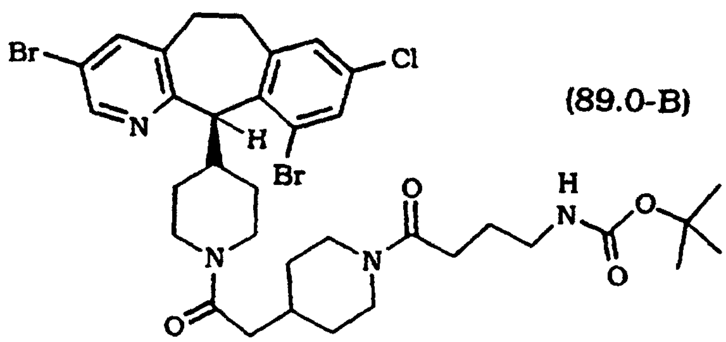

(89.0-B)

;

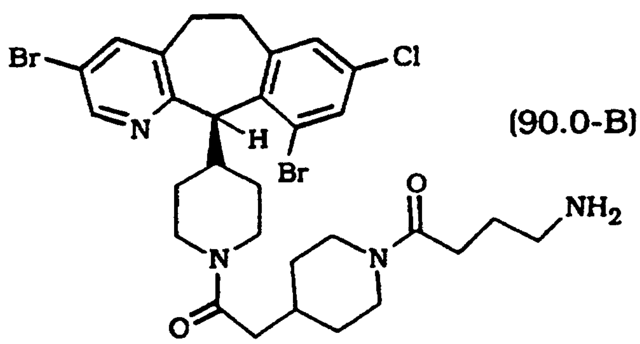

(90.0-B)

;

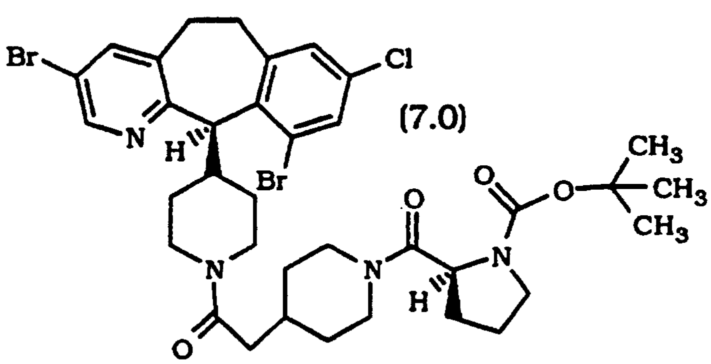

(7.0)

;

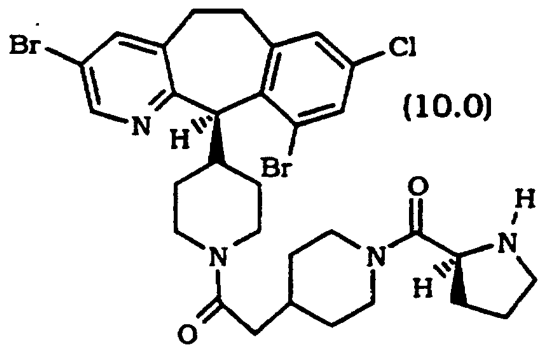

(10.0)

;

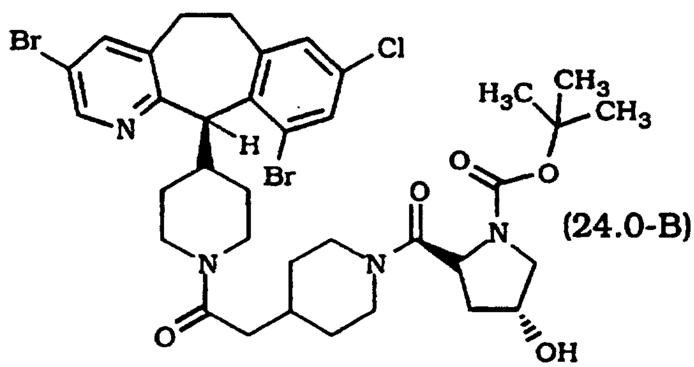

(24.0-B)

;

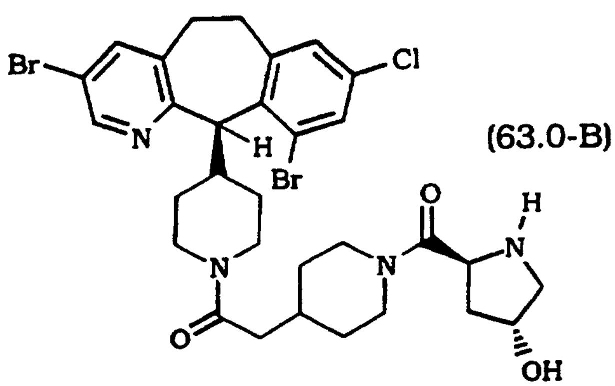

(63.0-B)

;

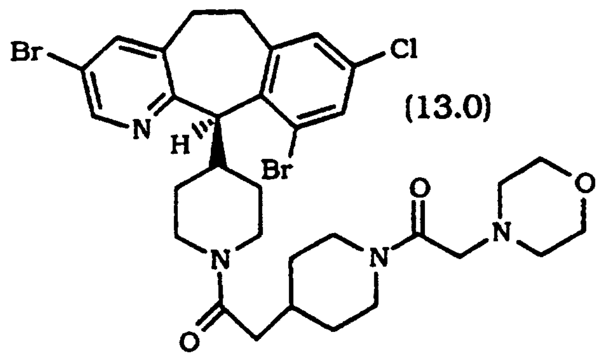

(13.0)

;

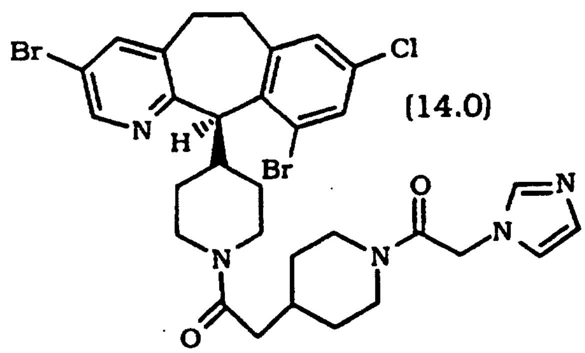

(14.0)

;

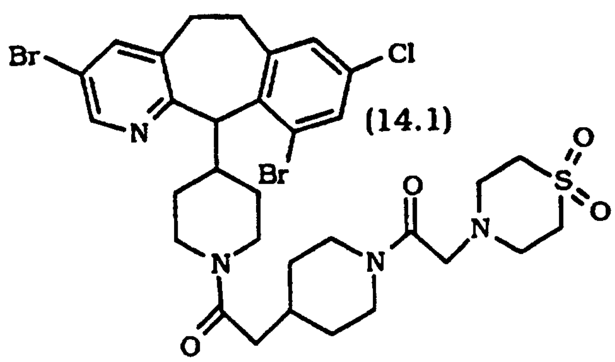

(14.1)

;

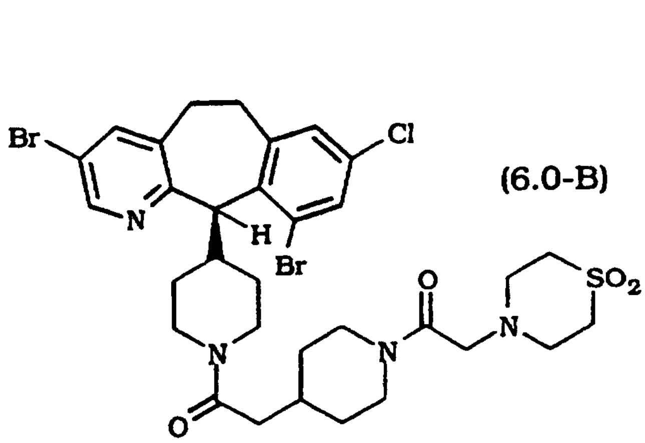

(6.0-B)

;

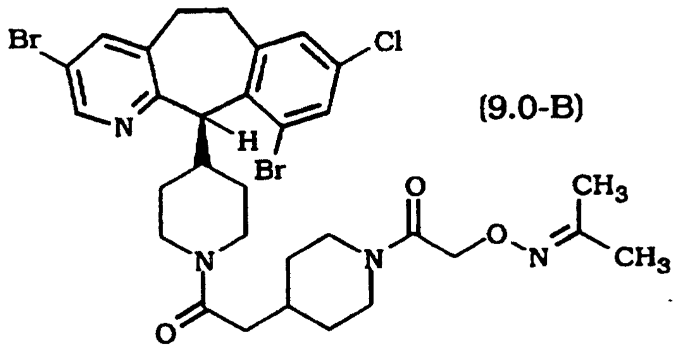

(9.0-B)

;

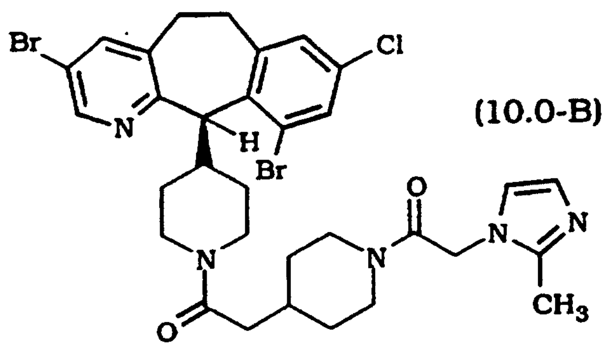

(10.0-B)

;

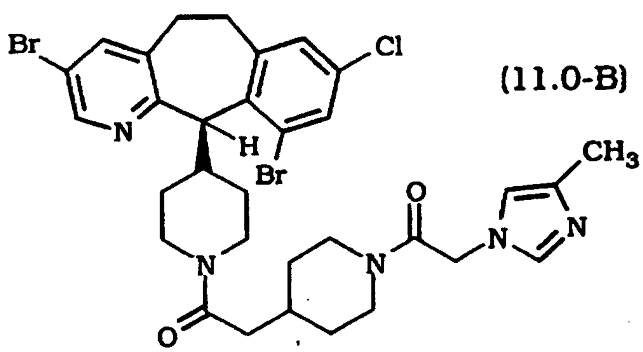

(11.0-B)

;

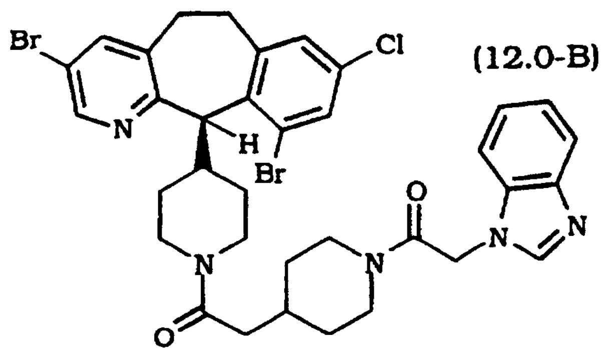

(12.0-B)

;

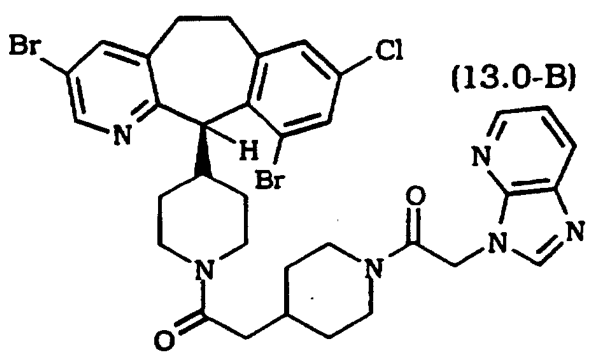

(13.0-B)

;

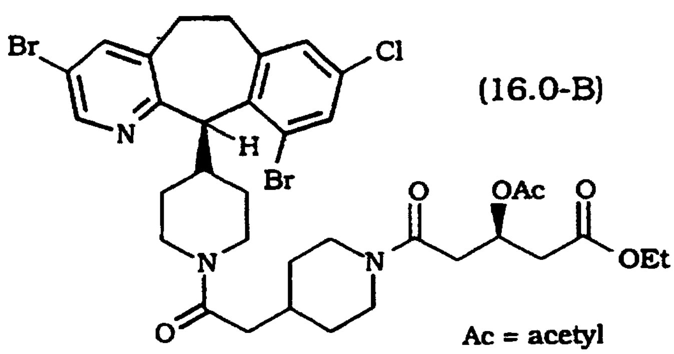

(16.0-B)

Ac = acetyl

;

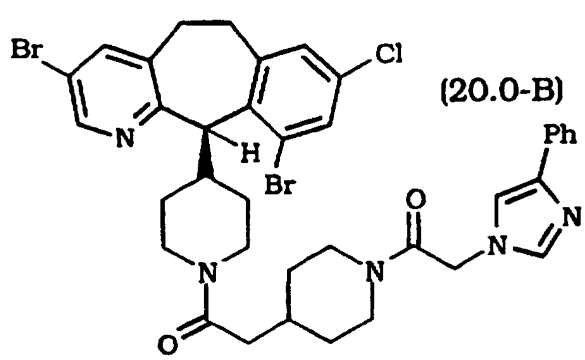

(20.0-B)

;

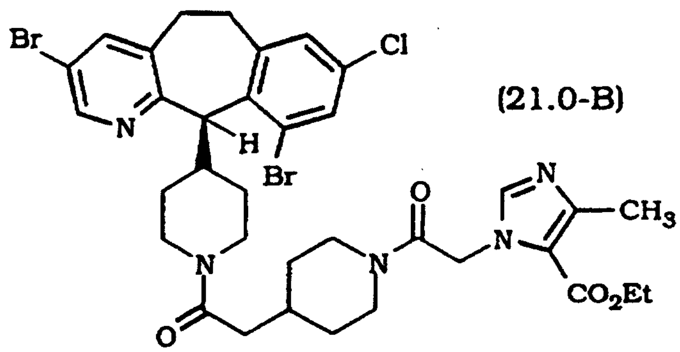

(21.0-B)

;

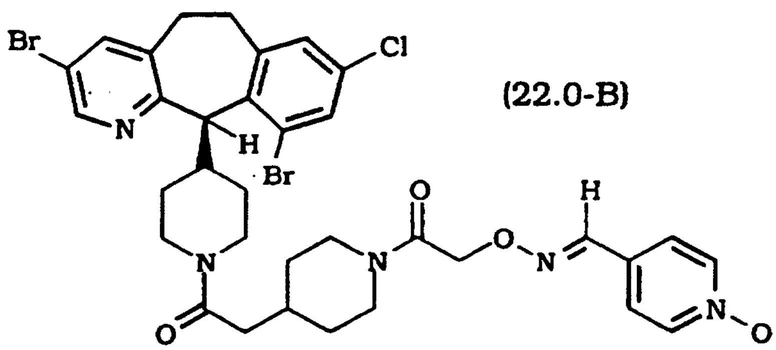

(22.0-B)

;

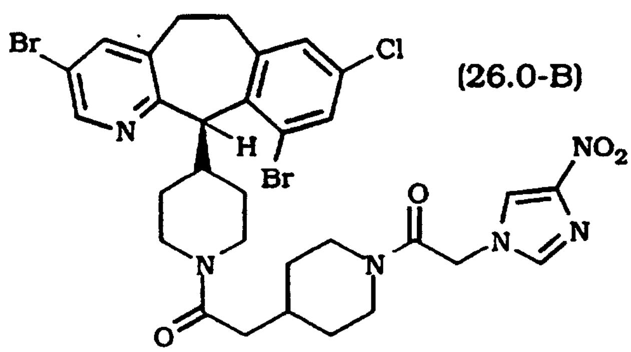

(26.0-B)

;

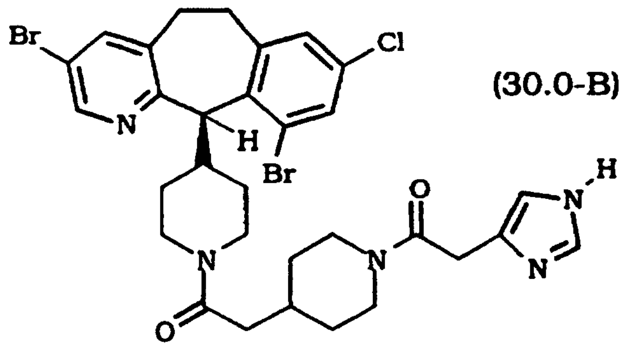

(30.0-B)

;

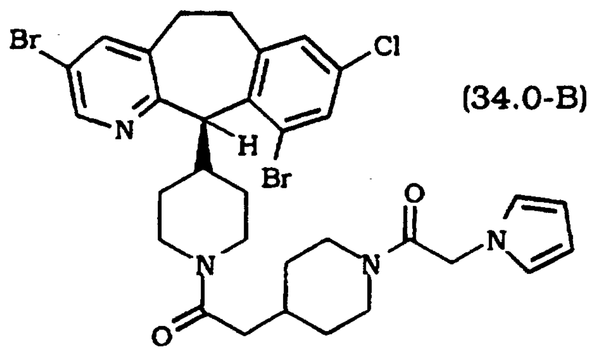

(34.0-B)

;

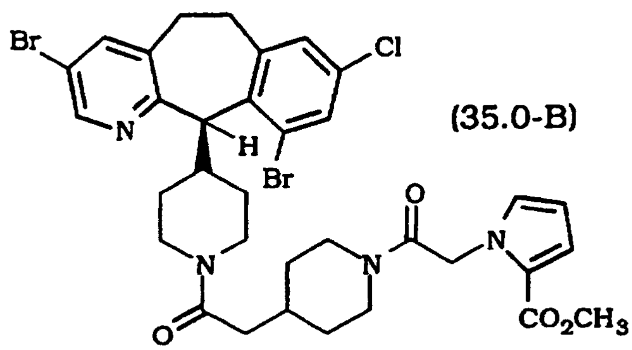

(35.0-B)

;

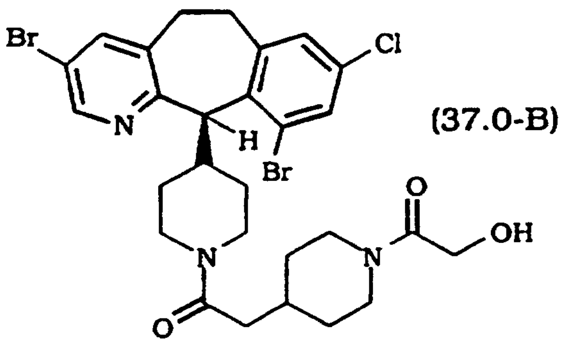

(37.0-B)

;

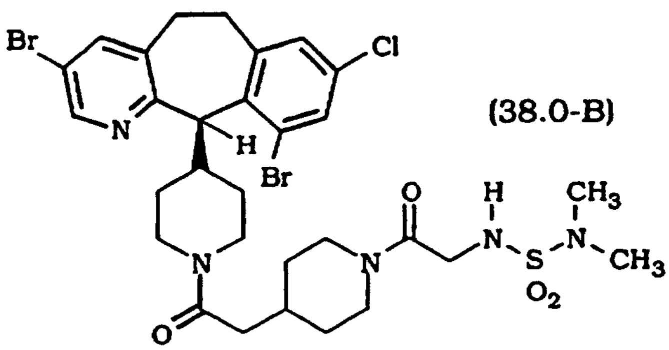

(38.0-B)

;

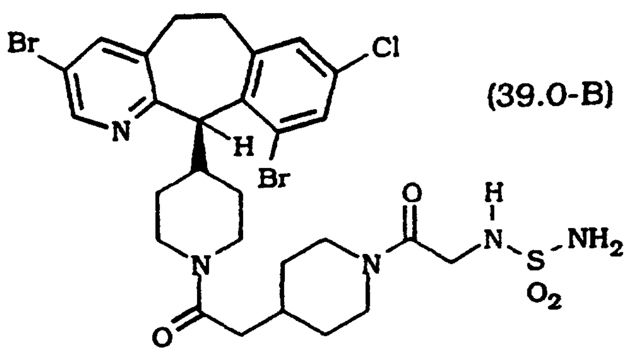

(39.0-B)

;

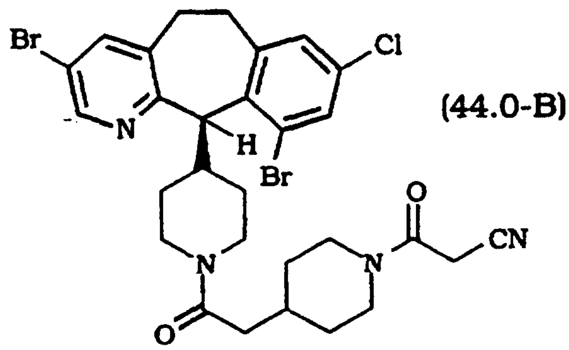

(44.0-B)

;

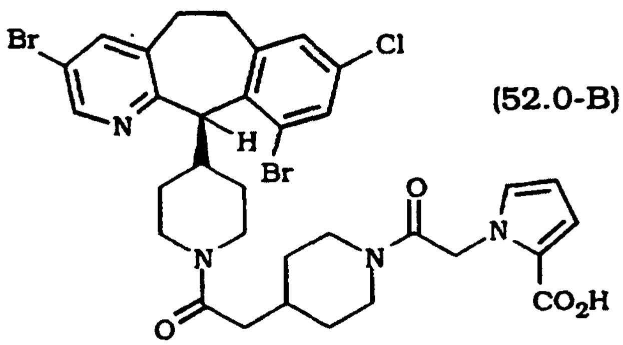

(52.0-B)

;

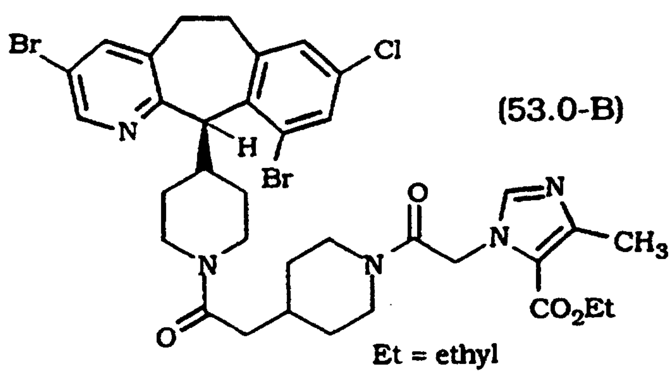

(53.0-B)

Et = ethyl

;

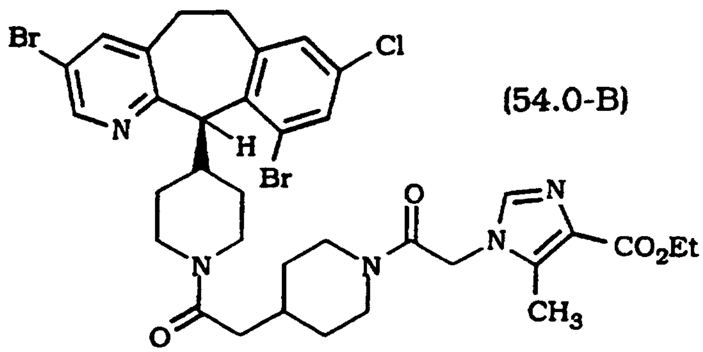

(54.0-B)

;

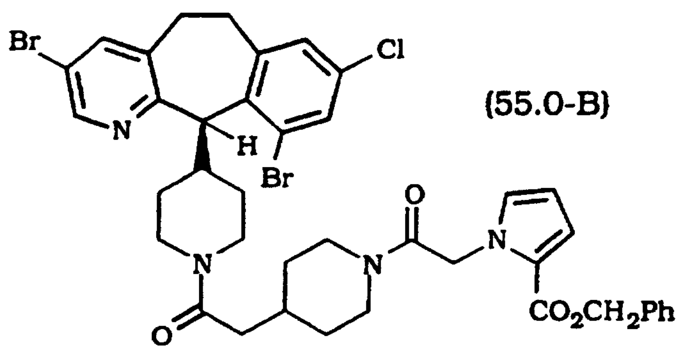

(55.0-B)

Ph = phenyl ;

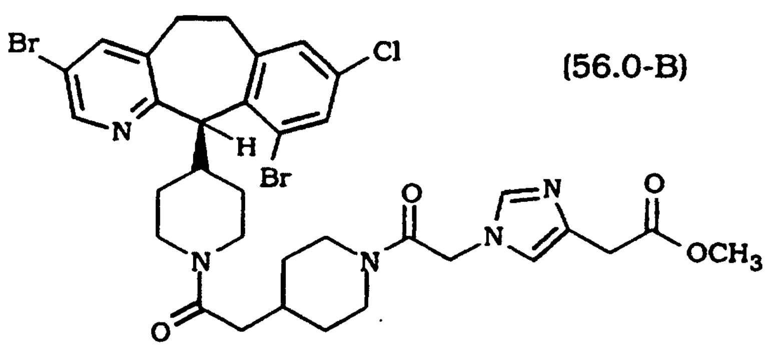

(56.0-B)

;

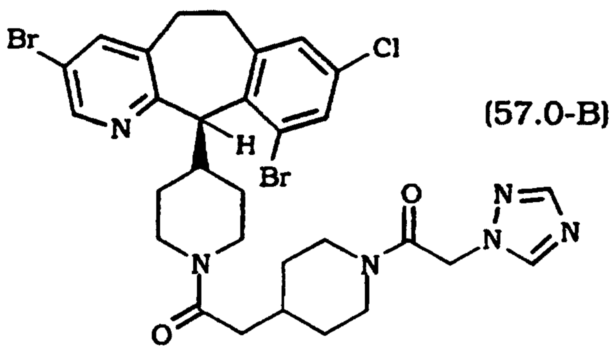

(57.0-B)

;

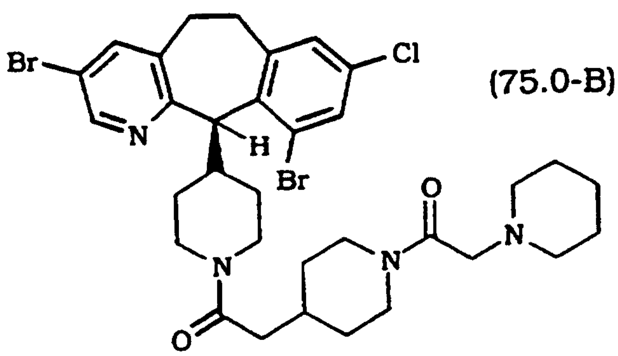

(75.0-B)

;

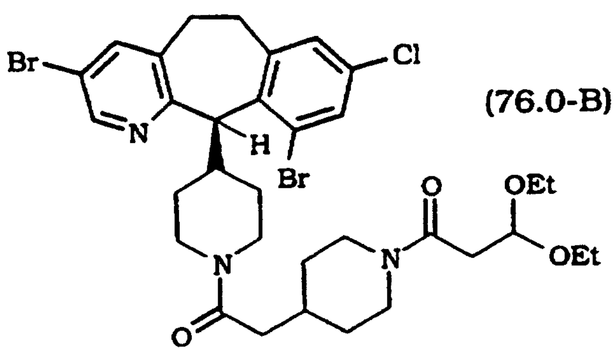

(76.0-B)

;

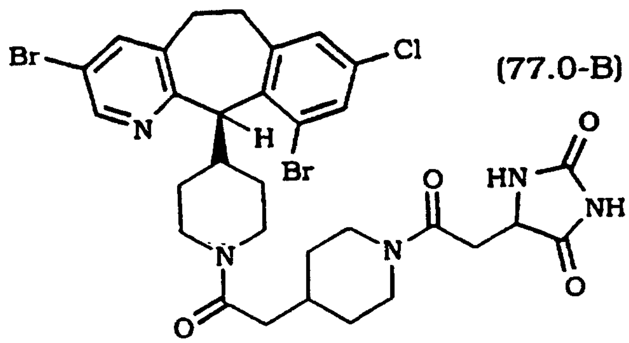

(77.0-B)

;

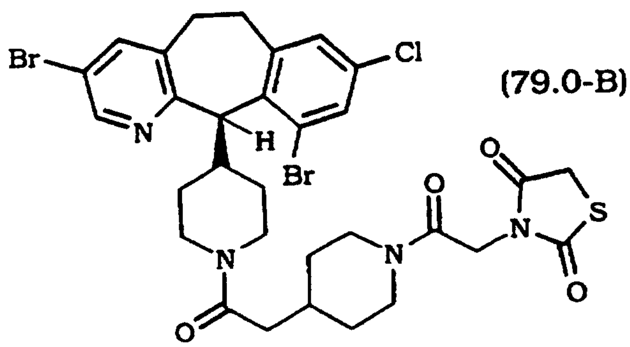

(79.0-B)

;

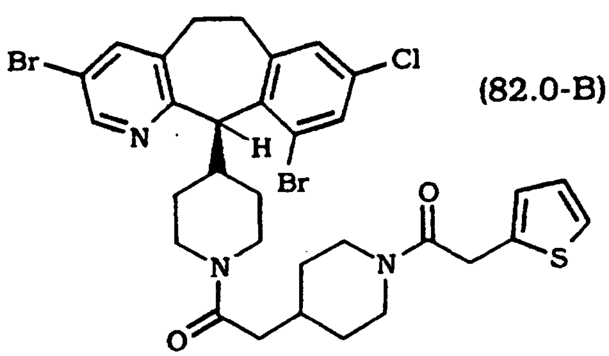

(82.0-B)

;

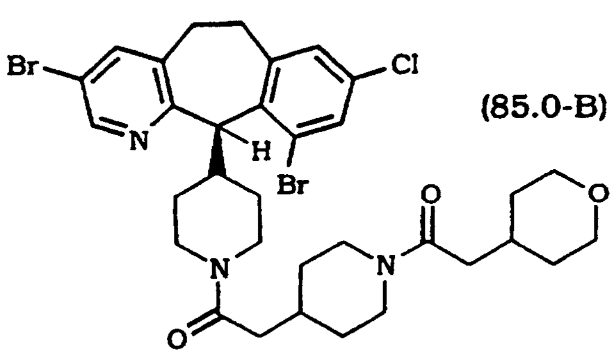

(85.0-B)

;

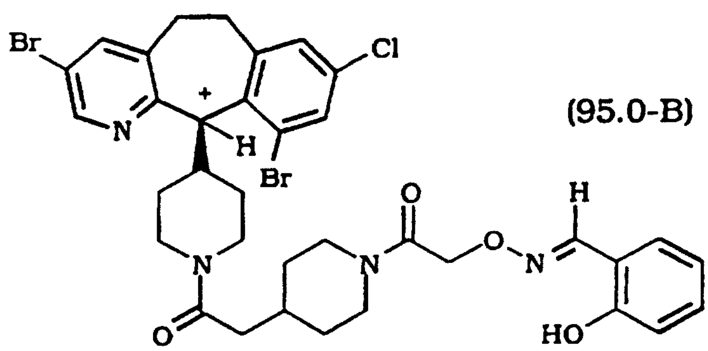

(95.0-B)

;

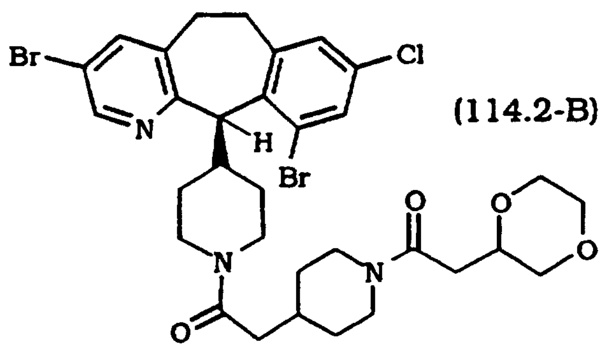

(114.2-B)

;

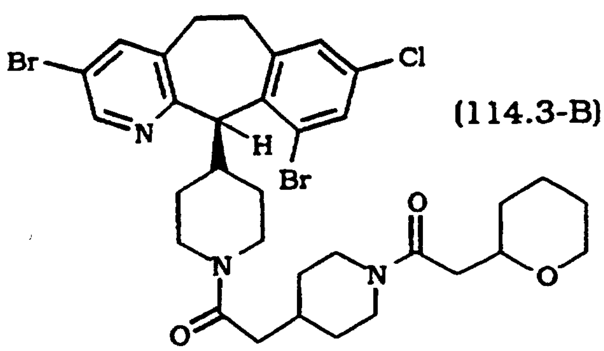

(114.3-B)

;

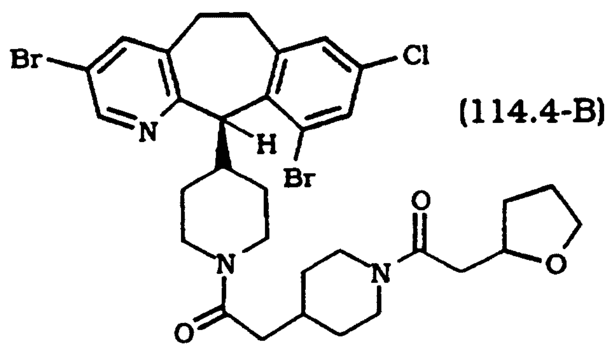

(114.4-B)

;

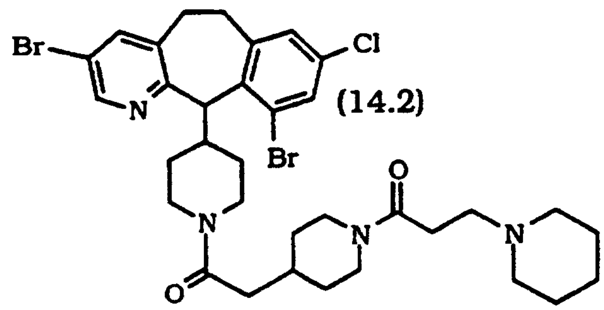

(14.2)

;

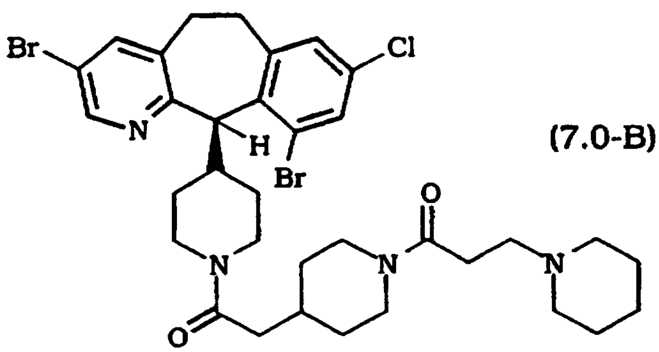

(7.0-B)

;

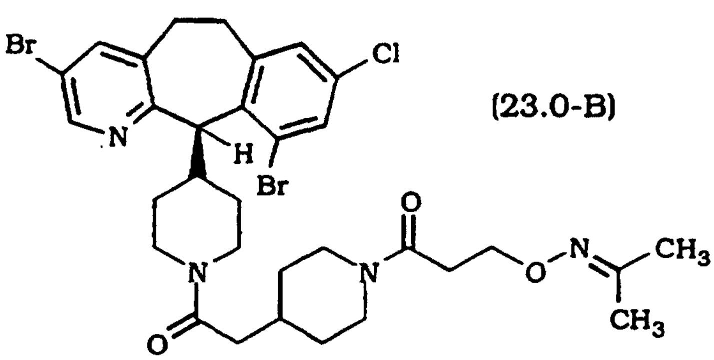

(23.0-B)

;

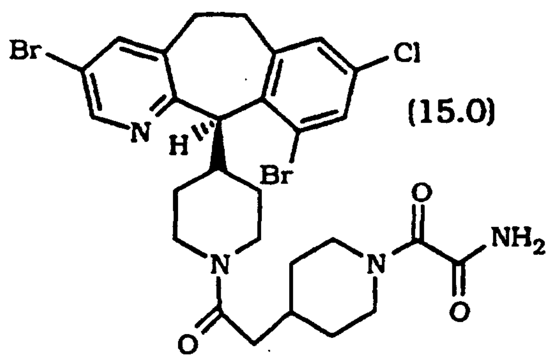

(15.0)

;

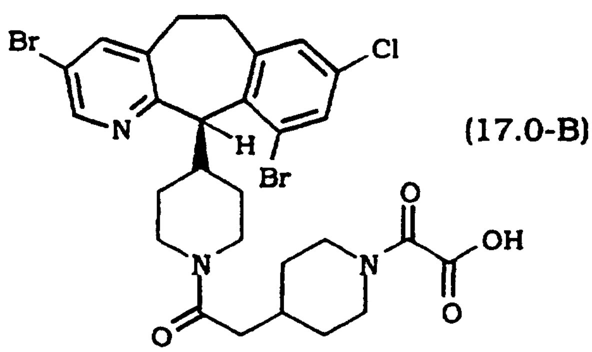

(17.0-B)

;

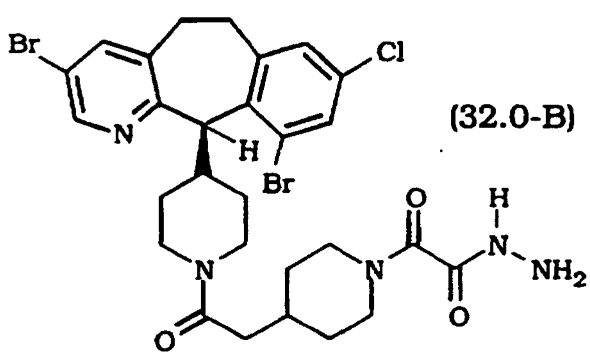

(32.0-B)

;

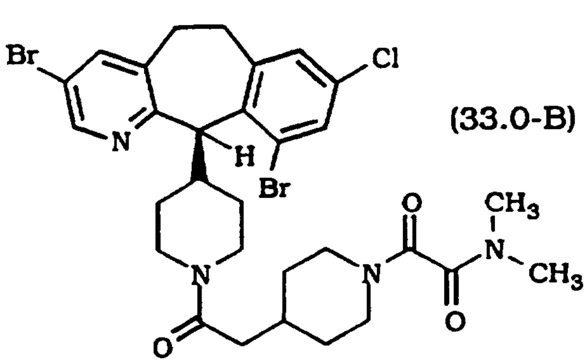

(33.0-B)

;

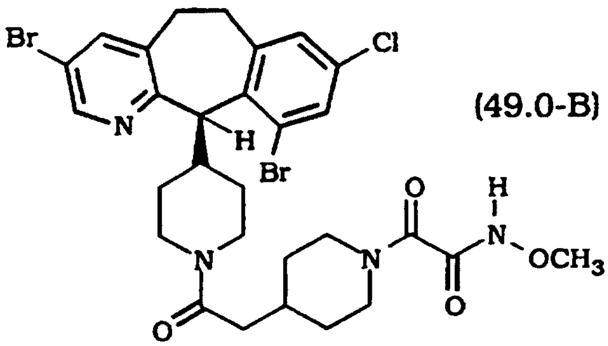

(49.0-B)

;

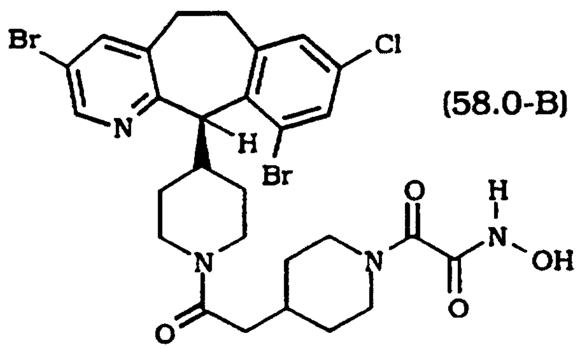

(58.0-B)

;

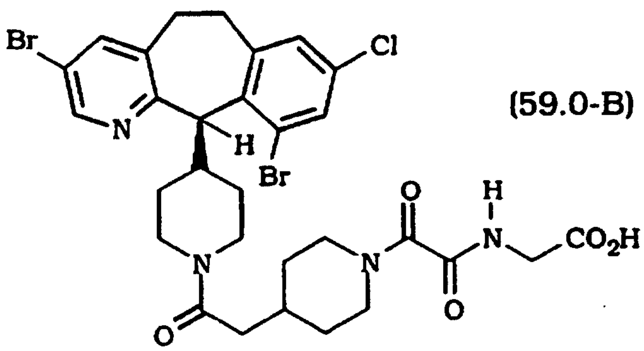

(59.0-B)

;

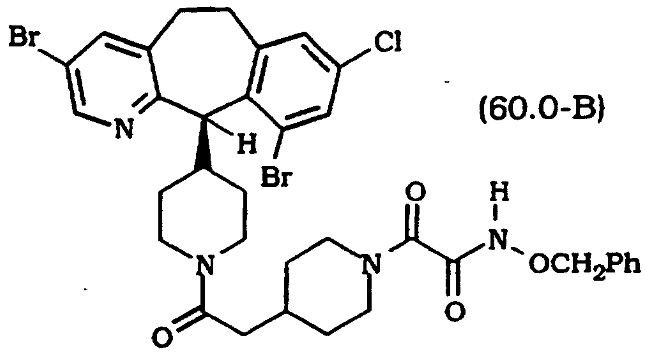

(60.0-B)

;

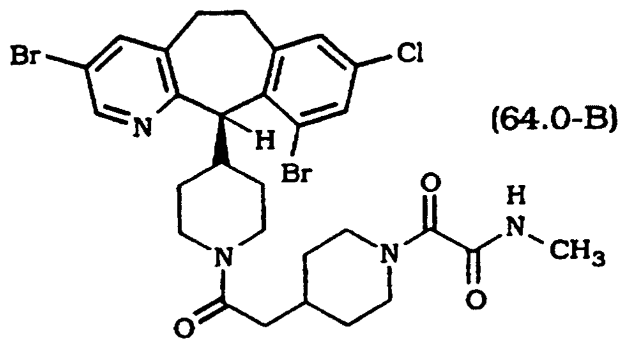

(64.0-B)

;

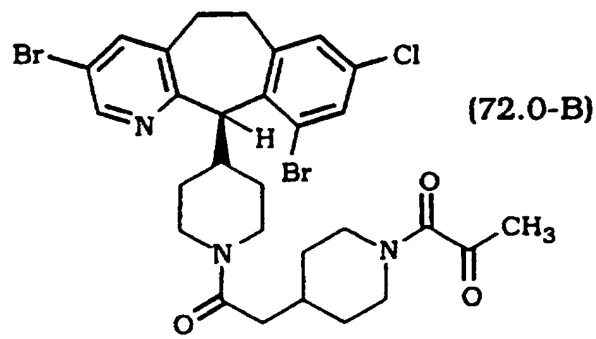

(72.0-B)

;

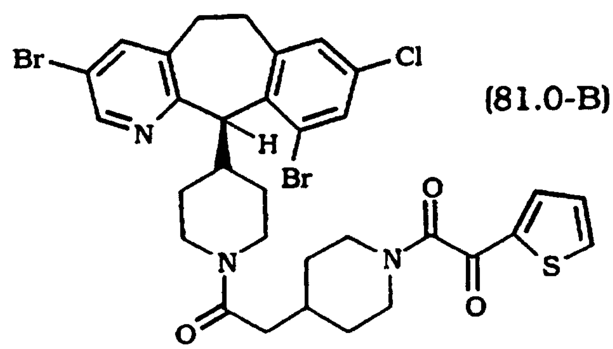

(81.0-B)

;

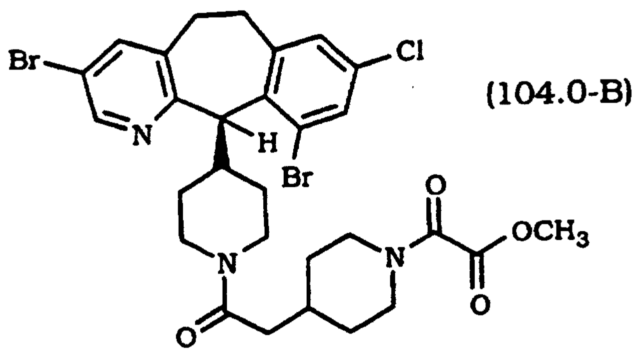

(104.0-B)

;

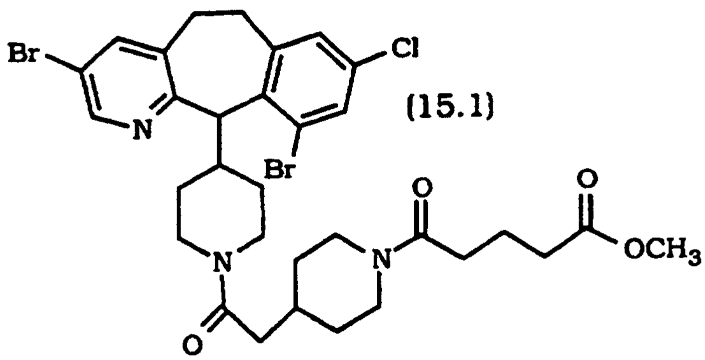

(15.1)

;

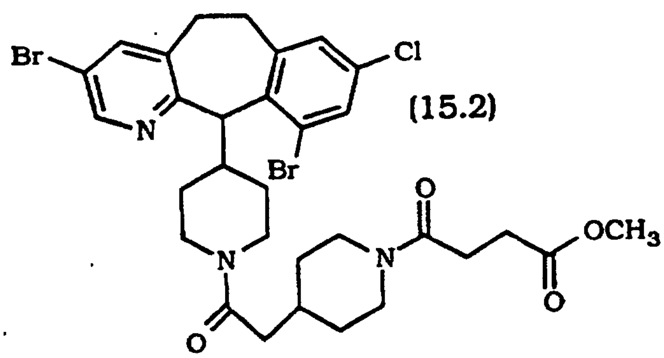

(15.2)

;

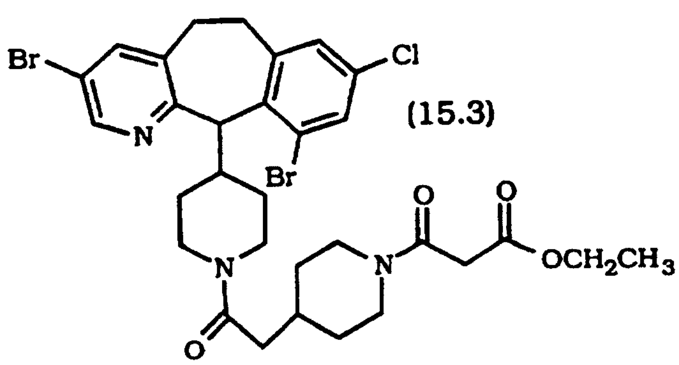

(15.3)

;

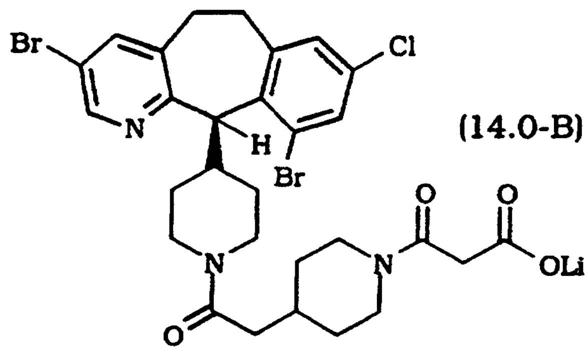

(14.0-B)

;

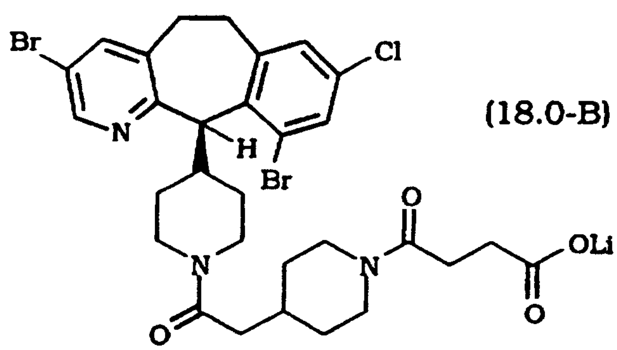

(18.0-B)

;

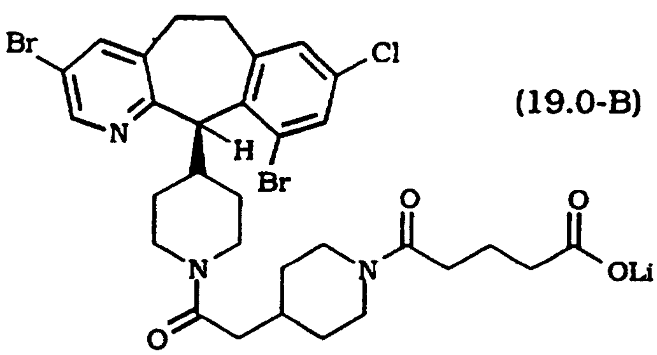

(19.0-B)

;

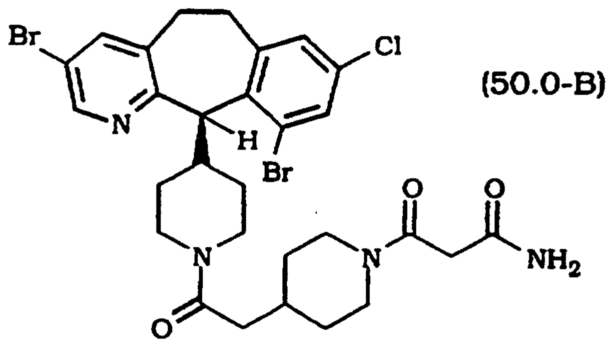

(50.0-B)

;

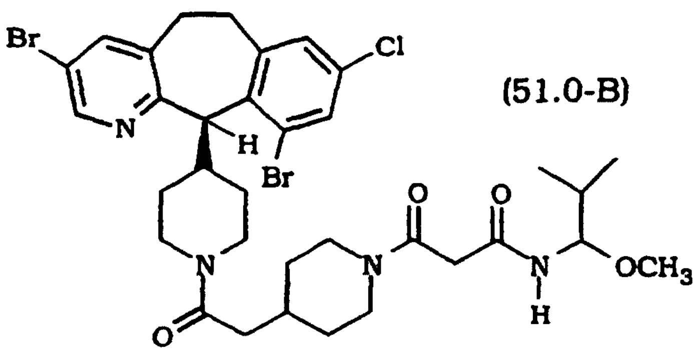

(51.0-B)

;

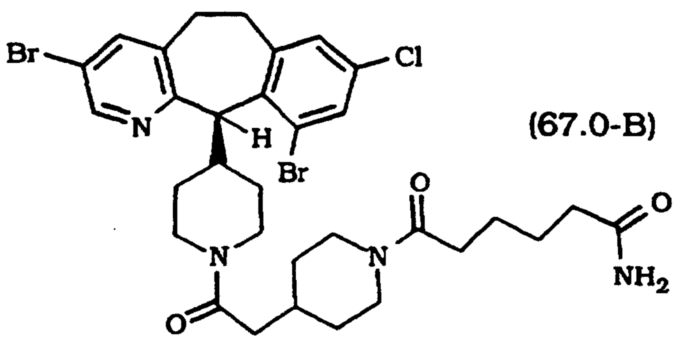

(67.0-B)

;

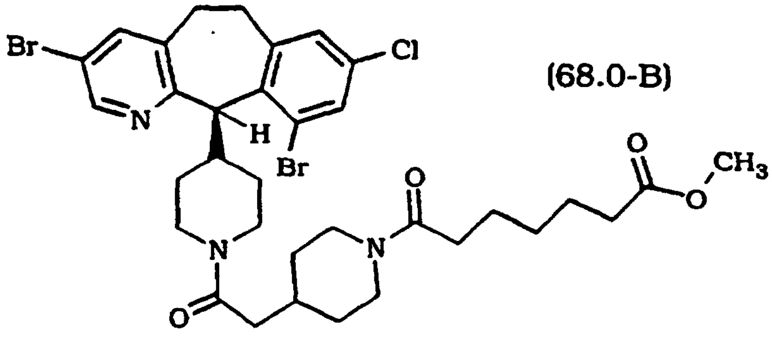

(68.0-B)

;

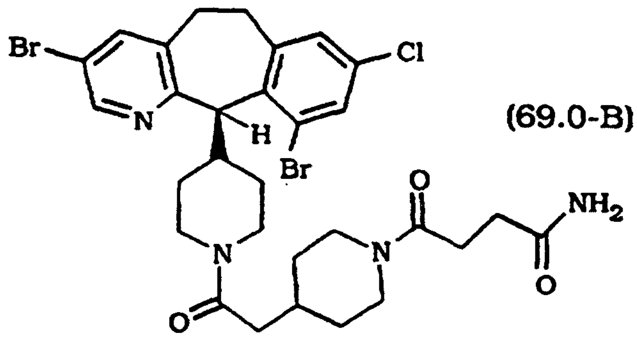

(69.0-B)

;

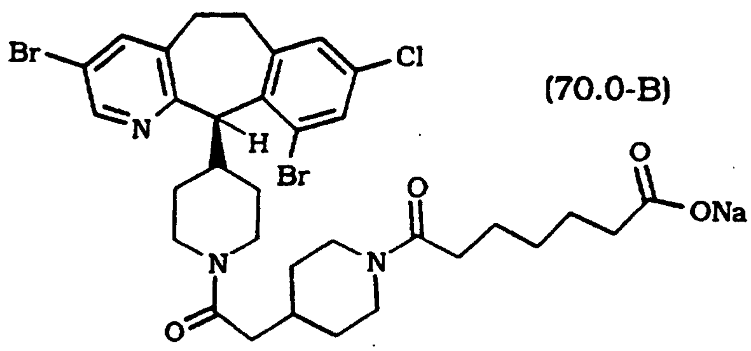

(70.0-B)

;

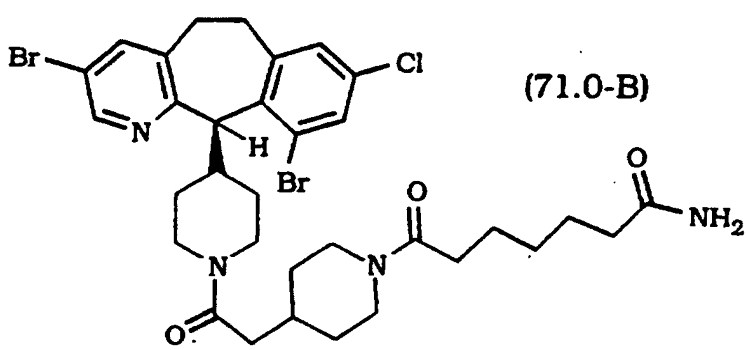

(71.0-B)

;

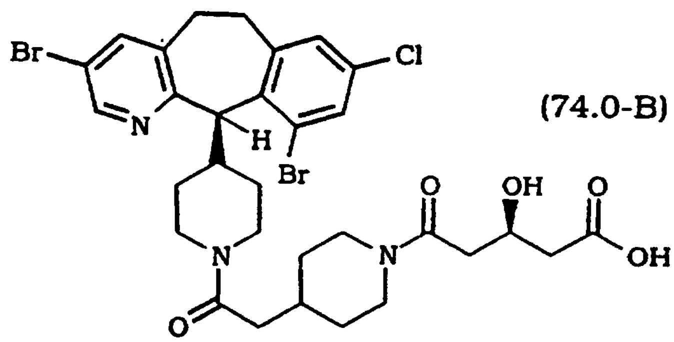

(74.0-B)

;

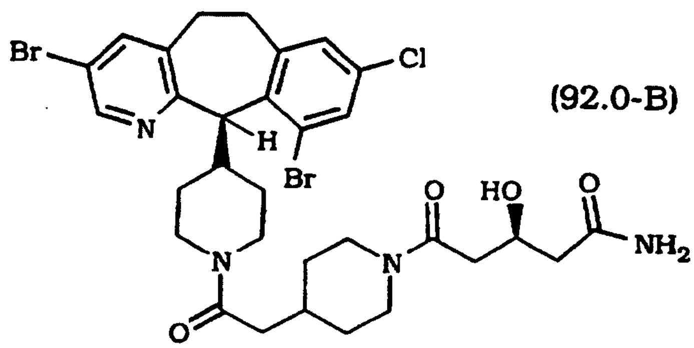

(92.0-B)

;

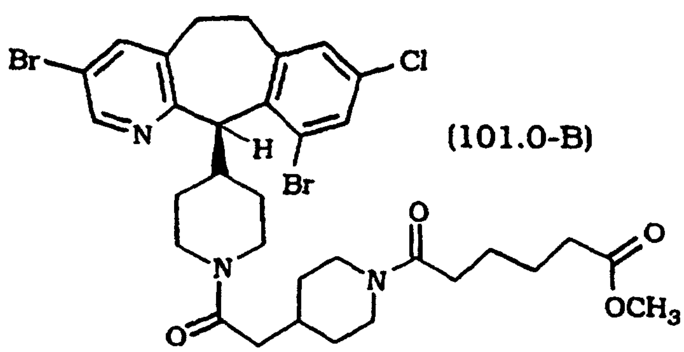

(101.0-B)

;

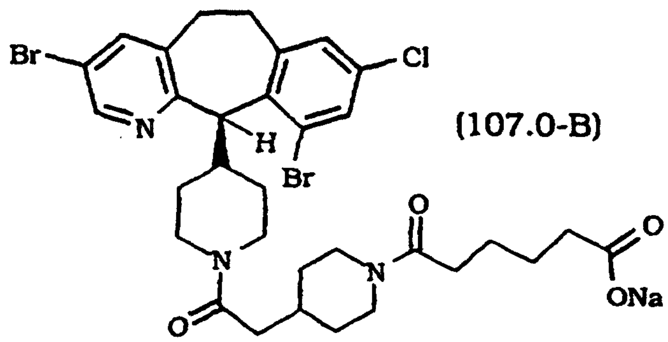

(107.0-B)

;

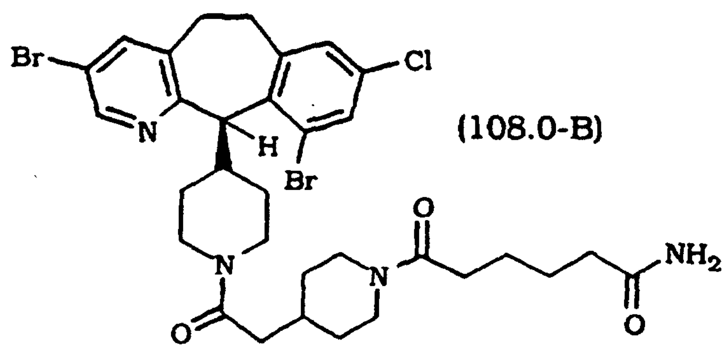

(108.0-B)

;

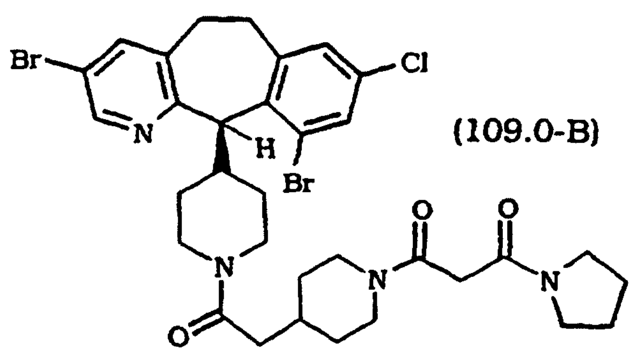

(109.0-B)

;

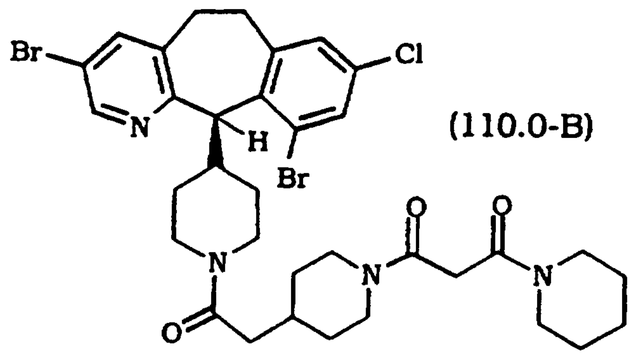

(110.0-B)

;

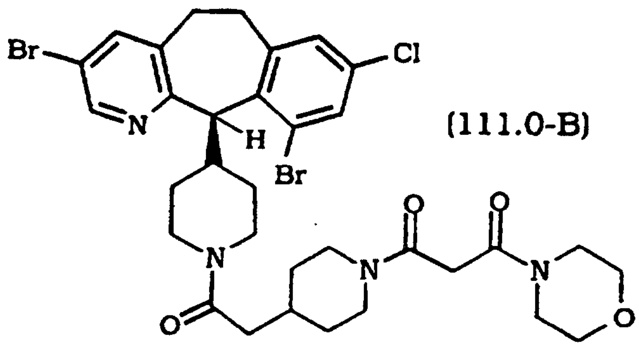

(111.0-B)

;

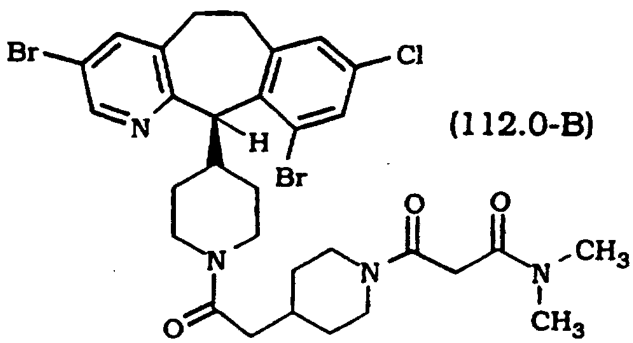

(112.0-B)

;

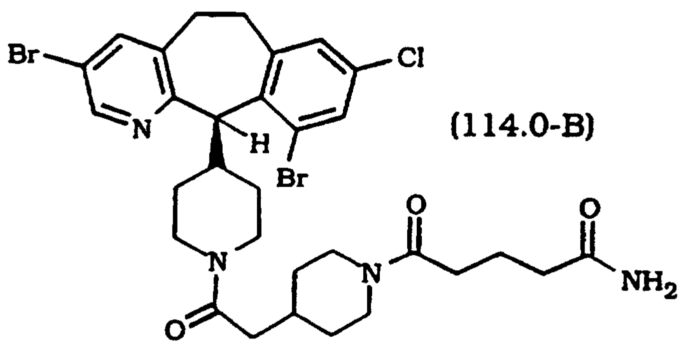

(114.0-B)

;

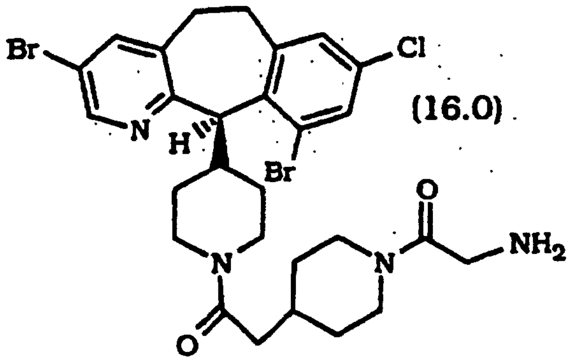

(16.0) ;

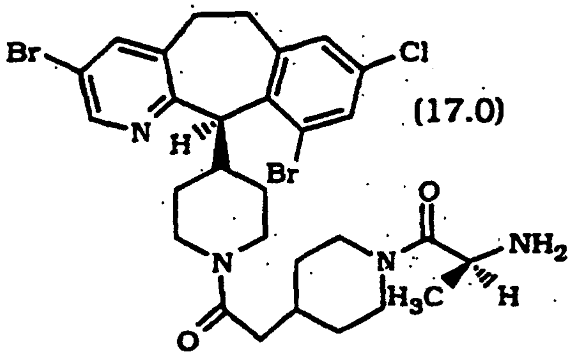

(17.0)

or

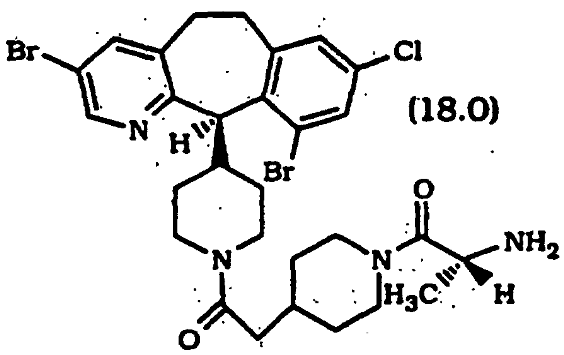

(18.0)

or phannaceutically acceptable salts or solvates thereof.

**10.** A pharmaceutical composition comprising an effective amount of a compound of any of Claims 1-9 in combination with a pharmaceutically acceptable carrier.

**11.** The use of a compound of any of Claims 1-9 for the manufacture of a medicament for the treatment of tumor cells.

**12.** Use according to Claim 11 wherein the cells treated are pancreatic tumor cells, lung cancer cells, myeloid leukemia tumor cells, thyroid follicular tumor cells, myelodysplastic tumor cells, epidermal carcinoma tumor cells, bladder carcinoma tumor cells, colon tumors cells, breast tumor cells or prostate tumor cells.

**13.** The use of a compound of any of Claims 1-10 for the manufacture of a medicament for the inhibition of farnesyl protein transferase.

**Patentansprüche**

1. Verbindung mit der Formel

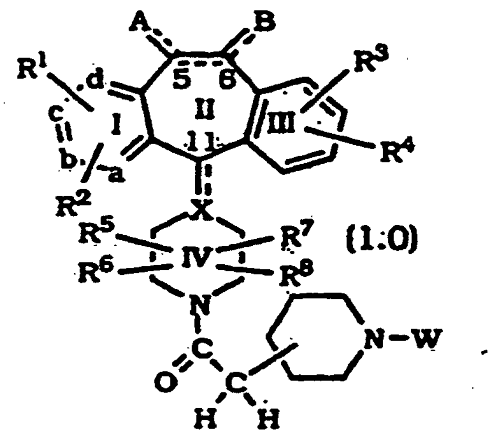

(1:0)

oder pharmazeutisch annehmbares Salz oder Solvat davon, wobei
einer von a, b, c und d für N oder $NR^9$ steht, wobei $R^9$ $O^-$, $-CH_3$ oder $-(CH_2)_nCO_2H$ ist, wobei n 1 bis 3 ist, und die verbleibenden Gruppen a, b, c und d für $CR^1$ oder $CR^2$ stehen; oder
jedes von a, b, c und d unabhängig ausgewählt ist aus $CR^1$ oder $CR^2$;
$R^2$ H ist und $R^1$, $R^3$ und $R^4$ Halogen sind;
$R^5$, $R^6$, $R^7$ und $R^8$ jeweils unabhängig für H, $-CF_3$, $-COR^{10}$, Alkyl oder Aryl stehen, wobei das Alkyl oder Aryl gegebenenfalls durch $-OR^{10}$, $-SR^{10}$, $-S(O)_tR^{11}$, $-NR^{10}COOR^{11}$, $-N(R^{10})_2$, $-NO_2$, $-COR^{10}$, $-OCOR^{10}$, $-OCO_2R^{11}$, $-CO_2R^{10}$, $OPO_3R^{10}$ substituiert ist, oder $R^5$ mit $R^6$ kombiniert ist, um =O oder =S wiederzugeben, und/oder $R^7$ mit $R^8$ kombiniert ist, um =O oder =S wiederzugeben;
$R^{10}$ für H, Alkyl, Aryl oder Aralkyl steht;
$R^{11}$ für Alkyl oder Aryl steht;
X für N, CH oder C steht, wobei C eine optionale Doppelbindung (dargestellt durch die punktierte Line) zu Kohlenstoffatom 11 enthalten kann;
die punktierte Linie zwischen den Kohlenstoffatomen 5 und 6 für eine optionale Doppelbindung steht, so dass, wenn eine Doppelbindung vorhanden ist, A und B unabhängig für $-R^{10}$, Halogen, $-OR^{11}$, $-OCO_2R^{11}$ oder $-OC(O)R^{10}$ stehen, und wenn keine Doppelbindung zwischen den Kohlenstoffatomen 5 und 6 vorhanden ist, A und B jeweils unabhängig für $H_2$, $-(OR^{11})_2$; H und Halogen, Dihalogen, Alkyl und H, $(Alkyl)_2$, -H und $-OC(O)R^{10}$, H und $-OR^{10}$, =O, Aryl und H, $=NOR^{10}$ oder $-O-(CH_2)_p-O-$ stehen, wobei p 2, 3 oder 4 ist, und
W für eine Gruppe ausgewählt aus der Gruppe bestehend aus

(1)

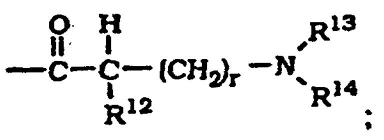

;

(2)

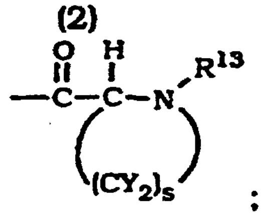

;

(3)

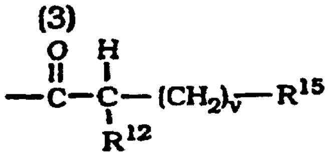

und

(4)

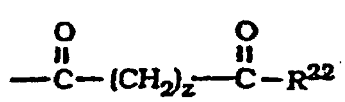

steht, worin
$R^{12}$ ausgewählt ist aus der Gruppe bestehend aus (a) H; (b) Alkyl; (c) Aralkyl und (d) Heteroarylalkyl;
$R^{13}$ und $R^{14}$ jeweils unabhängig ausgewählt sind aus der Gruppe bestehend aus (a) H; (b) -C(O)OR$^{16}$, wobei $R^{16}$ für Alkyl, Aralkyl und Heteroaralkyl steht; (c) -$SO_2R^{17}$, wobei $R^{17}$ ausgewählt ist aus der Gruppe bestehend aus $NH_2$, -N(alkyl)$_2$, wobei jedes Alkyl gleich oder unterschiedlich ist, Alkyl, Aryl, Aralkyl, Heteroaryl und Heteroaralkyl; (d) -C(O)R$^{18}$, wobei $R^{18}$ ausgewählt ist aus der Gruppe bestehend aus Aryl, Alkyl, Aralkyl, Heteroaryl und Heteroaralkyl; (e) $C_1$- bis $C_6$-Alkyl; (f) Alkaryl und (g) $C_3$- bis $C_6$-Cycloalkyl;
r 0, 1 oder 2 ist;
s für 1, 2, 3, 4 oder 5 steht; und jedes Y für jede -$CY_2$-Gruppe unabhängig ausgewählt ist aus H oder -OH mit der Maßgabe, dass nicht beide Y-Substituenten jeder -$CY_2$-Gruppe -OH sind, und mit der Maßgabe, dass bei der -$CY_2$-Gruppe a zu dem Stickstoff beide Y-Substituenten H sind, so dass die Gruppe

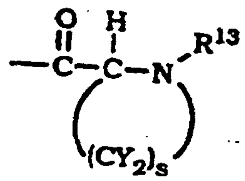

einen 3-, 4-, 5-, 6- oder 7-gliedrigen Ring bildet;
v 0, 1 oder 2 ist;
$R^{15}$ ausgewählt ist aus der Gruppe bestehend aus:

(a) Heteroaryl;
(b) einer Gruppe ausgewählt aus:

(1) 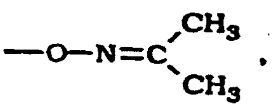 ,

(3) 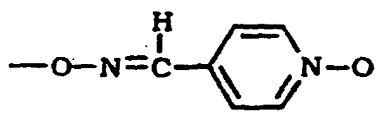 ,

(2) 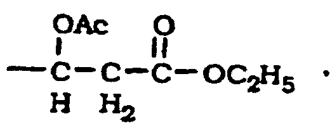 ,

(4) 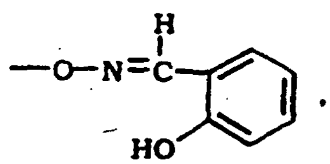 ,

(5) -$CH(OCH_2CH_3)_2$
(6) -OH und
(7) -CN und

(c) Heterocycloalkyl ausgewählt aus der Gruppe bestehend aus

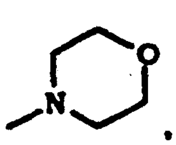 , 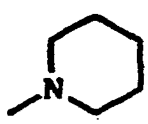 , 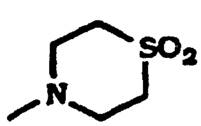 , 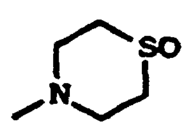 , 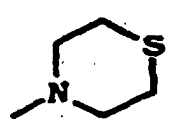 ,

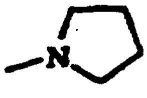, 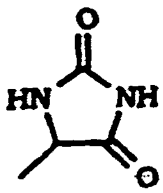, 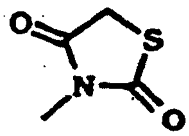, 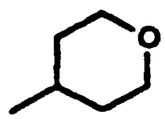, 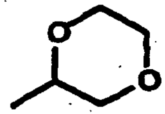, 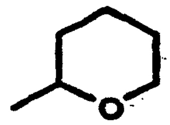

und

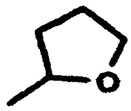;

z 0, 1, 2, 3, 4 oder 5 ist, wobei jede $-CH_2-$ Gruppe gegebenenfalls mit einer -OH Gruppe substituiert ist;
$R^{22}$ für eine Gruppe ausgewählt aus

(1) 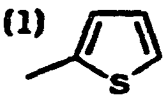, (2) 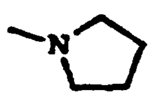, (3) 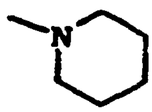, (4) 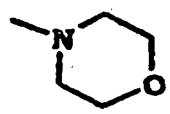,

(5) Alkyl (z. B. $-CH_3$),
(6) $-OR^{23}$, wobei $R^{23}$ ausgewählt ist aus der Gruppe bestehend aus Alkyl, Aryl und H, und
(7)

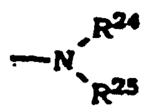

steht, wobei $R^{24}$ und $R^{25}$ unabhängig ausgewählt sind aus der Gruppe bestehend aus $-NH_2$, Alkoxy, -OH, $-CH_2CO_2H$, $-OCH_2Ph$, $-CH(OCH_3)CH(CH_3)_2$, Alkyl, Aryl, H, Aralkyl und Heteroaralkyl; oder $R^{24}$ und $R^{25}$ zusammengenommen eine Kohlenstoffkette mit 4 oder 5 $(-CH_2-)$ Gruppen bilden, so dass $R^{24}$ und $R^{25}$ zusammen mit dem Stickstoff, an den sie gebunden sind, einen 5-oder 6-gliedrigen Heterocycloalkylring bilden; wobei, wenn nicht anders gesagt,

Alkenyl für geradkettige und verzweigte Kohlenstoffketten mit mindestens einer Kohlenstoff-Kohlenstoff-Doppelbindung steht und 2 bis 12 Kohlenstoffatome enthält;
Alkinyl für geradkettige und verzweigte Kohlenstoffketten mit mindestens einer Kohlenstoff-Kohlenstoff-Dreifachbindung steht und 2 bis 12 Kohlenstoffatome enthält;
Alkyl (einschließlich der Alkylanteile von Aralkyl und Heteroarylalkyl) für geradkettige und verzweigte Kohlenstoffketten steht und ein bis zwanzig Kohlenstoffatome enthält:
Aralkyl für eine Arylgruppe wie nachfolgend definiert steht, die an eine Alkylgruppe wie bereits definiert gebunden ist;
Aryl (einschließlich des Arylanteils von Aralkyl) für eine carbocyclische Gruppe steht, die 6 bis 15 Kohlenstoffatome enthält und mindestens einen aromatischen Ring aufweist (Aryl ist z. B. ein Phenylring), wobei alle verfügbaren substituierbaren Kohlenstoffatome der carbocyclischen Gruppe als mögliche Bindungspunkte vorgesehen sind, wobei die carbocyclische Gruppe gegebenenfalls mit einem oder mehreren von Halogen, Alkyl, Hydroxy, Alkoxy, Phenoxy, $CF_3$, Amino, Alkylamino, Dialkylamino, $-COOR^{10}$ oder $-NO_2$ substituiert (z. B. 1 bis 3) ist;

Halogen für Fluor, Chlor, Brom und Iod steht;
Heteroaryl für cyclische Gruppen steht, die gegebenenfalls mit $R^3$, $R^4$, Phenyl und/oder $-CH_2C(O)OCH_3$ substituiert sind, wobei die cyclischen Gruppen mindestens ein Heteroarom ausgewählt aus 0, S oder N aufweisen, wobei das Heteroatom eine carbocyclische Ringstruktur unterbricht und eine ausreichende Anzahl delokalisierter n-Elektronen aufweist, um aromatischen Charakter zu liefern, wobei die aromatischen heterocyclischen Gruppen 2 bis 14 Kohlenstoffatome enthalten;
Heteroarylalkyl (Heteroaralkyl) für eine Heteroarylgruppe wie bereits definiert gebunden an eine Alkylgruppe wie bereits definiert steht;
Heterocycloalkyl für einen gesättigten, verzweigten oder unverzweigten carbocyclischen Ring steht, der 3 bis 15 Kohlenstoffatome, vorzugsweise 4 bis 6 Kohlenstoffatome enthält, wobei der carbocyclische Ring durch 1 bis 3 Heterogruppen ausgewählt aus -O-, -S- oder $-NR^{10}$ unterbrochen ist.

**2.** Verbindung nach Anspruch 1, wobei a N ist und b, c und d Kohlenstoff sind; A und B jeweils $H_2$ sind; die optionale Bindung zwischen C5 und C6 fehlt; X CH ist; und $R^5$, $R^6$, $R^7$ und $R^8$ H sind.

**3.** Verbindung nach einem der Ansprüche 1 bis 2, wobei W ausgewählt ist aus der Gruppe bestehend aus

(A)

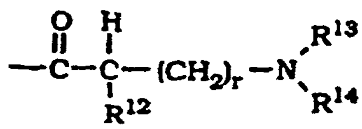

wobei

(1) r 0 ist;
(2) $R^{12}$ ausgewählt ist aus der Gruppe bestehend aus (a) H; (b) Alkyl; (c) Aralkyl und (d) Heteroaralkyl; und
(3) $R^{13}$ und $R^{14}$ unabhängig ausgewählt sind aus der Gruppe bestehend aus (a) H; (b) $-C(O)OR^{16}$, wobei $R^{16}$ Alkyl ist; (c) $-SO_2R^{17}$, wobei $R^{17}$ Alkyl oder Aryl ist; (d) $-C(O)R^{18}$,
wobei $R^{18}$ Aryl ist; und (e) Alkyl;

(B)

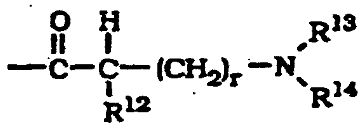

wobei:

(1) r 1 oder 2 ist;
(2) $R^{12}$ H ist und
(3) $R^{13}$ Alkyl ist und $R^{14}$ H, Alkyl oder $-C(O)OR^{16}$ ist,

wobei $R^{16}$ Alkyl ist;

(C)

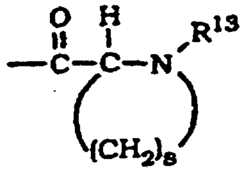

wobei

(1) s 1, 2, 3, 4 oder 5 ist; und
(2) $R^{13}$ ausgewählt ist aus der Gruppe bestehend aus

(a) H und -C(O)OR[16], wobei $R^{16}$ Alkyl ist;

(D) 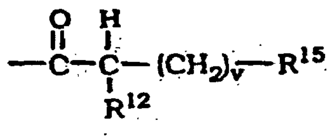

wobei

(1) v 0 ist;
(2) $R^{12}$ H ist; und
(3) $R^{15}$ ausgewählt ist aus der Gruppe bestehend aus:

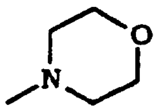, 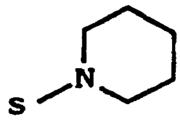, 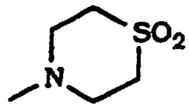, 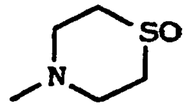,

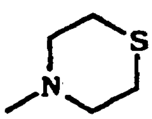, 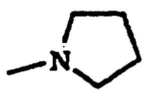, 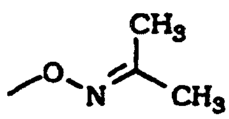, 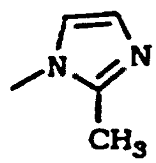,

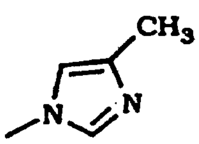, 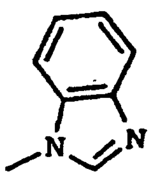, 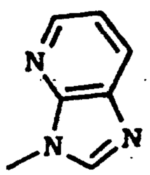, 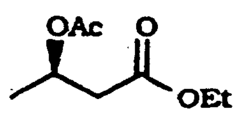,

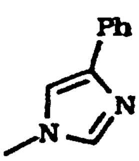, 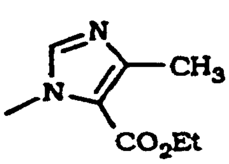, 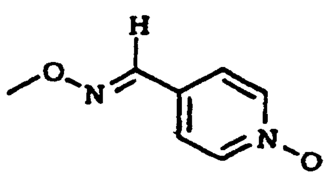.

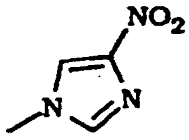, 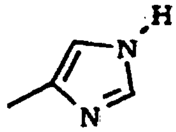, 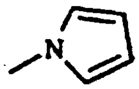, 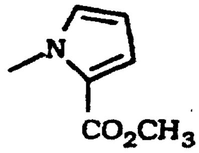,

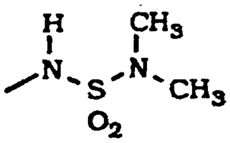, 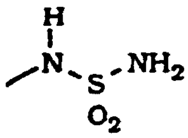, 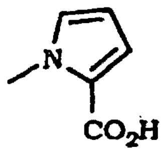, 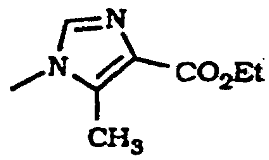,

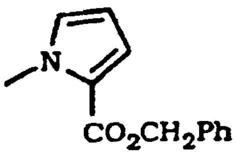, 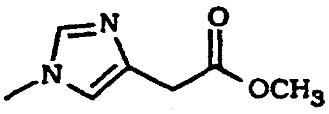, 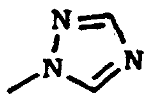, 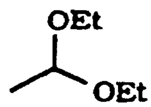,

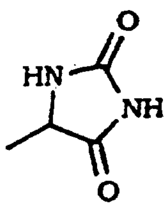, 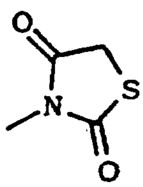, 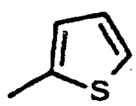, 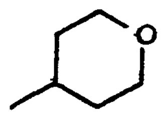,

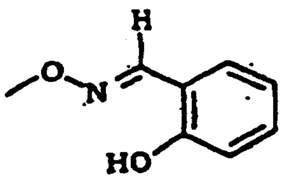, 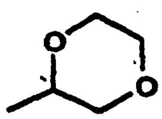, 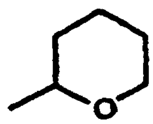, 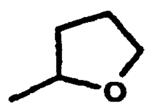.

und -OH, -CN;

(E)

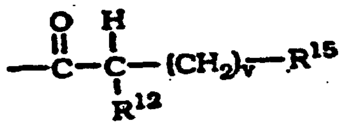

wobei

(1) v 1 oder 2 ist;

(2) $R^{12}$ H ist; und
(3) $R^{15}$ Heterocycloalkyl ist;

(F) 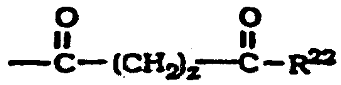

wobei

(1) z O ist;
(2) $R^{22}$ $-NR^{24}R^{25}$ ist; und
(3) $R^{24}$ und $R^{25}$ unabhängig ausgewählt sind aus H, $-NH_2$, Alkyl, Alkoxy, -OH, $-CH_2CO_2H$ oder $-OCH_2C_6H_5$ und

(G) 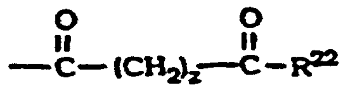 ;

wobei

(1) z 1, 2, 3, 4 oder 5 ist;
(2) $R^{22}$ ausgewählt ist aus $-OR^{23}$, -ONa, -OLi, Alkyl, $-NR^{24}R^{25}$ oder

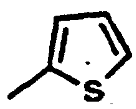

(3) $R^{23}$ Alkyl ist und
(4) $R^{24}$ und $R^{25}$ unabhängig ausgewählt sind aus H, $-CH(OCH_3)CH(CH_3)_2$

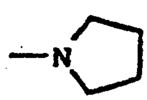 , 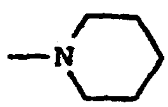 oder 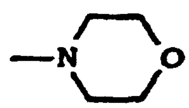 .

**4.** Verbindung nach einem der Ansprüche 1 bis 3, bei der $R^1$, $R^3$ und $R^4$ ausgewählt sind aus der Gruppe bestehend aus Cl oder Br.

**5.** Verbindung nach einem der Ansprüche 1 bis 4, bei der X CH ist.

**6.** Verbindung nach einem der Ansprüche 1 bis 5 ausgewählt aus

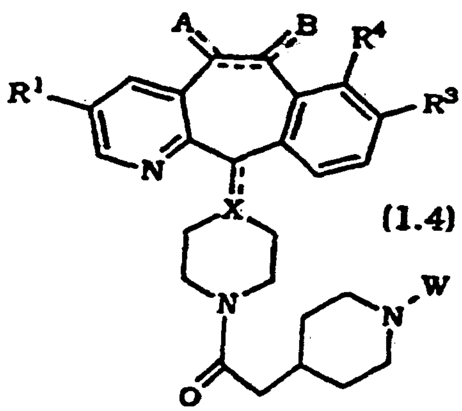

(1.4)

oder

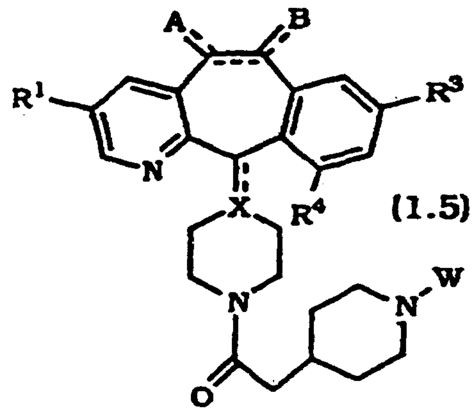

(1.5)

wobei A, B, X und W wie in Anspruch 1 definiert sind.

**7.** Verbindung nach einem der Ansprüche 1 bis 6, bei der $R^1$ Br ist, $R^3$ Cl ist und $R^4$ Br ist.

**8.** Verbindung nach einem der Ansprüche 1 bis 7, bei der die Verbindung die Formel

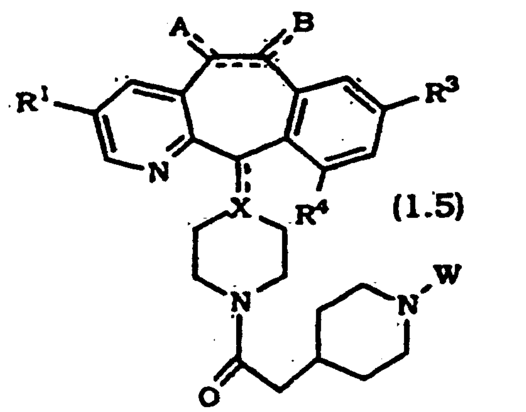

(1.5)

hat.

**9.** Verbindung nach Anspruch 1 ausgewählt aus:

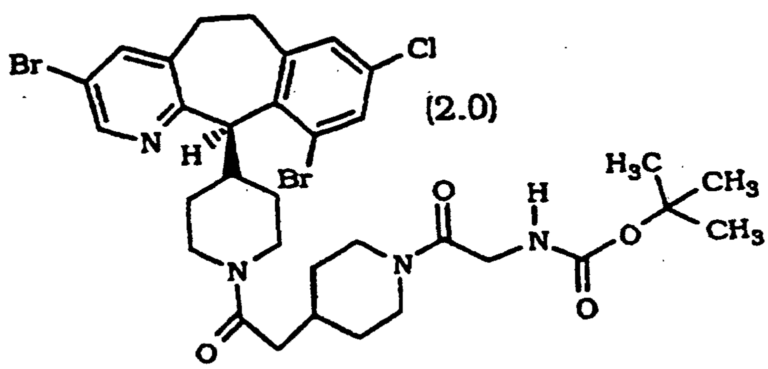

(2.0)

;

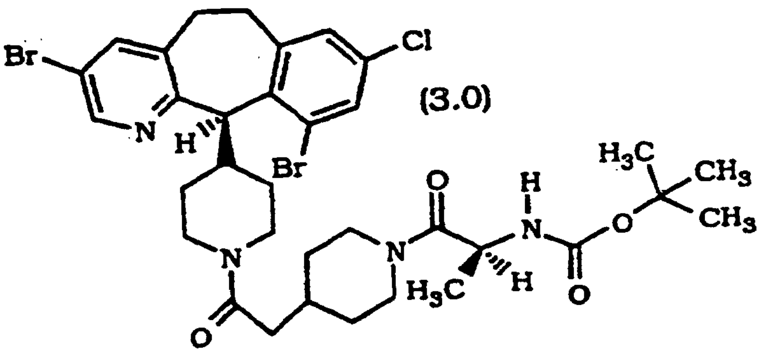

(3.0)

;

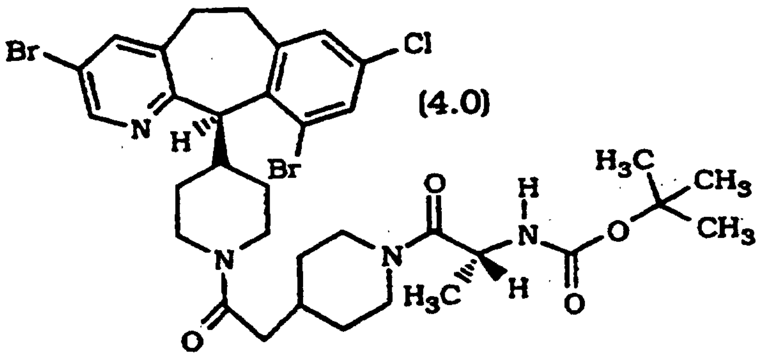

(4.0)

;

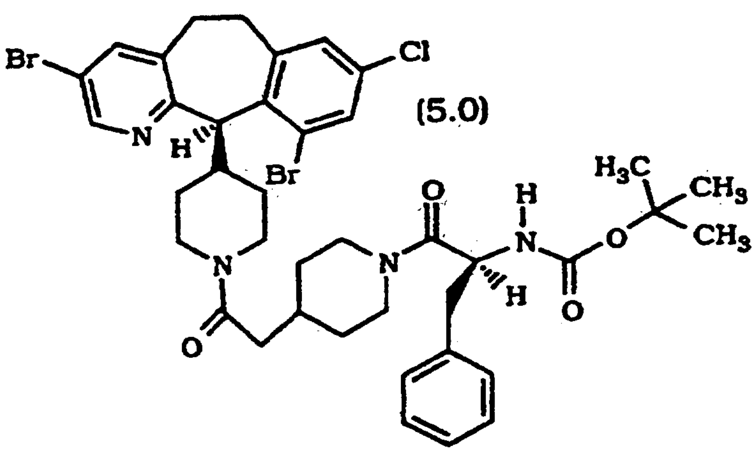

(5.0)

;

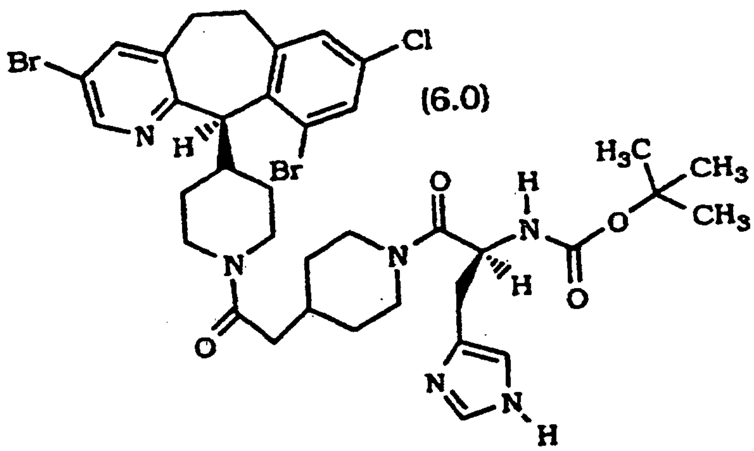

(6.0)

;

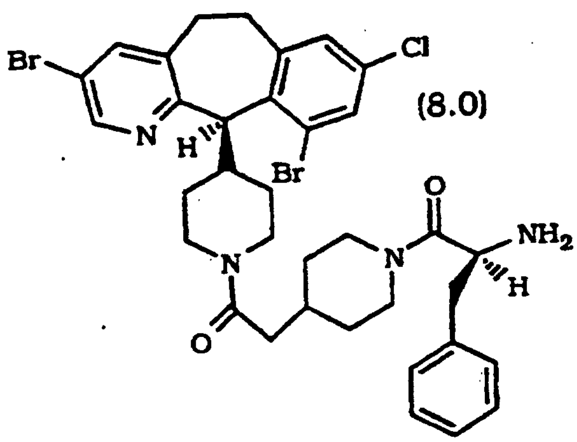

(8.0)

;

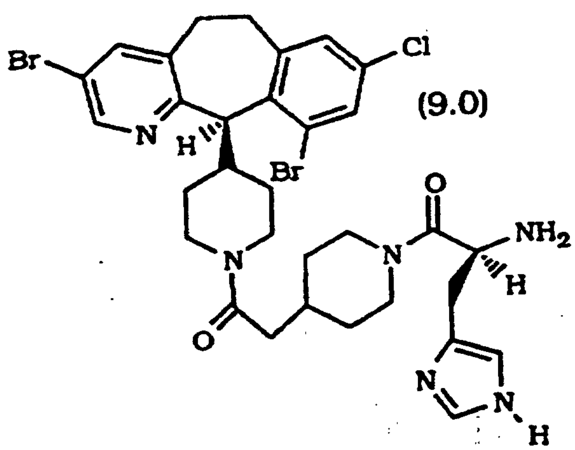

(9.0)

;

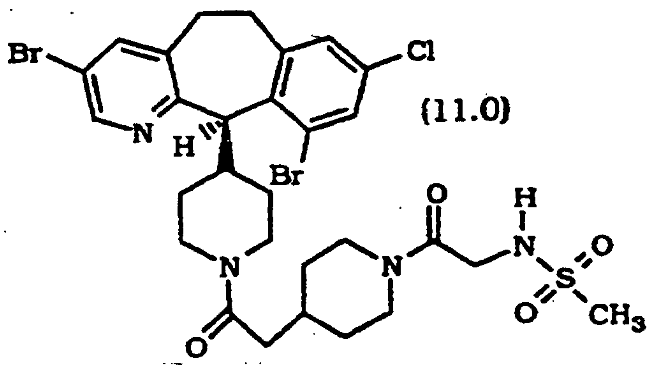

(11.0)

;

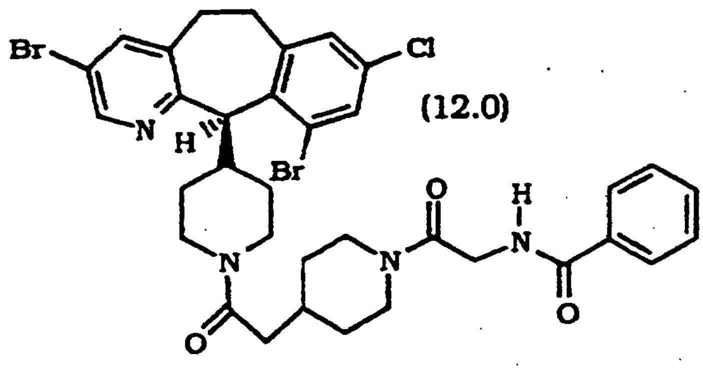

(12.0)

;

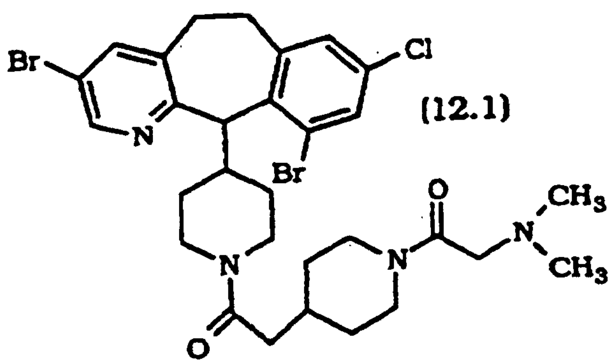

(12.1)

;

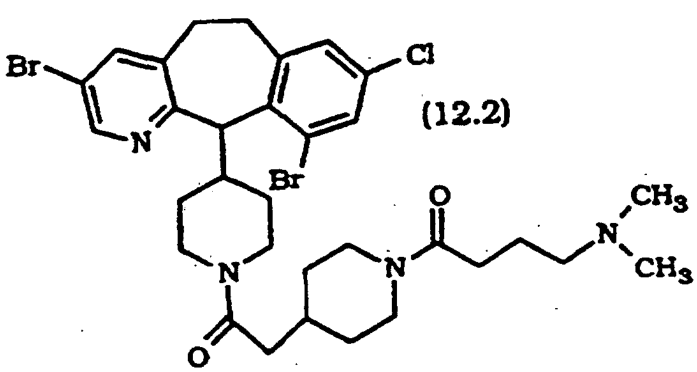

(12.2)

;

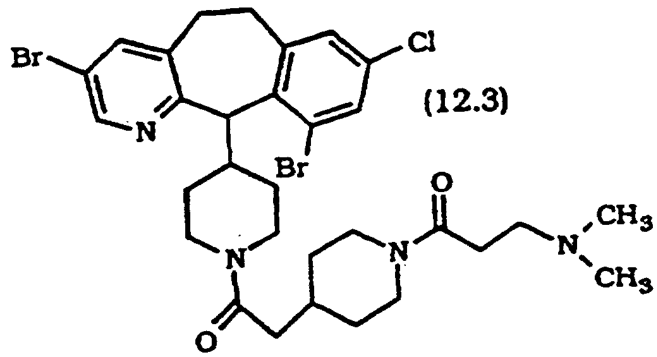

(12.3)

;

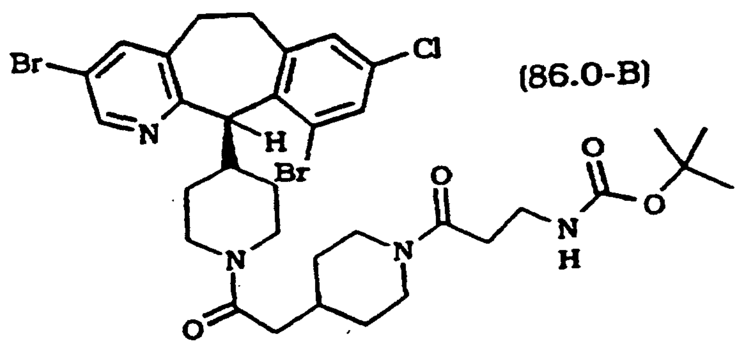

(86.0-B)

;

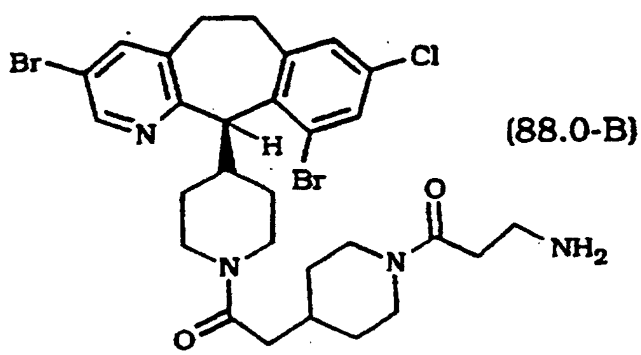

(88.0-B)

;

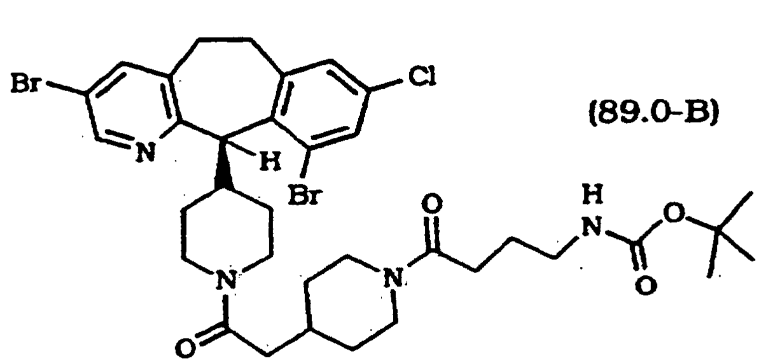

(89.0-B)

;

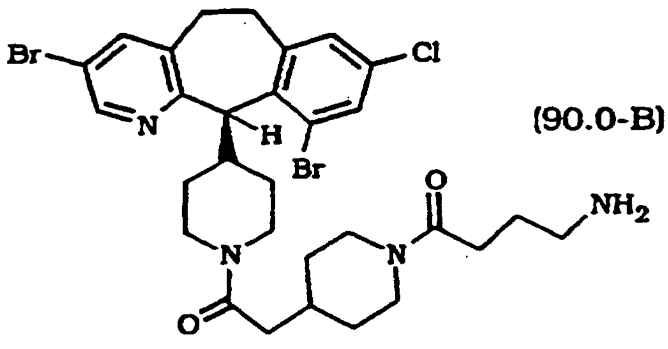

(90.0-B)

;

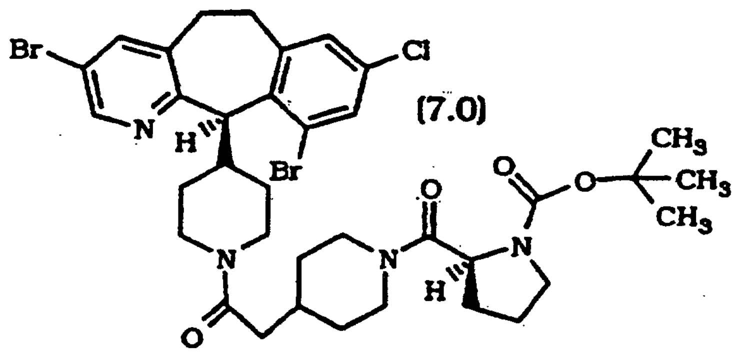

(7.0)

;

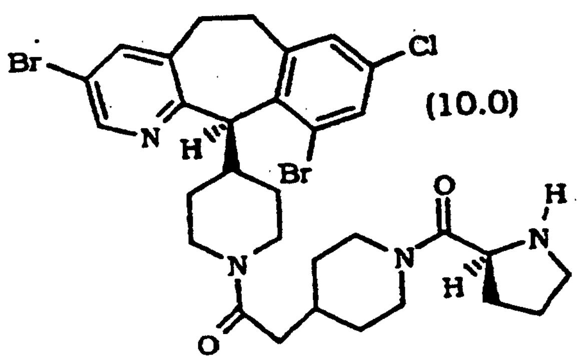

(10.0)

;

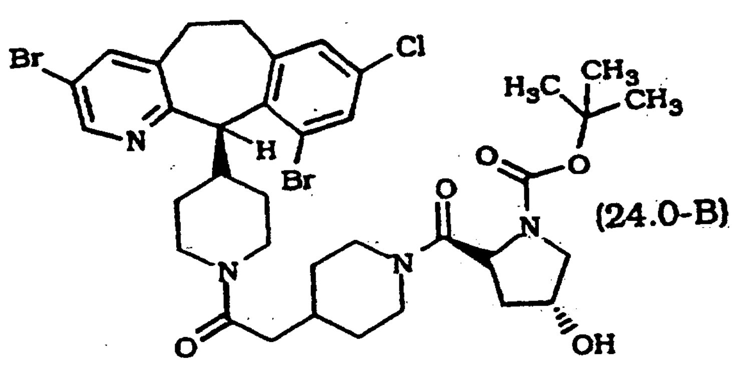

(24.0-B)

;

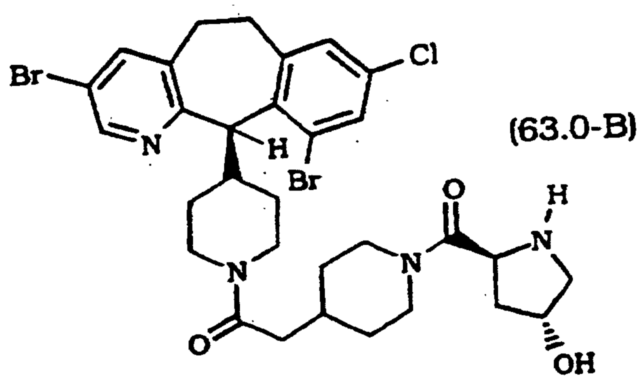

(63.0-B)

;

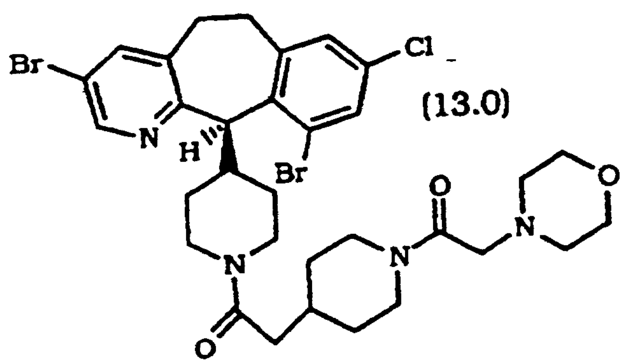

(13.0)

;

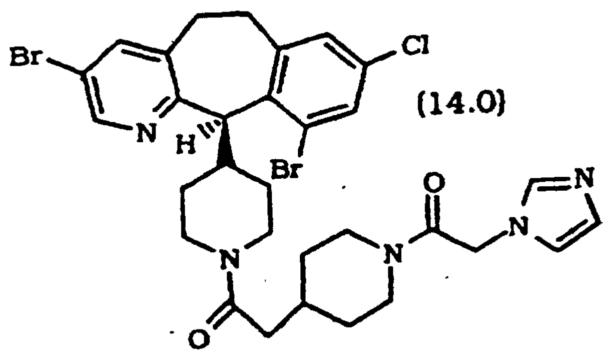

(14.0)

;

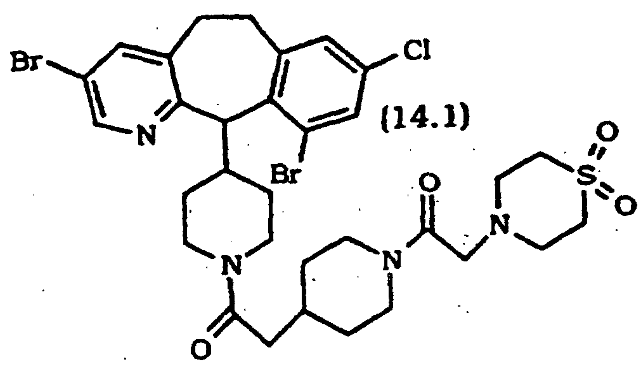

(14.1)

;

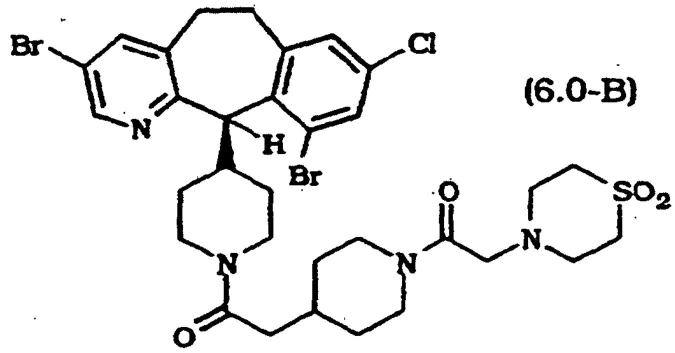

(6.0-B)

;

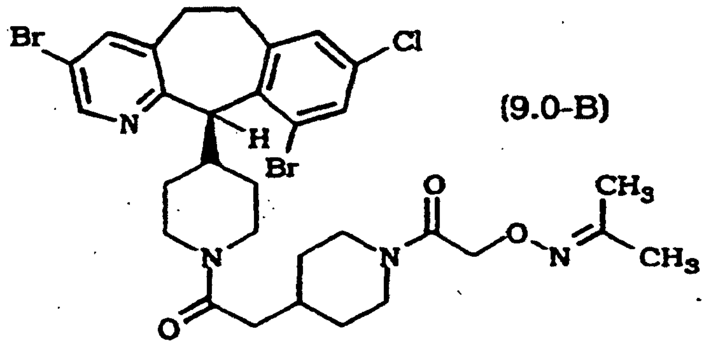

(9.0-B)

;

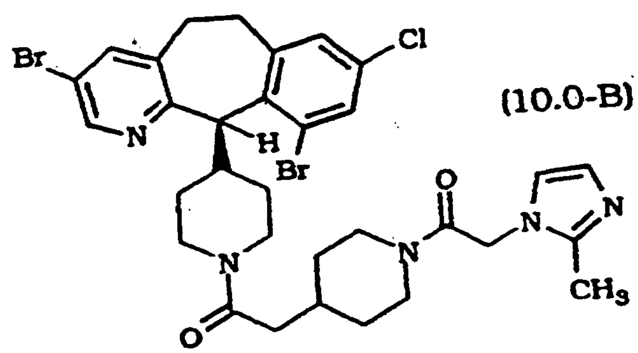

(10.0-B)

;

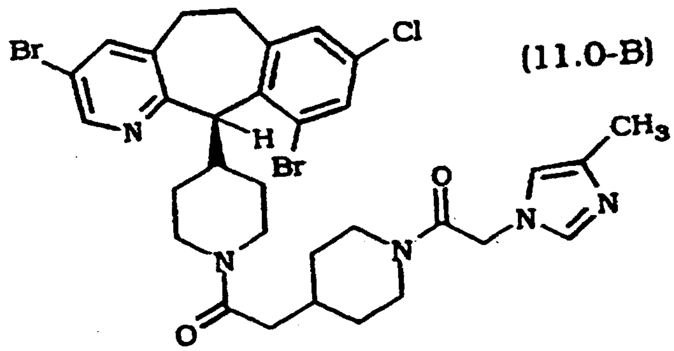

(11.0-B)

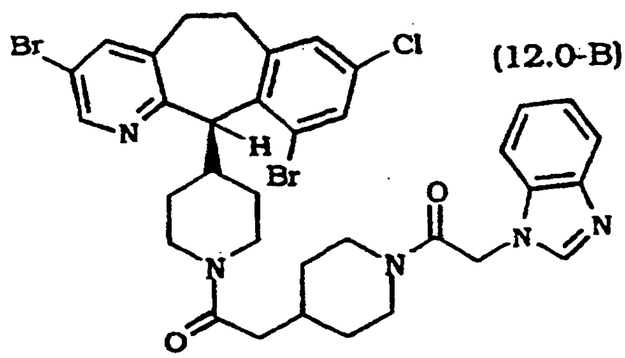

(12.0-B)

;

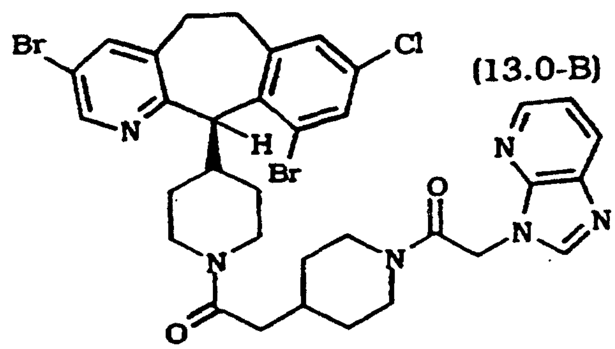

(13.0-B)

;

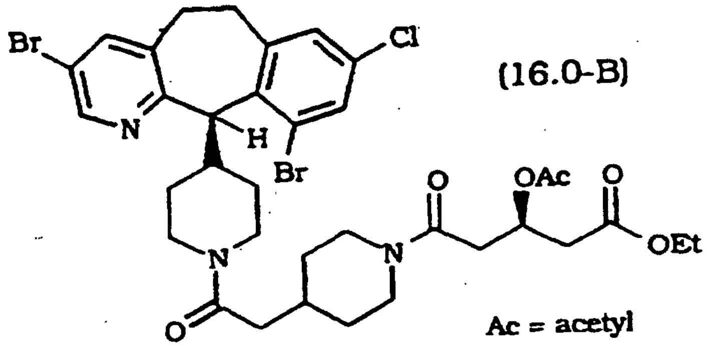

(16.0-B)

Ac = acetyl

;

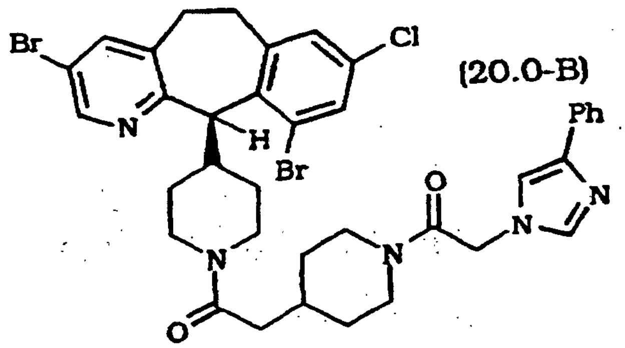

(20.0-B)

;

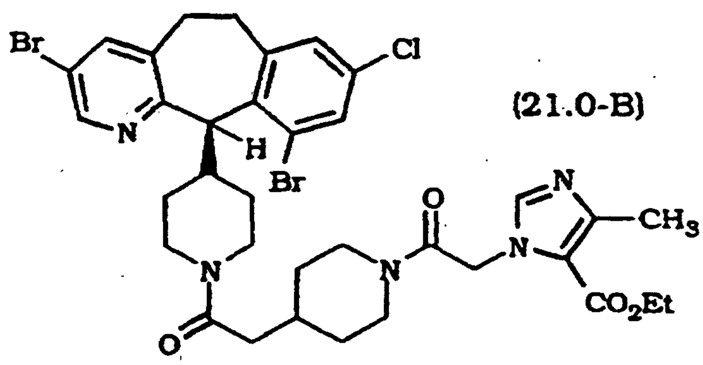

(21.0-B)

;

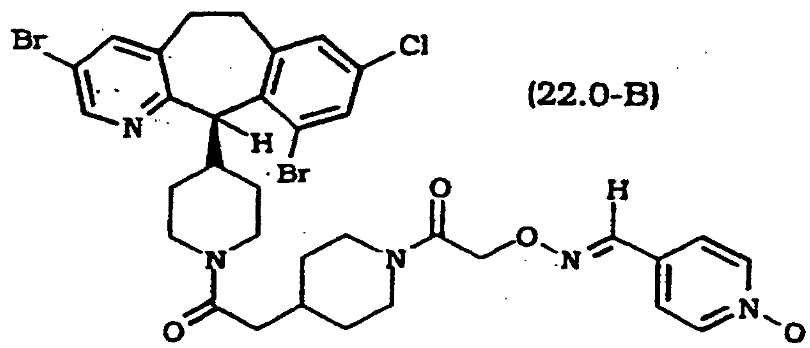

(22.0-B)

;

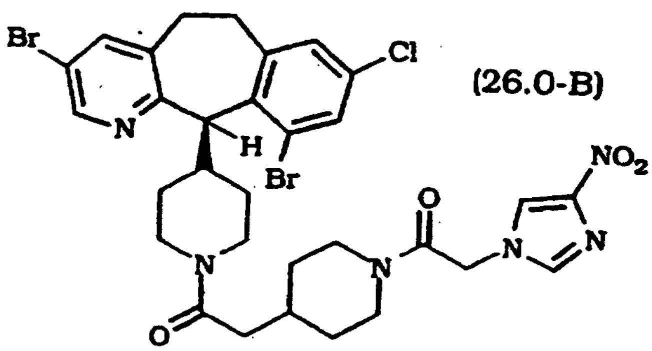

(26.0-B)

;

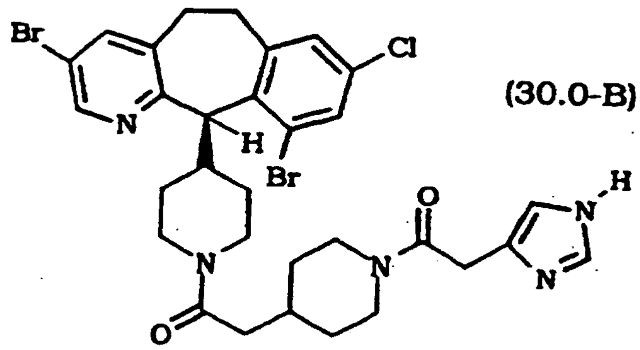

(30.0-B)

;

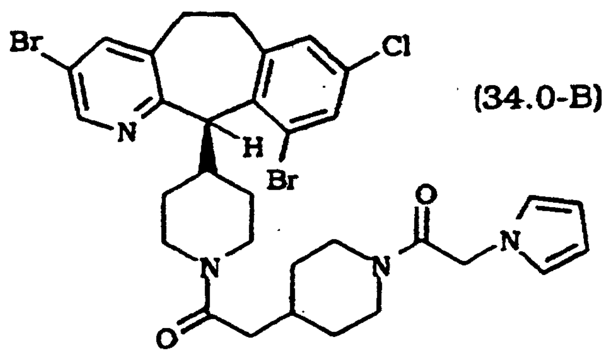

(34.0-B)

;

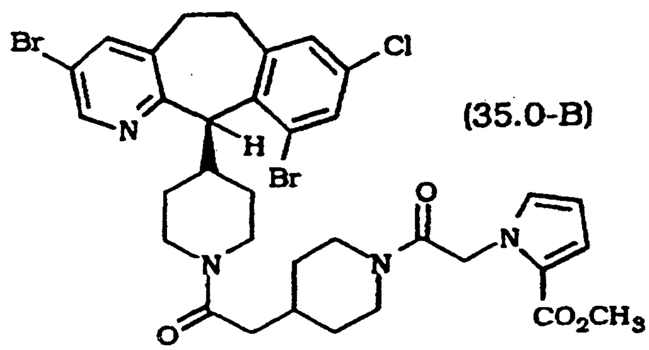

(35.0-B)

;

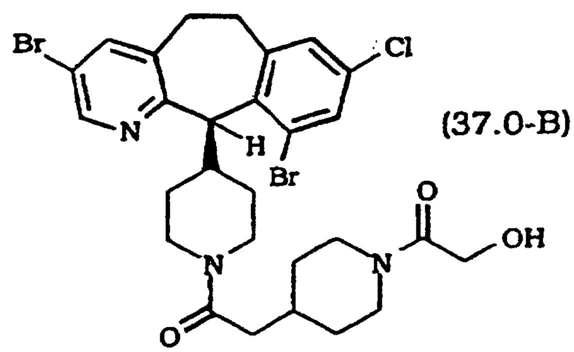

(37.0-B)

;

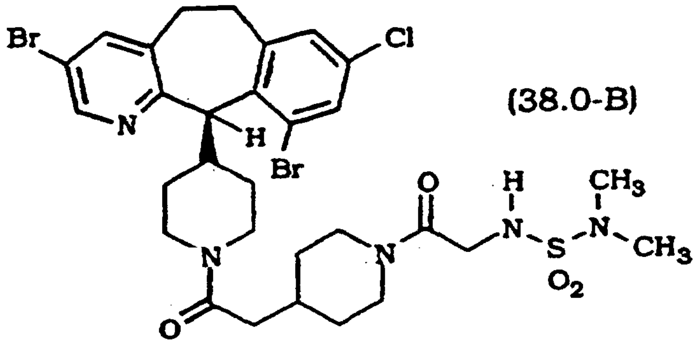

(38.0-B)

;

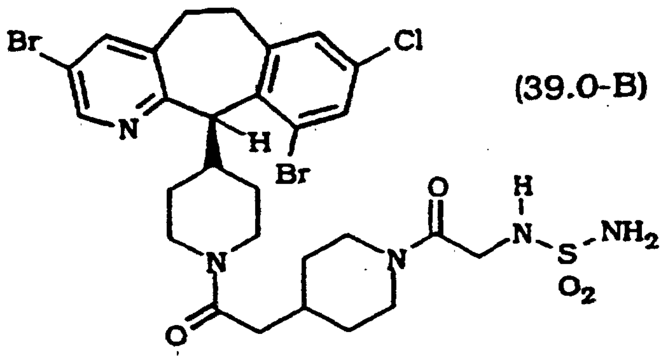

(39.0-B)

;

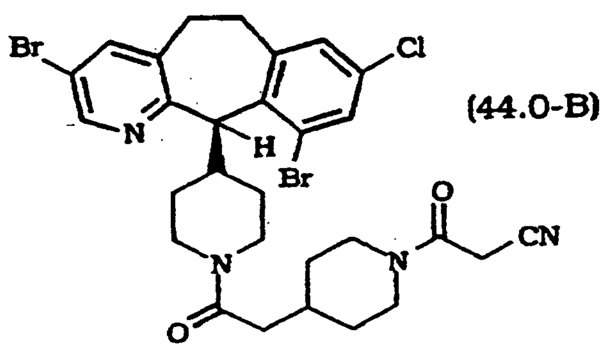

(44.0-B)

;

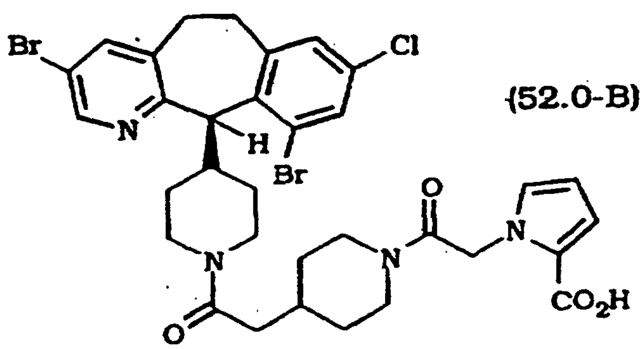

(52.0-B)

;

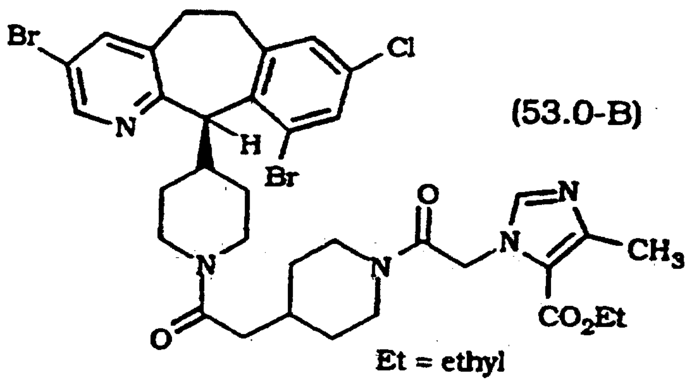

(53.0-B)

Et = ethyl

;

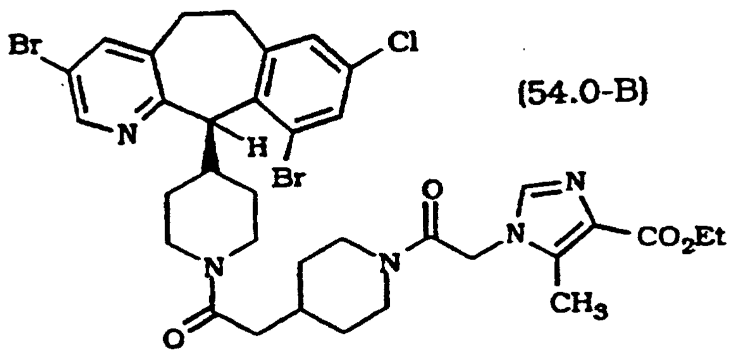

(54.0-B)

;

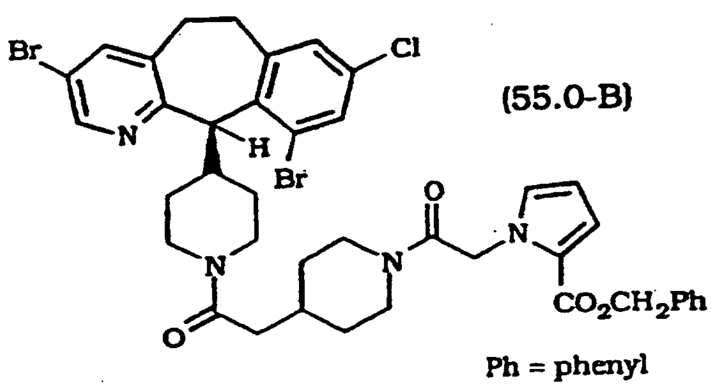

(55.0-B)

Ph = phenyl

;

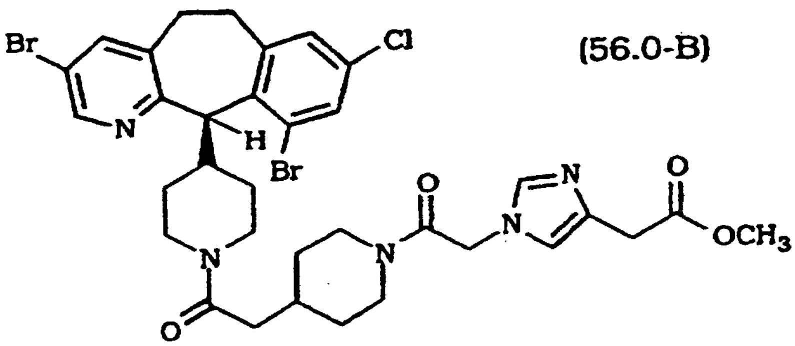

(56.0-B)

;

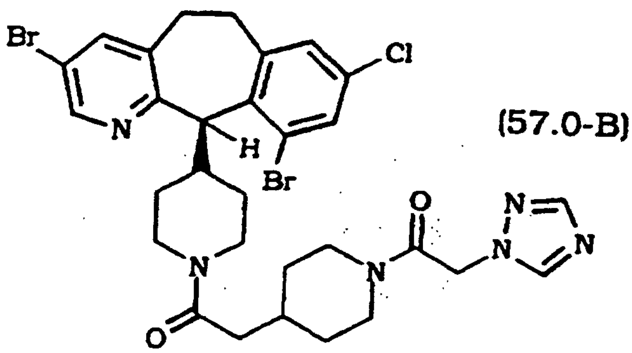

(57.0-B)

;

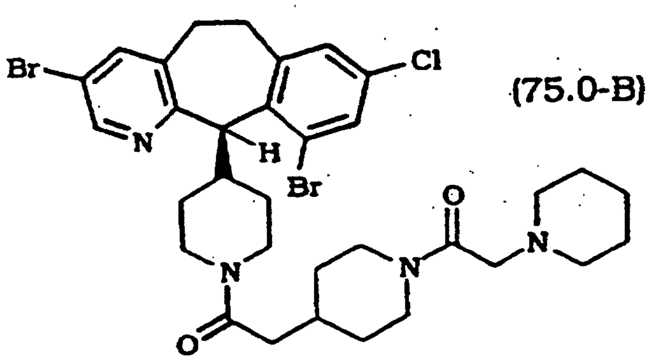

(75.0-B)

;

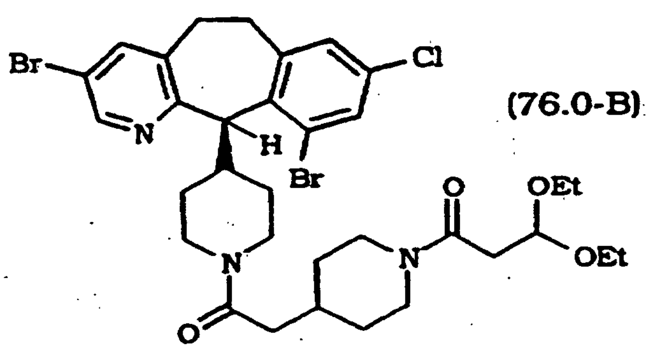

(76.0-B)

;

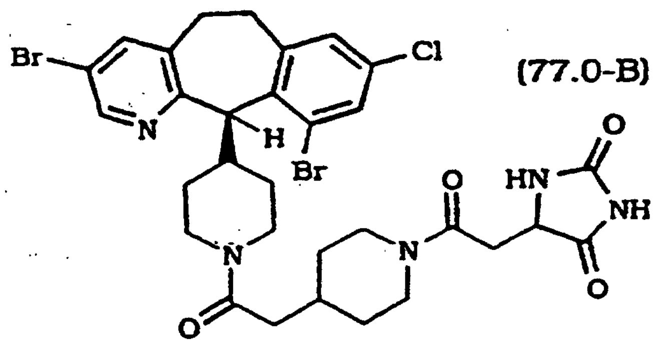

(77.0-B)

;

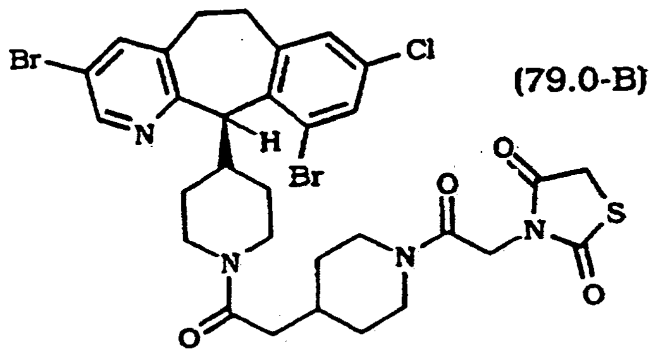

(79.0-B)

;

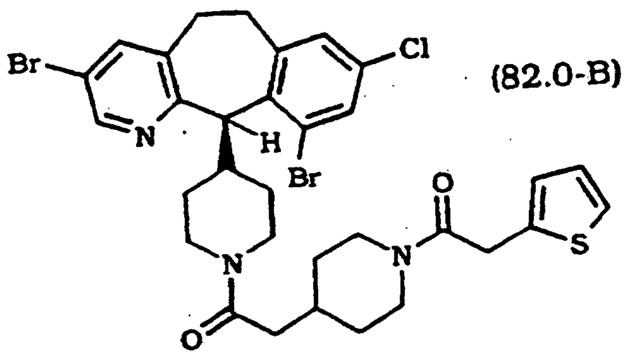

(82.0-B)

;

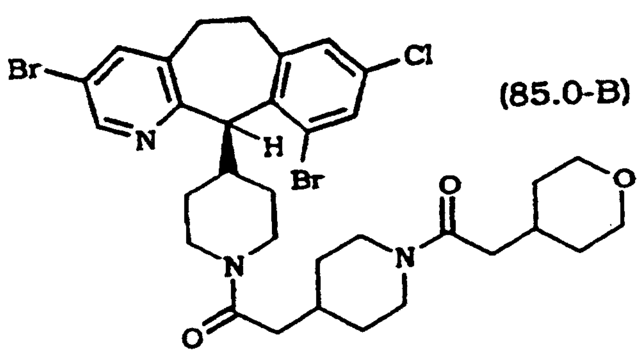

(85.0-B)

;

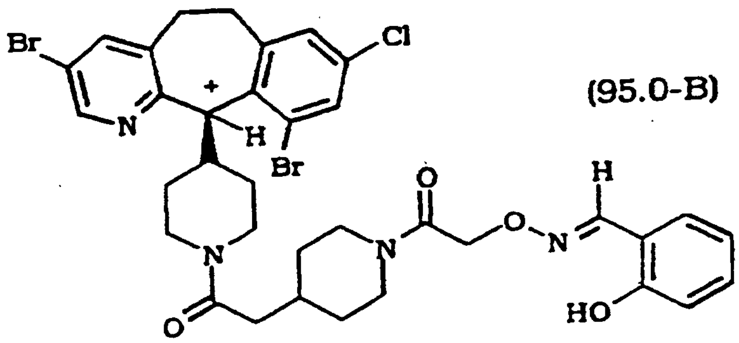

(95.0-B)

;

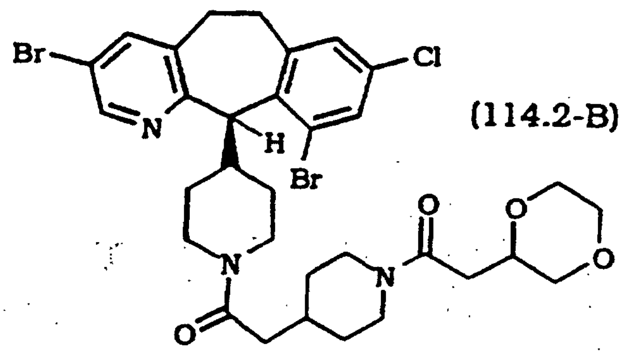

(114.2-B)

;

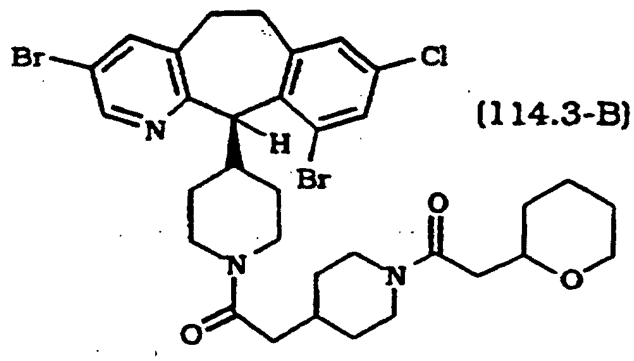

(114.3-B)

;

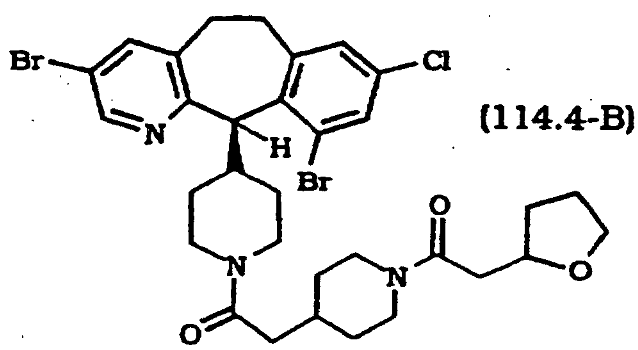

(114.4-B)

;

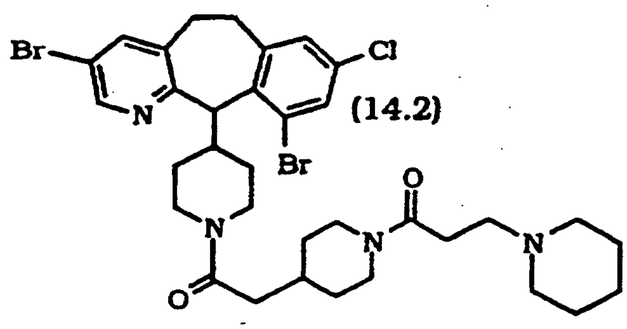

(14.2)

;

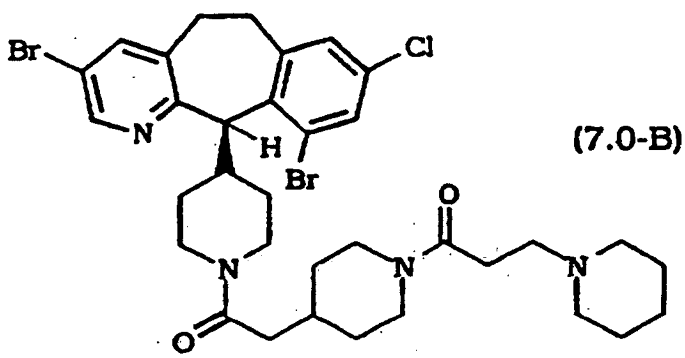

(7.0-B)

;

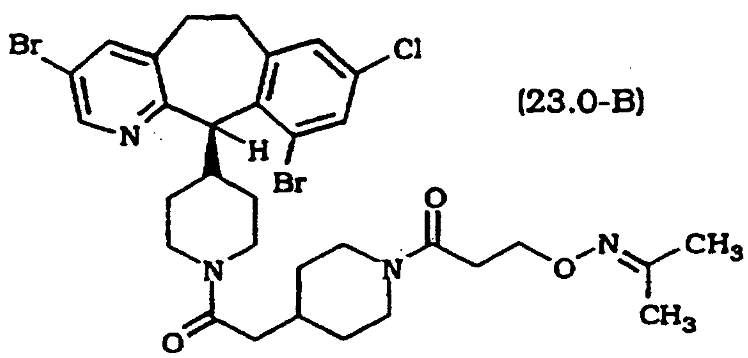

(23.0-B)

;

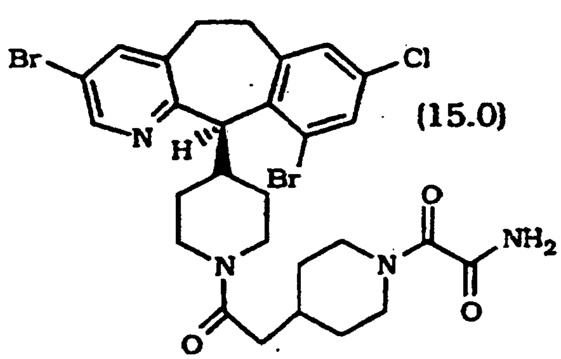

(15.0)

;

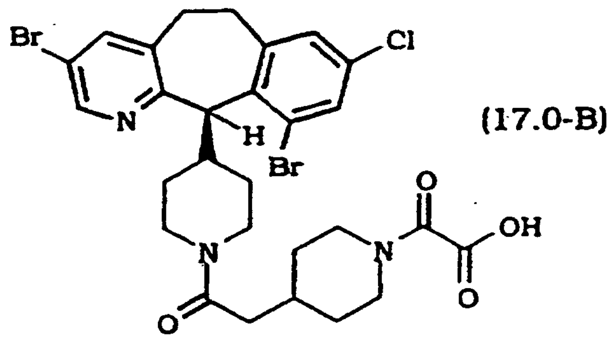

(17.0-B)

;

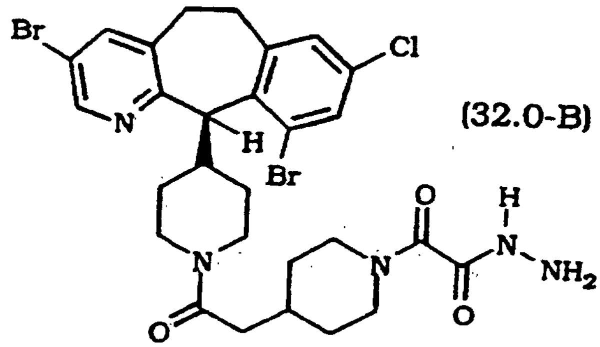

(32.0-B)

;

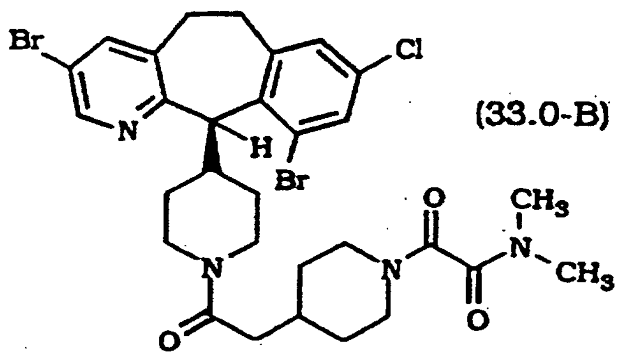

(33.0-B)

;

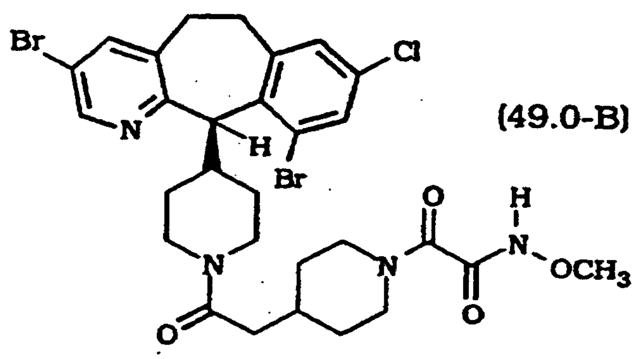

(49.0-B)

;

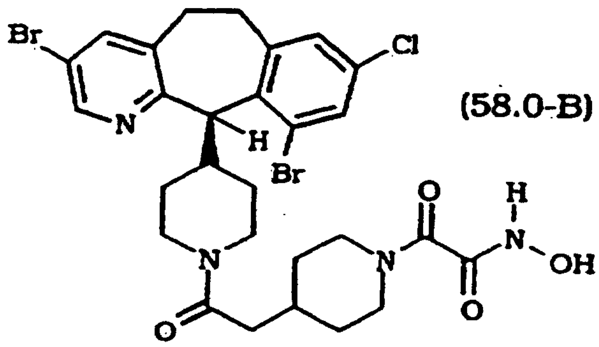

(58.0-B)

;

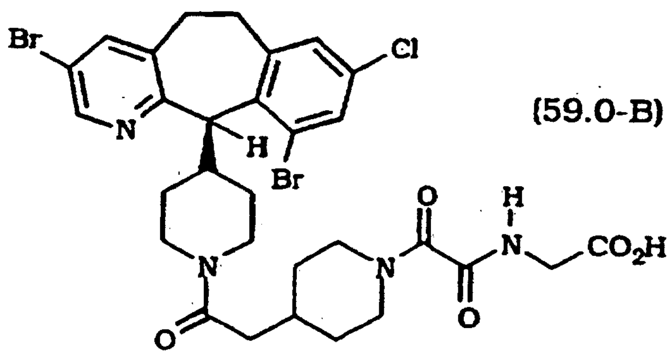

(59.0-B)

;

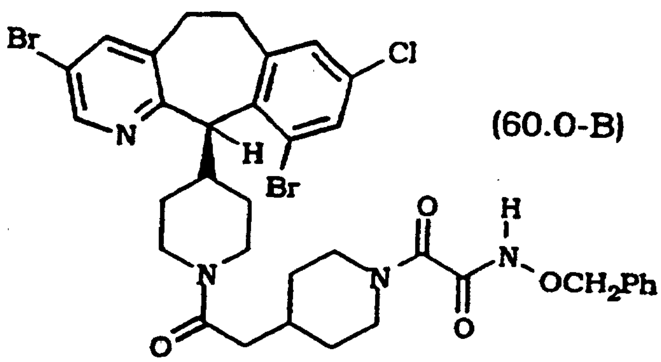

(60.0-B)

;

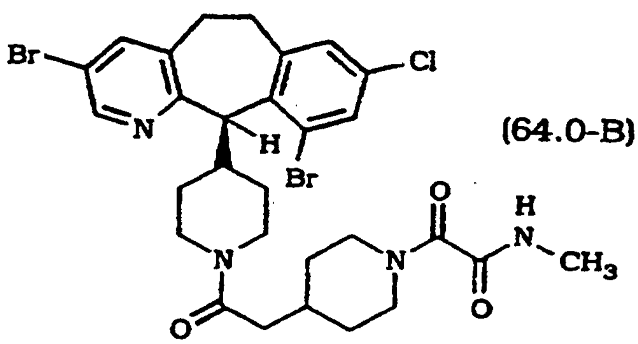

(64.0-B)

;

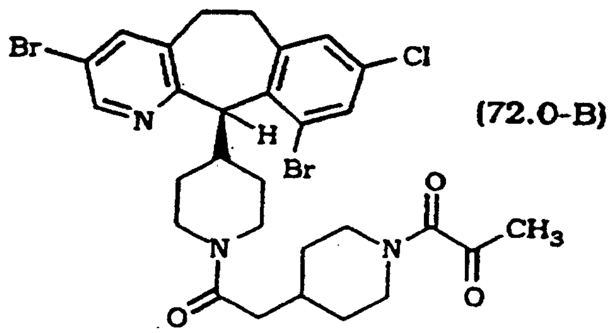

(72.0-B)

;

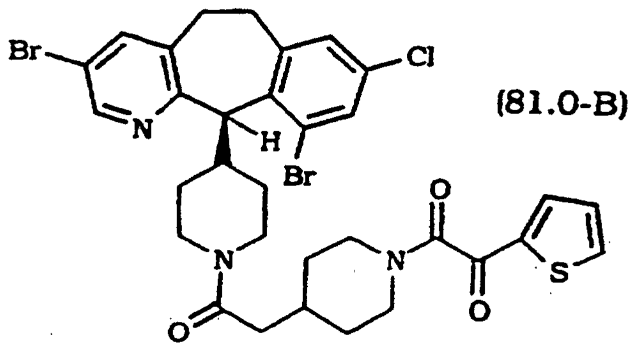

(81.0-B)

;

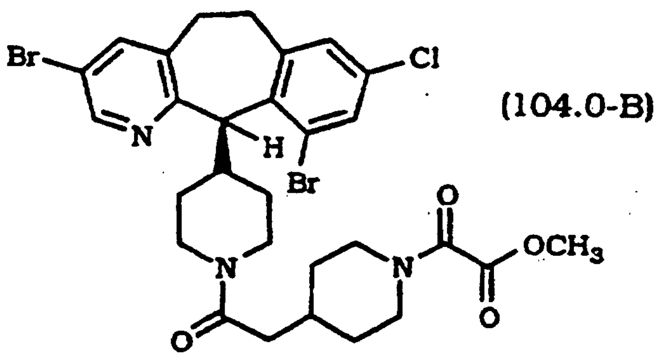

(104.0-B)

;

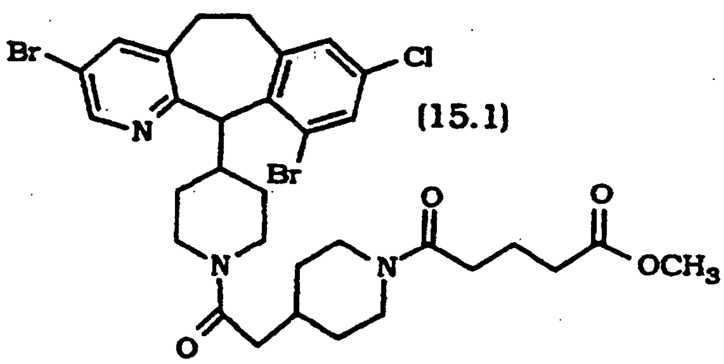

(15.1)

;

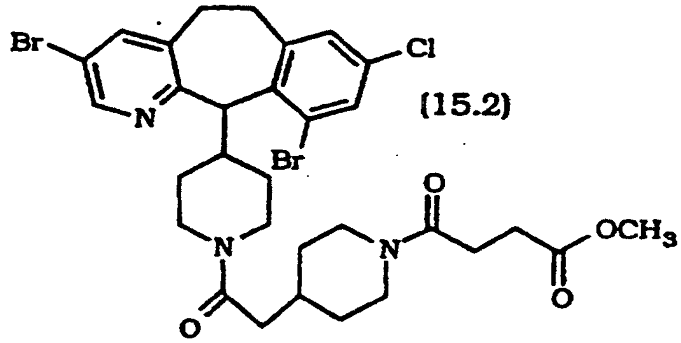

(15.2)

;

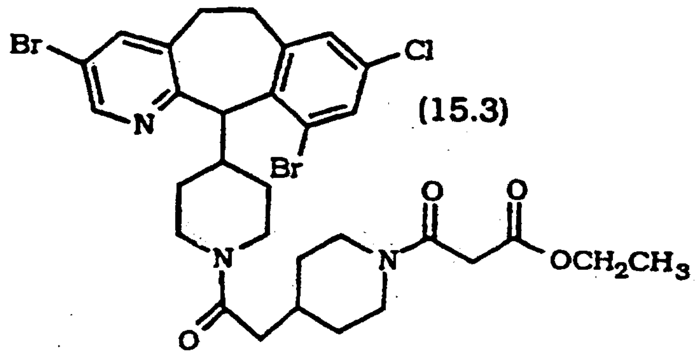

(15.3)

;

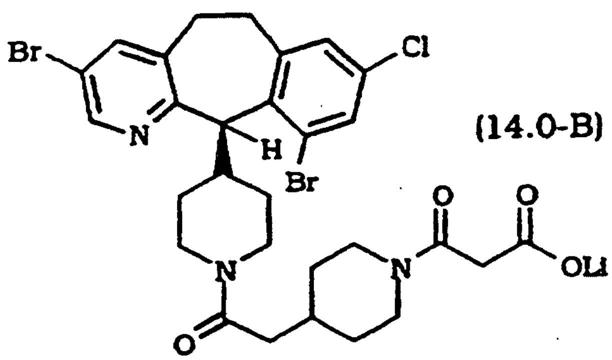

(14.0-B)

;

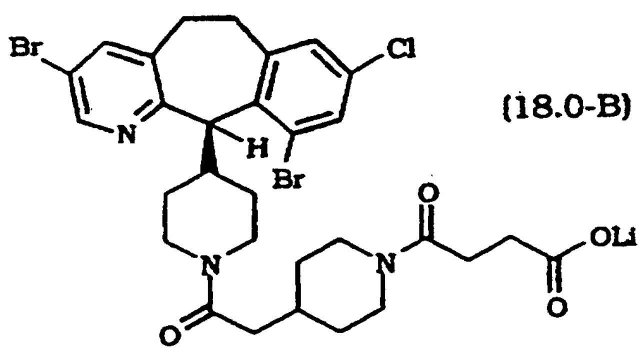

(18.0-B)

;

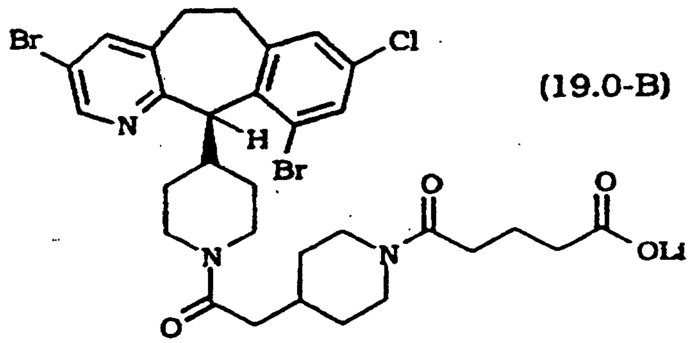

(19.0-B)

;

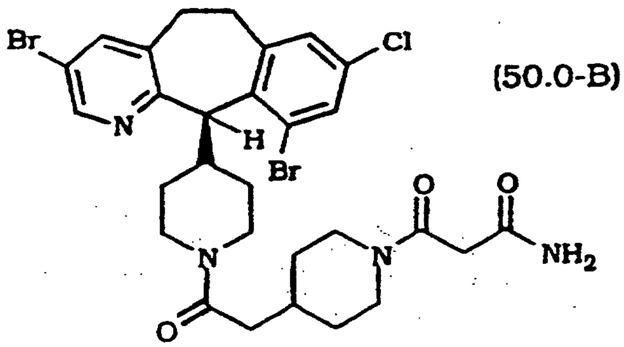

(50.0-B)

;

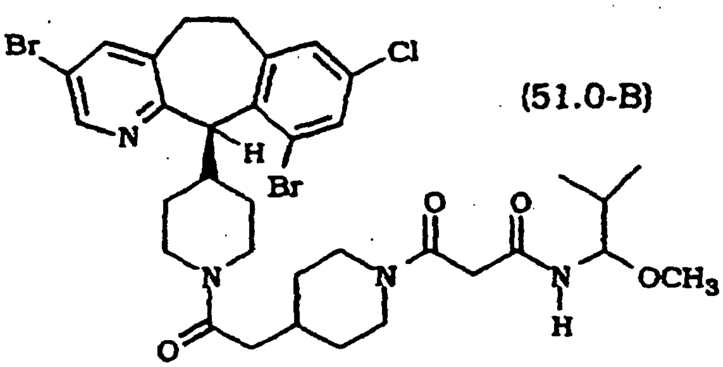

(51.0-B)

;

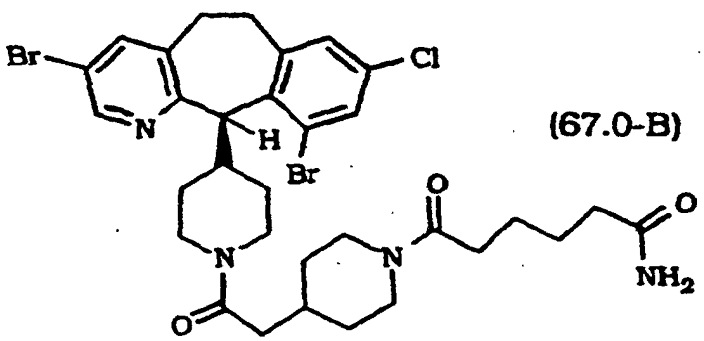

(67.0-B)

;

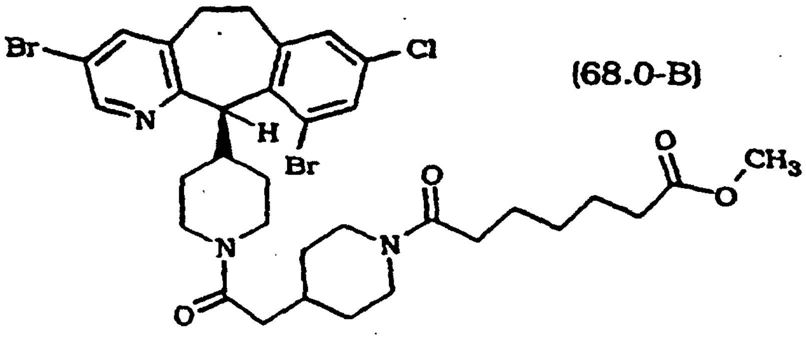

(68.0-B)

;

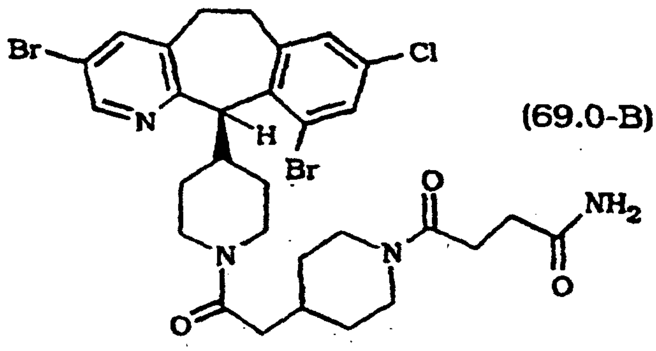

(69.0-B)

;

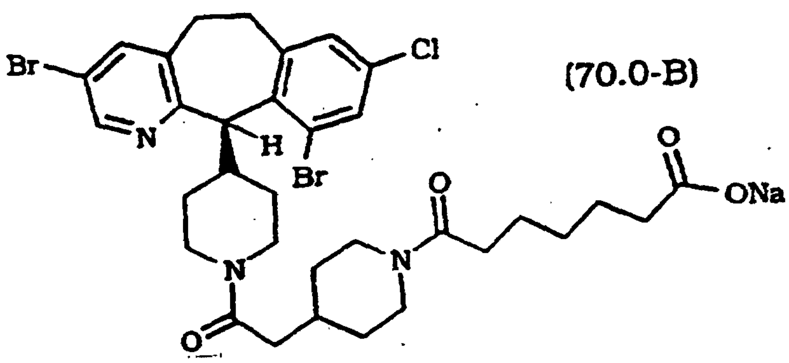

(70.0-B)

;

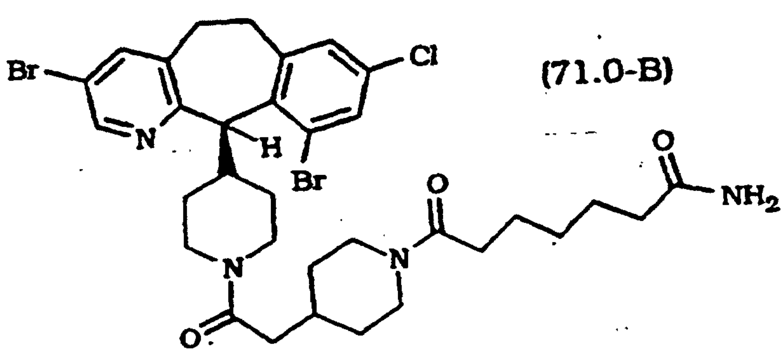

(71.0-B)

;

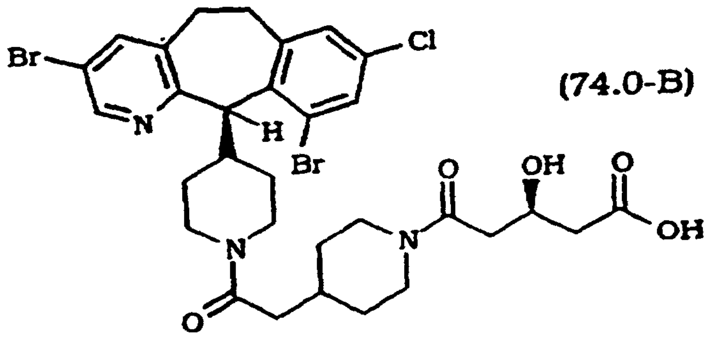

(74.0-B)

;

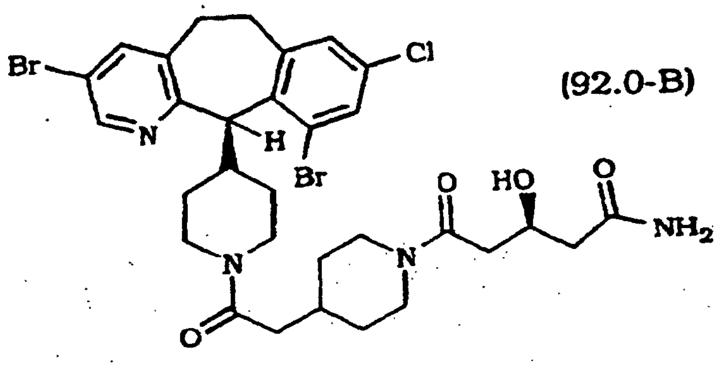

(92.0-B)

;

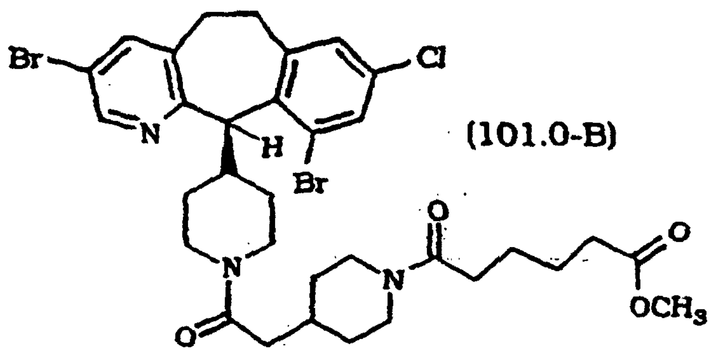

(101.0-B)

;

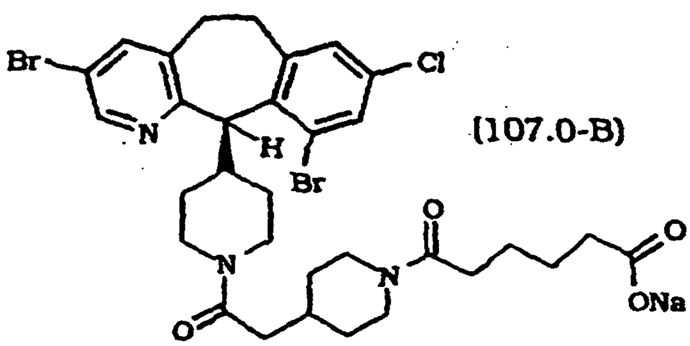

(107.0-B)

;

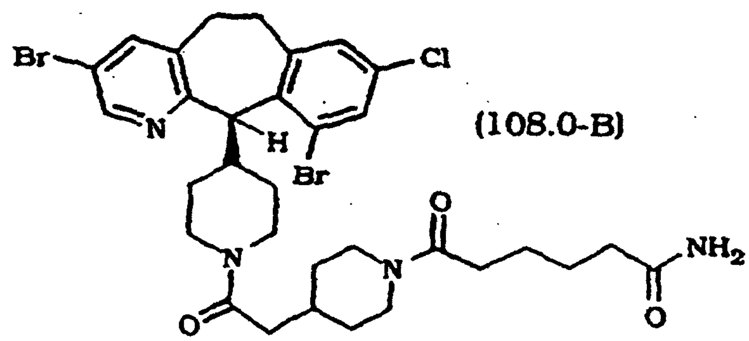

(108.0-B)

;

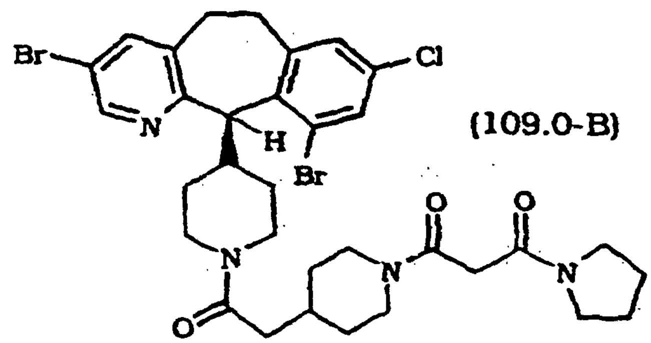

(109.0-B)

;

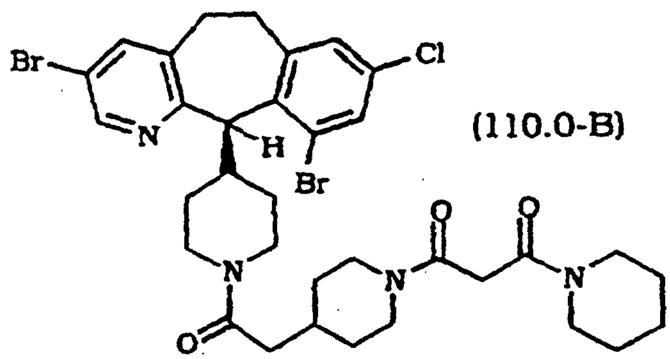

(110.0-B)

;

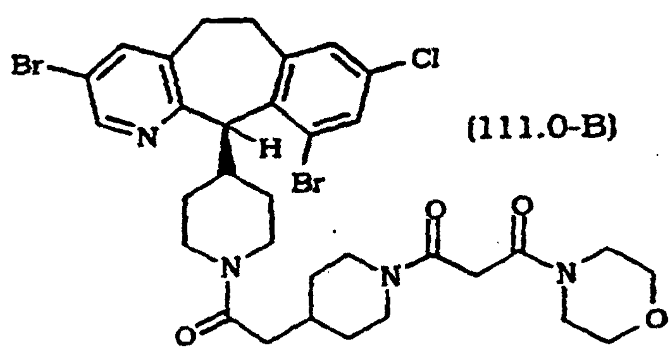

(111.0-B)

;

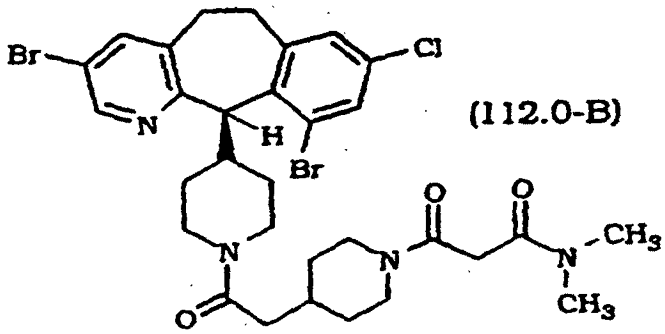

(112.0-B);

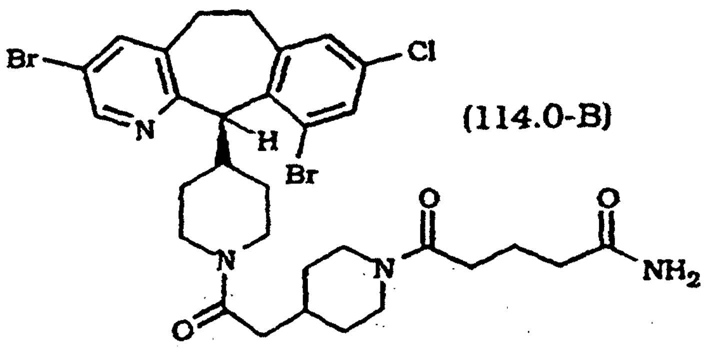

(114.0-B);

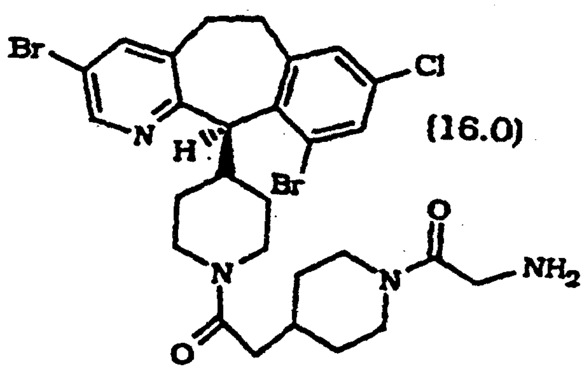

(16.0);

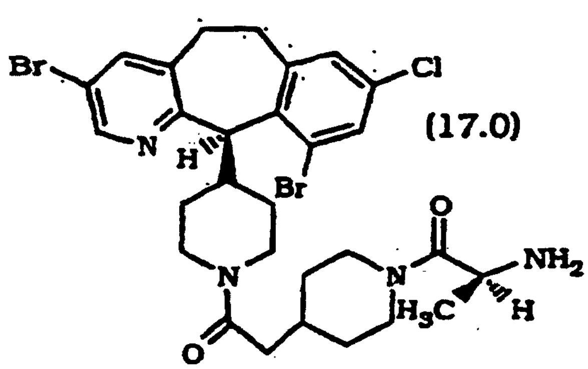

(17.0)

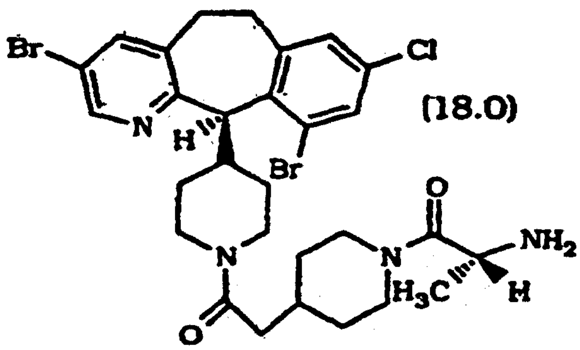

(18.0)

oder pharmazeutisch annehmbare Salze oder Solvate davon.

**10.** Pharmazeutische Zusammensetzung, die eine wirksame Menge einer Verbindung gemäß einem der Ansprüche 1 bis 9 in Kombination mit einem pharmazeutisch annehmbaren Träger enthält.

**11.** Verwendung einer Verbindung nach einem der Ansprüche 1 bis 9 zur Herstellung eines Medikaments zur Behandlung von Tumorzellen.

**12.** Verwendung nach Anspruch 11, bei der die behandelten Zellen Pankreastumorzellen, Lungenkrebszellen, myeloide Leukämie-Tumorzellen, Thyroidfollikel-Tumorzellen, myelodysplastische Tumorzellen, Epidermalcarcinom-Tumorzellen, Blasenkarzinom-Tumorzellen, Colon-Tumorzellen, Brust-Tumorzellen oder Prostatatumorzellen sind.

**13.** Verwendung einer Verbindung nach einem der Ansprüche 1 bis 10 zur Herstellung eines Medikaments zur Inhibierung der Farnesylproteintransferase.

## Revendications

**1.** Composé de formule :

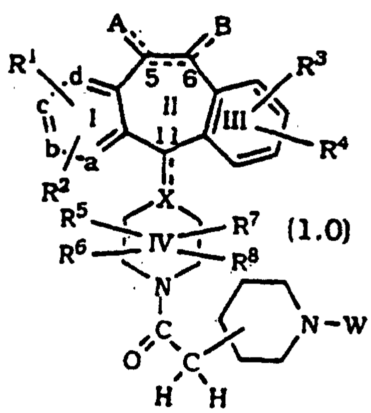

(1.0)

dans laquelle :

l'un des groupes a, b, c et d représente N ou $NR^9$ où $R^9$ désigne $O^-$, $-CH_3$ ou $-(CH_2)_nCO_2H$ où n est égal à 1 à 3, et les groupes a, b, c et d restants représentent $CR^1$ ou $CR^2$, ou bien
chacun de a, b, c et d représente indépendamment $CR^1$ ou $CR^2$,
$R^2$ représente H et $R^1$, $R^3$ et $R^4$ représentent des atomes d'halogène,
$R^5$, $R^6$, $R^7$ et $R^8$ représentent chacun indépendamment un atome d'hydrogène ou un groupe $-CF_3$, $-COR^{10}$,

alkyle ou aryle, ledit groupe alkyle ou aryle étant éventuellement substitué par $-OR^{10}$, $-SR^{10}$, $-S(O)_tR^{11}$, $-NR^{10}COOR^{11}$, $-N(R^{10})_2$, $-NO_2$, $-COR^{10}$, $-OCOR^{10}$, $-OCO_2R^{11}$, $-CO_2R^{10}$ ou $-OPO_3R^{10}$, ou bien $R^5$ est combiné avec $R^6$ pour représenter =O ou =S et/ou $R^7$ est combiné avec $R^8$ pour représenter =O ou =S,
$R^{10}$ représente un atome d'hydrogène ou un groupe alkyle, aryle ou aralkyle,
$R^{11}$ représente un groupe alkyle ou aryle,
X représente N, CH ou C qui peut échanger une deuxième liaison éventuelle (représentée par la ligne en pointillés) avec l'atome de carbone 11,
la ligne en pointillés entre les atomes de carbone 5 et 6 représente une deuxième liaison éventuelle, de sorte que, lorsqu'une double liaison est présente, A et B représentent chacun indépendamment $-R^{10}$, un atome d'halogène, $-OR^{11}$, $-OCO_2R^{11}$ ou $-OC(O)R^{10}$, et lorsqu'il n'y a pas de deuxième liaison entre les atomes de carbone 5 et 6, A et B représentent chacun indépendamment $H_2$, $-(OR^{11})_2$, H et un atome d'halogène, deux atomes d'halogène, un groupe alkyle et H, deux groupes alkyle, H et $-OC(O)R^{10}$, H et $-OR^{10}$, =O, un groupe aryle et H, $=NOR^{10}$ ou -O- $(CH_2)_p$-O- où p est égal à 2, 3 ou 4,
et W représente un groupe choisi parmi les groupes de formules :

(1)

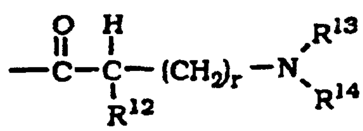

;

(2)

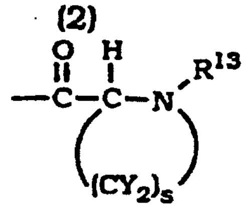

;

(3)

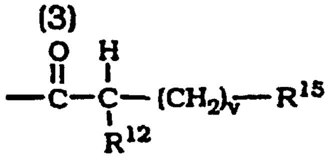

et

(4)

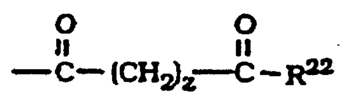

dans lesquelles:

$R^{12}$ représente un atome d'hydrogène, un groupe alkyle, un groupe aralkyle ou un groupe hétéroarylalkyle,
$R^{13}$ et $R^{14}$ représentent chacun indépendamment : (a) H ; (b) un groupe $-C(O)OR^{16}$ où $R^{16}$ représente un groupe alkyle, aralkyle ou hétéroaralkyle ; (c) un groupe $-SO_2R^{17}$ où $R^{17}$ représente un groupe $NH_2$, $-N(alkyl)_2$ où les deux alkyles sont identiques ou différents, alkyle, aryle, aralkyle, hétéroaryle ou hétéroaralkyle ; (d) un groupe $-C(O)R^{18}$ où $R^{18}$ représente un groupe aryle, alkyle, aralkyle, hétéroaryle ou hétéroaralkyle ; (e) un groupe alkyle en $C_1$ à $C_6$ ; (f) un groupe alkylaryle ; ou (g) un groupe cycloalkyle en $C_3$ à $C_6$,
r est égal à 0, 1 ou 2,
s représente 1, 2, 3, 4 ou 5, et chaque Y de chaque groupe $-CY_2-$ représente indépendamment H ou -OH, étant entendu que les deux substituants Y d'un groupe $-CY_2-$ déterminé ne sont pas simultanément -OH, et que les deux substituants Y du groupe $-CY_2-$ en position alpha par rapport à l'atome d'azote sont tous les deux H, de sorte que le groupe

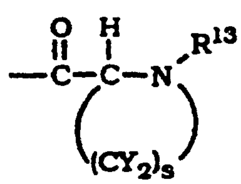

forme un cycle à 3, 4, 5, 6 ou 7 chaînons,

v est égal à 0, 1 ou 2,
$R^{15}$ représente :

(a) un groupe hétéroaryle,
(b) un groupe choisi parmi les groupes de formules :

(1)

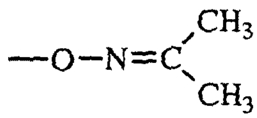
,

(2)

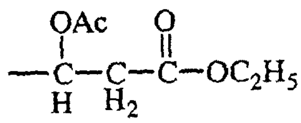
,

(3)

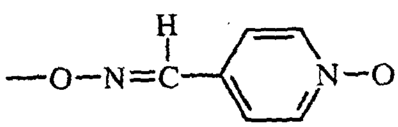
,

(4)

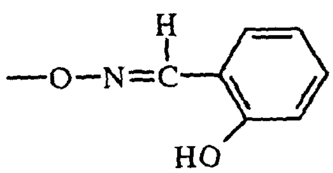
,

(5) $-CH(OCH_2CH_3)_2$ ,
(6) -OH, et
(7) -CN, ou

(c) un groupe hétérocycloalkyle choisi parmi les groupes de formules :

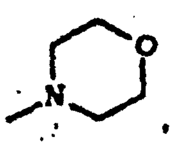 , 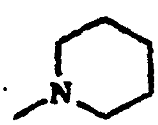 , 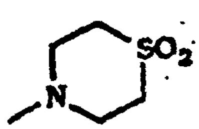 , 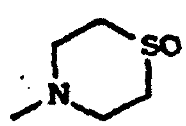 , 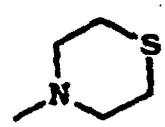 ,

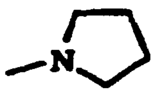, 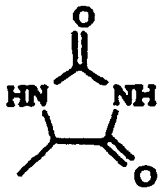, 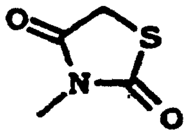, 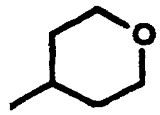, 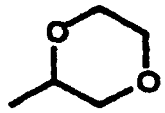, 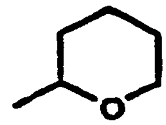

et

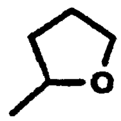

z représente 0, 1, 2, 3, 4 ou 5 et chaque groupe $-CH_2-$ est éventuellement substitué par un groupe -OH, $R^{22}$ représente un groupe choisi parmi :

(1) 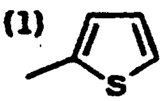, (2) 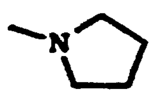, (3) 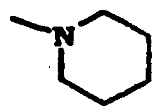, (4) 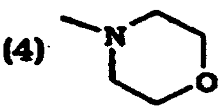

(5) les groupes alkyle (par exemple le groupe $-CH_3$),
(6) le groupe $-OR^{23}$ où $R^{23}$ représente un atome d'hydrogène ou un groupe alkyle ou aryle, et
(7) le groupe

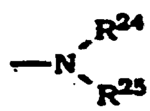

dans lequel $R^{24}$ et $R^{25}$ représentent chacun indépendamment $-NH_2$, un groupe alcoxy, -OH, $-CH_2CO_2H$, $-OCH_2Ph$, $-CH(OCH_3)CH(CH_3)_2$, un groupe alkyle, un groupe aryle, H, un groupe aralkyle ou un groupe hétéroaralkyle, ou bien $R^{24}$ et $R^{25}$ forment ensemble une chaîne carbonée ayant 4 ou 5 groupes ($-CH_2-$) de sorte que $R^{24}$ et $R^{25}$ forment ensemble avec l'atome d'azote auquel ils sont liés un cycle hétérocycloalkyle à 5 ou 6 chaînons,

les termes employés précédemment ayant, sauf indication contraire, les significations suivantes :

alcényle représente une chaîne carbonée droite ou ramifiée, ayant au moins une double liaison carbone-carbone et contenant 2 à 12 atomes de carbone,
alcynyle représente une chaîne carbonée droite ou ramifiée, ayant au moins une triple liaison carbone-carbone et contenant 2 à 12 atomes de carbone,
alkyle (y compris la partie alkyle des groupes aralkyle et hétéroarylalkyle) représente une chaîne carbonée droite ou ramifiée, ayant 1 à 20 atomes de carbone,
aralkyle représente un groupe aryle tel que défini ci-après, lié à un groupe alkyle tel que défini précédemment,
aryle (y compris la partie aryle des groupes aralkyle) représente un groupe carbocyclique contenant 6 à 15 atomes de carbone et ayant au moins un noyau aromatique (par exemple aryle désigne un cycle phényle), tous les atomes de carbone substituables disponibles du groupe carbocyclique étant des points possibles de rattachement et ledit groupe carbocyclique étant éventuellement substitué par un ou plusieurs (par exemple 1 à 3) substituants pris parmi les atomes d'halogène et les groupes alkyle, hydroxyle, alcoxy, phénoxy, $CF_3$, amino, alkylamino, dialkylamino, $-COOR^{10}$ et $-NO_2$,
halogène représente le fluor, le chlore, le brome et l'iode,
hétéroaryle représente un groupe cyclique, éventuellement substitué par $R^3$, $R^4$, un groupe phényle et/ou $-CH_2C(O)OCH_3$, qui présente au moins un hétéroatome pris parmi O, S et N, interrompant une structure carbocyclique, et qui a un nombre suffisant d'électrons pi délocalisés pour avoir le caractère aromatique, le groupe hétérocyclique aromatique contenant de 2 à 14 atomes de carbone,
hétéroarylalkyle (ou hétéroaralkyle) représente un groupe hétéroaryle tel que défini précédemment, lié à un groupe alkyle tel que défini précédemment,
hétérocycloalkyle représente un noyau carbocyclique saturé, ramifié ou non-ramifié, contenant 3 à 15 atomes de carbone, de préférence 4 à 6 atomes de carbone, qui est interrompu par 1 à 3 hétérogroupes pris parmi -O-, -S- et $-NR^{10}-$,
ou un sel ou produit de solvatation pharmaceutiquement acceptable d'un tel composé.

2. Composé selon la revendication 1, pour lequel a représente N, b, c et d représentent des atomes de carbone, A et B représentent chacun $H_2$, la deuxième liaison éventuelle entre C5 et C6 est absente, X représente CH, et $R^5$, $R^6$, $R^7$ et $R^8$ représentent des atomes d'hydrogène.

3. Composé selon la revendication 1 ou 2, pour lequel W est choisi parmi les groupes suivants :

(A)

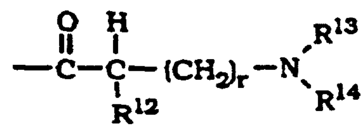

où :

(1) r est égal à 0,
(2) $R^{12}$ représente un atome d'hydrogène, un groupe alkyle, un groupe aralkyle ou un groupe hétéroaralkyle, et
(3) $R^{13}$ et $R^{14}$ représentent chacun indépendamment un atome d'hydrogène, un groupe $-C(O)OR^{16}$ où $R^{16}$ désigne un groupe alkyle, un groupe $-SO_2R^{17}$ où $R^{17}$ désigne un groupe alkyle ou aryle, un groupe $-C(O)R^{18}$ où $R^{18}$ désigne un groupe aryle, ou un groupe alkyle,

(B)

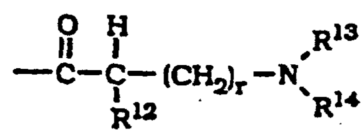

où :

(1) r est égal à 1 ou 2,
(2) $R^{12}$ représente H, et
(3) $R^{13}$ représente un groupe alkyle et $R^{14}$ représente H, un groupe alkyle ou un groupe $-C(O)OR^{16}$ où $R^{16}$ désigne un groupe alkyle,

(C)

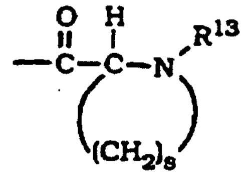

où :

(1) s est égal à 1, 2, 3, 4 ou 5, et
(2) $R^{13}$ représente H ou un groupe -C(O)O$R^{16}$ où $R^{16}$ désigne un groupe alkyle,

(D)

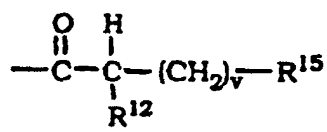

où :

(1) v est égal à 0,
(2) $R^{12}$ représente H, et
(3) $R^{15}$ représente un groupe choisi parmi les groupes :

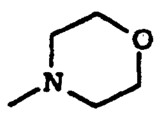 , s 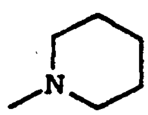 , 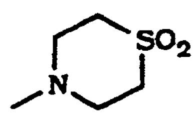 , 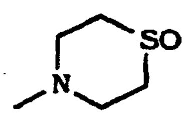 ,

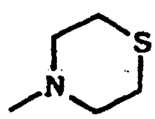 , 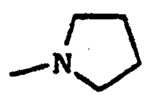 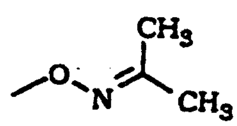 , 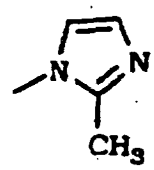 ,

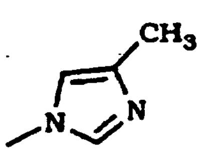 , 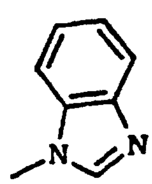 , 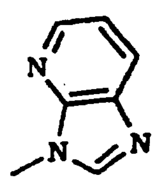 , 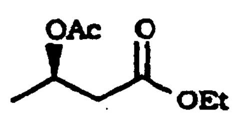 ,

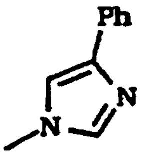 , 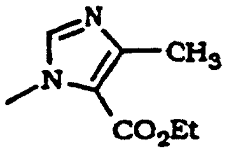 , 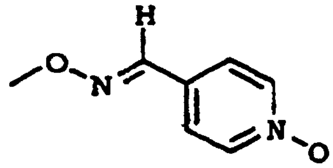 ,

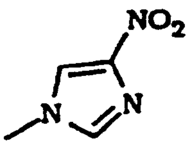 , 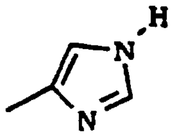 , 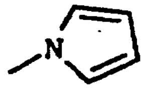 , 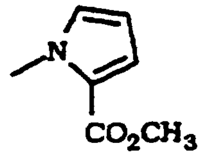 ,

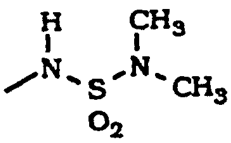 , 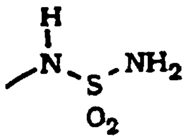 , 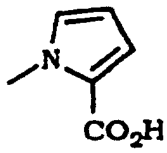 , 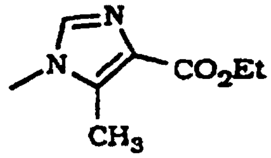 ,

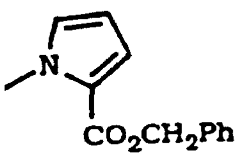 , 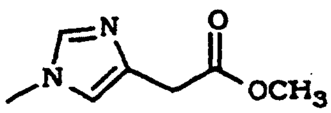 , 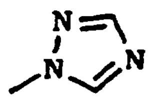 , 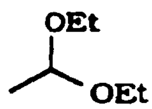 ,

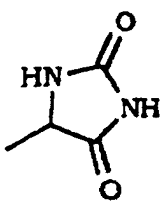 , 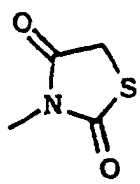 , 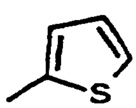 , 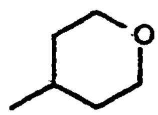 ,

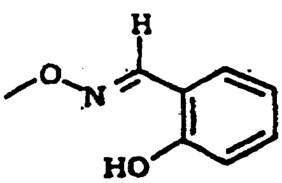 , 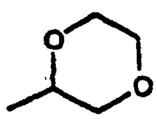 , 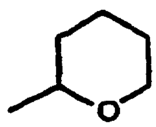 , 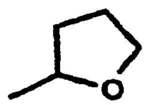 ,

-OH, et -CN;

(E)

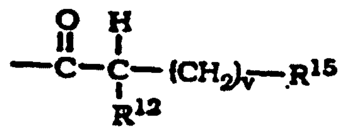

où :

(1) v est égal à 1 ou 2,
(2) $R^{12}$ représente H, et
(3) $R^{15}$ représente un groupe hétérocycloalkyle,

(F)

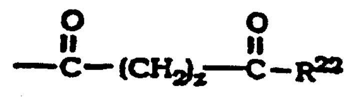

où :

(1) z est égal à 0,
(2) $R^{22}$ représente un groupe $-NR^{24}R^{25}$, et
(3) $R^{24}$ et $R^{25}$ représentent chacun indépendamment H, $-NH_2$, un groupe alkyle, un groupe alcoxy, -OH, $-CH_2CO_2H$ ou $-OCH_2C_6H_5$, et

(G)

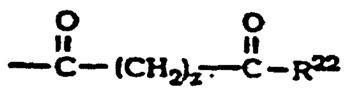

où :

(1) z est égal à 1, 2, 3, 4 ou 5,
(2) $R^{22}$ représente $-OR^{23}$, -ONa, -OLi, un groupe alkyle, $-NR^{24}R^{25}$ ou

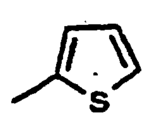

(3) $R^{23}$ représente un groupe alkyle, et
(4) $R^{24}$ et $R^{25}$ représentent chacun indépendamment H, $-CH(OCH_3)CH(CH_3)_2$,

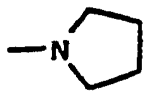
,
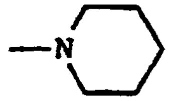
ou
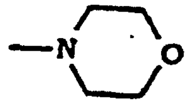

4. Composé selon l'une quelconque des revendications 1 à 3, pour lequel $R^1$, $R^3$ et $R^4$ représentent chacun Cl ou Br.

5. Composé selon l'une quelconque des revendications 1 à 4, pour lequel X représente CH.

6. Composé selon l'une quelconque des revendications 1 à 5, qu est choisi parmi les composés de formule :

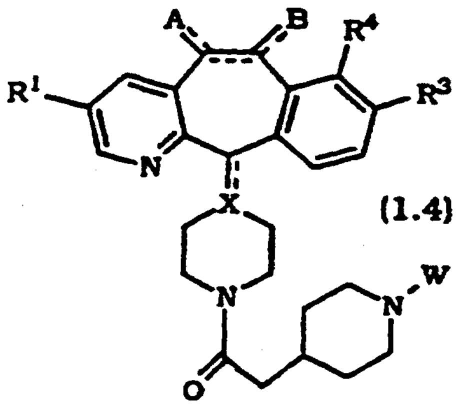
(1.4) ou
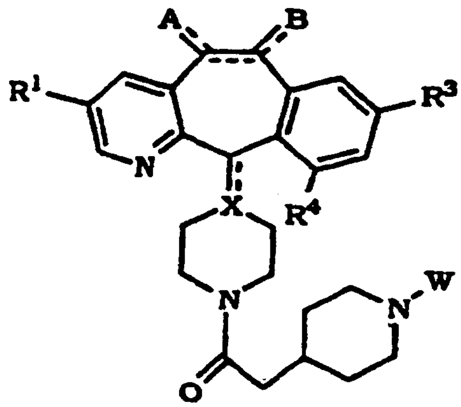
(1.5)

dans laquelle A, B, X et W ont les définitions indiquées dans la revendication 1.

7. Composé selon l'une quelconque des revendications 1 à 6, pour lequel $R^1$ représente Br, $R^3$ représente Cl, et $R^4$ représente Br.

8. Composé selon l'une quelconque des revendications 1 à 7, qui est un composé de formule :

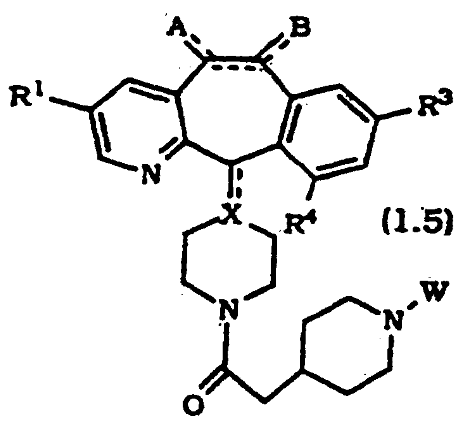
(1.5)

9. Composé selon la revendication 1, qui est choisi parmi les composés suivants :

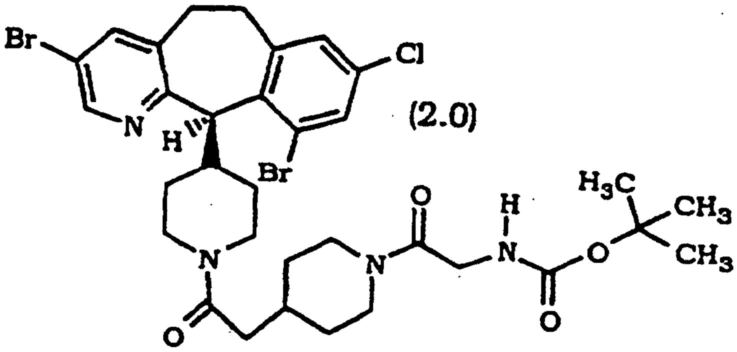

(2.0)

;

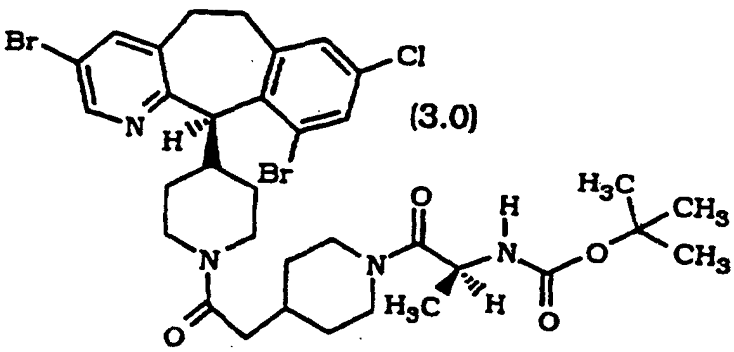

(3.0)

;

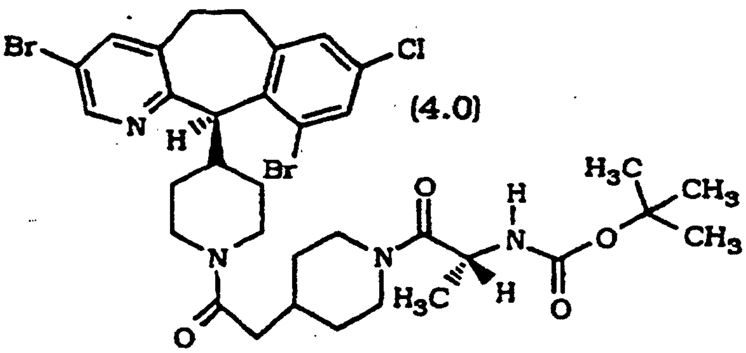

(4.0)

;

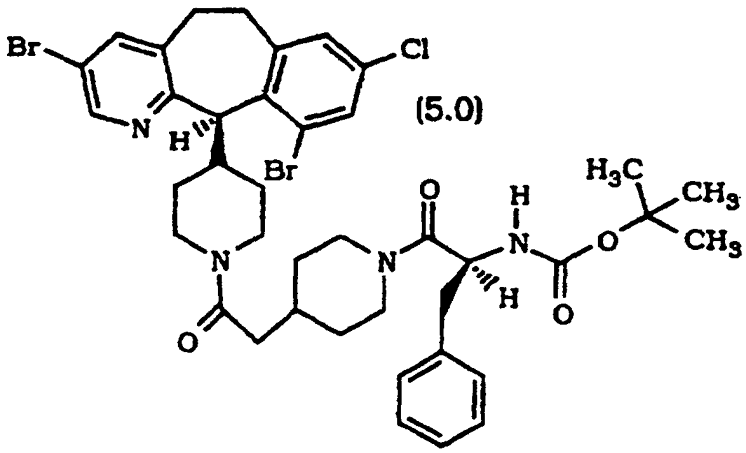

(5.0)

;

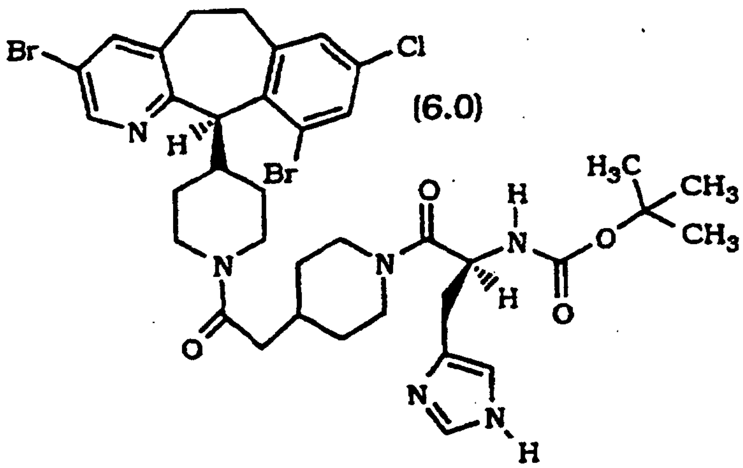

(6.0)

;

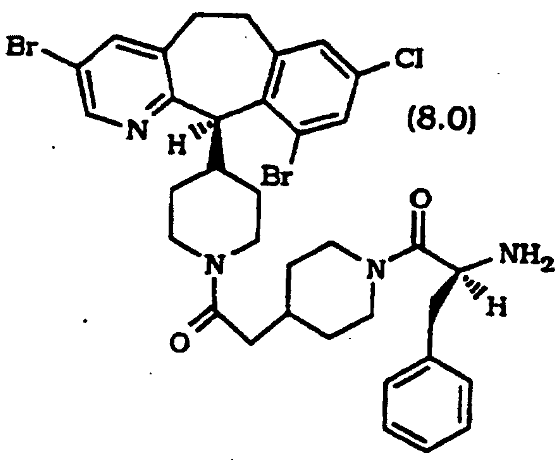

(8.0)

;

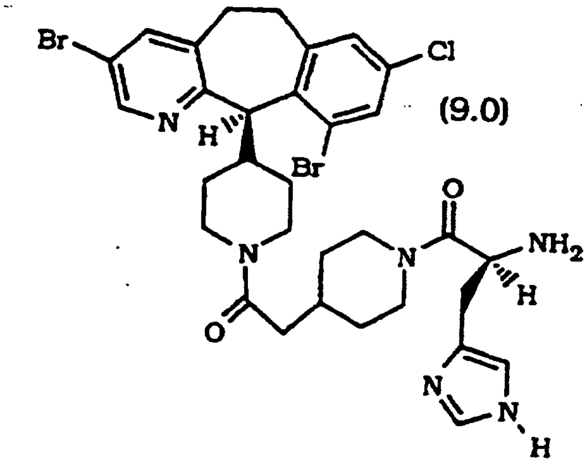

(9.0)

;

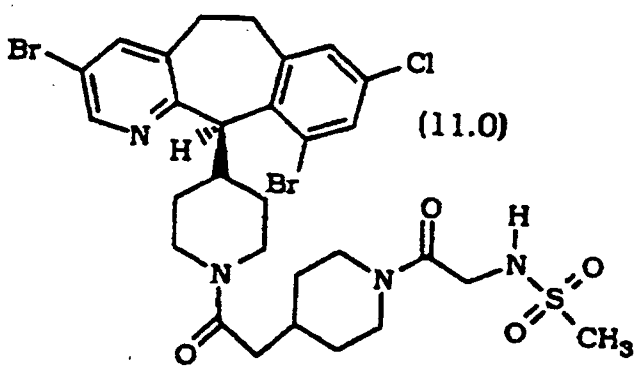

(11.0)

;

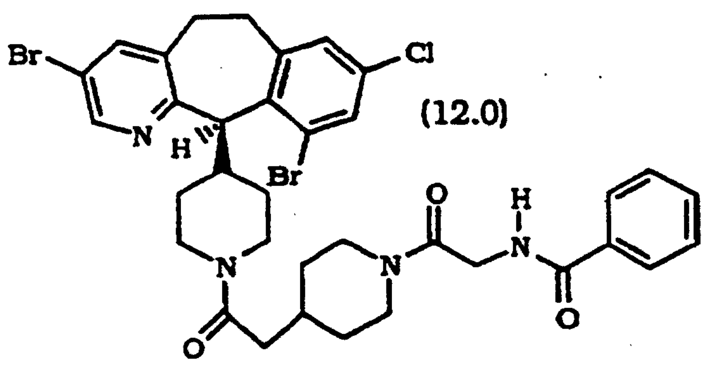

(12.0)

;

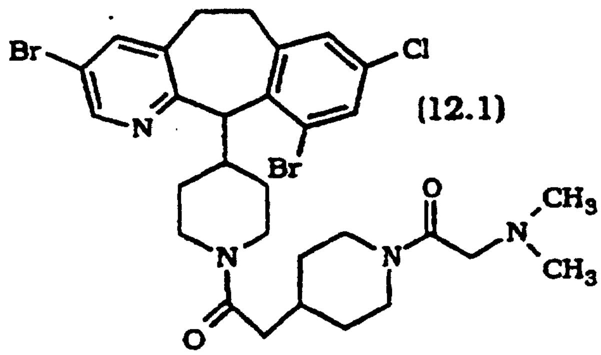

(12.1)

;

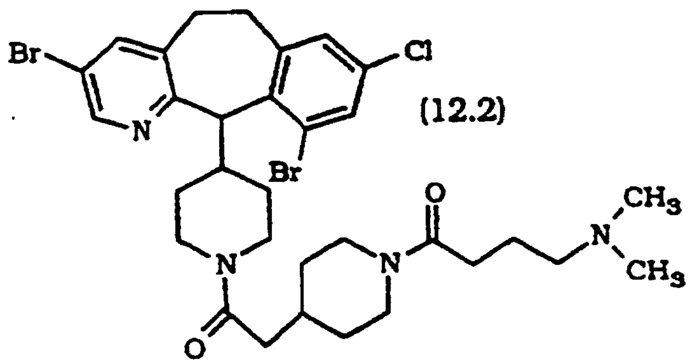

(12.2)

;

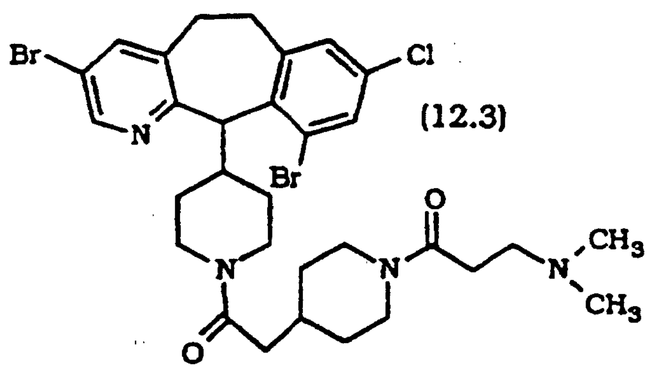

(12.3)

;

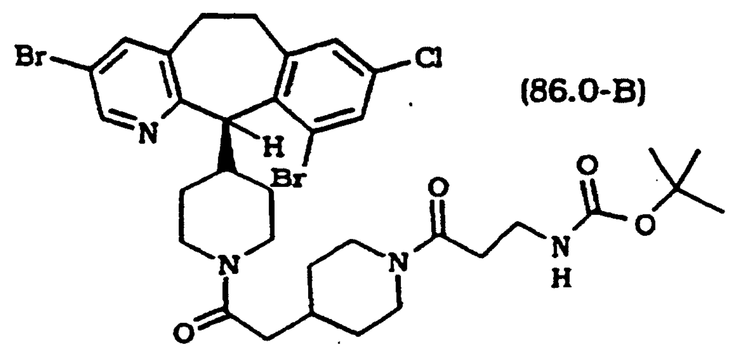

(86.0-B)

;

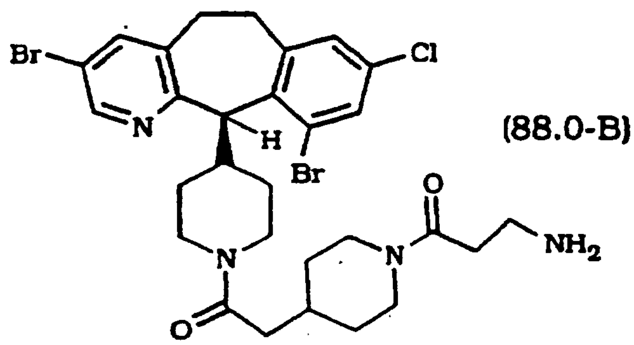

(88.0-B)

;

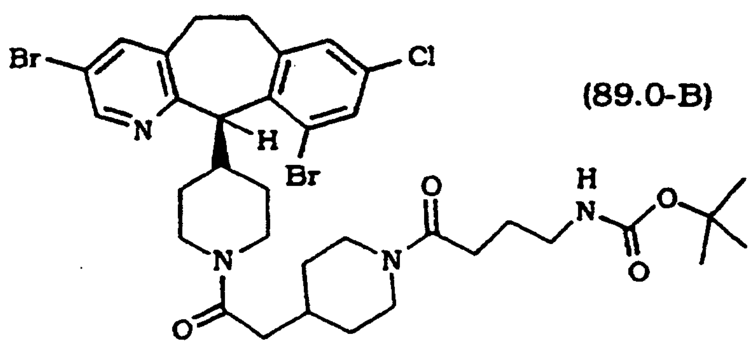

(89.0-B)

;

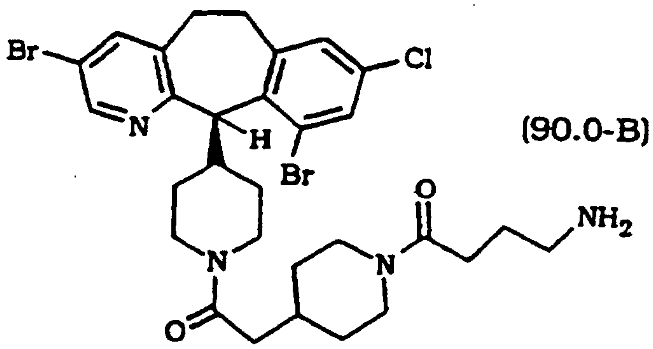

(90.0-B)

;

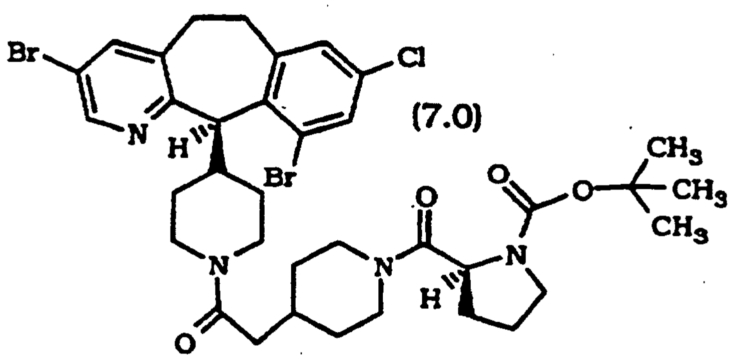

(7.0)

;

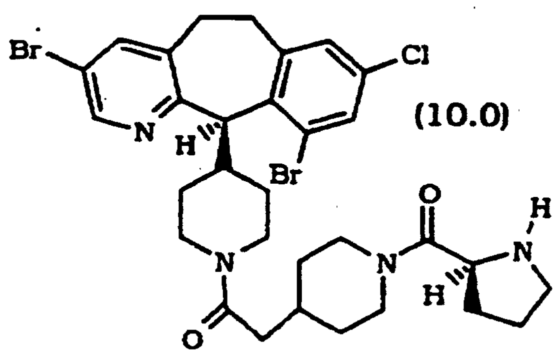

(10.0)

;

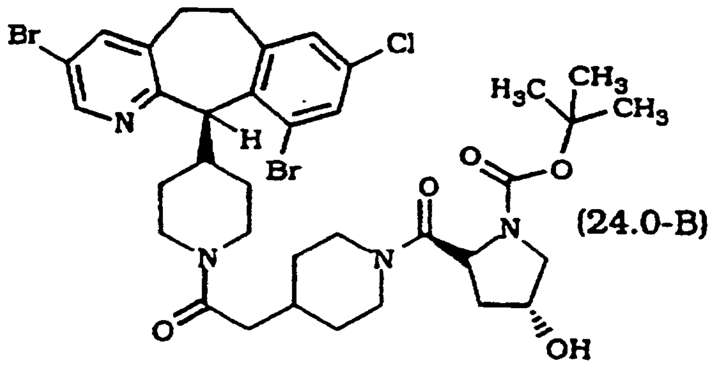

(24.0-B)

;

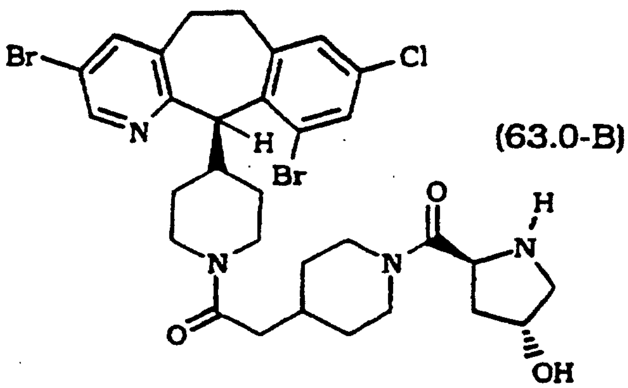

(63.0-B)

;

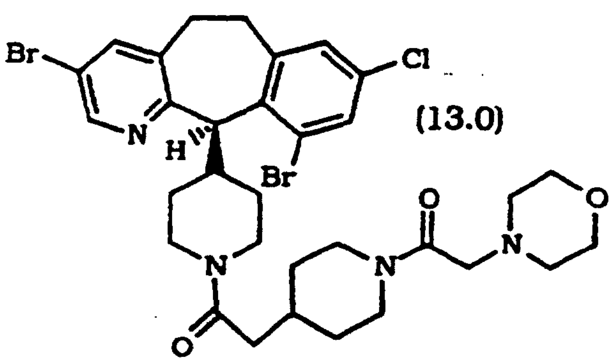

(13.0)

;

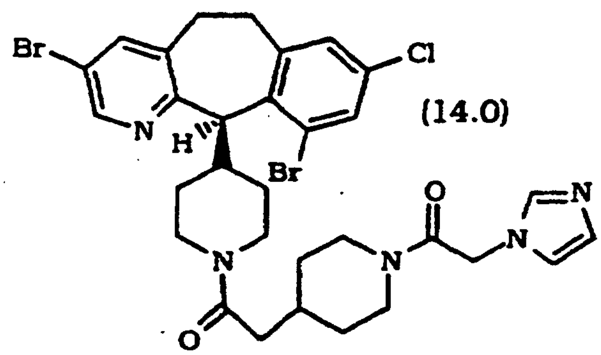

(14.0)

;

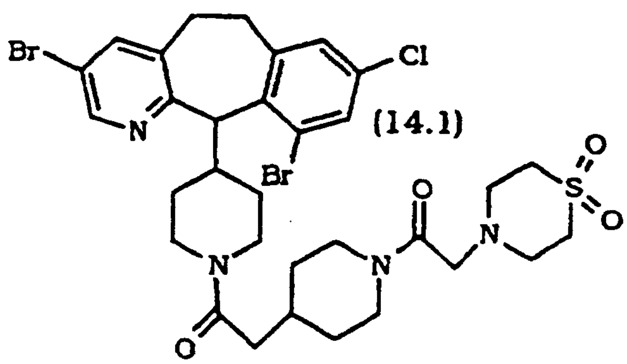

(14.1)

;

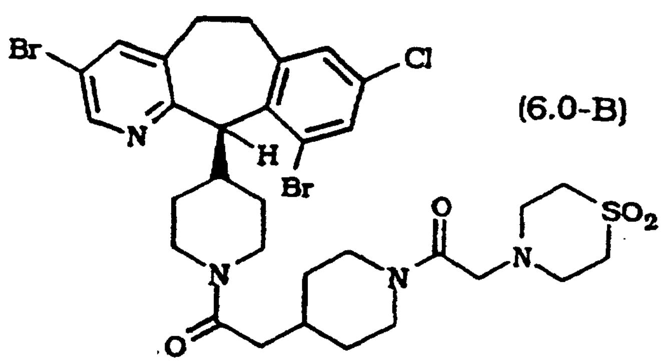

(6.0-B)

;

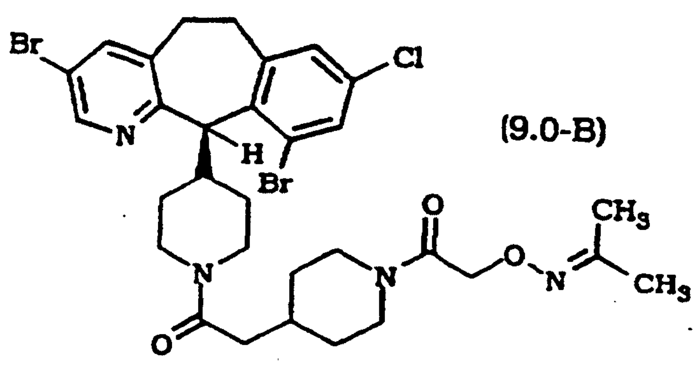

(9.0-B)

;

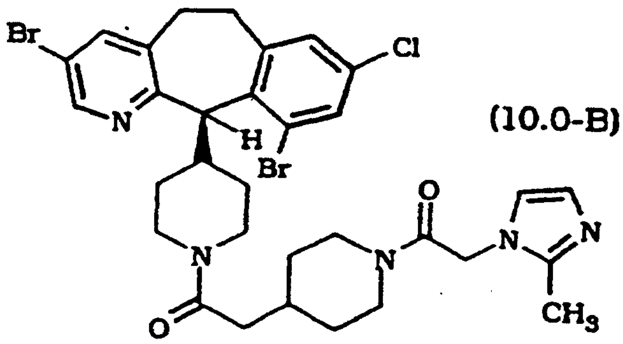

(10.0-B)

;

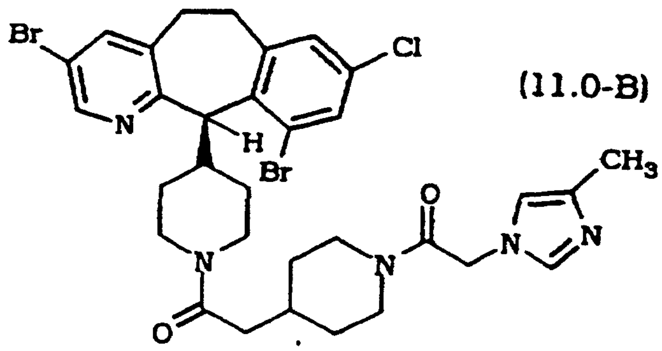

(11.0-B)

;

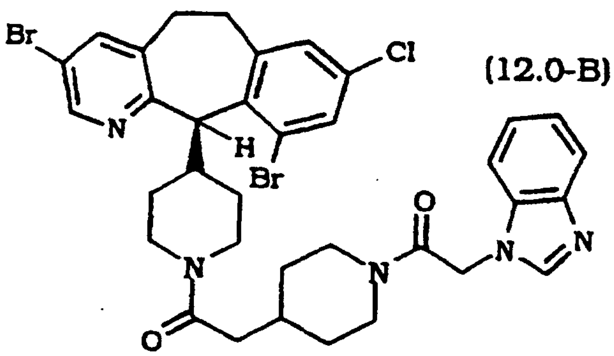

(12.0-B)

;

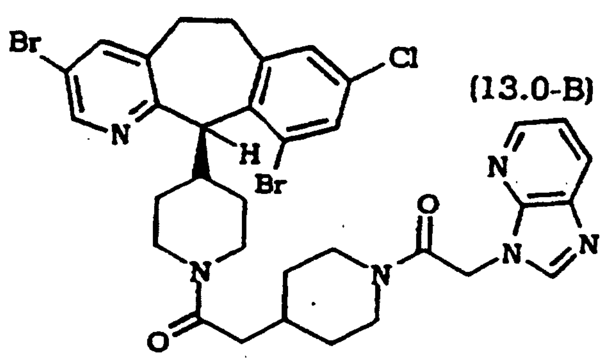

(13.0-B)

;

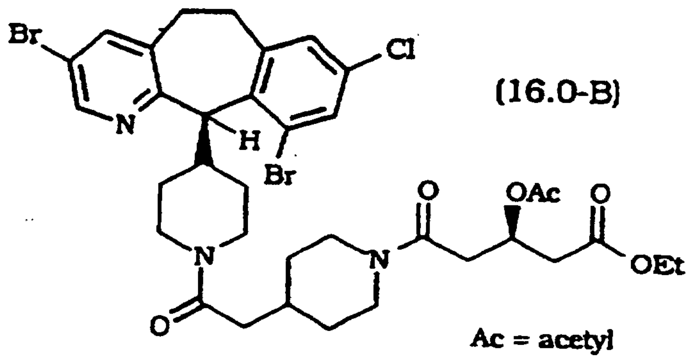

(16.0-B)

Ac = acetyl

;

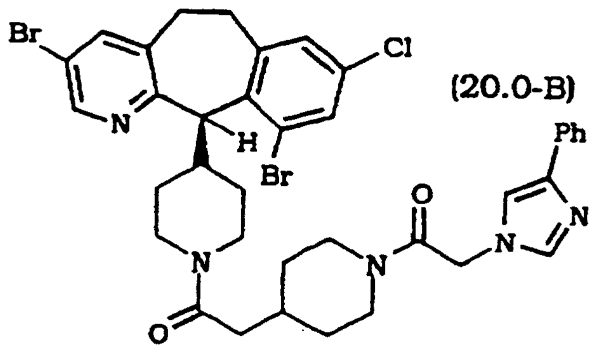

(20.0-B)

;

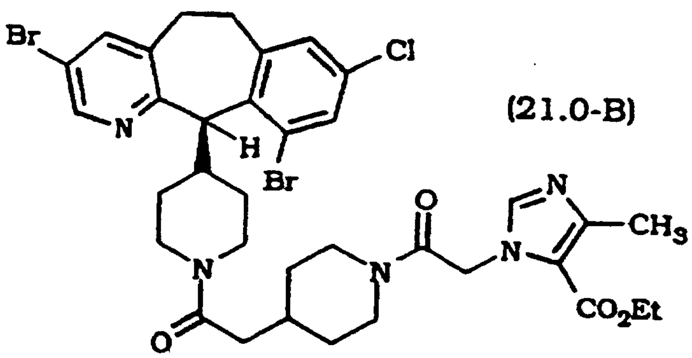

(21.0-B)

;

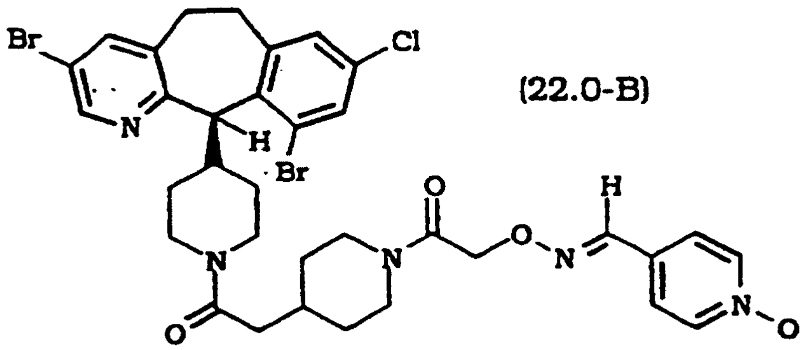

(22.0-B)

;

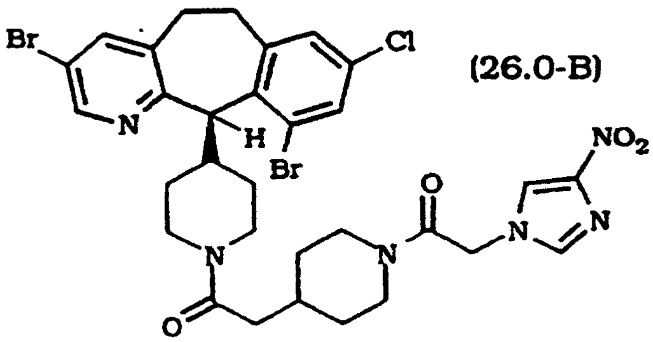

(26.0-B)

;

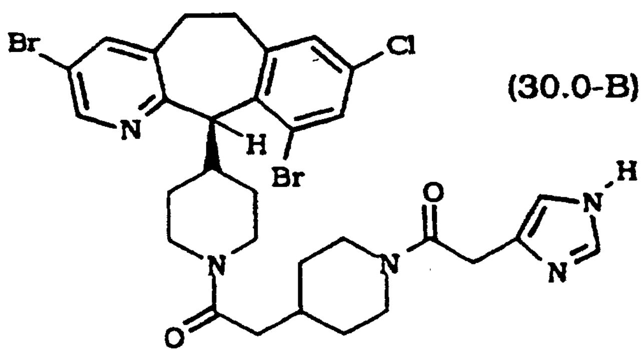

(30.0-B)

;

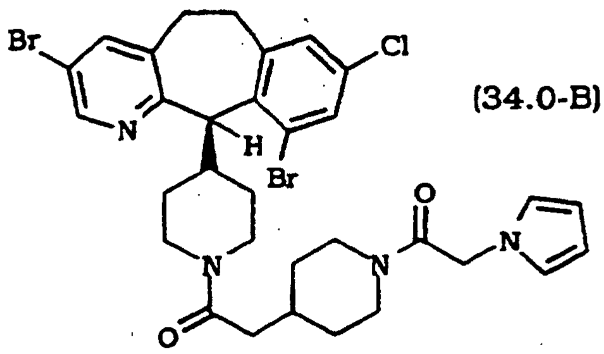

(34.0-B)

;

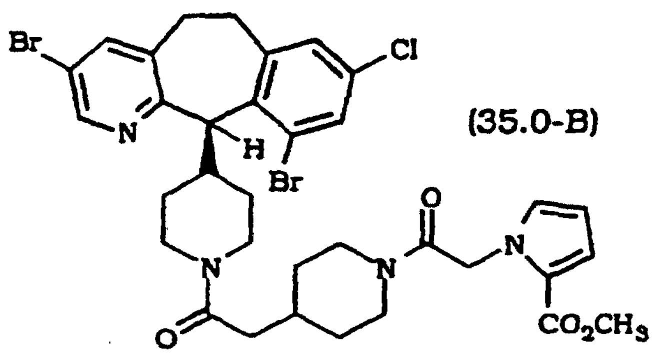

(35.0-B)

;

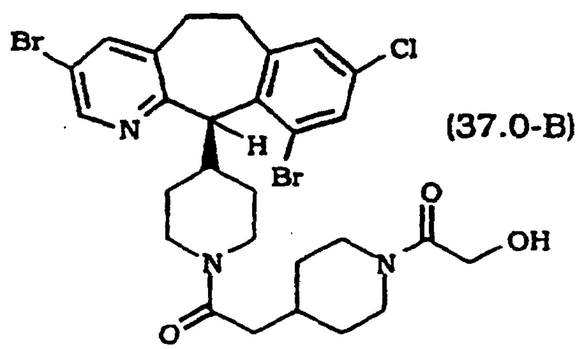

(37.0-B)

;

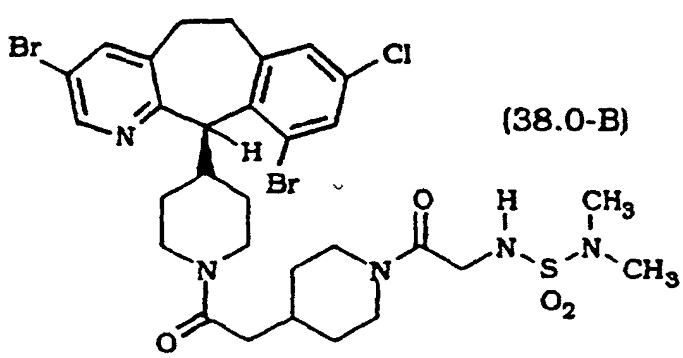

(38.0-B)

;

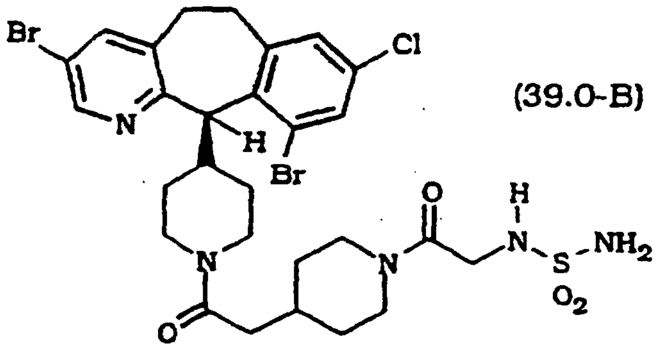

(39.0-B)

;

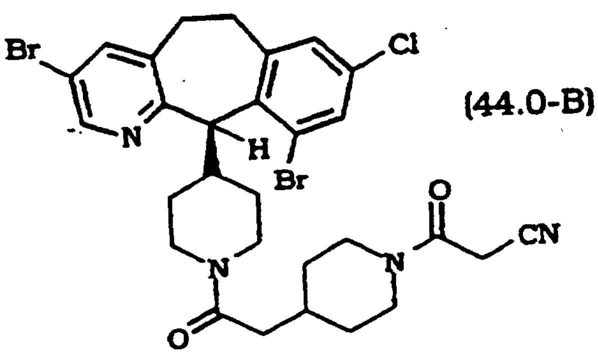

(44.0-B)

;

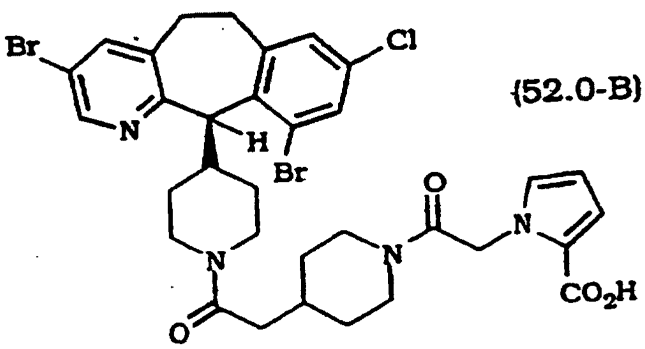

(52.0-B)

;

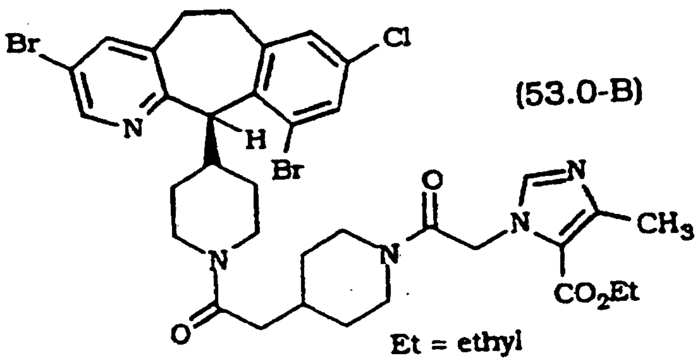

(53.0-B)

Et = ethyl

;

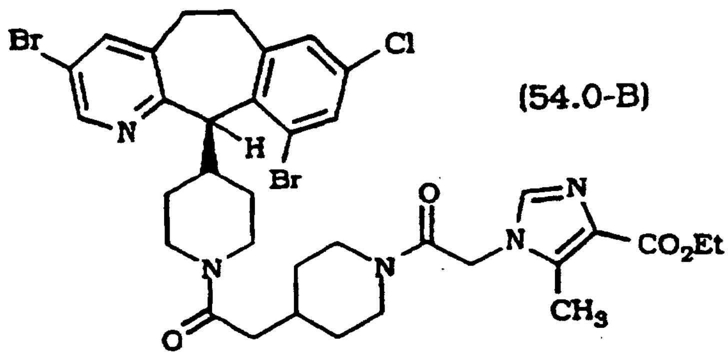

(54.0-B)

;

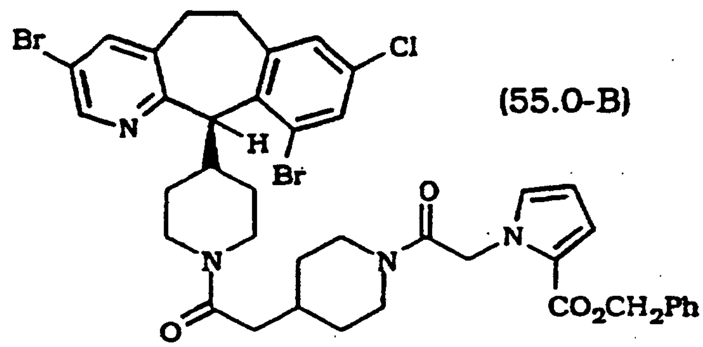

(55.0-B)

Ph = phenyl

;

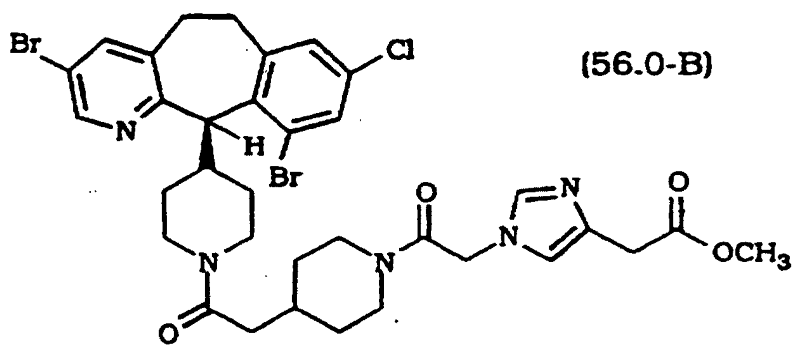

(56.0-B)

;

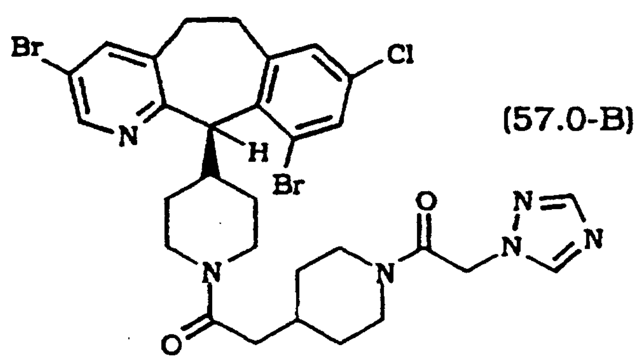

(57.0-B)

;

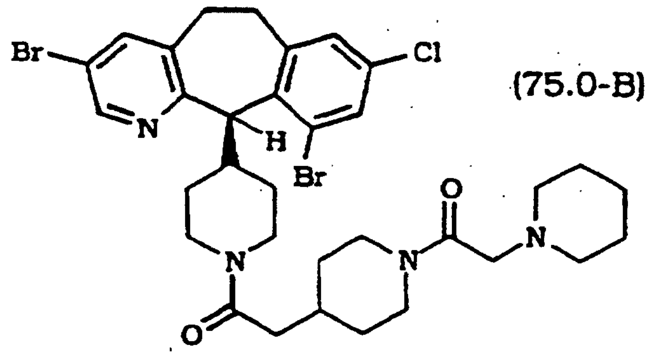

(75.0-B)

;

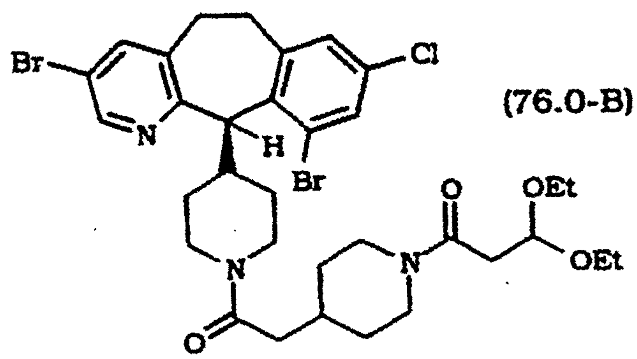

(76.0-B)

;

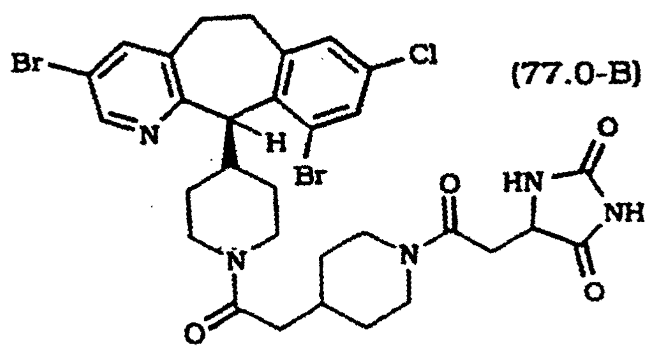

(77.0-B)

;

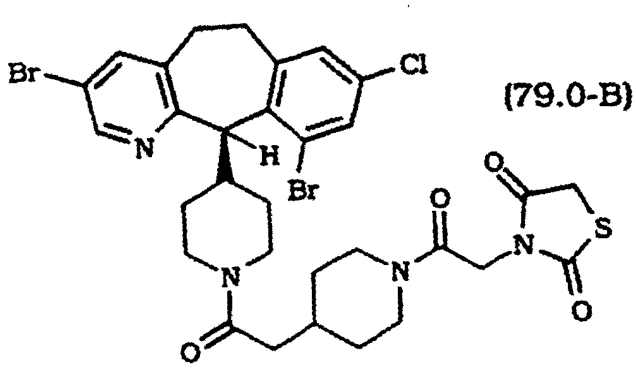

(79.0-B)

;

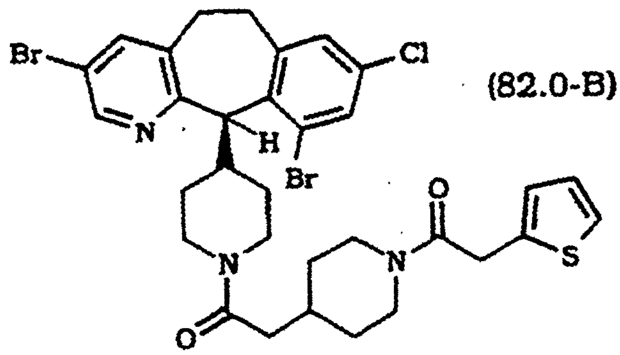

(82.0-B)

;

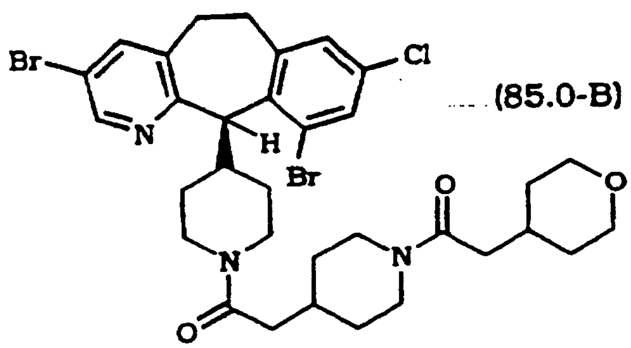

(85.0-B)

;

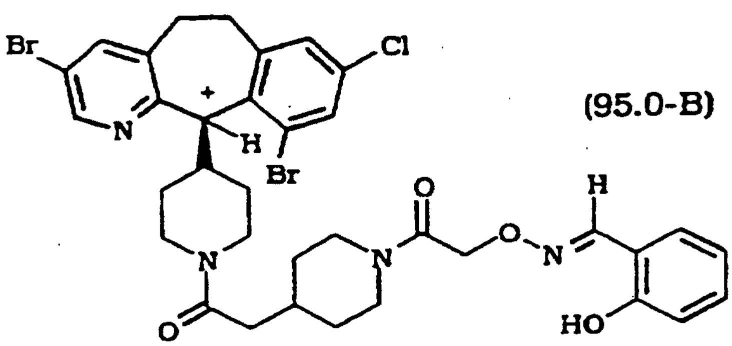

(95.0-B)

;

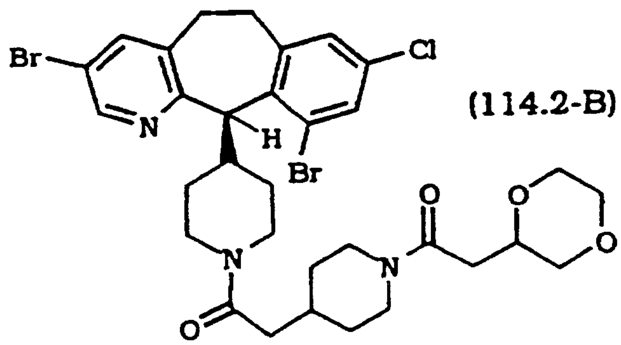

(114.2-B)

;

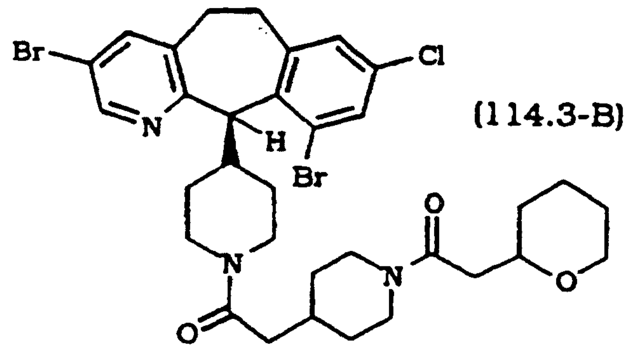

(114.3-B)

;

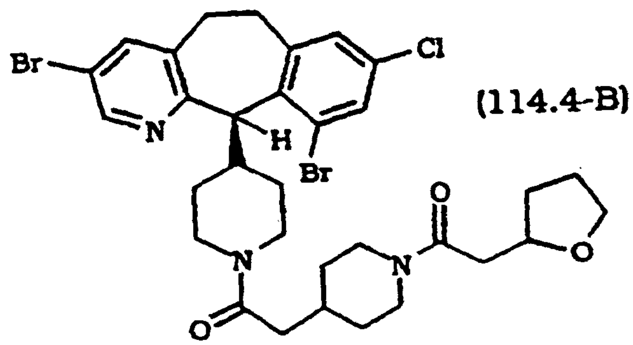

(114.4-B)

;

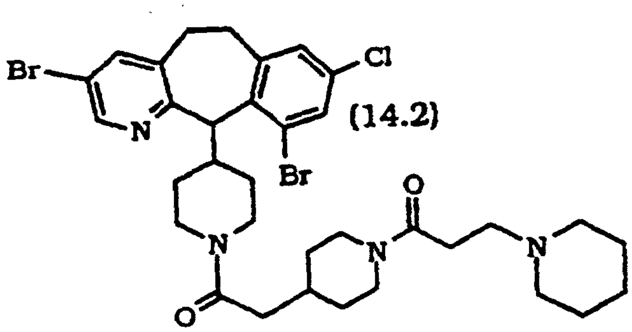

(14.2)

;

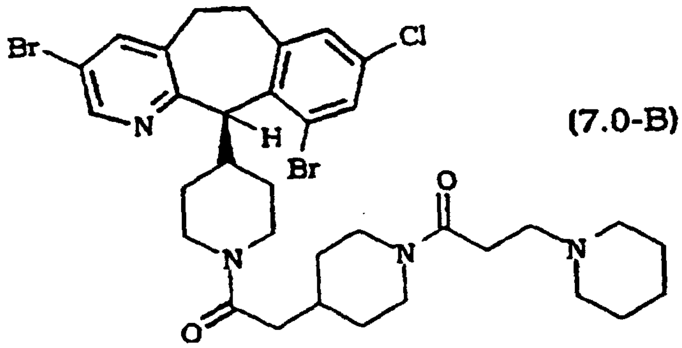

(7.0-B)

;

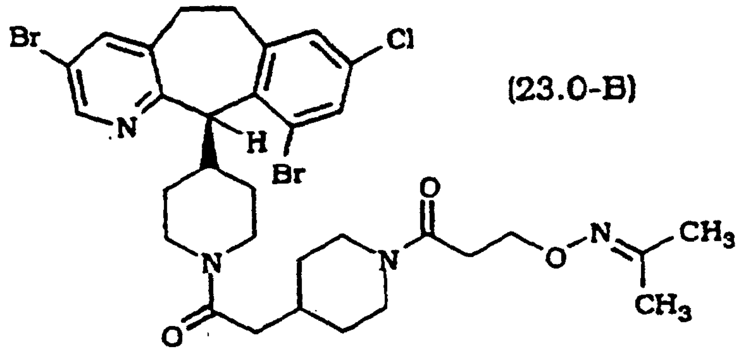

(23.0-B)

;

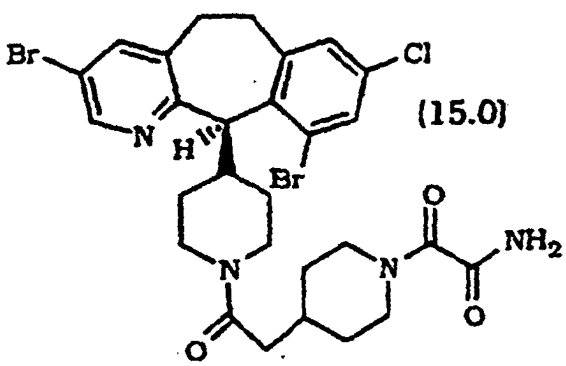

(15.0)

;

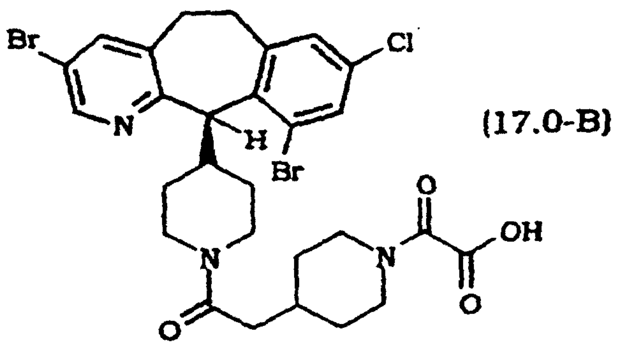

(17.0-B)

;

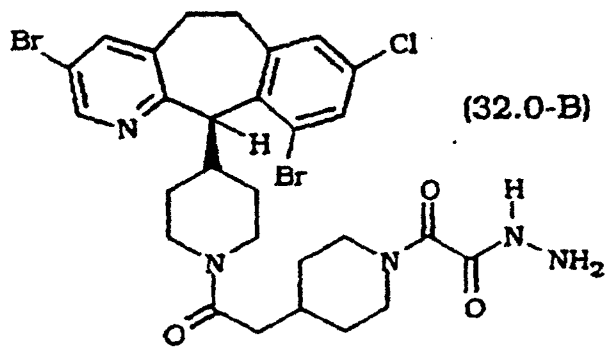

(32.0-B)

;

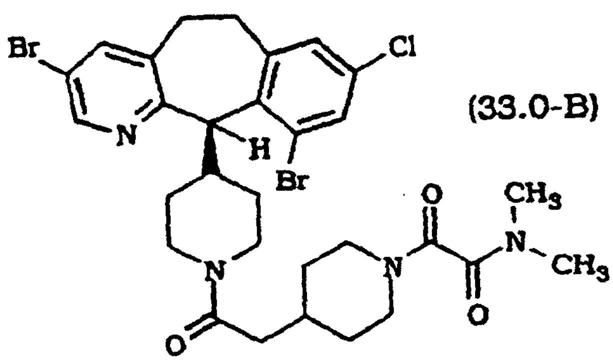

(33.0-B)

;

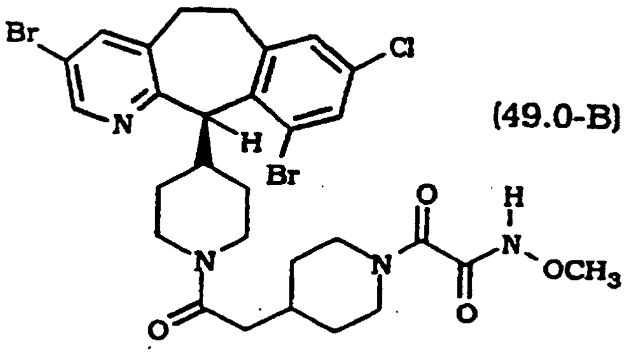

(49.0-B)

;

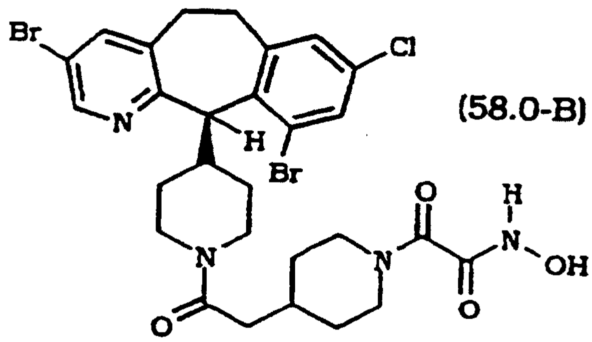

(58.0-B)

;

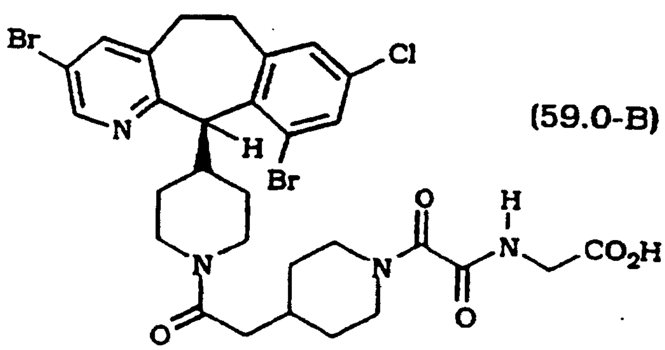

(59.0-B)

;

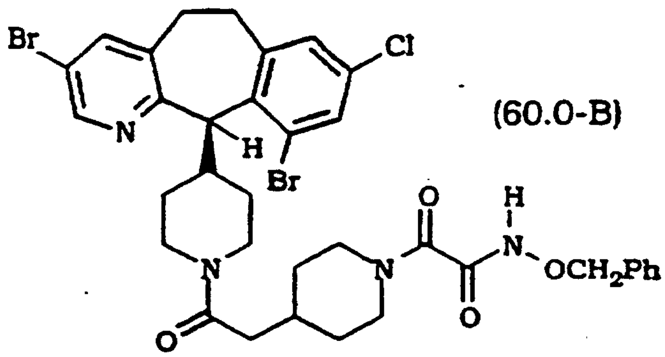

(60.0-B)

;

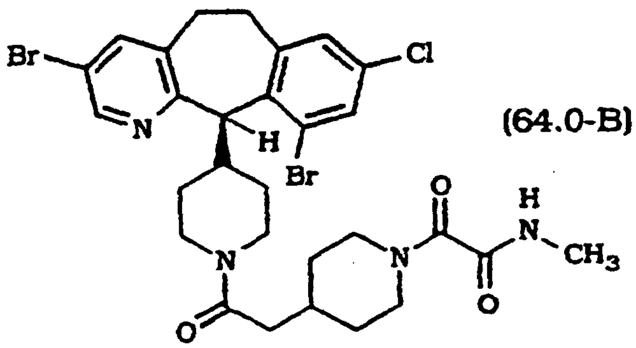

(64.0-B)

;

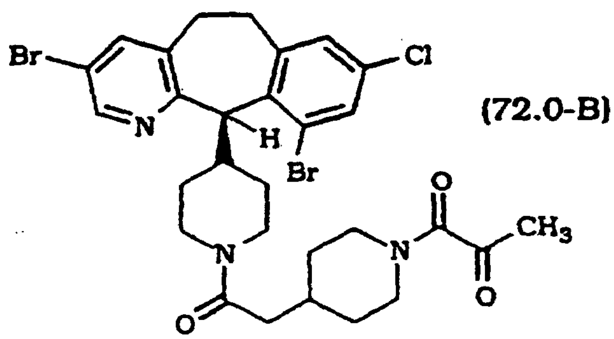

(72.0-B)

;

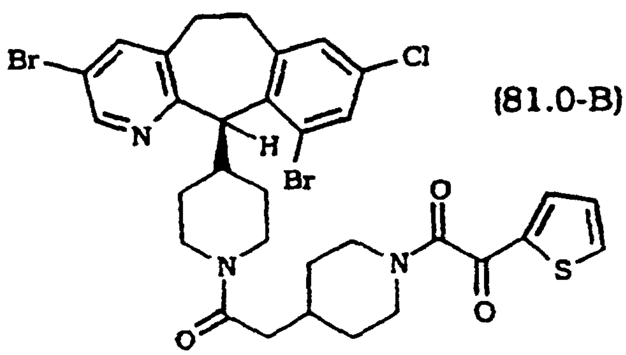

(81.0-B)

;

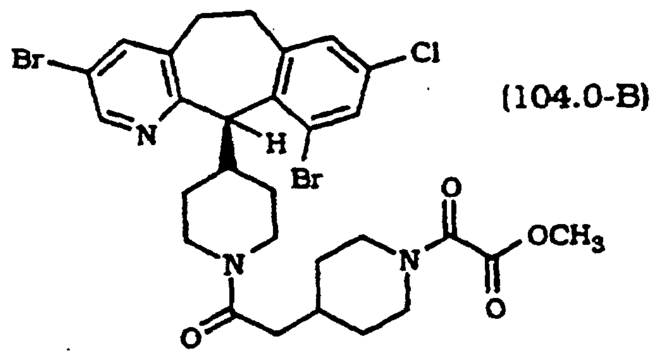

(104.0-B)

;

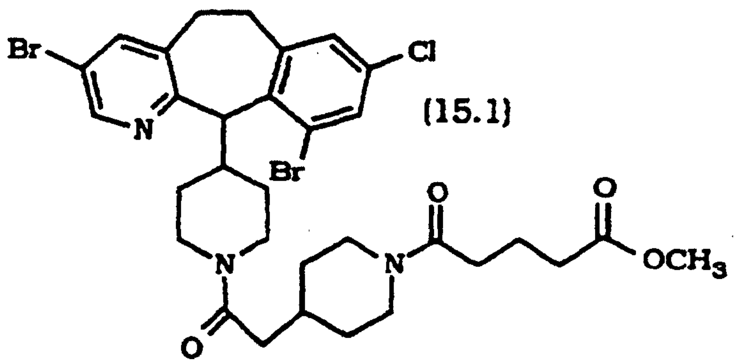

(15.1)

;

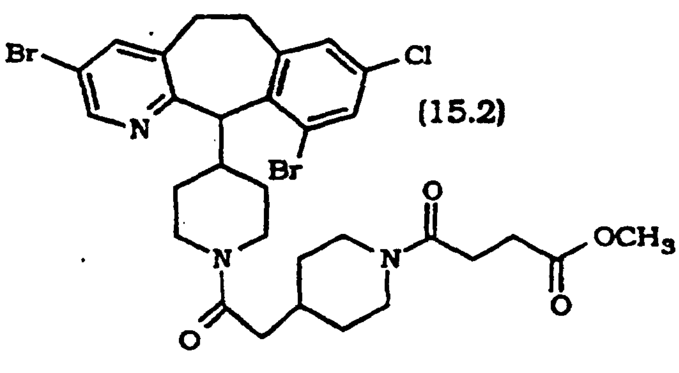

(15.2)

;

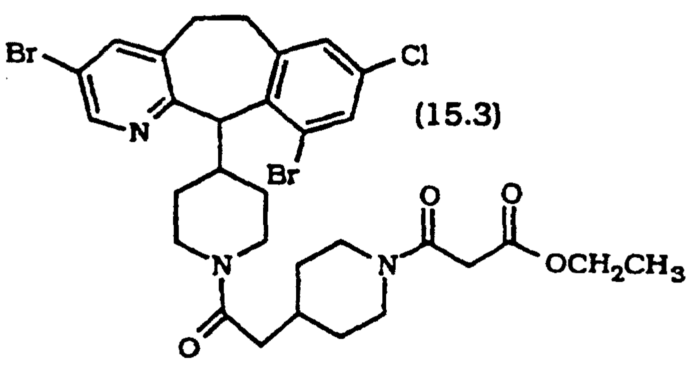

(15.3)

;

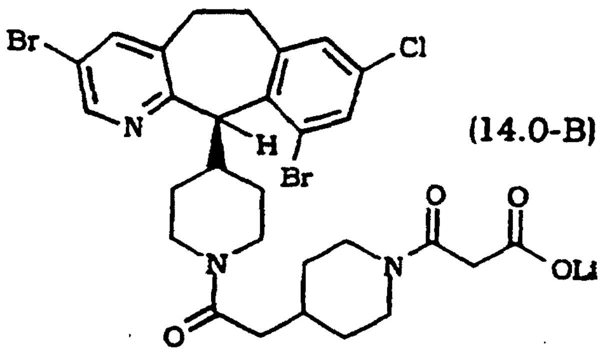

(14.0-B)

;

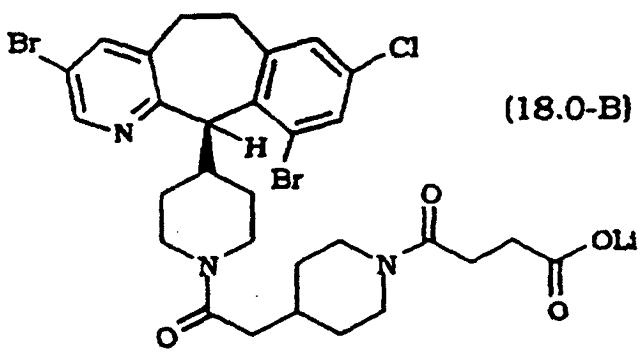

(18.0-B)

;

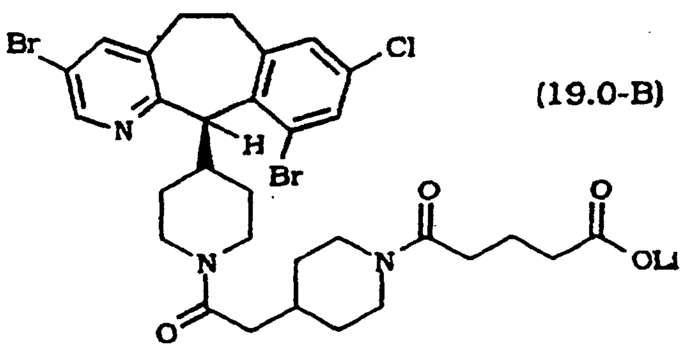

(19.0-B)

;

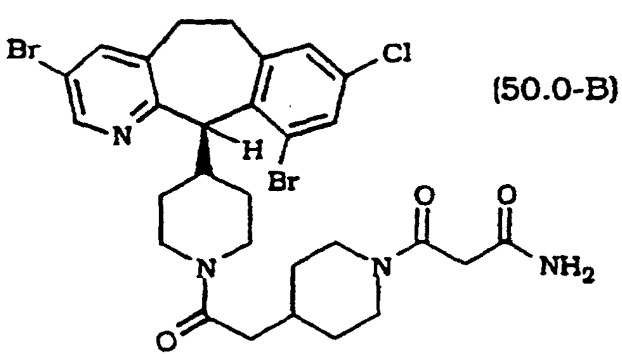

(50.0-B)

;

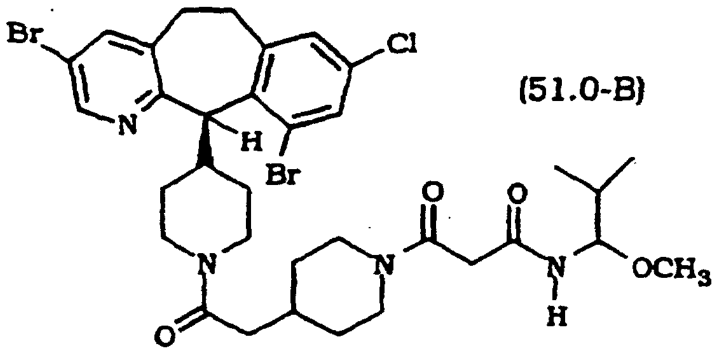

(51.0-B)

;

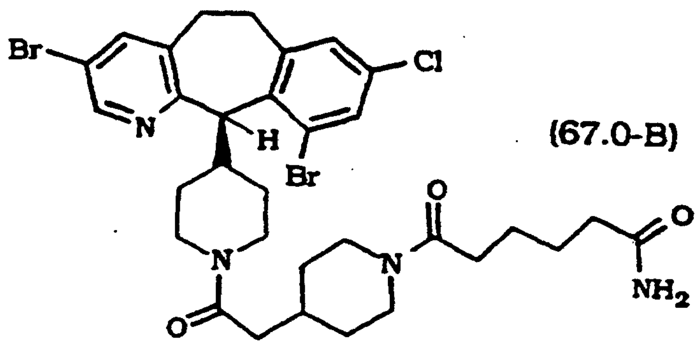

(67.0-B)

;

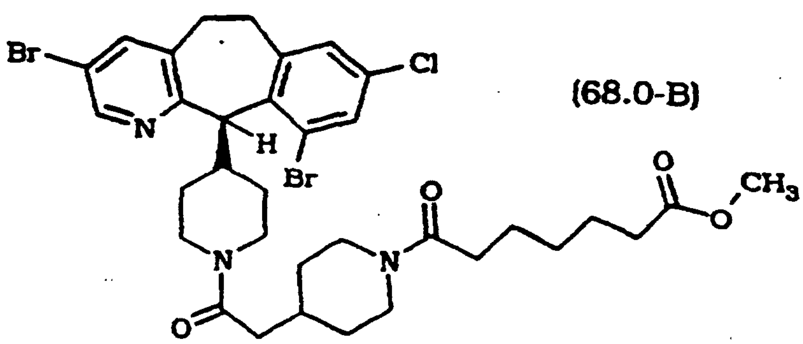

(68.0-B)

;

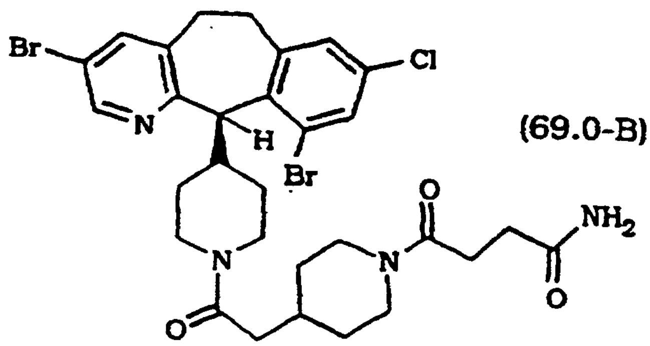

(69.0-B)

;

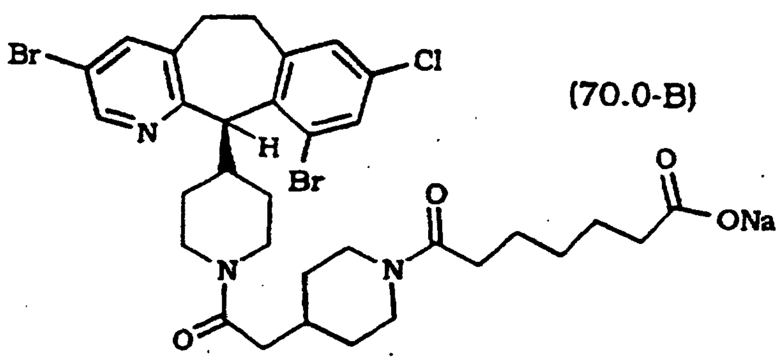

(70.0-B)

;

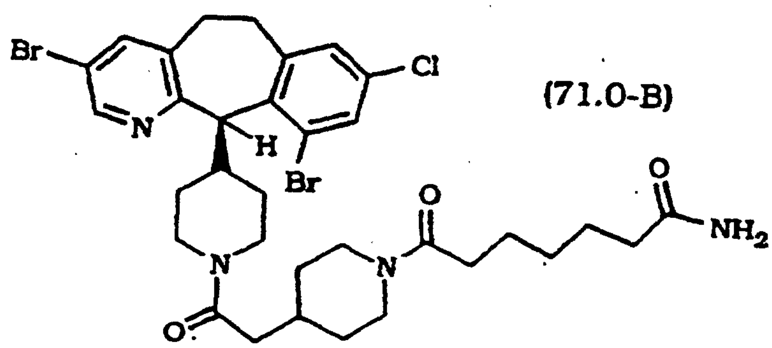

(71.0-B)

;

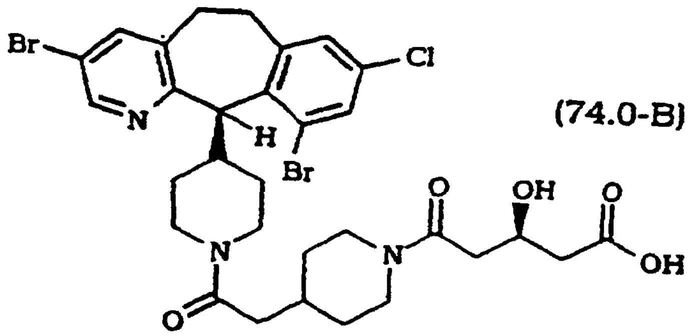

(74.0-B)

;

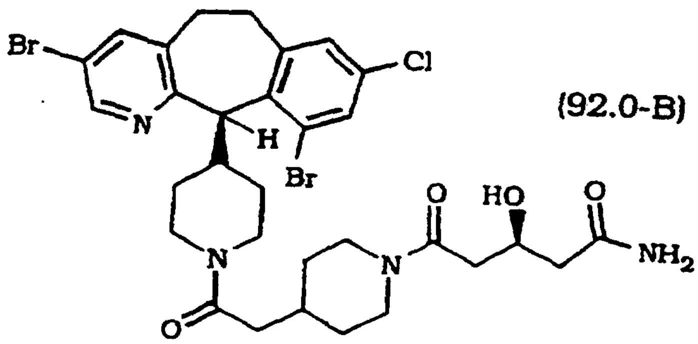

(92.0-B)

;

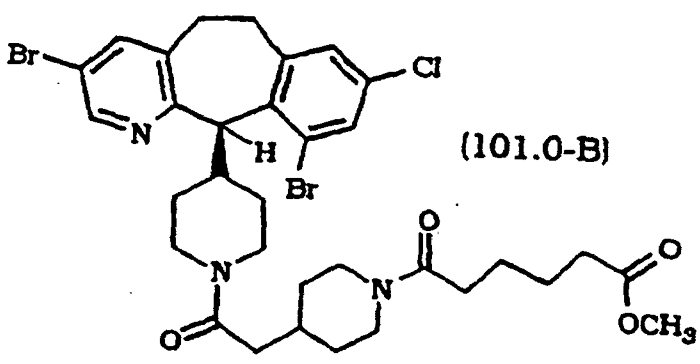

(101.0-B)

;

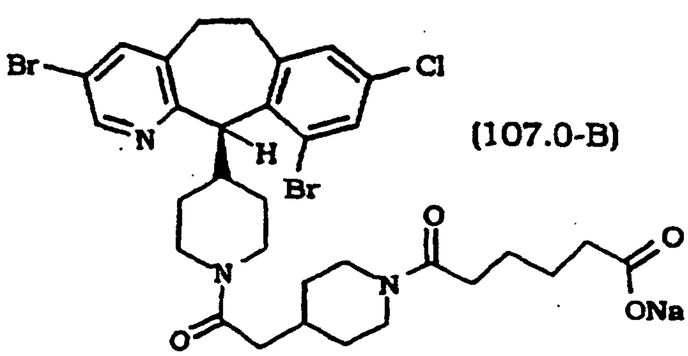

(107.0-B)

;

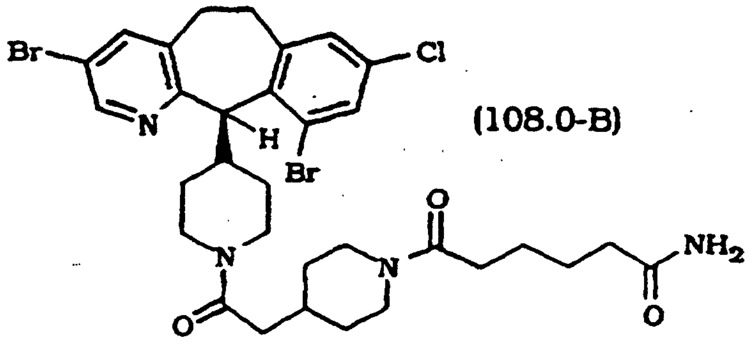

(108.0-B)

;

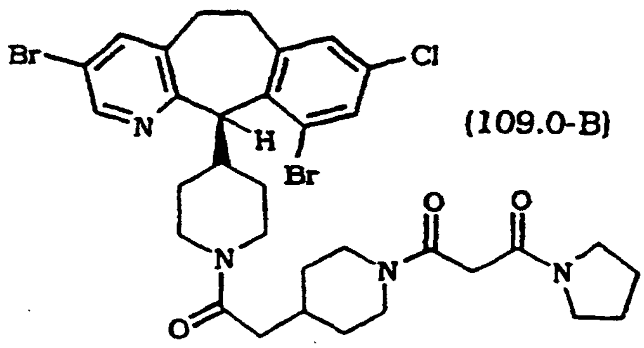

(109.0-B)

;

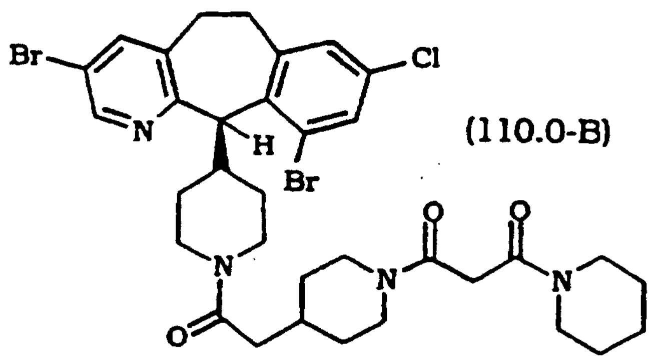

(110.0-B)

;

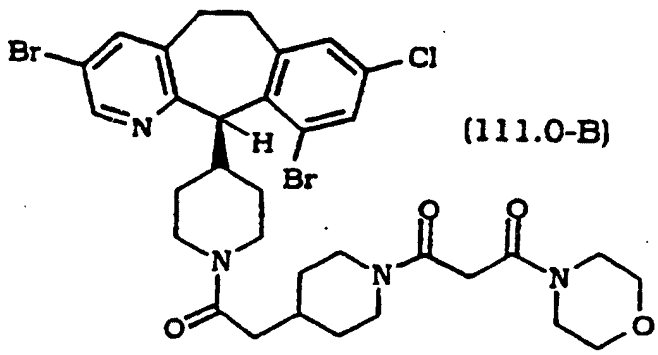

(111.0-B)

;

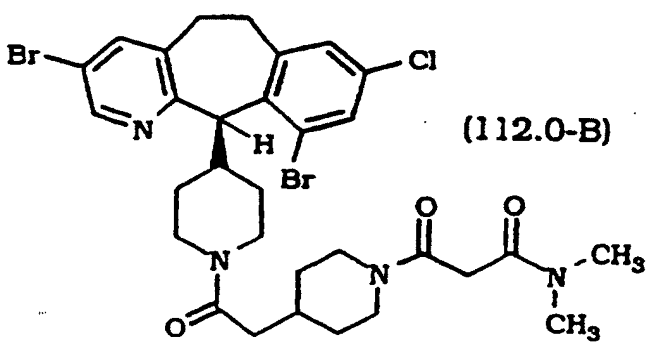

(112.0-B)

;

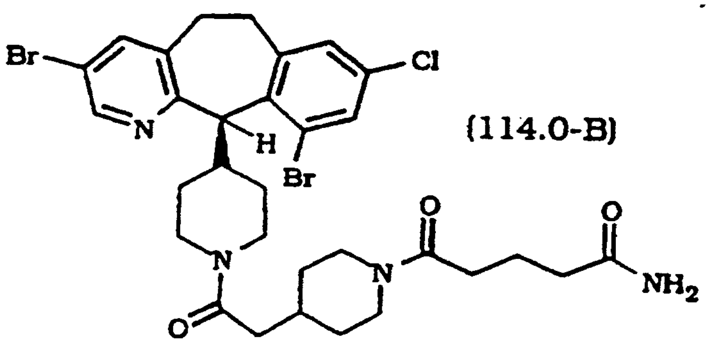

(114.0-B)

;

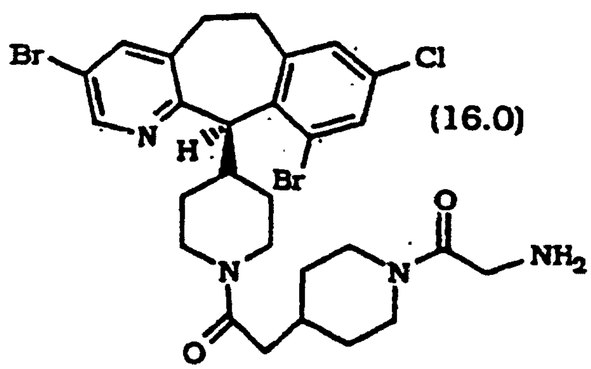

(16.0)

;

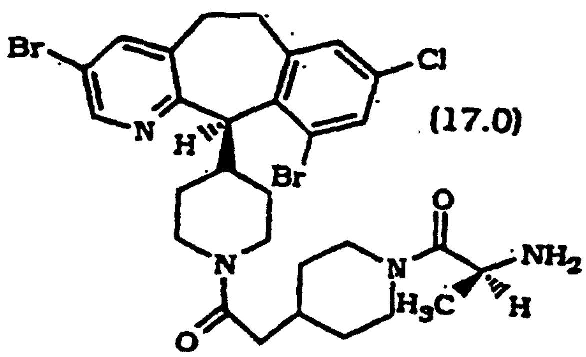

(17.0)

et

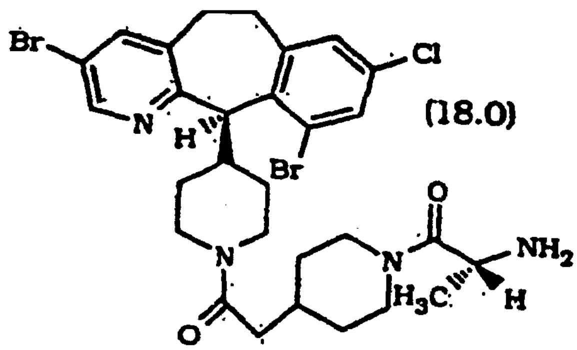

(18.0)

et leurs sels ou produits de solvatation pharmaceutiquement acceptables.

**10.** Composition pharmaceutique qui comprend une quantité efficace d'un composé selon l'une quelconque des revendications 1 à 9, en combinaison avec un support pharmaceutiquement acceptable.

**11.** Utilisation d'un composé selon l'une quelconque des revendications 1 à 9, pour la préparation d'un médicament destiné au traitement de cellules tumorales.

**12.** Utilisation selon la revendication 11, dans laquelle les cellules traitées sont des cellules de tumeur pancréatique, des cellules de cancer du poumon, des cellules de tumeur de leucémie myéloïde, des cellules de tumeur folliculaire de la thyroïde, des cellules de tumeurs myélodysplasiques, des cellules tumorales de carcinome de l'épiderme, des cellules tumorales de carcinome de la vessie, des cellules de tumeurs du colon, des cellules de tumeurs du sein ou des cellules de tumeurs de la prostate.

**13.** Utilisation d'un composé selon l'une quelconque des revendications 1 à 9, pour la préparation d'un médicament pour l'inhibition de la farnésyl-protéine transférase.